# EUROPEAN PATENT SPECIFICATION

(11) **EP 2 086 966 B1**
(45) Date of publication and mention of the grant of the patent: **14.09.2011**
(21) Application number: 07857221.1
(22) Date of filing: 15.11.2007
(51) Int. Cl.: C07D 409/04, C07D 409/10, C07D 409/12

(54) **2-CARBOXY THIOPHENE DERIVATIVES AS ANTI VIRAL AGENTS**
2-CARBOXY-THIOPHEN-DERIVATE ALS VIRUZIDE
DERIVES DU 2-CARBOXY THIOPHENE EN TANT QU'AGENTS ANTI-VIRAUX

(30) Priority: 17.11.2006 GB 0622992; 14.09.2007 GB 0718031
(43) Date of publication of application: 12.08.2009
(73) Proprietor: GlaxoSmithKline LLC, Philadelphia, PA 19102 (US)
(72) Inventor: GRIMES, Richard Martin, Stevenage Hertfordshire SG1 2NY (GB); HARTLEY, Charles David, Stevenage Hertfordshire SG1 2NY (GB); MORDAUNT, Jacqueline Elizabeth, Stevenage Hertfordshire SG1 2NY (GB); SHAH, Pritom, Stevenage Hertfordshire SG1 2NY (GB); SLATER, Martin John, Stevenage Hertfordshire SG1 2NY (GB); WHITE, Gemma Victoria, Stevenage Hertfordshire SG1 2NY (GB)
(74) Representative: McKinnell, Denise
(86) International application number: PCT/EP2007/062431
(87) International publication number: WO 2008/059042

(56) References cited:
- WO-A-02/100851
- WO-A-2005/092863

## Description

### FIELD OF THE INVENTION

The present invention relates to novel 2-carboxy thiophene derivatives useful as anti-viral agents. Specifically, the present invention involves novel inhibitors of Hepatitis C Virus (HCV) replication.

### BACKGROUND OF THE INVENTION

Infection with HCV is a major cause of human liver disease throughout the world. In the US, an estimated 4.5 million Americans are chronically infected with HCV. Although only 30% of acute infections are symptomatic, greater than 85% of infected individuals develop chronic, persistent infection. Treatment costs for HCV infection have been estimated at $5.46 billion for the US in 1997. Worldwide over 200 million people are estimated to be infected chronically. HCV infection is responsible for 40-60% of all chronic liver disease and 30% of all liver transplants. Chronic HCV infection accounts for 30% of all cirrhosis, end-stage liver disease, and liver cancer in the U.S. The CDC estimates that the number of deaths due to HCV will minimally increase to 38,000/year by the year 2010.

Due to the high degree of variability in the viral surface antigens, existence of multiple viral genotypes, and demonstrated specificity of immunity, the development of a successful vaccine in the near future is unlikely. Alpha-interferon (alone or in combination with ribavirin) has been widely used since its approval for treatment of chronic HCV infection. However, adverse side effects are commonly associated with this treatment: flu-like symptoms, leukopenia, thrombocytopenia, depression from interferon, as well as anemia induced by ribavirin (Lindsay, K.L. (1997) Hepatology 26 (suppl 1): 71S-77S). This therapy remains less effective against infections caused by HCV genotype 1 (which constitutes ~75% of all HCV infections in the developed markets) compared to infections caused by the other 5 major HCV genotypes. Unfortunately, only ~50-80% of the patients respond to this treatment (measured by a reduction in serum HCV RNA levels and normalization of liver enzymes) and, of responders, 50-70% relapse within 6 months of cessation of treatment. Recently, with the introduction of pegylated interferon (Peg-IFN), both initial and sustained response rates have improved substantially, and combination treatment of Peg-IFN with ribavirin constitutes the gold standard for therapy. However, the side effects associated with combination therapy and the impaired response in patients with genotype 1 present opportunities for improvement in the management of this disease.

First identified by molecular cloning in 1989 (Choo, Q-L et al (1989) Science 244:359-362), HCV is now widely accepted as the most common causative agent of post-transfusion non A, non-B hepatitis (NANBH) (Kuo, G et al (1989) Science 244:362-364). Due to its genome structure and sequence homology, this virus was assigned as a new genus in the *Flaviviridae* family. Like the other members of the *Flaviviridae,* such as flaviviruses (e.g. yellow fever virus and Dengue virus types 1-4) and pestiviruses (e.g. bovine viral diarrhea virus, border disease virus, and classic swine fever virus) (Choo, Q-L et al (1989) Science 244:359-362; Miller, R.H. and R.H. Purcell (1990) Proc. Natl. Acad. Sci. USA 87:2057-2061), HCV is an enveloped virus containing a single strand RNA molecule of positive polarity. The HCV genome is approximately 9.6 kilobases (kb) with a long, highly conserved, noncapped 5' nontranslated region (NTR) of approximately 340 bases which functions as an internal ribosome entry site (IRES) (Wang CY et al 'An RNA pseudoknot is an essential structural element of the internal ribosome entry site located within the hepatitis C virus 5' noncoding region' RNA- A Publication of the RNA Society. 1(5): 526-537, 1995 Jul.). This element is followed by a region which encodes a single long open reading frame (ORF) encoding a polypeptide of -3000 amino acids comprising both the structural and nonstructural viral proteins.

Upon entry into the cytoplasm of the cell, this RNA is directly translated into a polypeptide of -3000 amino acids comprising both the structural and nonstructural viral proteins. This large polypeptide is subsequently processed into the individual structural and nonstructural proteins by a combination of host and virally-encoded proteinases (Rice, C.M. (1996) in B.N. Fields, D.M.Knipe and P.M. Howley (eds) Virology 2nd Edition, p931-960; Raven Press, N.Y.). Following the termination codon at the end of the long ORF, there is a 3' NTR which roughly consists of three regions: an - 40 base region which is poorly conserved among various genotypes, a variable length poly(U)/polypyrimidine tract, and a highly conserved 98 base element also called the "3' X-tail" (Kolykhalov, A. et al (1996) J. Virology 70:3363-3371; Tanaka, T. et al (1995) Biochem Biophys. Res. Commun. 215:744-749; Tanaka, T. et al (1996) J. Virology 70:3307-3312; Yamada, N. et al (1996) Virology 223:255-261). The 3' NTR is predicted to form a stable secondary structure which is essential for HCV growth in chimps and is believed to function in the initiation and regulation of viral RNA replication.

The NS5B protein (591 amino acids, 65 kDa) of HCV (Behrens, S.E. et al (1996) EMBO J. 15:12-22), encodes an RNA-dependent RNA polymerase (RdRp) activity and contains canonical motifs present in other RNA viral polymerases. The NS5B protein is fairly well conserved both intra-typically (~95-98% amino acid (aa) identity across 1b isolates) and inter-typically (~85% aa identity between genotype 1a and 1b isolates). The essentiality of the HCV NS5B RdRp activity for the generation of infectious progeny virions has been formally proven in chimpanzees (A. A. Kolykhalov et al.. (2000) Journal of Virology, 74(4): 2046-2051). Thus, inhibition of NS5B RdRp activity (inhibition of RNA replication) is predicted to be useful to treat HCV infection.

Although the predominant HCV genotype worldwide is genotype 1, this itself has two main subtypes, denoted 1a and 1b. As seen from entries into the Los Alamos HCV database (www.hcv.lanl.gov) (Table 1) there are regional differences in the distribution of these subtypes: while genotype 1a is most abundant in the United States, the majority of sequences in Europe and Japan are from genotype 1b.

**Table 1**

| % of sequences in the database | World | USA | Europe | Japan |
|---|---|---|---|---|
| Genotype 1 | 71.8 | 87.8 | 75.9 | 80.2 |
| Genotype 1 a | 28.4 | 66.4 | 21.7 | 1.6 |
| Genotype 1 b | 43.4 | 21.4 | 54.2 | 78.6 |

Based on the foregoing, there exists a significant need to identify synthetic or biological compounds for their ability to inhibit replication of both genotype 1a and genotype 1 b of HCV.

Various compounds useful in the inhibition of genotype 1a or genotype 1b of HCV are known. For example, pyrazole derivatives described in WO2005/092863 and thiophene derivatives described in WO2002/100851

### SUMMARY OF THE INVENTION

The present invention involves novel 2-carboxy thiophene compounds represented hereinbelow, pharmaceutical compositions comprising such compounds and use of the compounds in treating viral infection, especially HCV infection.

### DETAILED DESCRIPTION OF THE INVENTION

In a first aspect, the present invention provides a compound of Formula (I) : 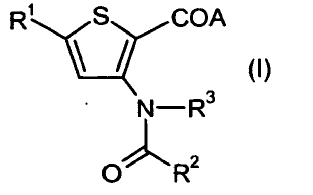 wherein:
A represents hydroxy;
R¹ represents -R^{X}-NHC(O)-R^{Y};
R^{x} represents phenyl (optionally substituted by one or more substituents selected independently from halo, methyl, ethyl, methoxy and trifluoromethyl), 5- or 6-membered heteroaryl (optionally substituted by one or more substituents selected independently from halo, methyl, ethyl, methoxy and trifluoromethyl) bonded through a carbon atom to the thiophene; the thiophene and amide groups being in a para relationship to each other;
R^{Y} represents phenyl (optionally substituted by one or more substituents selected independently from halo, methyl, ethyl, methoxy, trifluoromethyl, hydroxyl and amino) or 5- or 6-membered heteroaryl;
R² represents C₅₋₇cycloalkyl (optionally substituted by one or more substituents selected independently from -C₁₋₆alkyl [optionally substituted by one or more substituents selected independently from fluoro and -OR^{A}], halo and -OR^{A});
R^{A} represents hydrogen or -C₁₋₄alkyl; and
R³ represents C₁₋₆alkyl (optionally substituted by one or more substituents selected independently from fluoro, hydroxyl, methoxy, ethoxy, C₃₋₆cycloalkyl, and a 5 or 6-membered heteroaryl and heterocyclyl), tetrahydrofuranyl, pyranyl, C₃₋₇cycloalkyl or dioxanyl;
or salts, solvates or esters thereof.

It is to be understood that the present invention covers all combinations of aspects, suitable, convenient and preferred groups described herein.

As used herein, "acetyl" refers to -C(O)CH₃.

As used herein unless otherwise specified, "alkyl" refers to an optionally substituted hydrocarbon group. The alkyl hydrocarbon group may be linear or branched, saturated or unsaturated. Examples of such groups include methyl, ethyl, n-propyl, 1-methylethyl (isopropyl), n-butyl, isobutyl, sec-butyl, tert-butyl, n-pentyl, isopentyl, neopentyl or hexyl and the like. Where the alkyl hydrocarbon group is unsaturated, it will be understood that there will be a minimum of 2 carbon atoms in the group, for example an alkenyl or alkynyl group. In one aspect, alkyl moieties are saturated. In one aspect, alkyl moieties are -C₁₋₄alkyl. Unless otherwise stated, optional substituents include -C₁₋₆alkyl (unsubstituted), -C₃₋₇ cycloalkyl (unsubstituted), =CH(CH₂)ₜH, fluoro, -CF₃, -OR^{E}, -SR^{E}, -C(O)NR^{B}R^{C}, -C(O)R^{D}, -CO₂H, -CO₂R^{D}, -NR^{B}R^{C}, -NR^{A}C(O)R^{D}, -NR^{A}CO₂R^{D}, -NR^{A}SO₂R^{D}, -NR^{A}C(O)NR^{F}R^{G}, -SO₂NR^{F}R^{G}, -SO₂R^{D}, nitro, cyano, oxo, aryl, heteroaryl and heterocyclyl.

As used herein, the term "alkenyl" refers to a linear or branched hydrocarbon group containing one or more carbon-carbon double bonds. In one aspect the alkenyl group has from 2 to 6 carbon atoms. Examples of such groups include ethenyl, propenyl, butenyl, pentenyl or hexenyl and the like.

As used herein, the term "alkynyl" refers to a linear or branched hydrocarbon group containing one or more carbon-carbon triple bonds. In one aspect the alkynyl group has from 2 to 6 carbon atoms. Examples of such groups include ethynyl, propynyl, butynyl, pentynyl or hexynyl and the like.

As used herein unless otherwise specified, "cycloalkyl" refers to an optionally substituted, cyclic hydrocarbon group. The hydrocarbon group may be saturated or unsaturated, monocyclic or bridged bicyclic. Where the cycloalkyl group is saturated, examples of such groups include cyclopropyl, cyclobutyl, cyclopentyl, cyclohexyl, cycloheptyl or cyclooctyl and the like. Where the cycloalkyl group is unsaturated, examples of such groups include cyclobutenyl, cyclopentenyl, cyclohexenyl, cycloheptenyl or cyclooctenyl and the like. In one aspect, the cycloalkyl group has from 5 to 7 carbon atoms. In one aspect, cycloalkyl moieties are cyclohexenyl, cyclopentenyl and cyclohexyl. Unless otherwise stated, the cycloalkyl group may be substituted by one or more optional substituents including -C₁₋₆alkyl (unsubstituted), -C₃₋₇cycloalkyl (unsubstituted), =CH(CH₂)ₜH, fluoro, -CF₃, -OR^{E}, -SR^{E}, -C(O)NR^{B}R^{C}, -C(O)R^{D}, -CO₂H, -CO₂R^{D}, -NR^{B}R^{C}, -NR^{A}C(O)R^{D}, -NR^{A}CO₂R^{D}, -NR^{A}SO₂R^{D}, -NR^{A}C(O)NR^{F}R^{G}, -SO₂NR^{F}R^{G}, -SO₂R^{D}, nitro, cyano, oxo, phenyl and heterocyclyl.

As used herein, the term "alkoxy" refers to an -O-alkyl group wherein alkyl is as defined herein. Examples of such groups include methoxy, ethoxy, propoxy, butoxy, pentoxy or hexoxy and the like.

As used herein, "aryl" refers to an optionally substituted aromatic group with at least one ring having a conjugated pi-electron system, containing up to two conjugated or fused ring systems. "Aryl" includes carbocyclic aryl and biaryl groups, all of which may be optionally substituted. In one aspect, "aryl" moieties contain 6-10 carbon atoms. In one aspect, "aryl" moieties are unsubstituted, monosubstituted, disubstituted or trisubstituted phenyl. In one aspect, unless otherwise stated, "aryl" substituents are selected from the group consisting of -C₁₋₆alkyl, -C₃₋₇cycloalkyl, halo, -OR^{E}, -SR^{E}, -C(O)NR^{B}R^{C}, -C(O)R^{D}, -CO₂H, -CO₂R^{D}, -NR^{B}R^{C}, -NR^{A}C(O)R^{D}, -NR^{A}CO₂R^{D}, -NR^{A}SO₂R^{D}, -NR^{A}C(O)NR^{F}R^{G}, -SO₂NR^{F}R^{G}, -SO₂R^{D}, nitro, cyano, heterocyclyl, -CF₃, -OCF₃ and phenyl.

As used herein, "cyano" refers to -CN.

As used herein, "halogen" or "halo" refer to a fluorine, chlorine, bromine or iodine atom. References to "fluoro", "chloro", "bromo" or "iodo" should be construed accordingly.

As used herein, unless otherwise specified, "heteroaryl" refers to an optionally substituted, 5, 6, 8, 9 or 10 membered, aromatic group comprising one to four heteroatoms selected from N, O and S, with at least one ring having a conjugated pi-electron system, containing up to two conjugated or fused ring systems. In one aspect, "heteroaryl" moieties are unsubstituted, monosubstituted, disubstituted or trisubstituted (where applicable) pyridine, pyrazine, thiazole, thiophene, oxadiazole, oxazole, pyrimidine, pyridazine, benzodioxole, benzofuran, benzodioxin, indole, benzimidazole, benzofuran, indole, indazole, isoindole, benzothiophene, benzothiazole, benzoxazole, benzisoxazole, benzisothiazole, benzotriazole, furopyridine, furopyrimidine, furopyridazine, furopyrazine, furotriazine, pyrrolopyridine, pyrrolopyrimidine, pyrrolopyridazine, pyrrolopyrazine, pyrrolotriazine, thienopyridine, thienopyrimidine, thienopyridazine, thienopyrazine, thienotriazine, thiazolopyridine, thiazolopyrimidine, thiazolopyridazine, thiazolopyrazine, thiazolotriazine, oxazolopyridine, oxazolopyrimidine, oxazolopyridazine, oxazolopyrazine, oxazolotriazine, imidazopyridine, imidazopyrimidine, imidazopyridazine, imidazopyrazine, imidazotriazine, pyrazolopyridine, pyrazolopyrimidine, pyrazolopyridazine, pyrazolopyrazine, pyrazolotriazine, triazolopyridine, triazolopyrimidine, triazolopyridazine, triazolopyrazine, quinoline, naphthyridine, quinoxaline, quinazoline, isoquinoline, cinnoline, pyridopyridazine, pyridopyrimidine, pyridopyrazine, pyrazinopyrazine, pteridine, pyrazinopyridazine, pyrimidopyridazine, pyrimidopyrimidine, imidazothiazole, thiazolooxazole. All isomers of the above heteroaryls are within the scope of this invention. Each heteroaryl group may be attached at any ring carbon or may be attached through nitrogen when the nitrogen is part of a 5-membered ring. In one aspect, unless otherwise stated, "heteroaryl" substituents are selected from the group consisting of -C₁₋₆alkyl, -C₃₋₇cycloalkyl, halo, -OR^{E}, -SR^{E}, -C(O)NR^{B}R^{C}, -C(O)R^{D}, -CO₂R^{D}, -NR^{B}R^{C}, - NR^{A}C(O)R^{D}, -NR^{A}CO₂R^{D}, -NR^{A}SO₂R^{D}, -NR^{A}C(O)NR^{F}R^{G}, -SO₂NR^{F}R^{G}, -SO₂R^{D}, oxo, nitro, cyano, heterocyclyl, -CF₃ and phenyl.

As used herein, "heterocyclic" and "heterocyclyl" refer to an optionally substituted, 4, 5 or 6 membered, saturated or partially saturated, cyclic group containing 1 or 2 heteroatoms selected from N, optionally substituted by hydrogen, -C₁₋₆alkyl, -C₃₋₇cycloalkyl, -C(O)R^{D}, -C(O)NR^{B}R^{C}, -C(O)OH, -SO₂R^{D}, aryl or heteroaryl; O; and S, optionally substituted by one or two oxygen atoms. Ring carbon atoms may be optionally substituted by -C₁₋₆alkyl, -C₃₋₇ cycloalkyl, -OR^{A}, -C(O)R^{D}, or -SO₂R^{D}. In one aspect, unless otherwise stated, "heterocyclic" moieties are unsubstituted or monosubstituted tetrahydro-2H-pyran-4-yl, piperidinyl and tetrahydrofuran-3-yl.

As used herein, "nitro" refers to -NO₂.

As used herein, "oxo" refers to =O.

As used herein, "Et" refers to "ethyl", "iPr" refers to "isopropyl", "Me" refers to "methyl", "OBn" refers to "benzyloxy", and "Ph" refers to "phenyl".

R^{A} represents hydrogen, -C₁₋₆alkyl or -C₃₋₇cycloalkyl.

R^{B} and R^{C} independently represent hydrogen, -C₁₋₆alkyl, -C₃₋₇cycloalkyl, aryl, heterocyclyl or heteroaryl; or R^{B} and R^{C} together with the nitrogen atom to which they are attached form a 5- or 6-membered saturated cyclic group.

R^{D} represents -C₁₋₆alkyl, C₃₋₇cycloalkyl, aryl, heterocyclyl, heteroaryl, arylalkyl or heteroarylalkyl.

R^{E} represents hydrogen, -C₁₋₆alkyl, -C₃₋₇cycloalkyl, arylalkyl, heteroarylalkyl, aryl, heterocyclyl or heteroaryl.

R^{F} and R^{G} independently represent hydrogen, -C₁₋₆alkyl, -C₃₋₇cycloalkyl, aryl, heteroaryl, arylalkyl, or heteroarylalkyl; or R^{F} and R^{G} together with the nitrogen atom to which they are attached form a 5- or 6-membered saturated cyclic group.

As used herein, "arylalkyl" refers to one or more aryl groups attached to an alkyl radical. In one aspect, arylalkyl groups are benzyl or phenethyl.

As used herein, "heteroarylalkyl" refers to one or more heteroaryl groups attached to an alkyl radical. In one aspect, arylalkyl groups are pyridylmethyl or furanylmethyl.

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

As used herein, the term "substituted" refers to substitution with the named substituent or substituents, multiple degrees of substitution being allowed unless otherwise stated.

In one aspect, R^{X} represents phenyl (optionally substituted by one or more substituents selected independently from chloro, methoxy and trifluoromethyl), pyridinyl (optionally substituted by one or more substituents selected independently from halo, methyl, ethyl, methoxy and trifluoromethyl) bonded through a carbon atom to the thiophene, the thiophene and amide groups being in a *para* relationship to each other, or unsubstituted thienyl bonded to the amide group and the thiophene at the 2- and 5-positions.

In a further aspect, R^{X} represents phenyl (optionally substituted by one or more substituents selected independently from chloro, methoxy and trifluoromethyl), pyridinyl (optionally substituted by one or more substituents selected independently from chloro and methyl) bonded through a carbon atom to the thiophene, the thiophene and amide groups being in a para relationship to each other, or unsubstituted thienyl bonded to the amide group and the thiophene at the 2- and 5-positions.

In a further aspect, R^{X} represents unsubstituted phenyl, chlorophenyl (for example 2-chlorophenyl), (trifluoromethyl)phenyl (for example 3-(trifluoromethyl)phenyl), methoxyphenyl (for example 3-methoxyphenyl); unsubstituted pyridinyl (for example 2-pyridinyl or 3-pyridinyl), methylpyridinyl (for example 5-methyl-3-pyridinyl or 4-methyl-2-pyridinyl) or chloropyridinyl (for example 5-chloro-3-pyridinyl), wherein the pyridinyl ring in all cases is bonded through a carbon atom to the thiophene, the thiophene and amide groups being in a para relationship to each other; or unsubstituted thienyl bonded to the aminde group and the thiophene at the 2- and 5-positions.

In one aspect, R^{X} represents phenyl (optionally substituted by halo, methyl, ethyl, methoxy or trifluoromethyl) or pyridyl. In one aspect, R^{X} represents phenyl optionally substituted by halo, methyl, methoxy or trifluoromethyl. In a further aspect R^{X} represents unsubstituted phenyl.

In one aspect, R^{Y} represents phenyl (optionally substituted by one or more substituents selected from halo) or 5- or 6-membered heteroaryl.

In one aspect, R^{Y} represents phenyl (optionally substituted by one or more fluoro groups). In a further aspect, R^{Y} represents fluorophenyl. In a further aspect, R^{Y} represents 2-fluorophenyl, 3- fluorophenyl, 4 fluorophenyl or 2,6- difluorophenyl.

In one aspect, R^{Y} represents a 6-membered heteroaryl. In a further aspect, R^{Y} represents a 6-membered heteroaryl (optionally substituted by one or more substituents selected from methyl, ethyl, amino and hydroxymethyl). In a further aspect, R^{Y} represents pyridinyl, pyrazinyl, pyridazinyl or pyrimidinyl (all of which may be optionally substituted by one or more substituents selected independently from methyl and fluoro). In a further aspect, R^{Y} represents unsubstituted pyridinyl (for example 2-pyridinyl, 3-pyridinyl or 4-pyridinyl), methylpyridinyl (for example 3-methyl-2-pyridinyl, 6-methyl-2-pyridinyl, 2-methyl-3-pyridinyl, 6-methyl-3-pyridinyl, 5-methyl-3-pyridinyl or 2-methyl-4-pyridinyl) or fluoropyridinyl (for example 3-fluoro-2-pyridinyl or 5-fluoro-3-pyridinyl); unsubstituted pyrazinyl (for example 2-pyrazinyl) or methylpyrazinyl (for example 5-methyl-2-pyrazinyl); unsubstituted pyridazinyl (for example 3-pyridazinyl); or unsubstituted pyrimidinyl (for example 4-pyrimidinyl).

In one aspect, R^{Y} represents a 5-membered heteroaryl. In a further aspect, R^{Y} represents a 5-membered heteroaryl (optionally substituted by one or more substituents selected from methyl, ethyl, amino and hydroxymethyl). In a further aspect, R^{Y} represents furanyl (optionally substituted by one or more substituents selected independently from methyl and hydroxymethyl). In a further aspect, R^{Y} represents unsubstituted furanyl (for example 2-furanyl or 3-furanyl), methylfuranyl (for example 3-methyl-2-furanyl or 5-methyl-2-furanyl) or hydroxymethylfuranyl (for example 5-(hydroxymethyl)-2-furanyl).

In one aspect, R^{Y} represents oxazolyl or isoxazolyl, both of which may be optionally substituted by one or more substituents selected independently from methyl and ethyl. In a further aspect, R^{Y} represents unsubstituted oxazolyl (for example 1,3-oxazol-2-yl, 1,3-oxazol-4-yl or 1,3-oxazol-5-yl) or methyloxazolyl (for example 4-methyl-1,3-oxazol-5-yl or 2,4-dimethyl-1,3-oxazol-5-yl); unsubstituted isoxazolyl, methylisoxazolyl (for example 5-methyl-3-isoxazolyl, 3-methyl-4-isoxazolyl, 5-methyl-4-isoxazolyl, 3-methyl-5-isoxazolyl, or ethylisoxazolyl (for example 5-ethyl-3-isoxazolyl or 3,5-dimethyl-4-isoxazolyl).

In one aspect, R^{Y} represents thienyl, thiazolyl or isothiazolyl (all of which may be optionally substituted by one or more substituents selected independently from methyl and amino). In a further aspect, R^{Y} represents unsubstituted thienyl (for example 2-thienyl); unsubstituted thiazolyl (for example 1,3-thiazol-4-yl or 1,3-thiazol-5-yl), methylthiazolyl (for example 2-methyl-1,3-thiazol-4-yl or 4-methyl-1,3-thiazol-5-yl) or aminothiazolyl (for example 2-amino-1,3-thiazol-4-yl); or unsubstituted isothiazolyl (for example 5-isothiazolyl).

In one aspect, R^{Y} represents pyrazolyl or imidazolyl (both of which may be optionally substituted by one or more substituents selected independently from methyl and ethyl). In a further aspect, R^{Y} represents unsubstituted pyrazolyl (for example 1*H*-pyrazol-3-yl or 1*H-*pyrazol-4-yl), methyl pyrazolyl (for example 1-methyl-1*H*-pyrazol-3-yl, 1-methyl-1*H*-pyrazol-4-yl, 3-methyl-1*H*-pyrazol-4-yl, 5-methyl-1*H*-pyrazol-3-yl, 1,5-dimethyl-1*H*-pyrazol-3-yl, 1,5-dimethyl-1*H*-pyrazol-4-yl, 1,3-dimethyl-1*H*-pyrazol-4-yl or 1,3-dimethyl-1*H*-pyrazol-5-yl), ethyl pyrazolyl (for example 1-ethyl-1*H*-pyrazol-3-yl, 1-ethyl-1*H*-pyrazol-4-yl or 1-ethyl-1*H*-pyrazol-5-yl); unsubstituted imidazolyl (for example 1*H*-imidazol-2-yl or 1*H*-imidazol-4-yl), methyl imidazolyl (for example 1-methyl-1*H*-imidazol-2-yl, 1-methyl-1*H*-imidazol-4-yl or 1-methyl-1*H-*imidazol-5-yl), or ethyl imidazolyl (for example 1-ethyl-1*H* imidazol-5-yl).

In one aspect, R^{Y} represents phenyl (optionally substituted by one or more fluoro groups); or pyridinyl, pyrazinyl, pyridazinyl or pyrimidinyl (all of which may be optionally substituted by one or more substituents selected independently from methyl and fluoro); or furanyl (optionally substituted by one or more substituents selected independently from methyl and hydroxymethyl); or oxazolyl or isoxazolyl (both of which may be optionally substituted by one or more substituents selected independently from methyl and ethyl); or thienyl or thiazolyl (both of which may be optionally substituted by one or more substituents selected independently from methyl and amino); or pyrazolyl or imidazolyl (both of which may be optionally substituted by one or more substituents selected independently from methyl and ethyl).

In a further aspect, R^{Y} represents fluorophenyl; unsubstituted pyridinyl, methylpyridinyl or fluoropyridinyl; unsubstituted pyrazinyl or methylpyrazinyl; unsubstituted pyridazinyl; unsubstituted pyrimidinyl; unsubstituted furanyl, methylfuranyl or hydroxymethylfuranyl; unsubstituted oxazolyl, methyloxazolyl; unsubstituted isoxazolyl, methylisoxazolyl or ethylisoxazolyl; unsubstituted thienyl; unsubstituted thiazolyl, methylthiazolyl or aminothiazolyl; pyrazolyl, methyl pyrazolyl or ethyl pyrazolyl; unsubstituted imidazolyl, methylimidazolyl or ethylimidazolyl.

In a further aspect, R^{Y} represents 2-fluorophenyl, 3-fluorophenyl, 4-fluorophenyl, 2,6-difluorophenyl 2-pyridinyl, 3-pyridinyl, 4-pyridinyl, 3-methyl-2-pyridinyl, 6-methyl-2-pyridinyl, 2-methyl-3-pyridinyl, 6-methyl-3-pyridinyl, 5-methyl-3-pyridinyl, 2-methyl-4-pyridinyl, 3-fluoro-2-pyridinyl, 5-fluoro-3-pyridinyl, 2-pyrazinyl, 5-methyl-2-pyrazinyl, 3-pyridazinyl, 4-pyrimidinyl, 2-furanyl, 3-furanyl, 3-methyl-2-furanyl, 5-methyl-2-furanyl, 5-(hydroxymethyl)-2-furanyl, 1,3-oxazol-2-yl, 1,3-oxazol-4-yl, 1,3-oxazol-5-yl, 4-methyl-1,3-oxazol-5-yl, 2,4-dimethyl-1,3-oxazol-5-yl, 5-methyl-3-isoxazolyl, 3-methyl-4-isoxazolyl, 5-methyl-4-isoxazolyl, 3-methyl-5-isoxazolyl, 5-ethyl-3-isoxazolyl, 3,5-dimethyl-4-isoxazolyl, 2-thienyl, 1,3-thiazol-4-yl, 1,3-thiazol-5-yl, 2-methyl-1,3-thiazol-4-yl, 4-methyl-1,3-thiazol-5-yl, 2-amino-1,3-thiazol-4-yl, 5-isothiazolyl, 1*H*-pyrazol-3-yl, 1*H*-pyrazol-4-yl, 1-methyl-1*H*-pyrazol-3-yl, 1-methyl-1*H*-pyrazol-4-yl, 3-methyl-1*H*-pyrazol-4-yl, 5-methyl-1*H*-pyrazol-3-yl, 1,5-dimethyl-1*H*-pyrazol-3-yl, 1,5-dimethyl-1*H*-pyrazol-4-yl, 1,3-dimethyl-1*H*-pyrazol-4-yl, 1,3-dimethyl-1*H*-pyrazol-5-yl, 1-ethyl-1*H*-pyrazol-3-yl, 1-ethyl-1*H*-pyrazol-4-yl, 1-ethyl-1*H*-pyrazol-5-yl, 1*H*-imidazol-2-yl, 1*H-*imidazol-4-yl, 1-methyl-1*H*-imidazol-2-yl, 1-methyl-1*H*-imidazol-4-yl, 1-methyl-1*H*-imidazol-5-yl or 1-ethyl-1*H*-imidazol-5-yl.

In one aspect, R^{Y} represents 1,3-thiazol-4-yl, 3-pyridazinyl, 1*H*-pyrazol-3-yl, 1-methyl-1*H-*imidazol-4-yl or 1,5-dimethyl-1*H*-pyrazol-3-yl.

In one aspect, R^{Y} represents a 6-membered heteroaryl group. In one aspect, R^{Y} represents thiazolyl, pyridyl or phenyl. In one aspect, R^{Y} represents thiazolyl. In a further aspect R^{Y} represents thiazol-4-yl.

In one aspect, R² represents unsubstituted cyclohexyl or cyclohexyl substituted by one or more substituents selected independently from C₁₋₄alkyl (optionally substituted by one or more fluoro groups [for example methyl or trifluoromethyl]) and halo (for example, fluoro). In a further aspect, R² represents *trans*-4-methylcyclohexyl, *trans*-(4-trifluoromethyl)cyclohexyl or *cis*-(4-fluoro-4-methyl)cyclohexyl. In a further aspect, R² represents *trans*-4-methylcyclohexyl.

In one aspect, R³ represents C₁₋₆alkyl (optionally substituted by one or more substituents selected independently from fluoro, hydroxyl, methoxy, ethoxy, C₃₋₆cycloalkyl, and a 5 or 6- membered heteroaryl and heterocyclyl), tetrahydrofuranyl, pyranyl or dioxanyl.

In one aspect, R³ represents C₁₋₆alkyl (optionally substituted by one or more substituents selected independently from fluoro, methoxy and C₃₋₆cycloalkyl), C₃₋₇cycloalkyl or dioxan. In a further aspect, R³ represents 1-methylethyl, ethyl, 2,2-difluoroethyl, cyclopropylmethyl, 1,3-dioxan-5-yl, cyclobutyl, 1-methyl-2-(methyloxy)ethyl, 2-(methyloxy)ethyl or 2,2,2-trifluoroethyl.

In one aspect, R³ represents unsubstituted linear or branched C₁₋₆alkyl, methoxyethyl, tetrahydrofuran, pyran or C₃₋₇cycloalkyl. In one aspect, R³ represents unsubstituted linear or branched C₁₋₆alkyl. In a further aspect, R³ represents 1-methylethyl.

In one aspect, R^{A} represents hydrogen.

In one aspect, R^{X} represents phenyl (optionally substituted by one or more substituents selected independently from chloro, methoxy and trifluoromethyl), pyridinyl (optionally substituted by one or more substituents selected independently from halo, methyl, methoxy and trifluoromethyl) bonded through a carbon atom to the thiophene, the thiophene and amide groups being in a *para* relationship to each other, or unsubstituted thienyl bonded to the amide group and the thiophene at the 2- and 5-positions; R^{Y} represents phenyl (optionally substituted by one or more substituents selected from halo) or 5- or 6-membered heteroaryl; R² represents unsubstituted cyclohexyl or cyclohexyl substituted by one or more substituents selected independently from C₁₋₄alkyl (optionally substituted by one or more fluoro groups) and halo; and R³ represents C₁₋₆alkyl (optionally substituted by one or more substituents selected independently from fluoro, methoxy and C₃₋₆cycloalkyl), C₃₋₇cycloalkyl or dioxanyl.

In one aspect, R^{X} represents phenyl (optionally substituted by one or more substituents selected independently from chloro, methoxy and trifluoromethyl), pyridinyl (optionally substituted by one or more substituents selected independently from chloro and methyl) bonded through a carbon atom to the thiophene, the thiophene and amide groups being in a *para* relationship to each other, or unsubstituted thienyl bonded to the amide group and the thiophene at the 2- and 5-positions; R^{Y} represents phenyl optionally substituted by one or more fluoro groups; or pyridinyl, pyrazinyl, pyridazinyl or pyrimidinyl, all of which may be optionally substituted by one or more substituents selected independently from methyl and fluoro; or furanyl (optionally substituted by one or more substituents selected independently from methyl and hydroxymethyl); or oxazolyl or isoxazolyl, both of which may be optionally substituted by one or more substituents selected independently from methyl and ethyl; or thienyl or thiazolyl, both of which may be optionally substituted by one or more substituents selected independently from methyl and amino; or pyrazolyl or imidazolyl, both of which may be optionally substituted by one or more substituents selected independently from methyl and ethyl; R² represents unsubstituted cyclohexyl or cyclohexyl substituted by one or more substituents selected independently from C₁₋₄alkyl (optionally substituted by one or more fluoro groups) and halo; and R³ represents C₁₋₆alkyl (optionally substituted by one or more substituents selected independently from fluoro, methoxy and C₃₋₆cycloalkyl), C₃₋₇cycloalkyl or dioxan.

In one aspect, R^{X} represents unsubstituted phenyl, 2-chlorophenyl, 3-(trifluoromethyl)phenyl, 3-methoxyphenyl, 2-pyridinyl, 3-pyridinyl, 5-methyl-3-pyridinyl, 4-methyl-2-pyridinyl, chloropyridinyl, 5-chloro-3-pyridinyl, wherein the pyridinyl ring in all cases is bonded through a carbon atom to the thiophene, the thiophene and amide groups being in a para relationship to each other; or unsubstituted thienyl bonded to the amide group and the thiophene at the 2- and 5-positions; R^{Y} represents 2-fluorophenyl, 3- fluorophenyl, 4 fluorophenyl, 2,6-difluorophenyl 2-pyridinyl, 3-pyridinyl, 4-pyridinyl, 3-methyl-2-pyridinyl, 6-methyl-2-pyridinyl, 2-methyl-3-pyridinyl, 6-methyl-3-pyridinyl, 5-methyl-3-pyridinyl, 2-methyl-4-pyridinyl, 3-fluoro-2-pyridinyl, 2-pyrazinyl, 5-methyl-2-pyrazinyl, 3-pyridazinyl, 4-pyrimidinyl, 2-furanyl, 3-furanyl, 3-methyl-2-furanyl, 5-methyl-2-furanyl, 5-(hydroxymethyl)-2-furanyl, 1,3-oxazol-2-yl, 4-methyl-1,3-oxazol-5-yl, 2,4-dimethyl-1,3-oxazol-5-yl, 5-methyl-3-isoxazolyl, 3-methyl-4-isoxazolyl, 5-methyl-4-isoxazolyl, 3-methyl-5-isoxazolyl, 5-ethyl-3-isoxazolyl, 3,5-dimethyl-4-isoxazolyl, 2-thienyl, 1,3-thiazol-4-yl, 1,3-thiazol-5-yl, 2-methyl-1,3-thiazol-4-yl, 4-methyl-1,3-thiazol-5-yl, 2-amino-1,3-thiazol-4-yl, 1*H*-pyrazol-3-yl, 1*H*-pyrazol-4-yl, 1-methyl-1*H*-pyrazol-3-yl, 1-methyl-1*H*-pyrazol-4-yl, 3-methyl-1*H*-pyrazol-4-yl, 5-methyl-1*H*-pyrazol-3-yl, 1,5-dimethyl-1*H*-pyrazol-3-yl, 1,5-dimethyl-1*H*-pyrazol-4-yl, 1,3-dimethyl-1*H*-pyrazol-4-yl, 1,3-dimethyl-1*H*-pyrazol-5-yl, 1-ethyl-1*H*-pyrazol-3-yl, 1-ethyl-1*H*-pyrazol-4-yl, 1-ethyl-1*H-*pyrazol-5-yl, 1*H*-imidazol-2-yl, 1*H*-imidazol-4-yl, 1-methyl-1*H*-imidazol-2-yl, 1-methyl-1*H-*imidazol-4-yl, 1-methyl-1*H*-imidazol-5-yl or 1-ethyl-1*H*-imidazol-5-yl; R² represents *trans*-4-methylcyclohexyl, *trans*-(4-trifluoromethyl)cyclohexyl or *cis*-(4-fluoro-4-methyl)cyclohexyl; and R³ represents 1-methylethyl, ethyl, 2,2-difluoroethyl, cyclopropylmethyl, 1,3-dioxan-5-yl, cyclobutyl, 1-methyl-2-(methyloxy)ethyl, 2-(methyloxy)ethyl or 2,2,2-trifluoroethyl.

In one aspect, R^{X} represents unsubstituted phenyl; R^{Y} represents thiazolyl, pyridyl or phenyl; R² represents trans-4-methylcyclohexyl; and R³ represents 1-methylethyl.

In one aspect, there is provided at least one chemical entity chosen from compounds of Formula (Ia) : 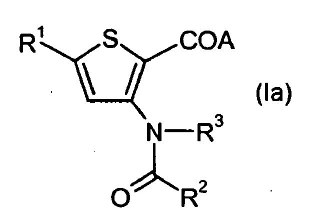 wherein:
A represents hydroxy;
R¹ represents -R^{X}-NHC(O)-R^{Y};
R^{X} represents phenyl (optionally substituted by halo, methyl, ethyl, methoxy or trifluoromethyl) or 6-membered heteroaryl bonded through a carbon atom to the thiophene; the thiophene and amide groups being in a para relationship to each other;
R^{Y} represents phenyl (optionally substituted by halo, methyl, ethyl, methoxy, trifluoromethyl, hydroxyl or amino) or 5- or 6-membered heteroaryl;
R² represents C₅₋₇cycloalkyl optionally substituted by one or more substituents selected from -C₁₋₆alkyl (optionally substituted with fluoro or -OR^{A}) and -OR^{A};
R^{A} represents hydrogen or -C₁₋₄alkyl; and
R³ represents linear, or branched C₁₋₆alkyl (optionally substituted by one or more substituents selected from fluoro, hydroxyl, methoxy, ethoxy, C₃₋₆cycloalkyl, or a 5 or 6- membered heteroaryl or heterocyclyl), tetrahydrofuran, pyran or C₃₋₇cycloalkyl;
and salts, solvates and esters thereof.

As used herein the term "at least one chemical entity" means at least one chemical substance chosen from the group of compounds consisting of compounds of Formula I and pharmaceutically acceptable salts, solvates and esters thereof.

In one aspect, the present invention provides a compound selected from the group consisting of:
3-[[(*trans*-4-Methylcyclohexyl)carbonyl](1-methylethyl)amino]-5-{4-[(1,3-thiazol-4-ylcarbonyl)amino]phenyl}-2-thiophenecarboxylic acid;
3-[[(*trans*-4-Methylcyclohexyl)carbonyl](1-methylethyl)amino]-5-{4-[(2-pyridinylcarbonyl)amino]phenyl}-2-thiophenecarboxylic acid;
5-{4-[(2-Furanylcarbonyl)amino]phenyl}-3-[[(*trans*-4-methylcyclohexyl)carbonyl](1-methylethyl)amino]-2-thiophenecarboxylic acid;
3-[[(*trans*-4-Methylcyclohexyl)carbonyl](1-methylethyl)amino]-5-(4-{[(2-methyl-1,3-thiazol-4-yl)carbonyl]amino}phenyl)-2-thiophenecarboxylic acid;
3-[[(*trans*-4-Methylcyclohexyl)carbonyl](1-methylethyl)amino]-5-{4-[(2-thienylcarbonyl)amino]phenyl}-2-thiophenecarboxylic acid;
3-[[(*trans*-4-Methylcyclohexyl)carbonyl](1-methylethyl)amino]-5-{4-[(2-pyrazinylcarbonyl)amino]phenyl}-2-thiophenecarboxylic acid;
5-(4-{[(4-Fluorophenyl)carbonyl]amino}phenyl)-3-[[(*trans*-4-methylcyclohexyl)carbonyl](1-methylethyl)amino]-2-thiophenecarboxylic acid;
3-[[(*trans*-4-Methylcyclohexyl)carbonyl](1-methylethyl)amino]-5-{4-[(1,3-oxazol-2-ylcarbonyl)amino]phenyl}-2-thiophenecarboxylic acid;
3-{(Cyclopropylmethyl)[(*trans*-4-methylcyclohexyl)carbonyl]amino}-5-{4-[(1,3-thiazol-4-ylcarbonyl)amino]phenyl}-2-thiophenecarboxylic acid;
3-((Cyclopropylmethyl){[*trans*-4-(trifluoromethyl)cyclohexyl]carbonyl}amino)-5-{4-[(1,3-thiazol-4-ylcarbonyl)amino]phenyl]-2-thiophenecarboxylic acid;
3-((1-Methylethyl){[*trans*-4-(trifluoromethyl)cyclohexyl]carbonyl}amino)-5{4-[(1,3-thiazol-4-ylcarbonyl)amino]phenyl}-2-thiophenecarboxylic acid;
3-[[(*trans*-4-Methylcyclohexyl)carbonyl](1-methylethyl)amino]-5-{4-[(3-pyridazinylcarbonyl)amino]phenyl}-2-thiophenecarboxylic acid;
3-[[(*trans*-4-Methylcyclohexyl)carbonyl](1-methylethyl)amino]-5-{4-[(4-pyrimidinylcarbonyl)amino]phenyl}-2-thiophenecarboxylic acid;
5-{3-Chloro-4-[(1,3-thiazol-4-ylcarbonyl)amino]phenyl}-3-[[(*trans*-4-methylcyclohexyl)carbonyl](1-methylethyl)amino]-2-thiophenecarboxylic acid;
3-[[(*trans*-4-Methylcyclohexyl)carbonyl](1-methylethyl)amino]-5-{4-[(3-thienylcarbonyl)amino]phenyl}-2-thiophenecarboxylic acid;
5-(3-Chloro-4-{[(4-fluorophenyl)carbonyl]amino}phenyl)-3-[[(*trans*-4-methylcyclohexyl)carbonyl](1-methylethyl)amino]-2-thiophenecarboxylic acid;
4-[[(*trans*-4-Methylcyclohexyl)carbonyl](1-methylethyl)amino]-5'-[(2-pyridinylcarbonyl)amino]-2,2'-bithiophene-5-carboxylic acid;
3-[[(*trans*-4-Methylcyclohexyl)carbonyl](1-methylethyl)amino]-5-(4-{[(1-methyl-1*H*-pyrazol-4-yl)carbonyl]amino}phenyl)-2-thiophenecarboxylic acid;
3-[[(*trans*-4-Methylcyclohexyl)carbonyl](1-methylethyl)amino]-5-(4-{[(5-methyl-3-isoxazolyl)carbonyl]amino}phenyl)-2-thiophenecarboxylic acid;
4-[[(*trans-*4-Methylcyclohexyl)carbonyl](1-methylethyl)amino]-5'-[(1,3-thiazol-4-ylcarbonyl)amino]-2,2'-bithiophene-5-carboxylic acid;
3-[[(*trans*-4-Methylcyclohexyl)carbonyl](1-methylethyl)amino]-5-{4-[(1*H*-pyrazol-3-ylcarbonyl)amino]phenyl}-2-thiophenecarboxylic acid;
3-[[(*trans*-4-Methylcyclohexyl)carbonyl](1-methylethyl)amino]-5-(4-{[(5-methyl-1*H*-pyrazol-3-yl)carbonyl]amino}phenyl}-2-thiophenecarboxylic acid;
5-{4-[(1*H*-Imidazol-4-ylcarbonyl)amino]phenyl}-3-[[(*trans*-4-methylcyclohexyl)carbonyl](1-methylethyl)amino]-2-thiophenecarboxylic acid;
5-(4-{[(1,5-Dimethyl-1*H*-pyrazol-3-yl)carbonyl]amino}phenyl)-3-[[(*trans*-4-methylcyclohexyl)carbonyl](1-methylethyl)amino]-2-thiophenecarboxylic acid;
3-[[(*trans*-4-Methylcyclohexyl)carbonyl](1-methylethyl)amino]-5-(4-{[(5-methyl-2-pyrazinyl)carbonyl]amino}phenyl)-2-thiophenecarboxylic acid;
3-[[(*trans*-4-Methylcyclohexyl)carbonyl](1-methylethyl)amino]-5-(4-{[(3-methyl-5-isoxazolyl)carbonyl]amino}phenyl)-2-thiophenecarboxylic acid;
3-[[(*trans*-4-Methylcyclohexyl)carbonyl](1-methylethyl)amino]-5-(4-{[(4-methyl-1,3-thiazol-5-yl)carbonyl]amino}phenyl)-2-thiophenecarboxylic acid;
3-[[(*trans*-4-Methylcyclohexyl)carbonyl](1-methylethyl)amino]-5-(4-{[(3-methyl-2-pyridinyl)carbonyl]amino}phenyl)-2-thiophenecarboxylic acid;
5-{4-[(1*H*-Imidazol-2-ylcarbonyl)amino]phenyl)-3-[[(*trans*-4-methylcyclohexyl)carbonyl](1-methylethyl)amino]-2-thiophenecarboxylic acid;
3-[[(*trans*-4-Methylcyclohexyl)carbonyl](1-methylethyl)amino]-5-(4-{[(6-methyl-2-pyridinyl)carbonyl]amino}phenyl)-2-thiophenecarboxylic acid;
3-[[(*trans*-4-Methylcyclohexyl)carbonyl](1-methylethyl)amino]-5-(4-{[(1-methyl-1*H*-imidazol-4-yl)carbonyl]amino}phenyl)-2-thiophenecarboxylic acid;
3-[[(*trans*-4-Methylcyclohexyl)carbonyl](1-methylethyl)amino]-5-(4-{[(2-methyl-4-pyridinyl)carbonyl]amino}phenyl)-2-thiophenecarboxylic acid;
5-(4-{[(3-Fluorophenyl)carbonyl]amino}phenyl)-3-[[(*trans*-4-methylcyclohexyl)carbonyl](1-methylethyl)amino]-2-thiophenecarboxylic acid;
3-[[(*trans*-4-Methylcyclohexyl)carbonyl](1-methylethyl)amino]-5-(4-{[(1-methyl-1*H*-imidazol-2-yl)carbonyl]amino}phenyl)-2-thiophenecarboxylic acid;
3-[[(*trans*-4-Methylcyclohexyl)carbonyl](1-methylethyl)amino]-5-(4-{[(1-methyl-1*H*-pyrazol-3-yl)carbonyl]amino}phenyl)-2-thiophenecarboxylic acid;
5-(4-{[(5-Ethyl-3-isoxazolyl)carbonyl]amino}phenyl)-3-[[(*trans*-4-methylcyclohexyl)carbonyl](1-methylethyl)amino]-2-thiophenecarboxylic acid;
5-{4-[(3-Furanylcarbonyl)amino]phenyl}-3-[[(*trans*-4-methylcyclohexyl)carbonyl](1-methylethyl)amino]-2-thiophenecarboxylic acid;
5-(4-{[(2-Fluorophenyl)carbonyl]amino}phenyl)-3-[[(*trans*-4-methylcyclohexyl)carbonyl](1-methylethyl)amino]-2-thiophenecarboxylic acid;
5-(4-{[(2,6-Difluorophenyl)carbonyl]amino}phenyl)-3-[[(*trans*-4-methylcyclohexyl)carbonyl](1-methylethyl)amino]-2-thiophenecarboxylic acid;
3-[[(*trans*-4-Methylcyclohexyl)carbonyl](1-methylethyl)amino]-5-(4-{[(3-methyl-2-furanyl)carbonyl]amino}phenyl)-2-thiophenecarboxylic acid;
5-(4-{[(2-Amino-1,3-thiazol-4-yl)carbonyl]amino}phenyl)-3-[[(*trans*-4-methylcyclohexyl)carbonyl](1-methylethyl)amino]-2-thiophenecarboxylic acid;
5-(4-{[(3-Fluoro-2-pyridinyl)carbonyl]amino}phenyl)-3-[[(*trans*-4-methylcyclohexyl)carbonyl](1-methylethyl)amino]-2-thiophenecarboxylic acid;
5-(4-{[(1-Ethyl-1*H*-pyrazol-3-yl)carbonyl]amino}phenyl)-3-[[(*trans*-4-methylcyclohexyl)carbonyl](1-methylethyl)amino]-2-thiophenecarboxylic acid;
3-[[(*trans*-4-Methylcyclohexyl)carbonyl](1-methylethyl)amino]-5-(4-{[(5-methyl-2-furanyl)carbonyl]amino}phenyl)-2-thiophenecarboxylic acid;
5-[4-({[5-(Hydroxymethyl)-2-furanyl]carbonyl}amino)phenyl]-3-[[(*trans*-4-methylcyclohexyl)carbonyl](1-methylethyl)amino]-2-thiophenecarboxylic acid;
3-[[(*trans*-4-Methylcyclohexyl)carbonyl](1-methylethyl)amino]-5-(4-{[(4-methyl-3-pyridinyl)carbonyl]amino}phenyl)-2-thiophenecarboxylic acid;
3-[[(*trans*-4-Methylcyclohexyl)carbonyl](1-methylethyl)amino]-5-(4-{[(3-methyl-4-isoxazolyl)carbonyl]amino}phenyl)-2-thiophenecarboxylic acid;
3-[[(*trans*-4-Methylcyclohexyl)carbonyl](1-methylethyl)amino]-5-{6-[(1,3-thiazol-4-ylcarbonyl)amino]-3-pyridinyl}-2-thiophenecarboxylic acid;
3-[[(*trans*-4-Methylcyclohexyl)carbonyl](1-methylethyl)amino]-5-{6-[(2-pyridinylcarbonyl)amino]-3-pyridinyl}-2-thiophenecarboxylic acid;
3-[[(*trans*-4-Methylcyclohexyl)carbonyl](1-methylethyl)amino]-5-{6-[(2-pyrazinylcarbonyl)amino]-3-pyridinyl}-2-thiophenecarboxylic acid;
5-(6-{[(4-Fluorophenyl)carbonyl]amino}-3-pyridinyl}-3-[[(*trans*-4-methylcyclohexyl)carbonyl](1-methylethyl)amino]-2-thiophenecarboxylic acid;
5-{6-[(2-Furanylcarbonyl)amino]-3-pyridinyl}-3-[[(*trans*-4-methylcyclohexyl)carbonyl](1-methylethyl)amino]-2-thiophenecarboxylic acid;
3-{(2,2-Difluoroethyl)[(*trans*-4-methylcyclohexyl)carbonyl]amino}-5-{4-[(1,3-thiazol-4-ylcarbonyl)amino]phenyl}-2-thiophenecarboxylic acid;
3-{Ethyl[(*trans*-4-methylcyclohexyl)carbonyl]amino}-5-{4-[(1,3-thiazol-4-ylcarbonyl)amino]phenyl}-2-thiophenecarboxylic acid;
3-[[(*cis*-4-Fluoro-4-methylcyclohexyl)carbonyl](1-methylethyl)amino]-5-{4-[(1,3-thiazol-4-ylcarbonyl)amino]phenyl}-2-thiophenecarboxylic acid;
3-{(Cyclopropylmethyl)[(trans-4-methylcyclohexyl)carbonyl]amino}-5-{4-[(2-pyridinylcarbonyl)amino]phenyl}-2-thiophenecarboxylic acid;
3-{(Cyclopropylmethyl)[(*trans*-4-methylcyclohexyl)carbonyl]amino}-5-{4-[(2-pyrazinylcarbonyl)amino]phenyl}-2-thiophenecarboxylic acid;
3-{(cyclopropylmethyl)[(*trans*-4-methylcyclohexyl)carbonyl]amino}-5-(4-{[(1-methyl-1*H-*imidazol-4-yl)carbonyl]amino}phenyl)-2-thiophenecarboxylic acid;
3-{(Cyclopropylmethyl)[(*trans*-4-methylcyclohexyl)carbonyl]amino}-5-{4-[(3-pyridazinylcarbonyl)amino]phenyl}-2-thiophenecarboxylic acid;
3-{(Cyclopropylmethyl)[(*trans*-4-methylcyclohexyl)carbonyl]amino}-5-(4-{[(1-methyl-1*H-*pyrazol-3-yl)carbonyl]amino}phenyl)-2-thiophenecarboxylic acid;
3-{(Cyclopropylmethyl)[(*trans*-4-methylcyclohexyl)carbonyl]amino}-5-(4-{[(1,5-dimethyl-1*H-*pyrazol-3-yl)carbonyl]amino}phenyl)-2-thiophenecarboxylic acid;
3-[[(*trans*-4-Methylcyclohexyl)carbonyl](1-methylethyl)amino]-5-[4-[(1,3-thiazol-4-ylcarbonyl)amino]-3-(trifluoromethyl)phenyl]-2-thiophenecarboxylic acid;
3-[[(*trans*-4-Methylcyclohexyl)carbonyl](1-methylethyl)amino]-5-{3-(methyloxy)-4-[(1,3-thiazol-4-ylcarbonyl)amino]phenyl}-2-thiophenecarboxylic acid;
3-{1,3-Dioxan-5-yl[(*trans*-4-methylcyclohexyl)carbonyl]amino}-5-{4-[(1,3-thiazol-4-ylcarbonyl)amino]phenyl}-2-thiophenecarboxylic acid;
3-{Cyclobutyl[(*trans*-4-methylcyclohexyl}carbonyl]amino}-5-{4-[(1,3-thiazol-4-ylcarbonyl)amino]phenyl}-2-thiophenecarboxylic acid;
3-{[(*trans*-4-Methylcyclohexyl)carbonyl][1-methyl-2-(methyloxy)ethyl]amino}-5-{4-[(1,3-thiazol-4-ylcarbonyl)amino]phenyl}-2-thiophenecarboxylic acid;
3-([1-Methyl-2-(methyloxy)ethyl]{[*trans*-4-(trifluoromethyl)cyclohexyl]carbonyl}amino)-5-{4-[(1,3-thiazol-4-ylcarbonyl)amino]phenyl}-2-thiophenecarboxylic acid;
3-[[(*trans*-4-Methylcyclohexyl)carbonyl](1-methylethyl)amino]-5-{5-methyl-6-[(1,3-thiazol-4-ylcarbonyl)amino]-3-pyridinyl}-2-thiophenecarboxylic acid;
5-(6-{[(4-Fluorophenyl)carbonyl]amino}-5-methyl-3-pyridinyl)-3-[[(*trans*-4-methylcyclohexyl)carbonyl](1-methylethyl)amino]-2-thiophenecarboxylic acid;
3-{Cyclobutyl[(*trans*-4-methylcyclohexyl)carbonyl]amino}-5-{4-[(1*H*-pyrazol-3-ylcarbonyl)amino]phenyl}-2-thiophenecarboxylic acid;
3-{Ethyl[(*trans*-4-methylcyclohexyl)carbonyl]amino}-5-{4-[(1*H*-pyrazol-3-ylcarbonyl)amino]phenyl}-2-thiophenecarboxylic acid;
3-{[(*trans*-4-Methylcyclohexyl)carbonyl][2-(methyloxy)ethyl]amino}-5-{4-[(1,3-thiazol-4-ylcarbonyl)amino]phenyl}-2-thiophenecarboxylic acid;
3-{[(*trans*-4-Methylcyclohexyl)carbonyl][1-methyl-2-(methyloxy)ethyl]amino}-5-(4-{[(1-methyl-1*H*-imidazol-4-yl)carbonyl]amino}phenyl)-2-thiophenecarboxylic acid;
3-{[(*trans*-4-Methylcyclohexyl)carbonyl][2-(methyloxy)ethyl]amino}-5-(4-{[(1-methyl-1*H-*imidazol-4-yl)carbonyl]amino}phenyl)-2-thiophenecarboxylic acid;
3-[[(*trans*-4-Methylcyclohexyl)carbonyl](2,2,2-trifluoroethyl)amino]-5-{4-[(1,3-thiazol-4-ylcarbonyl)amino]phenyl}-2-thiophenecarboxylic acid;
3-{(Cyclopropylmethyl)[(*trans*-4-methylcyclohexyl)carbonyl]amino}-5-{4-[(1*H*-pyrazol-3-ylcarbonyl)amino]phenyl}-2-thiophenecarboxylic acid;
3-{Cyclobutyl[(*trans*-4-methylcyclohexyl)carbonyl]amino}-5-(4-{[(1-methyl-1*H*-imidazol-4-yl)carbonyl]amino}phenyl)-2-thiophenecarboxylic acid;
3-{Ethyl[(*trans*-4-methylcyclohexyl)carbonyl]amino}-5-(4-{[(1-methyl-1*H*-imidazol-4-yl)carbonyl]amino}phenyl)-2-thiophenecarboxylic acid;
3-[[(*trans*-4-Methylcyclohexyl)carbonyl](1-methylethyl)amino]-5-{4-methyl-5-[(1,3-thiazol-4-ylcarbonyl)amino]-2-pyridinyl}-2-thiophenecarboxylic acid;
3-{Cyclobutyl[(*trans*-4-methylcyclohexyl}carbonyl]amino}-5-{4-[(3-pyridazinylcarbonyl)amino]phenyl}-2-thiophenecarboxylic acid;
5-{5-Chloro-6-[(1,3-thiazol-4-ylcarbonyl)amino]-3-pyridinyl}-3-[[(*trans*-4-methylcyclohexyl)carbonyl](1-methylethyl)amino]-2-thiophenecarboxylic acid;
5-(4-{[(3,5-dimethyl-4-isoxazolyl)carbonyl]amino}phenyl)-3-[[(*trans*-4-methylcyclohexyl)carbonyl](1-methylethyl)amino]-2-thiophenecarboxylic acid;
5-(4-{[(1-ethyl-1*H*-pyrazol-5-yl)carbonyl]amino}phenyl)-3-[[(*trans*-4-methylcyclohexyl)carbonyl](1-methylethyl)amino]-2-thiophenecarboxylic acid;
3-[[(*trans*-4-methylcyclohexyl)carbonyl](1-methylethyl)amino]-5-(4-{[(1-methyl-1*H*-imidazol-5-yl)carbonyl]amino}phenyl)-2-thiophenecarboxylic acid;
3-[[(*trans*-4-methylcyclohexyl)carbonyl](1-methylethyl)amino]-5-(4-{[(3-methyl-4-pyridinyl)carbonyl]amino}phenyl)-2-thiophenecarboxylic acid;
3-[[(*trans*-4-methylcyclohexyl)carbonyl](1-methylethyl)amino]-5-{4-[(1,3-thiazol-5-ylcarbonyl)amino]phenyl}-2-thiophenecarboxylic acid;
3-[[(*trans*-4-methylcyclohexyl)carbonyl](1-methylethyl)amino]-5-(4-{[(1-methyl-1*H*-pyrazol-5-yl)carbonyl]amino}phenyl)-2-thiophenecarboxylic acid;
5-(4-{[(2,4-dimethyl-1,3-oxazol-5-yl)carbonyl]amino}phenyl)-3-[[(*trans*-4-methylcyclohexyl)carbonyl](1-methylethyl)amino]-2-thiophenecarboxylic acid;
5-(4-{[(1,3-dimethyl-1*H*-pyrazol-5-yl)carbonyl]amino}phenyl)-3-[[(*trans*-4-methylcyclohexyl)carbonyl](1-methylethyl)amino]-2-thiophenecarboxylic acid;
3-[[(*trans*-4-methylcyclohexyl)carbonyl](1-methylethyl)amino]-5-(4-{[(4-methyl-1,3-oxazol-5-yl)carbonyl]amino}phenyl)-2-thiophenecarboxylic acid;
5-(4-{[(1,5-dimethyl-1*H*-pyrazol-4-yl)carbonyl]amino}phenyl)-3-[[(*trans*-4-methylcyclohexyl)carbonyl](1-methylethyl)amino]-2-thiophenecarboxylic acid;
3-[[(*trans*-4-methylcyclohexyl)carbonyl](1-methylethyl)amino]-5-(4-{[(2-methyl-3-pyridinyl)carbonyl]amino}phenyl)-2-thiophenecarboxylic acid;
3-[[(*trans*-4-methylcyclohexyl)carbonyl](1-methylethyl)amino]-5-(4-{[(6-methyl-3-pyridinyl)carbonyl]amino}phenyl)-2-thiophenecarboxylic acid;
3-[[(*trans-*4-methylcyclohexyl)carbonyl](1-methylethyl)amino]-5-{4-[(1*H*-pyrazol-4-ylcarbonyl)amino]phenyl}-2-thiophenecarboxylic acid;
5'-[(2-furanylcarbonyl)amino]-4-[[(*trans*-4-methylcyclohexyl)carbonyl](1-methylethyl)amino]-2,2'-bithiophene-5-carboxylic acid;
3-[[(*trans*-4-methylcyclohexyl)carbonyl](1-methylethyl)amino]-5-(4-{[(5-methyl-4-isoxazolyl)carbonyl]amino}phenyl)-2-thiophenecarboxylic acid;
5-(4-{[(1-ethyl-1*H*-pyrazol-4-yl)carbonyl]amino}phenyl)-3-[[(*trans*-4-methylcyclohexyl)carbonyl](1-methylethyl)amino]-2-thiophenecarboxylic acid;
3-[[(*trans*-4-methylcyclohexyl)carbonyl](1-methylethyl)amino]-5-(4-{[(5-methyl-3-pyridinyl)carbonyl]amino}phenyl)-2-thiophenecarboxylic acid;
5'-{[(4-fluorophenyl)carbonyl]amino}-4-[[(*trans*-4-methylcyclohexyl)carbonyl](1-methylethyl)amino]-2,2'-bithiophene-5-carboxylic acid;
3-[[(*trans*-4-methylcyclohexyl)carbonyl](1-methylethyl)amino]-5-{5-[(1,3-thiazol-4-ylcarbonyl)amino]-2-pyridinyl}-2-thiophenecarboxylic acid;
5-(4-{[(1,3-dimethyl-1*H*-pyrazol-4-yl)carbonyl]amino}phenyl)-3-[[(*trans*-4-methylcyclohexyl)carbonyl](1-methylethyl)amino]-2-thiophenecarboxylic acid;
5-(4-{[(1-ethyl-1*H*-imidazol-5-yl)carbonyl]amino}phenyl)-3-[[(*trans*-4-methylcyclohexyl)carbonyl](1-methylethyl)amino]-2-thiophenecarboxylic acid;
3-[[(*trans*-4-methylcyclohexyl)carbonyl](1-methylethyl)amino]-5-(4-{[(3-methyl-1*H*-pyrazol-4-yl)carbonyl]amino}phenyl)-2-thiophenecarboxylic acid;
and salts, solvates and esters, and individual enantiomers thereof where appropriate.

In a further aspect, the present invention provides a compound selected from the group consisting of:
3-[[(*trans*-4-Methylcyclohexyl)carbonyl](1-methylethyl)amino]-5-{4-[(1,3-thiazol-4-ylcarbonyl)amino]phenyl}-2-thiophenecarboxylic acid.

In a further aspect, the present invention provides a compound selected from the group consisting of:
Sodium 3-[[(*trans*-4-methylcyclohexyl)carbonyl](1-methylethyl)amino]-5-{4-[(1,3-thiazol-4-ylcarbonyl)amino]phenyl}-2-thiophenecarboxylate;
Lysine 3-[[(*trans*-4-methylcyclohexyl)carbonyl](1-methylethyl)amino]-5-{4-[(1,3-thiazol-4-ylcarbonyl)amino]phenyl}-2-thiophenecarboxylate;
Ammonium 3-[[(*trans*-4-methylcyclohexyl)carbonyl](1-methylethyl)amino]-5-{4-[(1,3-thiazol-4-ylcarbonyl)amino]phenyl}-2-thiophenecarboxylate;
N-Methyl D glucamine 3-[[(*trans-*4-methylcyclohexyl)carbonyl](1-methylethyl)amino]-5-{4-[(1,3-thiazol-4-ylcarbonyl)amino]phenyl}-2-thiophenecarboxylate;
Potassium 3-[[(*trans*-4-methylcyclohexyl)carbonyl](1-methylethyl)amino]-5-{4-[(1,3-thiazol-4-ylcarbonyl)amino]phenyl}-2-thiophenecarboxylate;
Choline 3-[[(*trans*-4-methylcyclohexyl)carbonyl](1-methylethyl)amino]-5-{4-[(1,3-thiazol-4-ylcarbonyl)amino]phenyl}-2-thiophenecarboxylate;
L-Arginine 3-[[(*trans*-4-methylcyclohexyl)carbonyl](1-methylethyl)amino]-5-{4-[(1,3-thiazol-4-ylcarbonyl)amino]phenyl}-2-thiophenecarboxylate;
Magnesium 3-[[(*trans*-4-methylcyclohexyl)carbonyl](1-methylethyl)amino]-5-{4-[(1,3-thiazol-4-ylcarbonyl)amino]phenyl}-2-thiophenecarboxylate;
and solvates and esters, and individual enantiomers thereof where appropriate.

In one aspect, the present invention provides a compound selected from the group consisting of:
3-[[(*trans*-4-Methylcyclohexyl)carbonyl](1-methylethyl)amino]-5-{4-[(1,3-thiazol-4-ylcarbonyl)amino]phenyl}-2-thiophenecarboxylic acid;
3-{(Cyclopropylmethyl)[(*trans*-4-methylcyclohexyl)carbonyl]amino}-5-{4-[(1,3-thiazol-4-ylcarbonyl)amino]phenyl}-2-thiophenecarboxylic acid;
3-[[(*trans*-4-Methylcyclohexyl)carbonyl](1-methylethyl)amino]-5-{4-[(3-pyridazinylcarbonyl)amino]phenyl}-2-thiophenecarboxylic acid;
3-[[(*trans*-4-methylcyclohexyl)carbonyl](1-methylethyl)amino]-5-{4-[(1*H*-pyrazol-3-ylcarbonyl)amino]phenyl}-2-thiophenecarboxylic acid;
3-[[(*trans*-4-methylcyclohexyl)carbonyl](1-methylethyl)amino]-5-(4-{[(1-methyl-1*H*-imidazol-4-yl)carbonyl]amino}phenyl)-2-thiophenecarboxylic acid;
3-{ethyl[(*trans*-4-methylcyclohexyl)carbonyl]amino}-5-{4-[(1,3-thiazol-4-ylcarbonyl)amino]phenyl}-2-thiophenecarboxylic acid;
and salts, solvates and esters, and individual enantiomers thereof where appropriate.

The compounds of the present invention may be in the form of their free base or pharmaceutically acceptable salts, pharmaceutically acceptable solvates or pharmaceutically acceptable esters thereof.

Also included in the present invention are pharmaceutically acceptable salt complexes. The present invention also covers the pharmaceutically acceptable salts of the compounds of Formula (I). As used herein, the term "pharmaceutically acceptable salts" refers to salts that retain the desired biological activity of the subject compound and exhibit minimal undesired toxicological effects. For a review on suitable salts see Berge et al, J. Pharm. Sci., 1977, 66, 1-19. The term "pharmaceutically acceptable salts" includes both pharmaceutically acceptable acid addition salts and pharmaceutically acceptable base addition salts. These pharmaceutically acceptable salts may be prepared *in situ* during the final isolation and purification of the compound, or by separately reacting the purified compound in its free acid or free base form with a suitable base or acid, respectively. The salt may precipitate from solution and be collected by filtration or may be recovered by evaporation of the solvent.

Therefore, according to a further aspect, the invention provides a pharmaceutically acceptable salt of a compound of Formula (I) and embodiments thereof.

In certain embodiments, compounds of Formula I may contain an acidic functional group and may therefore be capable of forming pharmaceutically acceptable base addition salts by treatment with a suitable base. A pharmaceutically acceptable base addition salt may be formed by reaction of a compound of Formula I with a suitable strong base, optionally in a suitable solvent such as an organic solvent, to give the base addition salt which may be isolated for example by crystallisation and filtration. Pharmaceutically acceptable base salts include ammonium salts (for example ammonium or tetraalkylammonium), metal salts, for example alkali-metal or alkaline-earth-metal salts (such as hydroxides, sodium, potassium, calcium or magnesium), organic amines (such as tris [also known as tromethamine or tris(hydroxymethyl)aminomethane], ethanolamine, diethylamine, triethanolamine, choline, isopropylamine, dicyclohexylamine or N-methyl-D-glucamine), cationic amino acids (such as arginine, lysine or histidine) or bases for insoluble salts (such as procaine or benzathine).

In certain embodiments, compounds according to Formula I may contain a basic functional group and may therefore be capable of forming pharmaceutically acceptable acid addition salts by treatment with a suitable acid. A pharmaceutically acceptable acid addition salt may be formed by reaction of a compound of Formula I with a suitable strong inorganic acid (such as hydrobromic, hydrochloric, sulfuric, nitric, phosphoric or perchloric) or a suitable strong organic acid, for example, sulfonic acids [such as p-toluenesulfonic, benzenesulfonic, methanesulfonic, ethanesulfonic, 2-hydroxyethanesulfonic, naphthalenesulfonic (e.g. 2-naphthalenesulfonic)], carboxylic acids (such as acetic, propionic, fumaric, maleic, benzoic, salicylic or succinic), anionic amino acids (such as glutamaic or aspartic), hydroxyl acids (such as citric, lactic, tartaric or glycolic), fatty acids (such as caproic, caprylic, decanoic, oleic or stearic) or acids for insoluble salts (such as pamoic or resinic [e.g. polystyrene sulfonate]), optionally in a suitable solvent such as an organic solvent, to give the salt which is usually isolated for example by crystallisation and filtration. In one embodiment, a pharmaceutically acceptable acid addition salt of a compound of Formula I is a salt of a strong acid, for example a hydrobromide, hydrochloride, hydroiodide, sulfate, nitrate, perchlorate, phosphate p-toluenesulfonic, benzenesulfonic or methanesulfonic salt.

In one aspect of the invention, there are provided suitable pharmaceutically acceptable salts of the compounds of Formula (I) including acid salts, for example sodium, potassium, calcium, magnesium, ammonium, tetraalkylammonium and tris (tromethamine - tris(hydroxymethyl)aminomethane) salts and the like, or mono- or di- basic salts with the appropriate acid for example organic carboxylic acids such as acetic, lactic, tartaric, malic, isethionic, lactobionic and succinic acids; organic sulfonic acids such as methanesulfonic, ethanesulfonic, benzenesulfonic and p-toluenesulfonic acids and inorganic acids such as hydrochloric, sulfuric, phosphoric and sulfamic acids and the like.

In one aspect of the invention, there is provided a pharmaceutically acceptable base addition salt of a compound of Formula (I) which is a salt of a strong base, for example, sodium, lysine, ammonium, N-methyl-D-glucamine, potassium, choline, arginine (for example L-arginine) or magnesium. In a further aspect, the salt is sodium, lysine, ammonium, N-methyl-D-glucamine, potassium, choline or arginine (for example L-arginine). In a further aspect, the salt is an ammonium salt or a lysine salt. In a further aspect, the salt is a lysine salt.

Other non-pharmaceutically acceptable salts, for example oxalates, may be used, for example in the isolation of compounds of Formula (I), and are included within the scope of this invention.

The invention includes within its scope all possible stoichiometric and non-stoichiometric forms of the salts of the compounds of Formula (I).

The salts of a compound of Formula (I) may be prepared by contacting appropriate stoichiometric amounts of the free acid with the appropriate base in a suitable solvent. The free acid of a compound of Formula (I) may for example be in solution with the appropriate base added as a solid or both the free acid of a compound of Formula (I) and the appropriate acid may independently be in solution.

Suitable solvents for solubilising a compound of Formula (I) free acid include for example alcohols such as isopropanol; ketones such as acetone; acetonitrile or toluene. If the base is to be added as a solution in a solvent, the solvent used may include acetone, methanol or water.

The salts of a compound of Formula (I) may be isolated in solid form by conventional means from a solution thereof obtained as above. For example, a non-crystalline salt may be prepared by precipitation from solution, spray drying or freeze drying of solutions, evaporating a solution to a glass, or vacuum drying of oils, or solidification of melts obtained from reaction of the free base and the acid.

The salts of a compound of Formula (I) may be prepared by directly crystallising from a solvent in which the salt has limited solubility, or by triturating or otherwise crystallising a non-crystalline salt. For example, organic solvents such as acetone, acetonitrile, butanone, 1-butanol, ethanol, 1-propanol or tetrahydrofuran or mixtures of such solvents may be used. An improved yield of the salts may be obtained by the evaporation of some or all of the solvent or by crystallisation at elevated temperature followed by controlled cooling, for example in stages. Careful control of the precipitation temperature and seeding may be used to improve the reproducibility of the production process and the particle size distribution and form of the product.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of Formula (I) are within the scope of the invention. Therefore, the present invention also relates to solvates of the compounds of Formula (I), for example hydrates.

Salts and solvates of compounds of Formula (I) which are suitable for use in medicine are those wherein the counterion or associated solvent is pharmaceutically acceptable. However, salts and solvates having non-pharmaceutically acceptable counterions or associated solvents are within the scope of the present invention, for example, for use as intermediates in the preparation of other compounds of the invention and their pharmaceutically acceptable salts and solvates.

It will be appreciated by those skilled in the art that certain protected derivatives of compounds of Formula (I), which may be made prior to a final deprotection stage, may not possess pharmacological activity as such, but may, in certain instances, be administered orally or parenterally and thereafter metabolised in the body to form compounds defined in the first aspect which are pharmacologically active. Such derivatives may therefore be described as "prodrugs". All protected derivatives and prodrugs of compounds defined in the first aspect are included within the scope of the invention. Examples of suitable pro-drugs for the compounds of the present invention are described in Drugs of Today, Volume 19, Number 9, 1983, pp 499 - 538 and in Topics in Chemistry, Chapter 31, pp 306 - 316 and in "Design of Prodrugs" by H. Bundgaard, Elsevier, 1985, Chapter 1 (the disclosures in which documents are incorporated herein by reference). It will further be appreciated by those skilled in the art, that certain moieties, known to those skilled in the art as "pro-moieties", for example as described by H. Bundgaard in "Design of Prodrugs" (the disclosure in which document is incorporated herein by reference) may be placed on appropriate functionalities when such functionalities are present within the compounds of Formula (I). Suitable prodrugs for compounds of the invention include : esters, carbonate esters, hemi-esters, phosphate esters, nitro esters, sulfate esters, sulfoxides, amides, carbamates, azo-compounds, phosphamides, glycosides, ethers, acetals and ketals.

The present invention also relates to pharmaceutically acceptable esters of the compounds of Formula (I), for example carboxylic acid esters -COOR, in which R is selected from straight or branched chain alkyl, for example n-propyl, n-butyl, alkoxyalkyl (e.g. methoxymethyl), aralkyl (e.g. benzyl), aryloxyalkyl (e.g. phenoxymethyl), aryl (e.g. phenyl optionally substituted by halogen, -C₁₋₄alkyl or -C₁₋₄alkoxy or amino); or for example - CH₂OC(O)R' or -CH₂OCO₂R' in which R' is alkyl (e.g. R' is *t*-butyl). Unless otherwise specified, any alkyl moiety present in such esters suitably contains 1 to 18 carbon atoms, particularly 1 to 4 carbon atoms. Any aryl moiety present in such esters suitably comprises a phenyl group.

In one aspect, the invention provides a pharmaceutically acceptable salt, solvate or prodrug of a compound of Formula (I).

In one aspect, the invention provides a compound of Formula (I) in the form of parent compound, a salt or a solvate.

Furthermore, some of the crystalline forms of the compounds of Formula (I) or salts and solvates thereof may exist in one or more polymorphic form, which are included in the present invention.

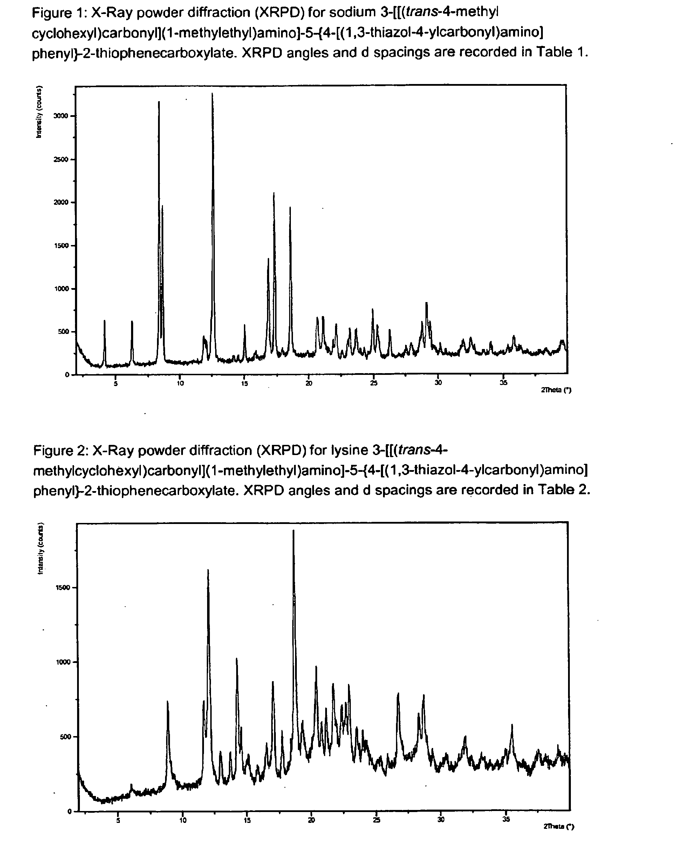

The present invention also provides sodium 3-[[(*trans*-4-methylcyclohexyl)carbonyl](1-methylethyl)amino]-5-{4-[(1,3-thiazol-4-ylcarbonyl)amino]phenyl}-2-thiophenecarboxylate characterised in that it provides an XRPD pattern substantially as illustrated in Figure 1.

The present invention further provides sodium 3-[[(*trans*-4-methylcyclohexyl)carbonyl)(1-methylethyl)amino]-5-{4-[(1,3-thiazol-4-ylcarbonyl)amino]phenyl}-2-thiophenecarboxylate characterised in that it provides an XRPD pattern with signals substantially as listed in Table 1.

The present invention also provides lysine 3-[[(*trans*-4-methylcyclohexyl)carbonyl](1-methylethyl)amino]-5-{4-[(1,3-thiazol-4-ylcarbonyl)amino]phenyl}-2-thiophenecarboxylate characterised in that it provides an XRPD pattern substantially as illustrated in Figure 2

The present invention further provides lysine 3-[[(*trans*-4-methylcyclohexyl)carbonyl](1-methylethyl)amino]-5-{4-[(1,3-thiazol-4-ylcarbonyl)amino]phenyl}-2-thiophenecarboxylate characterised in that it provides an XRPD pattern with signals substantially as listed in Table 2.

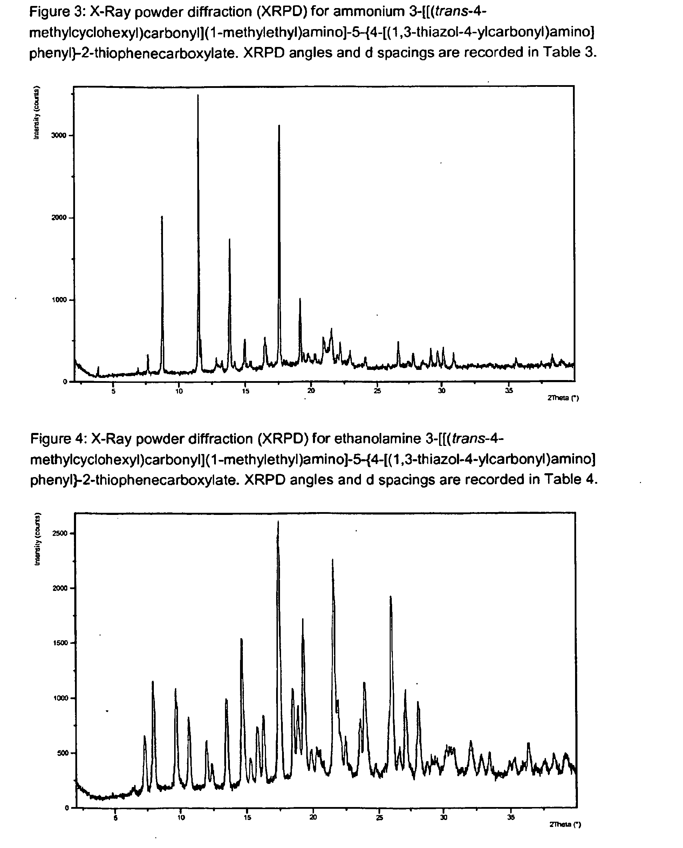

The present invention also provides ammonium 3-[[(*trans*-4-methylcyclohexyl)carbonyl](1-methylethyl)amino]-5-{4-[(1,3-thiazol-4-ylcarbonyl)amino]phenyl}-2-thiophenecarboxylate characterised in that it provides an XRPD pattern substantially as illustrated in Figure 3.

The present invention further provides ammonium 3-[[(*trans*-4-methylcyclohexyl)carbonyl](1-methylethyl)amino]-5-{4-[(1,3-thiazol-4-ylcarbonyl)amino]phenyl}-2-thiophenecarboxylate characterised in that it provides an XRPD pattern with signals substantially as listed in Table 3.

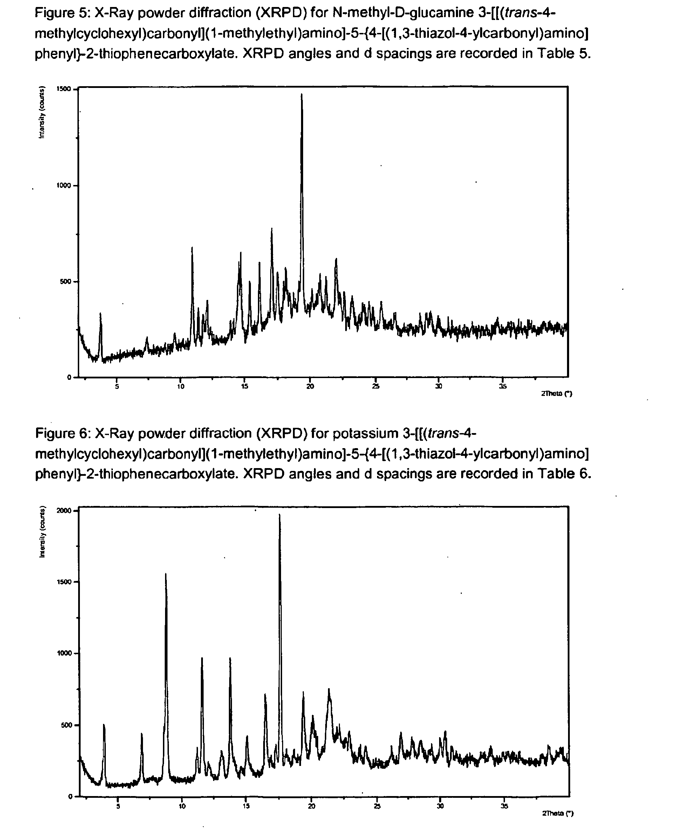

The present invention also provides N-methyl-D-glucamine 3-[[(trans-4-methylcyclohexyl)carbonyl](1-methylethyl)amino]-5-{4-[(1,3-thiazol-4-ylcarbonyl)amino]phenyl}-2-thiophenecarboxylate characterised in that it provides an XRPD pattern substantially as illustrated in Figure 5.

The present invention further provides N-methyl-D-glucamine 3-[[(*trans*-4-methylcyclohexyl)carbonyl](1-methylethyl)amino]-5-{4-[(1,3-thiazol-4-ylcarbonyl)amino]phenyl}-2-thiophenecarboxylate characterised in that it provides an XRPD pattern with signals substantially as listed in Table 5.

The present invention also provides potassium 3-[[(*trans*-4-methylcyclohexyl)carbonyl](1-methylethyl)amino]-5-{4-[(1,3-thiazol-4-ylcarbonyl)amino]phenyl}-2-thiophenecarboxylate characterised in that it provides an XRPD pattern substantially as illustrated in Figure 6.

The present invention further provides potassium 3-[[(*trans*-4-methylcyclohexyl)carbonyl](1-methylethyl)amino]-5-{4-[(1,3-thiazol-4-ylcarbonyl)amino]phenyl}-2-thiophenecarboxylate characterised in that it provides an XRPD pattern with signals substantially as listed in Table 6.

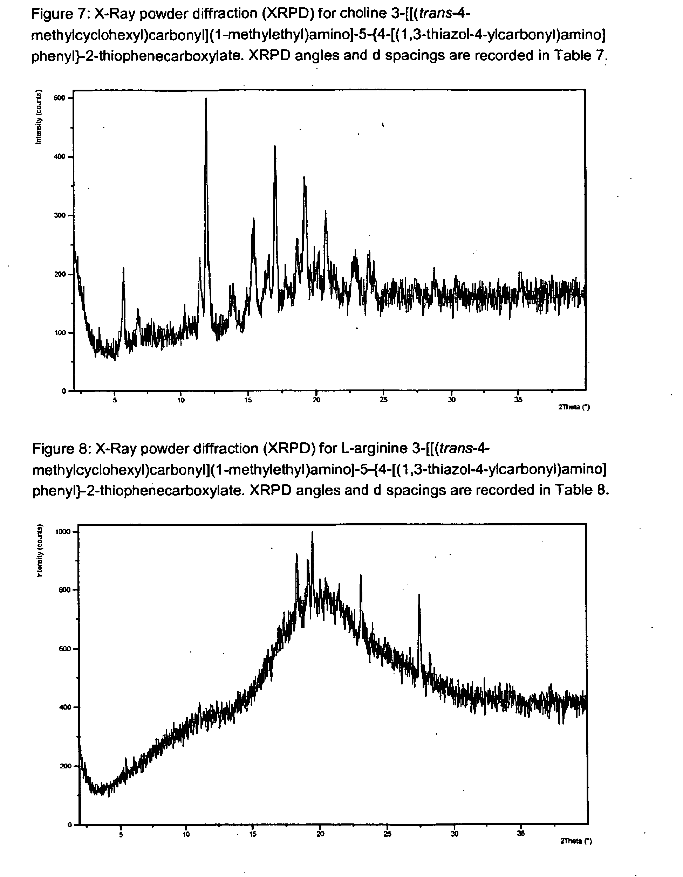

The present invention also provides choline 3-[[(*trans-*4-methylcyclohexyl)carbonyl](1-methylethyl)amino]-5-{4-[(1,3-thiazol-4-ylcarbonyl)amino]phenyl}-2-thiophenecarboxylate characterised in that it provides an XRPD pattern substantially as illustrated in Figure 7.

The present invention further provides choline 3-[[(*trans*-4-methylcyclohexyl)carbonyl](1-methylethyl)amino]-5-{4-[(1,3-thiazol-4-ylcarbonyl)amino]phenyl}-2-thiophenecarboxylate characterised in that it provides an XRPD pattern with signals substantially as listed in Table 7.

The present invention also provides L-arginine 3-[[(*trans*-4-methylcyclohexyl)carbonyl](1-methylethyl)amino]-5-{4-[(1,3-thiazol-4-ylcarbonyl)amino]phenyl}-2-thiophenecarboxylate characterised in that it provides an XRPD pattern substantially as illustrated in Figure 8.

The present invention further provides L-arginine 3-[[(*trans*-4-methylcyclohexyl)carbonyl](1-methylethyl)amino]-5-{4-[(1,3-thiazol-4-ylcarbonyl)amino]phenyl}-2-thiophenecarboxylate, characterised in that it provides an XRPD pattern with signals substantially as listed in Table 8.

It will be appreciated that the compounds of Formula (I) may contain one or more asymmetric carbon atoms and may exist in racemic, diastereoisomeric, and optically active forms. All of these racemic compounds, enantiomers and diastereoisomers are contemplated to be within the scope of the present invention. Racemic compounds may either be separated using preparative HPLC and a column with a chiral stationary phase or resolved to yield individual enantiomers utilising methods known to those skilled in the art. In addition, chiral intermediate compounds may be resolved and used to prepare chiral compounds of the invention.

The compounds of Formula (I) may exist in different tautomeric forms, i.e. one or more tautomeric forms. All tautomers, and mixtures thereof, are contemplated to be within the scope of the present invention. For example, a claim to 2-hydroxyquinolinyl would also cover its tautomeric form, α-quinolinonyl.

Diastereoisomers of compounds of Formula (I) may be obtained according to methods well known in the literature, for example by preparative HPLC or by chromatographic purifications. Racemic compounds may either be separated using preparative HPLC and a column with a chiral stationary phase or resolved to yield individual enantiomers utilising methods known to those skilled in the art. In addition, chiral intermediate compounds may be resolved and used to prepare chiral compounds of the invention.

As used herein, the term "pharmaceutically acceptable" used in relation to an ingredient (active ingredient such as an active ingredient, a salt thereof or an excipient) which may be included in a pharmaceutical formulation for administration to a patient, refers to that ingredient being acceptable in the sense of being compatible with any other ingredients present in the pharmaceutical formulation and not being deleterious to the recipient thereof.

The compounds of Formula (I) exhibit good potency against the replication of HCV (1a and 1b genotypes), and therefore have the potential to achieve greater efficacy in man. High potency in both genotypes is considered to be advantageous.

There is provided as a further aspect of the present invention a compound of Formula (I) or pharmaceutically acceptable salts, solvates or esters thereof for use in human or veterinary medical therapy, particularly in the treatment or prophylaxis of viral infection, particularly flavivirus infection, for example HCV infection.

It will be appreciated that reference herein to therapy and/or treatment includes, but is not limited to prevention, retardation, prophylaxis, therapy and cure of the disease. It will further be appreciated that references herein to treatment or prophylaxis of HCV infection include treatment or prophylaxis of HCV-associated disease such as liver fibrosis, cirrhosis and hepatocellular carcinoma.

In a further or alternative aspect, there is provided a method for the treatment of a human or animal subject with viral infection, particularly HCV infection, which method comprises administering to said human or animal subject an effective amount of a compound of Formula (I) or pharmaceutically acceptable salts, solvates or esters thereof.

According to another aspect of the invention, there is provided the use of a compound of Formula (I) or pharmaceutically acceptable salts, solvates or esters thereof in the manufacture of a medicament for the treatment and/or prophylaxis of viral infection, particularly HCV infection.

### PROCESSES

The compounds of Formula (I) or salts, solvates or esters thereof may be prepared by the processes described hereinafter, said processes constituting a further aspect of the invention.

Compounds of Formula (I) in which A is hydroxy and R¹, R² and R³ are as defined above for Formula (I), may be prepared from a compound of Formula (II) 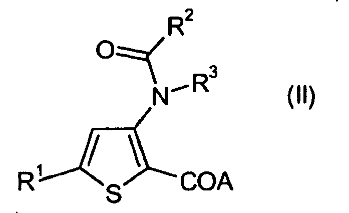 in which A is a protected hydroxy group, for example an alkoxy, benzyloxy or silyloxy group and R¹, R², and R³ are as defined above for Formula (I). For example when A is methoxy or ethoxy, and R¹, R² and R³ are as defined above for Formula (I), by treatment with an appropriate base, for example aqueous sodium hydroxide or lithium hydroxide, optionally in a solvent such as methanol, ethanol, tetrahydrofuran or combinations thereof. Suitably, the temperature is in the range 25 to 100°C, for example 25 to 50°C. Alternatively, when A is methoxy or ethoxy and R¹, R² and R³ are as defined above for Formula (I), by treatment with lithium iodide in a suitable solvent such as pyridine, lutidine or collidine, suitably in the temperature range 100-170°C.
For example when A is *tert*-butoxy, and R¹, R² and R³ are as defined above for Formula (I), by treatment with an appropriate acid, for example trifluoroacetic acid. Suitably, the reaction is carried out in a solvent, for example dichloromethane. Suitably, the temperature is in the range 0 to 50°C, for example 15 to 30°C.
For example when A is silyloxy, and R¹, R² and R³ are as defined above for Formula (I), by treatment with a suitable fluoride source for example tetrabutylammonium fluoride. The reaction is carried out in a suitable solvent, for example tetrahydrofuran. Suitably, the temperature is in the range 0 to 50°C, for example 15 to 30°C.

Compounds of Formula (I) in which A is hydroxy, or (II) in which A is an alkoxy, benzyloxy or silyloxy group and R¹, R² and R³ are as defined above for Formula (I), may be prepared by reaction of a compound of Formula (III) 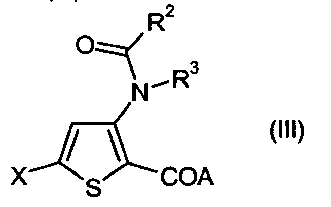 in which A is hydroxy or an alkoxy, benzyloxy or silyloxy group, and R² and R³ are as defined above for Formula (I) and X is a halogen such as bromide or iodide; with a suitable boronic acid R¹-B(OH)₂ or boronate ester R¹-B(OR')(OR"), in which R¹ is as defined above for Formula (I) and R' and R" are independently alkyl or R' and R" together with the oxygen atoms to which they are attached form a ring optionally substituted by alkyl, such as a pinacol ester, in the presence of a palladium catalyst such as tetrakistriphenyl phosphine palladium(0) or bis-[(diphenylphosphino)-ferrocene]-palladium(II) chloride, in the presence of a suitable base such as sodium carbonate, in a suitable solvent such as DMF, 1,4-dioxane or dimethyoxyethane, or combinations thereof, optionally in the presence of water, at a temperature in the range 50-100°C, optionally under an inert atmosphere.

Compounds of Formula (I) in which A is hydroxy, or (II) in which A is an alkoxy, benzyloxy or silyloxy group and R¹, R² and R³ are as defined above for Formula (I), may be prepared by reaction of a compound of Formula (III)' 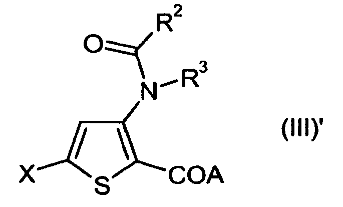 in which A is hydroxy or an alkoxy, benzyloxy or silyloxy group, and R² and R³ are as defined above for Formula (I) and X is a suitable boronic acid -B(OH)₂ or boronate ester -B(OR')(OR"), in which R' and R" are independently C₁₋₆alkyl or R' and R" together with the oxygen atoms to which they are attached form a ring optionally substituted by alkyl, such as a pinacol ester, with R¹-Hal wherein R¹ is as defined above for Formula (I) and Hal is a halogen such as bromide or iodide, in the presence of a palladium catalyst such as tetrakistriphenyl phosphine palladium(0) or bis-[(diphenylphosphino)-ferrocene]-palladium(II) chloride, in the presence of a suitable base such as cesium fluoride or sodium carbonate, in a suitable solvent such as DME, DMF, 1,4-dioxane or dimethyoxyethane, or combinations thereof, optionally in the presence of water, at a temperature in the range 50-100°C, optionally under an inert atmosphere.

Compounds of Formula (III) in which A is an alkoxy, benzyloxy or silyloxy may be prepared from compounds of Formula (IV) 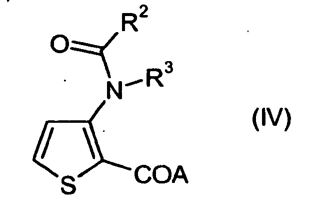 in which A is an alkoxy, benzyloxy or silyloxy, and R² and R³ are as defined above for Formula (I), by treatment with a suitable base such as lithium diisopropylamide and a halogen source such as iodine in a suitable solvent such as tetrahydrofuran, and at a temperature in the range -78°C to -20°C.

Compounds of Formula (III)' in which A is an alkoxy, benzyloxy or silyloxy group may be prepared from compounds of Formula (IV) 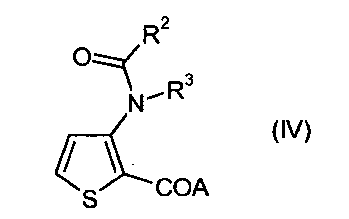 in which A is an alkoxy, benzyloxy or silyloxy, and R² and R³ are as defined above for Formula (I), by treatment with a suitable base such as lithium diisopropylamide and a boronate such as B(OR)₃ wherein R is an C₁₋₆alkyl group, for example methyl, in a suitable solvent such as tetrahydrofuran, and at a temperature in the range -78°C to -20°C.

Compounds of Formula (III) in which A is hydroxy may be prepared from compounds of Formula (III) in which A is an alkoxy, benyloxy or silyloxy group, for example by treatment with an appropriate base, acid or fluoride source as described in relation to the preparation of compounds of Formula (I) from compounds of Formula (II).

Compounds of Formula (IV) in which A is an alkoxy, benzyloxy or silyloxy group and R³ is as defined above may be prepared by reaction of a compound of Formula (V) 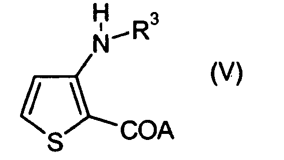 in which A an alkoxy, benzyloxy or silyloxy group, and R³ is as defined above for Formula (I); with a suitable acylating agent, for example R²-C(O)-Y, wherein Y is a halo atom, for example chloro or bromo, and R² is as defined above for Formula (I). The reaction may be carried out in a suitable solvent, for example dichloromethane, and optionally in the presence of a suitable base, for example pyridine or triethylamine. A phosphine such as triphenylphosphine may optionally be used in place of the base.

Compounds of Formula (V) in which A is an alkoxy, benzyloxy or silyloxy group may be prepared by reaction of a compound of Formula (VI) 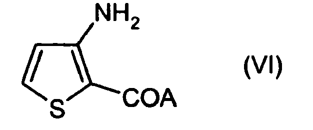 in which A an alkoxy, benzyloxy or silyloxy group, by treatment with a suitable vinyl ether, or a suitable aldehyde or a suitable ketone in the presence of a suitable acid, such as acetic acid, or alternatively a Lewis acid, for example TiCl₄, and a suitable reducing agent such as sodium triacetoxyborohydride, in a suitable solvent such as dichloromethane. Alternatively, compounds of Formula (V) in which A is an alkoxy, benzyloxy or silyloxy group may be prepared from compounds of Formula (VI) in which A is an alkoxy, benzyloxy or silyloxy are as defined above for Formula (I), by treatment with a suitable alkylating agent R³-X' where R³ is as defined above for Formula (I) and X' is a halo group such as chloride, bromide or iodide, or X' is a sulphonate ester such as methanesulfonate, in suitable solvent such as dimethylformamide in the presence of a suitable base such as triethylamine.

Compounds of Formula (V) may also be prepared by reacting a compound of Formula (VII) 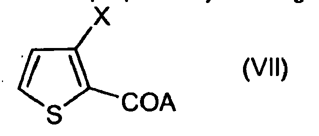 in which A is an alkoxy, benzyloxy or silyloxy group and X is a halo group such as bromo, with an amine R³NH₂ where R³ is as defined above for Formula (I), in the presence of a palladium catalyst such as tris(dibenzylidenacetone)dipalladium in the presence of a reagent such as 2,2'-bis(diphenylphosphino)-1,1'-binaphthyl (BINAP) and a base such as cesium carbonate, in a suitable solvent such as toluene and in a suitable temperature range such as 80-120°C.

Compounds of Formula (VI) and (VII) are commercially available or well known in the art.

Compounds of Formula (IV) in which A is an alkoxy, benzyloxy or silyloxy group and R² and R³ are as defined above for Formula (I), may also be prepared by reaction of a compound of Formula (VIII) 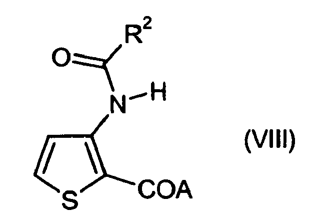 in which A an alkoxy, benzyloxy or silyloxy group, and R² is as defined above for Formula (I); with a suitable alkylating agent R³-X' in which R³ is as defined above for Formula (I) and X' is a halo atom such as chloro, bromo or iodo, or X' is a sulphonate ester such as methanesulfonate, in a suitable solvent such as dimethylformamide, in the presence of a suitable base such as sodium hydride optionally in the presence of triethylamine.

Compounds of Formula (VIII) in which A is an alkoxy, benzyloxy or silyloxy group may be prepared by reaction of a compound of Formula (VI) in which A an alkoxy, benzyloxy or silyloxy group, with a suitable acylating agent, for example R²-C(O)-Y, wherein Y is a halo atom, for example chloro or bromo, and R² is as defined above for Formula (I). The reaction may be carried out in a suitable solvent, for example dichloromethane, optionally in the presence of a suitable base, for example pyridine or triethylamine. A phosphine such as triphenylphosphine may optionally be used in place of the base.

Compounds of Formula (II) in which A is an alkoxy, benzyloxy or silyloxy group may also be prepared by reaction of a compound of Formula (IX) 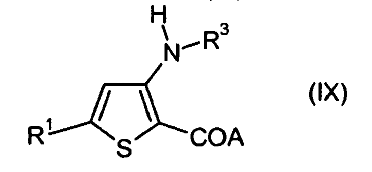 in which A an alkoxy, benzyloxy or silyloxy group, and R¹ and R³ are as defined above for Formula (I) with a suitable acylating agent, for example R²-C(O)-Y, wherein Y is a halo atom, for example chloro or bromo, and R² is as defined above for Formula (I). The reaction may be carried out in a suitable solvent, for example dichloromethane, in the presence of a suitable base, for example pyridine or triethylamine and thereafter removing any protecting group if desired. A phosphine such as triphenylphosphine may optionally be used in place of the base.

Compounds of Formula (IX) in which A is an alkoxy, benzyloxy or silyloxy group and R¹ and R³ are as defined above for Formula (I), may also be prepared by reaction of a compound of Formula (X) 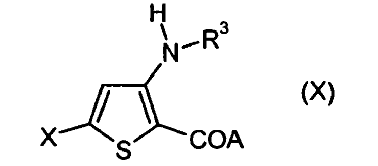 in which A is hydroxy or an alkoxy, benzyloxy or silyloxy group and X is a halogen such as ' bromide or iodide, with a suitable boronic acid R¹-B(OH)₂ or boronate ester R¹-B(OR')₂ wherein R³ is as defined above for Formula (I), in the presence of a palladium catalyst such as tetrakistriphenyl phosphine palladium(0) or bis-[(diphenylphosphino)-ferrocene]-palladium(II) chloride, in the presence of a suitable base such as sodium carbonate, in a suitable solvent such as DMF, methanol or toluene, or combinations thereof, at a temperature in the range 50-100°C, optionally under an inert atmosphere.

Compounds of Formula (IX) in which A is an alkoxy, benzyloxy or silyloxy group and R¹ and R³ are as defined above for Formula (I), may also be prepared by reaction of a compound of Formula (X) in which A is hydroxy or an alkoxy, benzyloxy or silyloxy group, and X is a suitable boronic acid -B(OH)₂ or boronate ester -B(OR')(OR"), in which R' and R" are independently C₁₋₆alkyl or R' and R" together with the oxygen atoms to which they are attached form a ring optionally substituted by alkyl, such as a pinacol ester, and R³ is as defined above for Formula (I), with R¹-Hal wherein R¹ is as defined above for Formula (I) and Hal is a halogen such as bromide or iodide, in the presence of a palladium catalyst such as tetrakistriphenyl phosphine palladium(0) or bis-[(diphenylphosphino)-ferrocene]-palladium(II) chloride, in the presence of a suitable base such as cesium fluoride or sodium carbonate, in a suitable solvent such as DME, DMF, 1,4-dioxane or dimethyoxyethane, or combinations thereof, optionally in the presence of water, at a temperature in the range 50-100°C, optionally under an inert atmosphere.

Compounds of Formula (X) in which A is an alkoxy, benzyloxy or silyloxy group and R³ is as defined above for Formula (I) and X is a halogen such as bromide or iodide, may be prepared by reaction of a compound of Formula (XI) 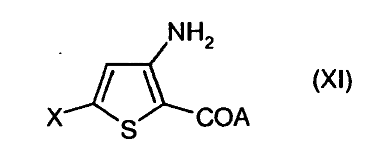 wherein X is a halogen such as bromide or iodide, by treatment with a suitable vinyl ether, or a suitable aldehyde or a suitable ketone in the presence of a suitable acid, such as acetic acid, and a suitable reducing agent such as sodium triacetoxyborohydride, in a suitable solvent such as dichloromethane. Alternatively, compounds of Formula (X) in which A is an alkoxy, benzyloxy or silyloxy group may be prepared from compounds of Formula (XI) in which A is an alkoxy, benzyloxy or silyloxy, by treatment with a suitable alkylating agent R³-X' where R³ is as defined above for Formula (I) and X' is a halo group such as chloride, bromide or iodide, or X' is a sulphonate ester such as methanesulfonate, in suitable solvent such as dimethylformamide in the presence of a suitable base such as triethylamine.

Compounds of Formula (X), in which A is an alkoxy, benzyloxy or silyloxy group, R³ is as defined above and X is a suitable halogen such as bromide or iodide, may also be prepared by reaction of a compound of Formula (V) in which A an alkoxy, benzyloxy or silyloxy group, and R³ is as defined above for Formula (I), by treatment with a suitable base such as lithium diisopropylamide and a halogen source such as iodine in a suitable solvent such as tetrahydrofuran, heptane, ethylbenzene or mixtures thereof and at a temperature in the range -78°C to -20°C.

Compounds of Formula (X) in which A is an alkoxy, benzyloxy or silyloxy group, R³ is as defined above for Formula (I) and X is a halogen such as bromide or iodide, may also be prepared by reaction of a compound of Formula (XII) 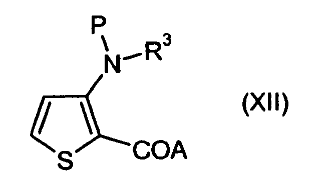 wherein P is -COCF₃ or -CO₂^{t}Bu and R³ is as defined above for Formula (I), by treatment with a halogen source, for example iodine, in a suitable solvent such as THF, heptane, ethylbenzene, or combinations thereof, in the presence of a suitable base such as LDA, at a suitable temperature for example -78 to -20°C, optionally in an inert atmosphere, and thereafter removing the protecting group P, for example with hydrochloric acid when P is CO₂^{t}Bu or with aqueous sodium carbonate solution when P is COCF₃.

Compounds of Formula (XII) in which P is -COCF₃ and R³ is as defined above for Formula (I), may be prepared from a compound of Formula (V) in which A an alkoxy, benzyloxy or silyloxy group, and R³ is as defined above for Formula (I), by treatment with trifluoroacetic anhydride in a suitable solvent such as diethylether.

Compounds of Formula (XII) in which P is CO₂^{t}Bu and R³ is as defined above for Formula (I), may be prepared from a compound of Formula (V), by treatment with di-tert-butyl dicarbonate in a suitable solvent such as ether, acetonitrile or acetone, optionally in the presence of a catalyst such as DMAP and a base such as triethylamine.

Compounds of Formula (XI), in which A is an alkoxy, benzyloxy or silyloxy group and X is a halogen such as iodide, may be prepared from a compound of Formula (XIII) 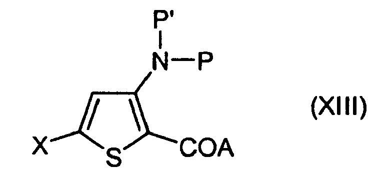 in which P is a suitable protecting group such as -COCF₃ and P' is hydrogen, or P is -CO₂^{t}Bu and P' is hydrogen or -CO₂^{t}Bu. For example, when P is -COCF₃ and P' is hydrogen, by treatment with a suitable base such as aqueous potassium carbonate optionally in the presence of an alcohol such as methanol, or when P is -CO₂^{t}Bu and P' is hydrogen or - CO₂^{t}Bu, by treatment with a suitable acid such as hydrochloric acid or trifluoroacetic acid in a suitable solvent such as 1,4-dioxane or dichloromethane.

Compounds of Formula (XIII), in which A is an alkoxy, benzyloxy or silyloxy group, X is a halo atom such as iodide and P is a suitable protecting group such as -COCF₃ and P' is hydrogen, or P is -CO₂^{t}Bu and P' is hydrogen or -CO₂^{t}Bu, may be prepared by reaction of a compound of Formula (XIV) 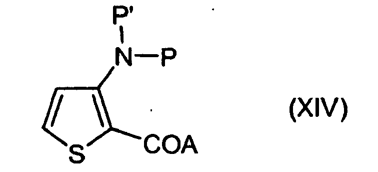 in which A is an alkoxy, benzyloxy or silyloxy group, P is a suitable protecting group such as -COCF₃ and P' is hydrogen, or P is -CO₂^{t}Bu and P' is hydrogen or -CO₂^{t}Bu, with a suitable base such as lithium diisopropylamide and a halogen source such as iodine, in a suitable solvent such as tetrahydrofuran, and at a temperature in the range -78°C to -20°C.

Compounds of Formula (XIV), in which A is an alkoxy, benzyloxy or silyloxy group and P and P' are as described above for Formula (XIII), may be prepared by treating compounds of Formula (VI) with trifluoroacetic anhydride or di-tert-butyl dicarbonate in a suitable solvent such as ether, acetonitrile or acetone, optionally in the presence of a catalyst such as DMAP and a base such as triethylamine.

Compounds of Formula (X), in which A is an alkoxy, benzyloxy or silyloxy group, R³ is -CF₃, - CF₂H or -CH₂F and X is a halogen such as bromide or iodide, may also be prepared by reaction of a compound of Formula (XV) 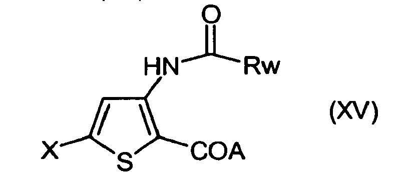 in which A is an alkoxy, benzyloxy or silyloxy group, X is a halogen such as bromide or iodide and Rw is -CF₃, -CF₂H or -CH₂F, by treatment with a reducing agent such as sodium borohydride in the presence of an acid such as acetic acid, in a suitable solvent such as dioxane and at a temperature in the range 0-25°C.

Compounds of Formula (XV) in which A is an alkoxy, benzyloxy or silyloxy group, X is a halogen such as bromide or iodide and Rw is -CF₃, -CF₂H or -CH₂F, may be prepared by reaction of a compound of Formula (XI) wherein X is a halogen such as bromide or iodide, with a suitable acylating agent, for example Rw-C(O)-Y, wherein Y is a halo atom, for example chloro or bromo, and Rw is CF₃, -CF₂H or -CH₂F. The reaction may be carried out in a suitable solvent, for example dichloromethane, optionally in the presence of a suitable base, for example pyridine or triethylamine. A phosphine such as triphenylphosphine may optionally be used in place of the base.

Compounds of Formula (V), in which A is an alkoxy, benzyloxy or silyloxy group, R³ is -CF₃, -CF₂H or -CH₂F and X is a halogen such as bromide or iodide, may also be prepared by reaction of a compound of Formula (XVI) 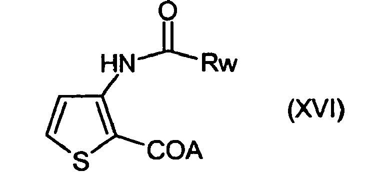 in which A is an alkoxy, benzyloxy or silyloxy group, X is a halogen such as bromide or iodide and Rw is -CF₃, -CF₂H or -CH₂F, by treatment with a reducing agent such as sodium borohydride in the presence of an acid such as acetic acid, in a suitable solvent such as dioxane and at a temperature in the range 0-25°C.

Compounds of Formula (XVI) in which A is an alkoxy, benzyloxy or silyloxy group and Rw is - CF₃, -CF₂H or -CH₂F, may be prepared by reaction of a compound of Formula (VI), with a suitable acylating agent, for example Rw-C(O)-Y, wherein Y is a halo atom, for example chloro or bromo, and Rw is CF₃, -CF₂H or -CH₂F. The reaction may be carried out in a suitable solvent, for example dichloromethane, optionally in the presence of a suitable base, for example pyridine or triethylamine. A phosphine such as triphenylphosphine may optionally be used in place of the base.

Compounds of Formula (II), in which A is an alkoxy, benzyloxy or silyloxy group and R¹, R² and R³ are as defined above for Formula (I), may also be prepared from compounds of Formula (XVII) 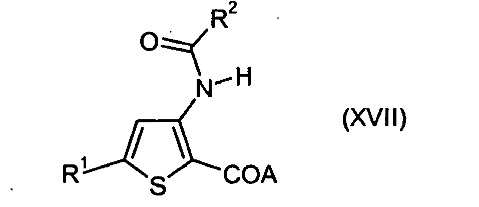 in which A is an alkoxy, benzyloxy or silyloxy, and R¹ and R² are as defined above for Formula (I), by treatment with a suitable alkylating agent R³-X' where X' is a halo group such as chloride, bromide or iodide, or X' is a sulphonate ester such as methanesulfonate or trifluoromethylsulphonate, and R³ is as defined above for Formula (I), in a suitable solvent such as dimethylformamide in the presence of a suitable base such as triethylamine or sodium hydride or combinations thereof.

Compounds of Formula (XVII), in which A is an alkoxy, benzyloxy or silyloxy group and R¹ and R² are as defined above for Formula (I), may be prepared from compounds of Formula (XVIII) 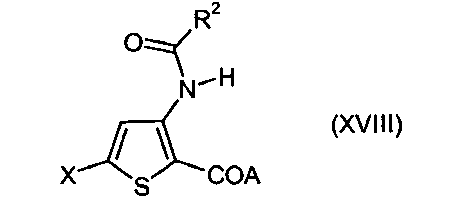 in which A is an alkoxy, benzyloxy or silyloxy, R² is as defined above for Formula (I) and X is a halogen such as bromide or iodide, by treatment with a suitable boronic acid R¹-B(OH)₂ or boronate ester R¹-B(OR')(OR"), in which R' and R" are independently C₁₋₆alkyl or R' and R" together with the carbon atoms to which they are attached form a ring optionally substituted by C₁₋₆alkyl, such as a pinacol ester, in the presence of a palladium catalyst such as tetrakistriphenyl phosphine palladium(0) or bis-[(diphenylphosphino)-ferrocene]-palladium(II) chloride, in the presence of a suitable base such as sodium carbonate, in a suitable solvent such as DMF, 1,4-dioxane or dimethyoxyethane, or combinations thereof, optionally in the presence of water, at a temperature in the range 50-100°C, optionally under an inert atmosphere.

Compounds of Formula (XVIII), in which A is an alkoxy, benzyloxy or silyloxy group, R² is as defined above for Formula (I) and X is a halogen such as bromide or iodide, may be prepared from compounds of Formula (XI) in which A is an alkoxy, benzyloxy or silyloxy, and X is a halogen such as bromide or iodide, by treatment with a suitable acylating agent, for example R²-C(O)-Y, wherein Y is a halo atom, for example chloro or bromo, and R² is as defined above for Formula (I). The reaction may be carried out in a suitable solvent, for example dichloromethane or dichloroethane, optionally in the presence of a suitable base, for example pyridine or triethylamine. The reaction may be carried out at a suitable temperature, for example in the range 20°C to 100°C. A phosphine such as triphenylphosphine may optionally be used in place of the base.

Compounds of Formula (XVIII), in which A is an alkoxy, benzyloxy or silyloxy group, R² is as defined above and X is a suitable halogen such as bromide or iodide, may be prepared by reaction of a compound of Formula (VIII) in which A an alkoxy, benzyloxy or silyloxy group, and R² is as defined above for Formula (I), by treatment with a suitable base such as lithium diisopropylamide and a halogen source such as iodine in a suitable solvent such as tetrahydrofuran, heptane, ethylbenzene or mixtures thereof and at a temperature in the range -78°C to -20°C.

Compounds of Formula (III) in which A is an alkoxy, benzyloxy or silyloxy group, R² and R³ are as defined above for Formula (I) and X is a halogen such as bromide or iodide, may also be prepared from compounds of Formula (XVIII) in which A is an alkoxy, benzyloxy or silyloxy, X is a halogen such as bromide or iodide and R² is as defined above for Formula (I), by treatment with a suitable alkylating agent R³-X' where X' is a halo group such as chloride, bromide or iodide, or X' is a sulphonate ester such as methanesulphonate or trifluoromethanesulphonate, and R³ is as defined above for Formula (I), in suitable solvent such as dimethylformamide in the presence of a suitable base such as triethylamine or sodium hydride or combinations thereof.

Compounds of Formula (III) in which A is an alkoxy, benzyloxy or silyloxy group, X is a halogen such as bromide or iodide and R² and R³ are as defined above for Formula (I), may also be prepared by reaction of a compound of Formula (X), in which A an alkoxy, benzyloxy or silyloxy group, R³ is as defined above for Formula (I) and X is a halogen such as bromide or iodide, with a suitable acylating agent, for example R²-C(O)-Y, wherein Y is a halo atom, for example chloro or bromo, and R² is as defined above for Formula (I). The reaction may be carried out in a suitable solvent, for example dichloromethane, in the presence of a suitable base, for example pyridine or triethylamine. A phosphine such as triphenylphosphine may optionally be used in place of the base.

Compounds of Formula (III)' in which A is an alkoxy, benzyloxy or silyloxy group, R³ is as defined above for Formula (I) and X is a suitable boronic acid -B(OH)₂ or boronate ester - B(OR')(OR"), in which R' and R" are independently C₁₋₆alkyl or R' and R" together with the carbon atoms to which they are attached form a ring optionally substituted by C₁₋₆alkyl, such as a pinacol ester, may also be prepared by reaction of a compound of Formula (IXX) 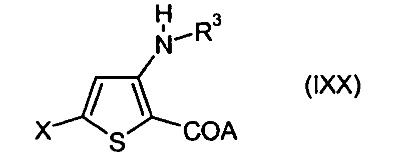 in which A an alkoxy, benzyloxy or silyloxy group, and R³ is as defined above for Formula (I) and X is a suitable boronate ester -B(OR')(OR"), in which R' and R" are independently C₁₋₆alkyl or R' and R" together with the carbon atoms to which they are attached form a ring optionally substituted by C₁₋₆alkyl, such as a pinacol ester, with a suitable acylating agent, for example R²-C(O)-Y, wherein Y is a halo atom, for example chloro or bromo, and R² is as defined above for Formula (I). The reaction may be carried out in a suitable solvent, for example dichloromethane, in the presence of a suitable base, for example pyridine or triethylamine. A phosphine such as triphenylphosphine may optionally be used in place of the base.

Compounds of Formula (IXX), in which A is an alkoxy, benzyloxy or silyloxy group, R³ is as defined above and X is a suitable boronate ester -B(OR')(OR"), in which R' and R" are independently C₁₋₆alkyl or R' and R" together with the carbon atoms to which they are attached form a ring optionally substituted by C₁₋₆alkyl, such as a pinacol ester, may also be prepared by reaction of a compound of Formula (V) in which A an alkoxy, benzyloxy or silyloxy group, and R³ is as defined above for Formula (I), by treatment with a suitable base such as lithium diisopropylamide and a boronate source such as B(OR)₃ wherein R is an alkyl group, for example methyl, in a suitable solvent such as tetrahydrofuran, and at a temperature in the range -78°C to -20°C.

Compounds of Formula (II) in which A is an alkoxy, benzyloxy or silyloxy group, may be prepared by reaction of a compound of Formula (II)' 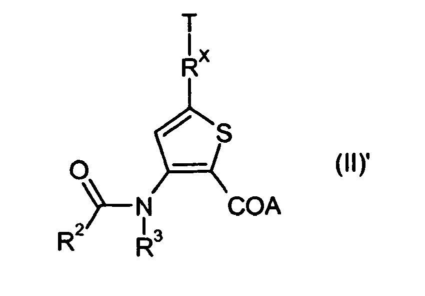 in which R^{x}, R², R³, are as defined for Formula (II), T is -NHP and P is H or a suitable protecting group (for example a tert-butyloxycarbonyl group) by reaction with a suitable carboxylic acid, R^{Y}-CO₂H, in the presence of a suitable coupling reagent such as O-(7-azabenzotriazol-1-yl)-*N,N,N',N*'-tetramethyluronium hexafluorophosphate (HATU) in the presence of a suitable base such as triethylamine or diisopropylethylamine, in a suitable solvent such as DMF, or by reaction with a suitable acid chloride, bromide or anhydride in the presence of a suitable base such as triethylamine, diisopropylethylamine or pyridine in a suitable solvent such as dichloromethane. Compounds of Formula (II)' in which T is -NHP and P is a protecting group can be converted into compounds of Formula (II)' in which P is H by a suitable deprotection reaction. For example if P is a tert-butyloxycarbonyl group, the deprotection may be carried out by treatment with acid (for example trifluoroacetic acid or hydrochloric acid). Compounds of Formula (II)' may be prepared in an analogous fashion to compounds of Formula (II) as described above.

Compounds of Formula (II)' in which T is -NHP and P is H may be prepared from compounds of Formula (II)' in which T is a nitro group, by reduction using methods well known in the art, for example using palladium, carbon or hydrogen. For example, the reduction can be carried out using iron and acetic acid in a suitable solvent, for example ethanol, at a suitable temperature, for example 50-70°C.

Compounds of Formula R'-B(OR')₂ for use in the preparation of compounds of Formula (II), (IX) or (XVII) are available commercially or may be prepared from compounds of Formula R^{Y}-R^{X}-hal or R^{Y}-hal by methods well known in the art.

Compounds of Formula R¹-hal for use in the preparation of compounds of Formula (II) are available commercially or may be prepared by methods well known in the art. Representative examples of heteroaryl halide preparation are given below (but are not exhaustive).

Esters of compounds of Formula (I), in which A is -OR where R is selected from straight or branched chain alkyl, aralkyl, aryloxyalkyl, or aryl, may also be prepared by esterification of a compound of Formula (I) in which A is hydroxy by standard literature procedures for esterification.

It will be appreciated that compounds of Formula (I), (II), (II)', (III), (IV), (VIII); (IX), (X), (XVII), (VIII) and (IXX) which exist as diastereoisomers may optionally be separated by techniques well known in the art, for example by column chromatography or recrystallisation. For example, the formation of an ester using a chiral alcohol, separation of the resulting diastereoisomers, and subsequent hydrolysis of the ester to yield the individual enantiomeric acid of Formula (I), (II), (II)', (III), (IV), (VIII), (IX), (X), (XVII), (VIII) and (IXX).

It will be appreciated that racemic compounds of Formula (I), (II), (II)', (III), (IV), (VIII), (IX), (X), (XVII), (VIII) and (IXX) may be optionally resolved into their individual enantiomers. Such resolutions may conveniently be accomplished by standard methods known in the art. For example, a racemic compound of Formula (I), (II), (II)', (III), (IV), (VIII), (IX), (X), (XVII), (VIII) and (IXX) may be resolved by chiral preparative HPLC. Alternatively, racemic compounds of Formula (I), (II), (II)', (III), (IV), (VIII), (IX), (X), (XVII), (VIII) and (IXX) which contain an appropriate acidic or basic group, such as a carboxylic acid group or amine group may be resolved by standard diastereoisomeric salt formation with a chiral base or acid reagent respectively as appropriate. Such techniques are well established in the art. For example, a racemic compound may be resolved by treatment with a chiral acid such as (R)-(-)-1,1'-binaphthyl-2,2'-diyl-hydrogen phosphate or (-)-di-O,O'-p-tolyl-L-tartaric acid, in a suitable solvent, for example isopropanol. The free enantiomer may then be obtained by treating the salt with a suitable base, for example triethylamine, in a suitable solvent, for example methyl tert-butyl ether. Alternatively, racemic acid compounds may be resolved using a chiral base, for example (S)-alpha methylbenzylamine, (S)-alpha phenylethylamine, (1S, 2S)-(+)-2-amino-1-phenyl-1,3-propane-diol, (-) ephidrine, quinine, brucine. Individual enantiomers of Formula (I), (II), (II)', (III), (IV), (VIII), (IX), (X), (XVII), (VIII) and/or (IXX) may then be progressed to an enantiomeric compound of Formula (I) by the chemistry described above in respect of racemic compounds.

With appropriate manipulation and protection of any chemical functionality, synthesis of compounds of Formula (I) is accomplished by methods analogous to those above and to those described in the Experimental section. Suitable protecting groups can be found, but are not restricted to, those found in T W Greene and P G M Wuts 'Protective Groups in Organic Synthesis', 3^{rd} Ed (1999), J Wiley and Sons.

Several of the synthetic procedures described above in general terms (and below in specific terms) may involve heating the reactants. It will be appreciated that heating may be carried out by various conventional methods but also with the use of a microwave reactor.

### EXAMPLES

| **Abbreviations** | |
|---|---|
| AcOH | acetic acid |
| Biotage | Biotage flash equipment for use with pre-packed silica cartridges |
| DCE | 1,2-dichloroethane |
| DCM | dichloromethane |
| DEF | N,N-diethylformamide |
| Diglyme | diethylene glycol dimethyl ether |
| DIPEA | N,N-diisopropylethylamine |
| DMAP | 4-(dimethylamino)pyridine |
| DME | 1,2-dimethoxyethane |
| DMF | *N*,*N*-dimethylformamide |
| DMSO | dimethylsulfoxide |
| EtOAc | ethyl acetate |
| Et₂O | diethyl ether |
| Greenhouse | A greenhouse parallel synthesiser as manufactured by Radleys Discovery |
| HATU | *O*-(7-azabenzotriazol-1-yl)-*N,N,N,N'*-tetramethyluronium hexafluorophosphate |
| IPA | 2-propanol/isopropanol |
| ISCO Companion^{™} | Automated flash chromatography equipment with fraction analysis by UV absorption available from Presearch. |
| LDA | lithium diisopropylamide |
| MDAP HPLC | reverse phase HPLC on a C₁₈ column using a two-solvent gradient elution with (A) water containing formic acid (0.1 %) and (B) acetonitrile-water (95:5 v/v) containing formic acid (0.05%) as the eluents, and analysis of the fractions by electrospray mass spectroscopy. |
| MeOH | methanol |
| MIBK | methyl isobutyl ketone |
| NH₂ SPE | aminopropyl ion exchange cartridge |
| OASIS^{™} HLB cartridge | sample extraction cartridge available from Waters |
| Pd₂(dba)₃ | tris(dibenzylideneacetone)dipalladium |
| PLM | polarised light microscopy |
| SCX | propylsulphonic acid ion exchange cartridge |
| Reactivial^{™} | a small volume, thick walled glass reaction vial sealed with a screw cap, available from Pierce |
| SPE | solid phase extraction column |
| Strata | Dual NH₂ and SO₃H solid phase extraction cartridge available from Phenomenex. |
| THF | tetrahydrofuran |
| TFA | trifluoroacetic acid |

All mass spectroscopy was performed using electrospray as the method of ionisation. For ISCO Companion^{™} chromatography using normal phase silica, a gradient of ethyl acetate in cyclohexane is used unless otherwise stated. For purification of Examples using ion exchange chromatography, unless otherwise described, the crude product was applied to an aminopropyl cartridge which was washed with methanol. The cartridge was eluted with 10% 2N HCl in MeOH and the appropriate fractions were evaporated *in vacuo.*

### Intermediate 1

### trans-4-Methylcyclohexanecarbonyl chloride

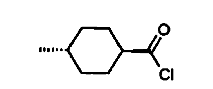

A solution of trans-4-methylcyclohexanecarboxylic acid (40 g) in dry DCM (400 mL) was stirred at 21°C under nitrogen, and was treated with DEF (2 drops), followed by dropwise addition of oxalyl chloride (24.5 mL). An effervescence was observed. The reaction was stirred overnight. The solvent was evaporated *in vacuo* to give the title compound.
¹H NMR (CDCl₃): δ 2.65 (1H, tt), 2.20-2.12 (2H, m), 1.86-1.78 (2H, m), 1.58-1.44 (2H, m), 1.44-1.31 (1H, m), 1.04-0.94 (2H, m), 0.91 (3H, d).

### Intermediate 2

### Methyl 3-[(1-methylethyl)amino]-2-thiophenecarboxylate

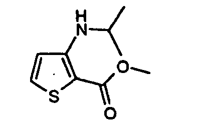

A solution of methyl 3-amino-2-thiophenecarboxylate (25 g) in dry DCM (500 mL) under nitrogen with stirring was treated slowly with 2-methoxypropene (34.1 g), followed by dropwise addition of acetic acid (28 mL), taking care not to let the temperature rise above 25°C. Sodium triacetoxyborohydride (50.55 g) was added in portions, keeping the temperature below 25°C. The resulting mixture was stirred overnight. The reaction was poured into saturated sodium bicarbonate solution (700 mL) and the phases were separated. The aqueous phase was extracted with DCM (x 2). The combined organic phases were washed with saturated sodium bicarbonate solution and brine, dried over sodium sulphate and evaporated *in vacuo* to give the title compound.
MS calcd for (C₉H₁₃NO₂S + H)⁺: 200
MS found (electrospray): (M+H)⁺ = 200

### Intermediate 3

### Methyl 3-[[(trans-4-methylcyclohexyl)cabonyl](1-methylethyl)amino]-2-thiophenecarboxylate

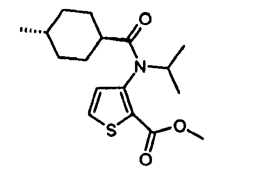

A solution of Intermediate 2 (33.16 g) and Intermediate 1 (31.28 g) in DCE (470 mL) was stirred and heated at reflux overnight. The reaction was then heated at reflux for a further 36 h, and was left to stand at 21 °C for a further 36 h. The reaction was poured into a mixture of DCM and saturated sodium bicarbonate solution, and the layers were separated. The aqueous phase was extracted with DCM (x 2) and the combined organic phases were washed with saturated sodium bicarbonate solution, followed by brine. The organic phases were dried over sodium sulphate and were evaporated *in vacuo.* The crude material was triturated with cyclohexane, filtered, washed with more cyclohexane (x 3) and was dried in a vacuum oven to give the title compound.
MS calcd for (C₁₇H₂₅NO₃S + H)⁺: 324
MS found (electrospray): (M+H)⁺ = 324

### Intermediate 4

### {4-[[(trans-4-Methylcyclohexyl)carbonyl](1-methylethyl)amino]-5-[(methyloxy)carbonyl]-2-thienyl}boronic acid

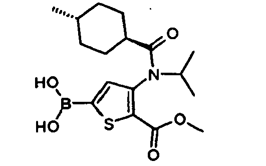

A solution of LDA (2.0M solution in THF/heptane/ethyl benzene, 76 mL) was stirred under nitrogen and cooled to -78°C. A solution of methyl 3-[[(*trans*-4-methylcyclohexyl)carbonyl](1-methylethyl)amino]-2-thiophenecarboxylate (16.3 g, a synthesis of which is described above as Intermediate 3) in dry THF (175 mL) was added dropwise, keeping the temperature below -70°C. The resulting mixture was stirred at -78°C for 1 h, and was then treated dropwise with a solution of trimethyl borate (15.5 g) in dry THF (40 mL) keeping the temperature below - 70°C. The reaction was stirred for 20 mins and was then quenched by the dropwise addition of 2M HCl solution (240 mL). The reaction was allowed to warm to 21°C and the phases were separated. The aqueous layer was extracted with EtOAc (x 2), and the combined organics were washed with brine, dried over sodium sulphate and evaporated *in vacuo* to give a solid. This was triturated with IPA/water (1:1) and was filtered and washed with a further portion of IPA/water (1:1). The material was then dried in a vacuum oven at 50°C to give the title compound.
MS calcd for (C₁₇H₂₆BNO₅S + H)⁺: 368
MS found (electrospray): (M+H)⁺ = 368

### Intermediate 5

### N-(4-iodophenyl)-1,3-thiazole-4-carboxamide

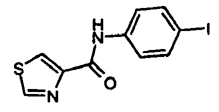

1,3-Thiazole-4-carboxylic acid (200 mg) was dissolved in DMF (6 mL). HATU (650 mg) and DIPEA (0.539 mL) were added and the reaction mixture was stirred at room temperature for 15 mins. 4-lodoaniline (508 mg) was added and the reaction mixture was stirred at room temperature for 1 h. The reaction mixture was evaporated *in vacuo* and the residue was dissolved in DCM. This was washed with sodium bicarbonate solution (x 2) followed by 2N HCl (x 2). The DCM was separated, dried using a hydrophobic frit and was evaporated *in vacuo* to give the title compound.
MS calcd for (C₁₀H₇IN₂O₅ + H)⁺: 331
MS found (electrospray): (M+H)⁺ = 331

### Intermediate 6

### Methyl 3-[[(trans-4-methylcyclohexyl)carbonyl](1-methylethyl)amino]-5-{4-[(1,3-thiazol-4-ylcarbonyl)amino]phenyl}-2-thiophenecarboxylate

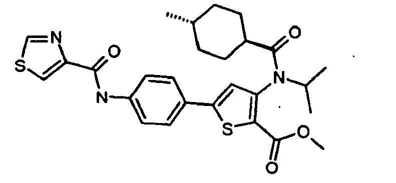

Intermediate 5 (250 mg), Intermediate 4 (330 mg), cesium fluoride (693 mg) and tetrakis(triphenylphosphine)palladium(0) (104 mg) in DME/water (1:1, 6 mL) were heated to 90°C for 2 h and were then allowed to cool to room temperature. The mixture was partitioned between EtOAc and water. The aqueous layer was washed with EtOAc (x 2) and the combined organics were dried using a hydrophobic frit and were evaporated *in vacuo.* The crude material was purified by ISCO Companion^{™} silica chromatography, eluting with a gradient 5-100% EtOAc in cyclohexane to give the title compound.
MS calcd for (C₂₇H₃₁N₃O₄S₂ + H)⁺: 526
MS found (electrospray): (M+H)⁺= 526

### Intermediate 7

### Methyl 5-iodo-3-[[(trans-4-methylcyclohexyl)carbonyl](1-methylethyl)amino]-2-thiophenecarboxylate

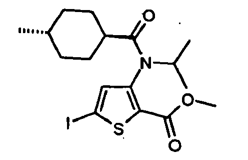

A solution of [methyl 3-[[(*trans*-4-methylcyclohexyl)carbonyl](1-methylethyl)amino]-2-thiophenecarboxylate] (10.0 g, a synthesis of which is described as Intermediate 3) in dry THF (100 mL) was added dropwise under nitrogen to a cold (-78°C) 2M solution of LDA in THF/heptane/ethyl benzene (46.5 mL) maintaining an internal temperature below -70°C. The reaction was stirred at -78°C for 2.5 h. A solution of iodine (15.75 g) in dry THF (100 mL) was added dropwise over 30 mins to the stirred reaction mixture, maintaining an internal temperature below -70°C. After stirring under nitrogen for 1.5 h, the reaction mixture was quenched by addition of saturated ammonium chloride solution (50 mL) and warmed to 0°C. 5% Sodium thiosulphate solution (200 mL) was added and the organic phases were separated. The aqueous phase was extracted with EtOAc (x 2) and the combined organic phases were washed with brine, dried over sodium sulphate and evaporated *in vacuo*. The crude material was purified by ISCO Companion^{™} silica chromatography, eluting with a gradient 0-40% EtOAc in cyclohexane to give the title compound.
MS calcd for (C₁₇H₂₄NIO₃S + H)⁺: 450
MS found (electrospray): (M+H)⁺ = 450

### Intermediate 8

### Methyl 5-[4-({[(1,1-dimethylethyl)oxy]carbonyl}amino)phenyl]-3-[[(trans-4-methylcyclohexyl)carbonyl](1-methylethyl)amino]-2-thiophenecarboxylate

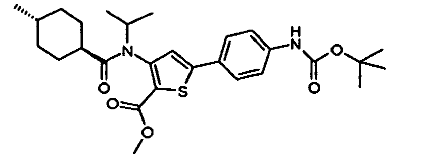

A mixture of Intermediate 7 (3.5 g), [4-({[(1,1-dimethylethyl)oxy]carbonyl}amino)phenyl]boronic acid (2.0 g), sodium carbonate (3.3 g, dissolved in water 20 mL), and tetrakis(triphenylphosphine)palladium(0) (900 mg) were dissolved in DMF (100 mL) and the reaction mixture was stirred at 100°C for 3 h. The mixture was evaporated *in vacuo* and the residue partitioned between water and DCM. The organic layer was washed with water (x 2), dried using a hydrophobic frit and evaporated *in vacuo.* The crude material was purified by ISCO Companion^{™} silica chromatography, eluting with a gradient 5-50% EtOAc in cyclohexane to give the title compound.
MS calcd for (C₂₈H₃₈N₂O₅S + H)⁺: 515
MS found (electrospray): (M+H)⁺ = 515

### Intermediate 9

### Methyl 5-(4-aminophenyl)-3-[[(trans-4-methylcyclohexyl)carbonyl](1-methylethyl)amino]-2-thiophenecarboxylate

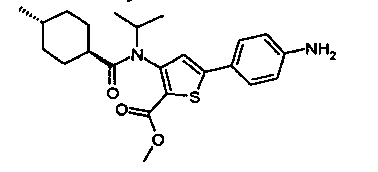

Intermediate 8 (3.9 g) was stirred in DCM (40 mL) and TFA (10 mL) at room temperature under nitrogen for 2 h. The reaction was evaporated *in vacuo* and the residue partitioned between sodium bicarbonate solution and DCM. The organic phases were dried using a hydrophobic frit and evaporated *in vacuo* to give the title compound.
MS calcd for (C₂₃H₃₀N₂O₃S + H)⁺: 415
MS found (electrospray): (M+H)⁺=415

### Intermediate 10

### Methyl 3-[[(trans-4-methylcyclohexyl)carbonyl](1-methylethyl)amino]-5-{4-[(2-pyridinylcarbonyl)amino]phenyl}-2-thiophenecarboxylate

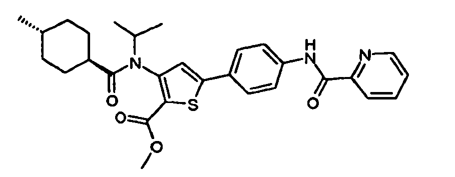

To a solution of 2-pyridinecarboxylic acid (89 mg) in DMF (6 mL) was added DIPEA (129 mg), followed by HATU (209 mg). The reaction was stirred at room temperature for 15 mins. Intermediate 9 (200 mg) was added and the reaction mixture was stirred at room temperature overnight. The crude material was purified by ISCO Companion^{™} silica chromatography, eluting with a gradient 5-100% EtOAc in cyclohexane to give the title compound.
MS calcd for (C₂₉H₃₃N₃O₄S + H)⁺: 520
MS found (electrospray): (M+H)⁺ = 520

### Intermediate 11

### Methyl 5-{4-[(2-furanylcarbonyl)amino]phenyl}-3-[[(trans-4-methylcyclohexyl)carbonyl](1-methylethyl)amino]-2-thiophenecarboxylate

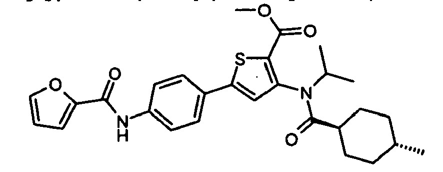

To a solution of 2-furancarboxylic acid (103 mg) in DMF (6 mL) was added DIPEA (129 mg), followed by HATU (209 mg). The reaction was stirred at room temperature for 15 mins. Intermediate 9 (200 mg) was added and the reaction mixture was stirred at room temperature overnight. The crude material was purified by ISCO Companion^{™} silica chromatography, eluting with a gradient 5-100% EtOAc in cyclohexane to give the title compound.
MS calcd for (C₂₈H₃₂N₂O₅S + H)⁺: 509
MS found (electrospray): (M+H)⁺= 509

### Intermediate 12

### Methyl 3-[[(trans-4-methylcyclohexyl)carbonyl](1-methylethyl)amino]-5(4-{[(2-methyl-1,3-thiazol-4-yl)carbonyl]amino}phenyl)-2-thiophenecarboxylate

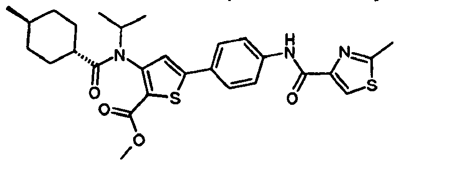

To a solution of 2-methyl-1,3-thiazole-4-carboxylic acid (81 mg) in DMF (6 mL) was added DIPEA (129 mg), followed by HATU (209 mg). The reaction was stirred at room temperature for 15 mins. Intermediate 9 (200 mg) was added and the reaction mixture was stirred at room temperature overnight. The crude material was purified by ISCO Companion^{™} silica chromatography, eluting with a gradient 5-100% EtOAc in cyclohexane to give the title compound.
MS calcd for (C₂₈H₃₃N₃O₄S₂ + H)⁺: 540
MS found (electrospray): (M+H)⁺ = 540

### Intermediate 13

### Methyl 3-[[(trans-4-methylcyclohexyl)carbonyl](1-methylethyl)amino]-5-{4-[(2-thienylcarbonyl)amino]phenyl}-2-thiophenecarboxylate

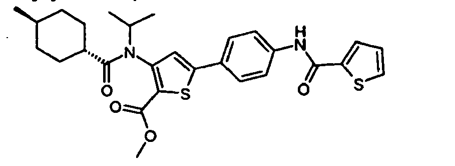

To a solution of 2-thiophenecarboxylic acid (92 mg) in DMF (6 mL) was added DIPEA (129 mg), followed by HATU (209 mg). The reaction was stirred at room temperature for 15 mins. Intermediate 9 (200 mg) was added and the reaction mixture was stirred at room temperature overnight. The crude material was purified by ISCO Companion^{™} silica chromatography, eluting with a gradient 5-100% EtOAc in cyclohexane to give the title compound.
MS calcd for (C₂₈H₃₂N₄O₄S + H)⁺: 521
MS found (electrospray): (M+H)⁺ 521

### Intermediate 14

### Methyl 3-[[(trans-4-methylcyclohexyl)carbonyl](1-methylethyl)amino]-5-{4-[(2-pyrazinylcarbonyl)amino]phenyl}-2-thiophenecarboxylate

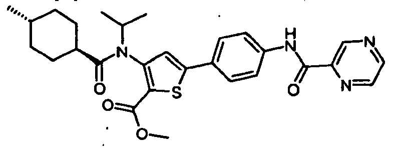

To a solution of 2-pyrazinecarboxylic acid (89 mg) in DMF (6 mL) was added DIPEA (129 mg), followed by HATU (209 mg). The reaction was stirred at room temperature for 15 mins. Intermediate 9 (200 mg) was added and the reaction mixture was stirred at room temperature overnight. The crude material was purified by ISCO Companion^{™} silica chromatography, eluting with a gradient 5-100% EtOAc in cyclohexane to give the title compound.
MS calcd for (C₂₈H₃₂N₂O₄S₂ + H)⁺: 525
MS found (electrospray): (M+H)⁺= 525

### Intermediate 15

### Methyl 5-(4-{[(4-fluorophenyl)carbonyl]amino}pheny)-3-[[(trans-4-methylcyclohexyl)carbonyl](1-methylethyl)amino]-2-thiophenecarboxylate

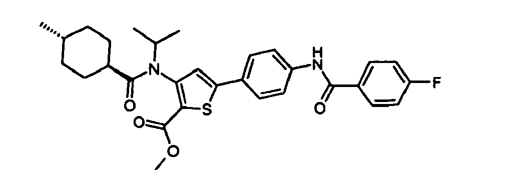

To a solution of 4-fluorobenzoic acid (105 mg) in DMF (6 mL) was added DIPEA (129 mg), followed by HATU (209 mg). The reaction was stirred at room temperature for 15 mins. Intermediate 9 (200 mg) was added and the reaction mixture was stirred at room temperature for 4.5 h. The reaction was evaporated *in vacuo* and the residue partitioned between DCM and sodium bicarbonate solution. The organic phases were dried by passing through a hydrophobic frit and evaporated *in vacuo.* The crude material was purified by ISCO Companion^{™} silica chromatography, eluting with a gradient 5-100% EtOAc in cyclohexane to give the title compound.
MS calcd for (C₃₀H₃₃FN₂O₄S + H)⁺: 537
MS found (electrospray): (M+H)⁺= 537

### Intermediate 16

### Methyl 3-[[(trans-4-methylcyclohexyl)carbonyl](1-methylethyl)amino]-5-{4-[(1,3-oxazol-2-ylcarbonyl)amino]phenyl}-2-thiophenecarboxylate

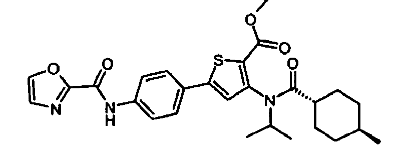

To a solution of 1,3-oxazole-2-carboxylic acid (85 mg) in DMF (6 mL) was added DIPEA (129 mg), followed by HATU (209 mg). The reaction was stirred at room temperature for 15 mins. Intermediate 9 (200 mg) was added and the reaction mixture was stirred at room temperature for 4.5 h. The reaction was evaporated *in vacuo* and the residue partitioned between DCM and sodium bicarbonate solution. The organic phases were dried by passing through a hydrophobic frit and evaporated *in vacuo.* The crude material was purified by ISCO Companion^{™} silica chromatography, eluting with a gradient 5-100% EtOAc in cyclohexane to give the title compound.
MS calcd for (C₂₇H₃₁N₃O₅S + H)⁺: 510
MS found (electrospray): (M+H)⁺ = 510

### Intermediate 17

### Methyl 3-[(cyclopropylmethyl)amino]-2-thiophenecarboxylate

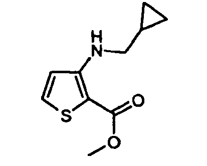

To methyl 3-amino-2-thiophenecarboxylate (10 g) was added DCM (200 mL), acetic acid (14.6 mL), cyclopropanecarbaldehyde (5.35 g) and sodium triacetoxyborohydride (27 g). The reaction was stirred at room temperature under nitrogen for 16 h. The reaction was cautiously quenched with saturated sodium bicarbonate solution and was extracted with DCM. The layers were separated and the organic layers passed through a hydrophobic frit. The organic layers were evaporated *in vacuo* to give the title compound.
MS calcd for (C₁₀H₁₃NO₂S + H)⁺: 212
MS found (electrospray): (M+H)⁺= 212

### Intermediate 18

### {4-[(1,3-Thiazol-4-ylcarbonyl)amino]phenyl}boronic acid

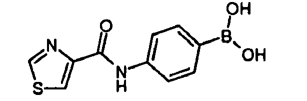

To 1,3-thiazole-4-carboxylic acid (2.5 g) was added dry DMF (68 mL), DIPEA (6.67 mL) and HATU (13.1 g). The reaction was stirred at room temperature for 1 h under nitrogen. (4-Aminophenyl)boronic acid (5.1 g) was added and the reaction was stirred for 24 h, then partitioned between EtOAc and saturated sodium bicarbonate solution. The organic phases were washed further with saturated sodium bicarbonate solution, water, 2N HCl (x 2) and brine. The organic phases were passed through a hydrophobic frit and evaporated *in vacuo.* The crude material was purified by ISCO Companion^{™} silica chromatography, eluting with a gradient 0-100% EtOAc in cyclohexane to give the title compound.
MS calcd for (C₁₀H₉BN₂O₃S + H)⁺: 331
MS found (electrospray): (M+H)⁺= 331

### Intermediate 19

### Methyl 3-[(cyclopropylmethyl)amino]-5-iodo-2-thiophenecarboxylate

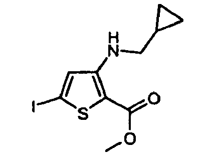

To Intermediate 17 (10.1 g) in dry THF (200 mL) at -78°C under nitrogen was added a solution of LDA (2.0M solution in THF/heptane, 95.6 mL). The reaction was stirred for 45 mins, and then a solution of iodine (14.5 g) in dry THF (60 mL) was added slowly. The resulting suspension was stirred for 1 h. The reaction was neutralised with saturated ammonium chloride and was extracted with EtOAc. The organic phases were washed with 5% sodium thiosulphate solution and brine. The organic phases were dried using a hydrophobic frit and were evaporated *in vacuo.* The crude material was purified by reverse phase ISCO Companion^{™} chromatography, using a C18 cartridge, to give the title compound.
MS calcd for (C₁₀H₁₂INO₂S + H)⁺: 338
MS found (electrospray): (M+H)⁺ = 338

### Intermediate 20

### Methyl 3-[(cyclopropylmethyl)amino]-5-{4-[(1,3-thiazol-4-ylcarbonyl)amino]phenyl}-2-thiophenecarboxylate

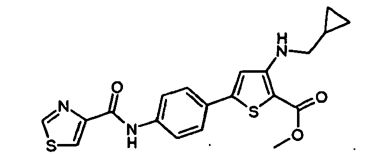

To Intermediate 19 (800 mg) was added DME (16 mL), water (4 mL), potassium phosphate (1.0 g), Intermediate 18 (1.17 g) and tetrakis(triphenylphosphine)palladium(0) (274 mg). The reaction was stirred at 80°C under nitrogen for 16 h. The reaction was cooled and partitioned between water and DCM. The phases were separated using a hydrophobic frit and the organic phases were evaporated *in vacuo.* The crude material was purified by ISCO Companion^{™} silica chromatography, eluting with a gradient 20-80% EtOAc in cyclohexane to give the title compound.
MS calcd for (C₂₀H₁₉N₃O₃S₂ + H)⁺: 414
MS found (electrospray): (M+H)⁺= 414

### Intermediate 21

### trans-4-(Trifluoromethyl)cyclohexanecarboxylic acid

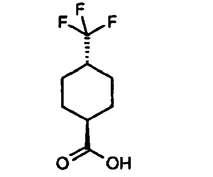

A solution of 4-(trifluoromethyl)cyclohexanecarboxylic acid (20 g) in diglyme (50 mL) and MeOH (12 mL) was treated with a solution of potassium hydroxide (12.3 g) in water (12 mL). The resulting solution was heated to 170°C using Dean-Stark apparatus and the solvent started to azeotrope over at 148°C. When 40 mL had azeotroped over, the temperature was reduced to 165°C and the reaction was heated for 1 h. The temperature was increased to 170°C and the reaction heated at reflux for 3 h (with the Dean-Stark apparatus removed). The reaction mixture was allowed to cool to room temperature and was diluted with water (50 mL) and EtOAc (50 mL). 2N HCl was added until the mixture was acidic (pH 1). A precipitate formed and the mixture was filtered through a thin pad of Celite to remove the solid. The Celite was washed through with water (50 mL) and EtOAc (50 mL). The layers were separated and the aqueous layer extracted further with EtOAc (2 x 100 mL). The combined organic phases were dried over sodium sulphate and evaporated *in vacuo.* The residue was azeotroped with toluene (3 x 50 mL). The material was re-crystallised from cyclohexane (30 mL) and the solid was filtered off, air-dried, then dried at 45°C for 5 h to give the title compound.
¹H NMR (d₆-DMSO): δ 12.13 (1H, s), 2.33-2.12 (2H, m), 1.91 (4H, dd), 1.43-1.18 (4H, m).

### Intermediate 22

### trans-4-(Trifluoromethyl)cyclohexanecarbonyl chloride

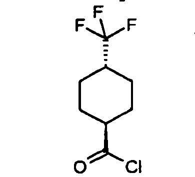

Oxalyl chloride (4.59 mL) was added dropwise to a solution of *trans*-4-(trifluoromethyl)cyclohexanecarboxylic acid (6.85 g, a synthesis of which is described as Intermediate 21) in dry DCM (100 mL) at room temperature under nitrogen. After 10 mins an effervescence was observed and the reaction was stirred at room temperature overnight. The solvent was evaporated *in vacuo* to give the title compound.
¹H NMR (d₆-DMSO) δ 2.38-2.19 (2H, m), 1.92 (4H, dd), 1.44-1.22 (4H, m).

### Intermediate 23

### Methyl 3-((1-methylethyl){[trans-4-(trifluoromethyl)cyclohexyl]carbonyl}amino)-2-thiophenecarboxylate

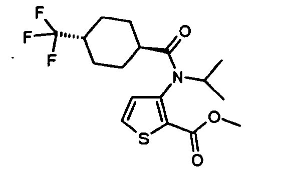

A mixture of methyl 3-[(1-methylethyl)amino]-2-thiophenecarboxylate (1.26 g, a synthesis of which is described above as Intermediate 2), *trans*-4-(trifluoromethyl)cyclohexanecarbonyl chloride (2.03 g, a synthesis of which is described above as Intermediate 22) and triphenylphoshine (3.31 g) in DCM was stirred at 50°C under nitrogen overnight. A further portion of *trans*-4-(trifluoromethyl)cyclohexanecarbonyl chloride (1.35 g, a synthesis of which is described above as Intermediate 22) was added and the mixture was stirred at 50°C under nitrogen overnight. Crystals precipitated out and these were collected by filtration. The filtrate was partitioned between DCM and water. The aqueous layer was separated and extracted with DCM. The remaining organic phases were then washed with water. The combined organic phases were evaporated *in vacuo* and were purified by ISCO Companion^{™} silica chromatography, eluting with a gradient 0-60% EtOAc in cyclohexane. Product containing fractions were evaporated *in vacuo* and were combined with the crystals obtained above to give the title compound.
MS calcd for (C₁₇H₂₂F₃NO₃S + H)⁺: 378
MS found (electrospray): (M+H)⁺= 378

### Intermediate 24

### {4-((1-Methylethyl){[trans-4-(trifluoromethyl)cyclohexyl]carbonyl}amino)-5-[(methyloxy)carbonyl]-2-thienyl}boronic acid

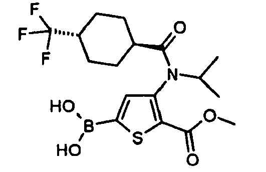

Methyl 3-((1-methylethyl){[*trans*-4-(trifluoromethyl)cyclohexy]carbonyl}amino)-2-thiophenecarboxylate (1.0 g, a synthesis of which is described as Intermediate 23) in THF (5 mL) was added dropwise to a solution of LDA (1.8M solution in THF/heptane/ethyl benzene, 4.41 mL) in THF (5 mL) at -78°C under nitrogen. The mixture was left to stir at -78°C for 0.75 h. Trimethylborate (0.887 mL) in THF (5 mL) was added dropwise, maintaining the internal temperature at -78°C. After 30 mins, 2N HCl (15 mL) was added dropwise to quench the reaction and the mixture was allowed to warm to room temperature over 1 h. EtOAc (40 mL) was added and the layers were separated. The aqueous layer was extracted further with EtOAc (2 x 10 mL). The combined organic phases were dried over sodium sulphate and evaporated *in vacuo.* The material was triturated with Et₂O (20 mL) and the mixture was stirred at room temperature for 15 mins. The solid was filtered off to give the title compound. ¹H NMR (CD₃OD): δ 7.40 (1H, s), 3.83 (3H, s), 2.12-1.92 (2H, m), 1.92-1.69 (4H, m), 1.68-1.53 (1H, m), 1.45-1.26 (2H, m), 1.14 (3H, d), 1.09-0.84 (5H, m), 2 exchangeable protons not seen.

### Intermediate 25

### Methyl 3-((1-methylethyl){[trans-4-(trifluoromethyl)cyclohexyl]carbonyl}amino)-5-{4-[(1,3-thiazol-4-ylcarbonyl)amino]phenyl}-2-thiophenecarboxylate

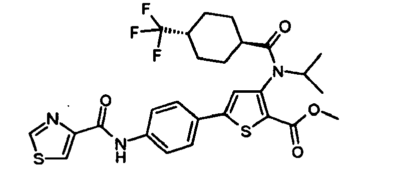

A solution of *N*-(4-iodophenyl)-1,3-thiazole-4-carboxamide (235 mg, a synthesis of which is described as Intermediate 5), {4-((1-methylethyl){[*trans*-4-(trifluoromethyl)cyclohexyl]carbonyl}amino)-5-[(methyloxy)carbonyl]-2-thienyl}boronic acid (300 mg, a synthesis of which is described as Intermediate 24), potassium phosphate (450 mg) and tetrakis(triphenylphosphine)palladium(0) (6 mg) in DMF (10 mL) was stirred under nitrogen at 100°C for 20 h. The reaction was evaporated *in vacuo* and was partitioned between DCM and water. The organic phases were evaporated and the crude material was purified by ISCO Companion^{™} silica chromatography, eluting with 30% EtOAc in cyclohexane to give the title compound.
MS calcd for (C₂₇H₂₈F₃NO₄S₂ + H)⁺: 580
MS found (electrospray): (M+H)⁺= 580

### Intermediate 26

### Methyl 3-[[(trans-4-methylcyclohexyl)carbonyl](1-methylethyl)amino]-5-{4-[(3-pyridazinylcarbonyl)amino]phenyl}-2-thiophenecarboxylate

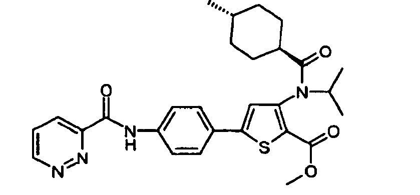

A mixture of 3-pyridazinecarboxylic acid (66 mg), HATU (220 mg) and DIPEA (248 mg) were dissolved in DMF (5 mL) and the reaction was stirred at room temperature for 1 h. Methyl 5-(4-aminophenyl)-3-[[(*trans*-4-methylcyclohexyl)carbonyl](1-methylethyl)amino]-2-thiophenecarboxylate (200 mg, a synthesis of which is described as Intermediate 9) was added and the reaction was stirred at room temperature for 18 h, then at 50°C over a weekend. HATU (220 mg), DIPEA (248 mg) and 3-pyridazinecarboxylic acid (66 mg) were added and the reaction was stirred at 50°C for 4 h. The reaction was evaporated *in vacuo* and partitioned between DCM and sodium bicarbonate solution. The organic phases were separated, dried using a hydrophobic frit and evaporated *in vacuo.* The crude material was purified by ISCO Companion^{™} silica chromatography, eluting with a gradient 5-100% EtOAc in cyclohexane to give the title compound.
MS calcd for (C₂₈H₃₂N₄O₄S + H)⁺: 521
MS found (electrospray): (M+H)⁺= 521

### Intermediate 27

### Methyl 3-[[(trans-4-methylcyclohexyl)carbonyl](1-methylethyl)amino]-5-{4-[(4-pyrimidinylcarbonyl)amino]phenyl}-2-thiophenecarboxylate

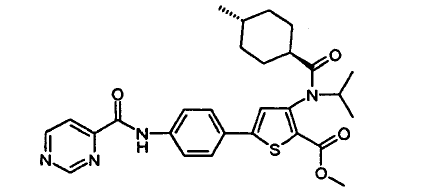

A mixture of 4-pyrimidinecarboxylic acid (66 mg), HATU (220 mg) and DIPEA (248 mg) were dissolved in DMF (5 mL) and the reaction was stirred at room temperature for 1 h. Methyl 5-(4-aminophenyl)-3-[[(*trans*-4-methylcyclohexyl)carbonyl](1-methylethyl)amino]-2-thiophenecarboxylate (200 mg, a synthesis of which is described as Intermediate 9) was added and the reaction was stirred at room temperature for 18 h, then at 50°C over a weekend. HATU (220 mg), DIPEA (248 mg) and 4-pyrimidinecarboxylic acid (66 mg) were added and the reaction was stirred at 50°C for 4 h. The reaction was evaporated *in vacuo* and partitioned between DCM and sodium bicarbonate solution. The organic phases were separated, dried using a hydrophobic frit and evaporated *in vacuo.* The crude material was purified by ISCO Companion^{™} silica chromatography, eluting with a gradient 5-100% EtOAc in cyclohexane to give the title compound.
MS calcd for (C₂₈H₃₂N₄O₄S + H)⁺: 521
MS found (electrospray): (M+H)⁺ = 521

### Intermediate 28

### Methyl 5-(4-amino-3-chlorophenyl)-3-[[(trans-4-methylcyclohexyl)carbonyl](1-methylethyl)amino]-2-thiophenecarboxylate

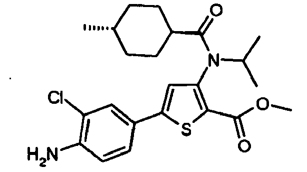

A mixture of 2-chloro-4-iodoaniline (1.3 g), {4-[[(*trans*-4-methylcyclohexyl)carbonyl](1-methylethyl)amino]-5-[(methyloxy)carbonyl]-2-thienyl}boronic acid (2 g, a synthesis of which is described as Intermediate 4), cesium fluoride (4.4 g) and tetrakis(triphenylphosphine)palladium(0) (687 mg) in DME/water (1:1, 30 mL) was heated at 100°C for 1 h. The mixture was partitioned between EtOAc and water. The organic phases were collected, dried using a hydrophobic frit and evaporated *in vacuo.* The crude material was purified by SCX SPE cartridge, eluting with 10% aqueous ammonia (0.88) in MeOH to give the title compound.
MS calcd for (C₂₃H₂₉CIN₂O₃S + H)⁺: 449/451
MS found (electrospray): (M+H)⁺ = 449/451

### Intermediate 29

### Methyl 5-{3-chloro-4-[(1,3-thiazol-4-ylcarbonyl)amino]phenyl}-3-[[(trans-4-methylcyclohexyl)carbonyl](1-methylethyl)amino]-2-thiophenecarboxylate

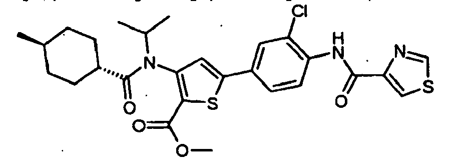

1,3-Thiazole-4-carboxylic acid (65 mg) was dissolved in DMF (4 mL) then HATU (867 mg) and DIPEA (0.175 mL) were added. The reaction was stirred for 15 mins then Intermediate 28 (250 mg) was added. The reaction was stirred at room temperature overnight, then at 70°C for 4 h. DMAP (4 mg) was added and the reaction was stirred at 70°C for 18 h. The reaction was evaporated *in vacuo* and was partitioned between DCM and sodium bicarbonate solution. The organic phases were dried by passing through a hydrophobic frit and were evaporated *in vacuo.* The crude material was purified by ISCO Companion^{™} silica chromatography, eluting with a gradient 5-100% EtOAc in cyclohexane to give the title compound.
MS calcd for (C₂₇H₃₀CIN₃O₄S₂ + H)⁺: 560/562
MS found (electrospray): (M+H)⁺ = 560/562

### Intermediate 30

### Methyl 3-[[(trans-4-methylcyclohexyl)carbonyl](1-methylethyl)amino]-5-{4-[(3-thienylcarbonyl)amino]phenyl}-2-thiophenecarboxylate

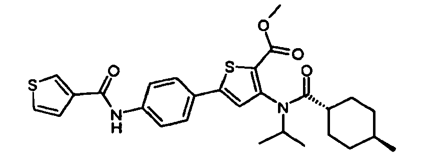

3-Thiophenecarboxylic acid (93 mg) was dissolved in DMF (5 mL) then HATU (293 mg) and DIPEA (0.336 mL) were added. The reaction was stirred for 15 mins then methyl 5-(4-aminophenyl)-3-[[(*trans*-4-methylcyclohexyl)carbonyl](1-methylethyl)amino]-2-thiophenecarboxylate (200 mg, a synthesis of which is described as Intermediate 9) was added. The reaction was stirred at room temperature overnight. The reaction was evaporated *in vacuo* and the residue was partitioned between DCM and sodium bicarbonate solution. The organic phases were collected and dried using a hydrophobic frit. The crude material was purified by ISCO Companion^{™} silica chromatography, eluting with a gradient 5-100% EtOAc in cyclohexane to give the title compound.
MS calcd for (C₂₈H₃₂N₂O₄S₂ + H)⁺: 525
MS found (electrospray): (M+H)⁺ = 525

### Intermediate 31

### Methyl 5-(3-chloro-4-{[(4-fluorophenyl)carbonyl]amino}phenyl)-3-[[(trans-4-methylcyclohexyl)carbonyl](1-methylethyl)amino]-2-thiophenecarboxylate

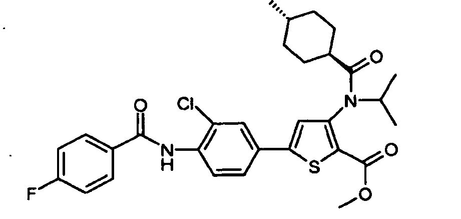

Intermediate 28 (200 mg) was dissolved in DCM (4 mL). 4-Fluorobenzoyl chloride (142 mg) and triphenylphosphine (295 mg) were added and the reaction mixture was stirred at 45°C for 2 days. The mixture was partitioned with sodium bicarbonate solution, separated, dried using a hydrophobic frit and evaporated *in vacuo.* The crude material was purified by ISCO Companion^{™} silica chromatography, eluting with a gradient 5-100% EtOAc in cyclohexane to give the title compound.
MS calcd for (C₃₀H₃₂CIFN₂O₄S + H)⁺: 571/573
MS found (electrospray): (M+H)⁺ = 571/573

### Intermediate 32

### Methyl 4-[[(trans-4-methylcyclohexyl)carbonyl](1-methylethyl)amino]-5'-nitro-2,2'-bithiophene-5-carboxylate

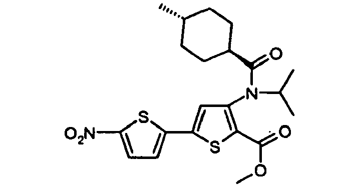

2-Bromo-5-nitrothiophene (2.2 g), {4-[[(*trans*-4-methylcyclohexyl)carbonyl](1-methylethyl)amino]-5-[(methyloxy)carbonyl]-2-thienyl}boronic acid (4.0 g, a synthesis of which is described as Intermediate 4) and cesium fluoride (8.0 g) were dissolved in DME/water (100 mL, 1:1), then tetrakis(triphenylphosphine)palladium(0) (616 mg) was added. The reaction was heated at 90°C for 2 h and was then cooled and partitioned between EtOAc and water. The organic phases were separated, dried using a hydrophobic frit and evaporated *in vacuo.* The crude material was purified by ISCO Companion^{™} silica chromatography, eluting with a gradient 5-60% EtOAc in cyclohexane to give the title compound.
MS calcd for (C₂₁H₂₆N₂O₅S₂ + H)⁺: 541
MS found (electrospray): (M+H)⁺ = 541

### Intermediate 33

### Methyl 5'-amino-4-[[(trans-4-methylcyclohexyl)carbonyl](1-methylethyl)amino]-2,2'-bithiophene-5-carboxylate

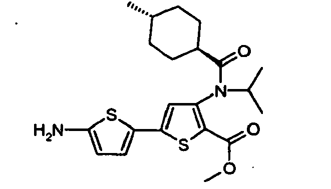

Intermediate 32 (3.2 g) was dissolved in ethanol (24 mL) and glacial acetic acid (8 mL). Iron powder (1.4 g) was added and the reaction was stirred at 70°C under nitrogen for 2 h. The reaction was cooled and evaporated *in vacuo*. The residue was basified with sodium bicarbonate which was then extracted with EtOAc (x 2). The combined organic phases were dried by passing through a hydrophobic frit and were evaporated *in vacuo* to give the title compound.
MS calcd for (C₂₁H₂₈N₂O₃S₂ + H)⁺: 421
MS found (electrospray): (M+H)⁺ = 421

### Intermediate 34

### Methyl 4-[[(trans-4-methylcyclohexyl)carbonyl](1-methylethyl)amino]-5'-[(2-pyridinylcarbonyl)amino]-2,2'-bithiophene-5-carboxylate

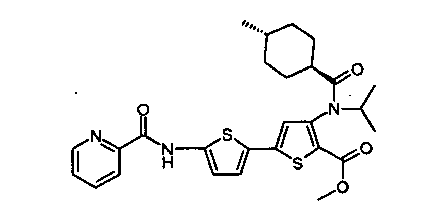

To a solution of 2-pyridinecarboxylic acid (72 mg) in DMF (6 mL) was added DIPEA (0.407 mL) and HATU (245 mg). The reaction was stirred at room temperature for 30 mins. Intermediate 33 (300 mg) was added and the reaction was stirred at room temperature overnight. The mixture was evaporated *in vacuo* and the residue was partitioned between DCM and water. The organic phases were separated, dried using a hydrophobic frit and evaporated *in vacuo.* The crude material was purified by ISCO Companion^{™} silica chromatography, eluting with a gradient 5-100% EtOAc in cyclohexane to give the title compound.
MS calcd for (C₂₇H₃₁N₃O₄S₂ + H)⁺: 526
MS found (electrospray): (M+H)⁺= 526

### Intermediate 35

### Methyl 3-[[(trans-4-methylcyclohexyl)carbonyl](1-methylethyl)amino]-5-(4-{[(1-methyl-1H-pyrazol-4-yl)carbonyl]amino}phenyl)-2-thiophenecarboxylate

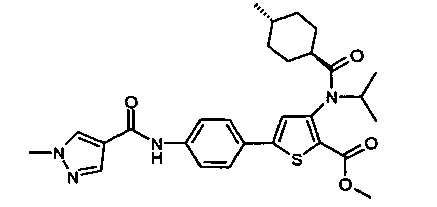

1-Methyl-1*H*-pyrazole-4-carboxylic acid (91 mg) was dissolved in DMF (5 mL). HATU (274 mg) and DIPEA (0.336 mL) were added and the mixture was stirred at room temperature for 15 mins. Methyl 5-(4-aminophenyl)-3-[[(*trans*-4-methylcyclohexyl)carbonyl](1-methylethyl)amino]-2-thiophenecarboxylate (200 mg, a synthesis of which is described as Intermediate 9) was added and the reaction was stirred at 45°C overnight. The reaction was cooled and evaporated *in vacuo*. The residue was partitioned between DCM and sodium bicarbonate solution. The organic phases were separated, dried using a hydrophobic frit. The crude material was purified by ISCO Companion^{™} silica chromatography, eluting with a gradient 5-100% EtOAc/cyclohexane to give the title compound.
MS calcd for (C₂₈H₃₄N₄O₄S + H)⁺: 523
MS found (electrospray): (M+H)⁺= 523

### Intermediate 36

### Methyl 3-[[(trans-4-methylcyclohexyl)carbonyl](1-methylethyl)amino]-5-(4-{[(5-methyl-3-isoxazolyl)carbonyl]amino}phenyl)-2-thiophenecarboxylate

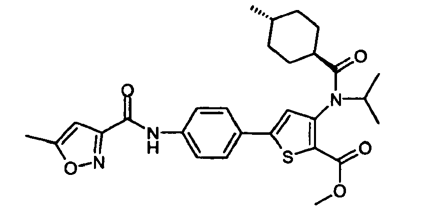

Methyl 5-(4-aminophenyl)-3-[[(*trans*-4-methylcyclohexyl)carbonyl](1-methylethyl)amino]-2-thiophenecarboxylate (200 mg, a synthesis of which is described as Intermediate 9) was dissolved in DCM (5 mL), then triphenylphosphine (317 mg) and 5-methyl-3-isoxazolecarbonyl chloride (140 mg) were added. The reaction was stirred at 45°C for 4 h. The mixture was diluted with DCM and sodium bicarbonate solution was added. The mixture was stirred at room temperature for 5 mins, then the organic phases were separated, dried using a hydrophobic frit and evaporated *in vacuo.* The crude material was purified by ISCO Companion^{™} silica chromatography, eluting with a gradient 5-100% EtOAc in cyclohexane to give the title compound.
MS calcd for (C₂₈H₃₃N₃O₅S + H)⁺: 524
MS found (electrospray): (M+H)⁺ = 524

### Intermediate 37

### Methyl 4-[[(trans-4-methylcyclohexyl)carbonyl](1-methylethyl)amino]-5'-[(1,3-thiazol-4-ylcarbonyl)amino]-2,2'-bithiophene-5-carboxylate

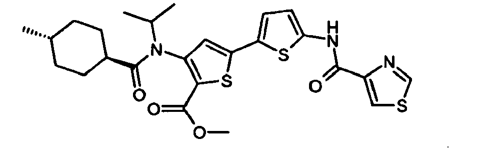

1,3-Thiazole-4-carboxylic acid (40.9 mg) was dissolved in DMF (5 mL). HATU (132 mg) and DIPEA (150 mg) were added and the mixture was stirred at room temperature for 15 mins. Methyl 5'-amino-4-[[(*trans*-4-methylcyclohexyl)carbonyl](1-methylethyl)amino]-2,2'-bithiophene-5-carboxylate (130 mg, a synthesis of which is described as Intermediate 33) was added and the reaction mixture was stirred at room temperature over a weekend. The reaction was evaporated *in vacuo* and was partitioned between DCM and sodium bicarbonate solution. The organic phases were dried by passing through a hydrophobic frit and were evaporated *in vacuo*. The crude material was purified by ISCO Companion^{™} silica chromatography, eluting with a gradient 5-100% EtOAc in cyclohexane to give the title compound.
MS calcd for (C₂₅H₂₉N₃O₄S₃ + H)⁺: 532
MS found (electrospray): (M+H)⁺ = 532

### Intermediate 38

### Methyl 5-[4-({[2-({[(1,1-dimethylethyl)oxy]carbonyl}amino)-1,3-thiazol-4-yl]carbonyl}amino)phenyl]-3-[[(trans-4-methylcyclohexyl)carbonyl](1-methylethyl)amino]-2-thiophenecarboxylate

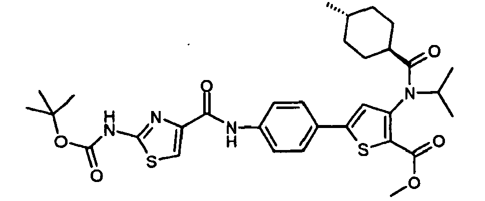

2-({[(1,1-Dimethylethyl)oxy]carbonyl}amino)-1,3-thiazole-4-carboxylic acid (212 mg) was dissolved in DMF (6 mL). DIPEA (0.505 mL) and HATU (330 mg) were added and the reaction was stirred at room temperature for 15 mins. Methyl 5-(4-aminophenyl)-3-[[(*trans*-4-methylcyclohexyl)carbonyl](1-methylethyl)amino]-2-thiophenecarboxylate (300 mg, a synthesis of which is described as Intermediate 9) was added and the reaction mixture was stirred at room temperature for 6 h. The mixture was evaporated *in vacuo* and the residue was partitioned between DCM and sodium bicarbonate solution. The organic phases were separated, dried using a hydrophobic frit and evaporated *in vacuo.* The crude material was purified by ISCO Companion^{™} silica chromatography, eluting with a gradient 5-100% EtOAc in cyclohexane to give the title compound.
MS calcd for (C₃₂H₄₀N₄O₆S₂ + H)⁺: 641
MS found (electrospray): (M+H)⁺ = 641

### Intermediate 39

### Methyl 5-(4-{[(2-amino-1,3-thiazol-4-yl)carbonyl]amino}phenyl)-3-[[(trans-4-methylcyclohexyl)carbonyl](1-methylethyl)amino]-2-thiophenecarboxylate

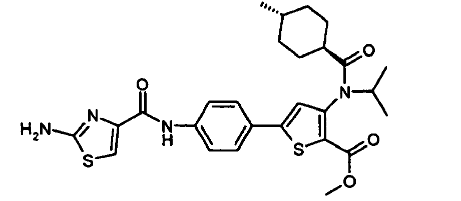

To Intermediate 38 (369 mg) was added a solution of 4M HCl (5 mL in 1,4-dioxane). DCM (10 mL) was added and the reaction mixture was stirred at room temperature for 2 days. The reaction was evaporated *in vacuo* and the residue was dissolved in DCM (5 mL) and TFA (1 mL). The mixture was stirred over a weekend and was evaporated *in vacuo*. The residue was partitioned between DCM and sodium bicarbonate solution. The organic phases were separated, dried using a hydrophobic frit and evaporated *in vacuo* to give the title compound.
MS calcd for (C₂₇H₃₂N₄O₄S₂ + H)⁺: 541
MS found (electrospray): (M+H)⁺ = 541

### Intermediate 40

### Methyl 5-(6-amino-3-pyridinyl)-3-[[(trans-4-methylcyclohexyl)carbonyl](1-methylethyl)amino]-2-thiophenecarboxylate

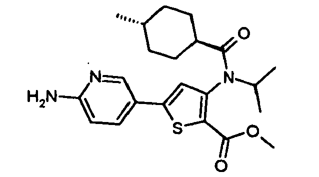

A mixture of 5-iodo-2-pyridinamine (1.2 g), {4-[[(*trans*-4-methylcyclohexyl)carbonyl](1-methylethyl)amino]-5-[(methyloxy)carbonyl]-2-thienyl}boronic acid (2.0 g, a synthesis of which is described as Intermediate 4), cesium fluoride (4.1 g) and tetrakis(triphenylphosphine)palladium(0) (314 mg) in DME/water (1:1, 30 mL) were heated at 100°C for 3 h under nitrogen. The mixture was then allowed to cool to room temperature. The mixture was partitioned between EtOAc and saturated sodium bicarbonate solution. The organic layer separated, washed with brine, dried (Na₂SO₄) and concentrated to give a brown gum. The crude material was purified by ISCO Companion^{™} silica chromatography, eluting with a gradient of 3-100% EtOAc in cyclohexane to give the title compound.
MS calcd for (C₂₂H₂₉N₃O₃S + H)⁺: 416
MS found (electrospray): (M+H)⁺ = 416

### Intermediate 41

### Methyl 3-[[(trans-4-methylcyclohexyl)carbonyl](1-methylethyl)amino]-5-{6-[(1,3-thiazol-4-ylcarbonyl)amino]-3-pyridinyl}-2-thiophenecarboxylate

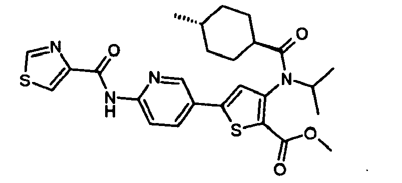

1,3-Thiazole-4-carboxylic acid (93 mg) was dissolved in DMF (4 mL). HATU (293 mg) and DIPEA (248 mg) were added and the mixture was stirred at room temperature for 10 mins. A solution of Intermediate 4 (200 mg) in DMF (2 mL) was added and the reaction mixture stirred at room temperature for 3 days. The reaction mixture was evaporated *in vacuo* and the residue partitioned between DCM and saturated sodium bicarbonate solution. The organic phase was separated and dried by passing through a hydrophobic frit and evaporated *in vacuo.* The crude material was purified by ISCO Companion^{™} silica chromatography, eluting with a gradient of 3-100% EtOAc in cyclohexane to give the title compound.
MS calcd for (C₂₆H₃₀N₄O₄S₂ + H)⁺: 526
MS found (electrospray): (M+H)⁺ = 526

### Intermediate 42

### Methyl 3-[[(trans-4-methylcyclohexyl)carbonyl](1-methylethyl)amino]-5-{6-[(2-pyridinylcarbonyl)amino]-3-pyridinyl}-2-thiophenecarboxylate

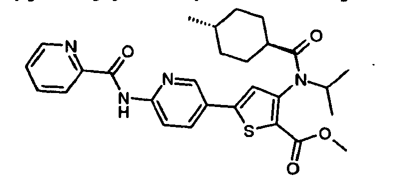

2-Pyridinecarboxylic acid (89 mg) was dissolved in DMF (4 mL). HATU (293 mg) and DIPEA (248 mg) were added and the mixture was stirred at room temperature for 10 mins. A solution of Intermediate 4 (200 mg) in DMF (2 mL) was added and the reaction mixture stirred at room temperature for 3 days. The reaction mixture was evaporated *in vacuo* and the residue partitioned between DCM and saturated sodium bicarbonate solution. The organic phase was separated and dried by passing through a hydrophobic frit and evaporated *in vacuo.* The crude material was purified by ISCO Companion^{™} silica chromatography, eluting with a gradient of 3-100% EtOAc in cyclohexane to give the title compound.
MS calcd for (C₂₈H₃₂N₄O₄S + H)⁺: 520
MS found (electrospray): (M+H)⁺ = 520

### Intermediate 43

### Methyl 3-[[(trans-4-methylcyclohexyl)carbonyl](1-methylethyl)amino]-5-{6-[(2-pyrazinylcarbony)]amino]-3-pyridinyl}-2-thiophenecarboxylate

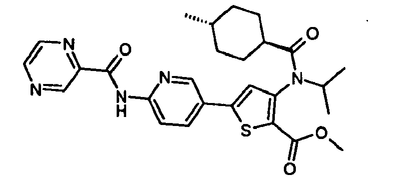

2-Pyrazinecarboxylic acid (89 mg) was dissolved in DMF (4 mL). HATU (293 mg) and DIPEA (248 mg) were added and the mixture was stirred at room temperature for 10 mins. A solution of Intermediate 4 (200 mg) in DMF (2 mL) was added and the reaction mixture stirred at room temperature for 3 days. The reaction mixture was evaporated *in vacuo* and the residue partitioned between DCM and saturated sodium bicarbonate solution. The organic phase was separated and dried by passing through a hydrophobic frit and evaporated *in vacuo.* The crude material was purified by ISCO Companion^{™} silica chromatography, eluting with a gradient of 3-100% EtOAc in cyclohexane to give the title compound.
MS calcd for (C₂₇H₃₁N₅O₄S + H)⁺: 521
MS found (electrospray): (M+H)⁺ = 521

### Intermediate 44

### Methyl 5-(6-{[(4-fluorophenyl)carbonyl]amino}-3-pyridinyl)-3-[[(trans-4-methylcyclohexyl)carbonyl](1-methylethyl)amino]-2-thiophenecarboxylate

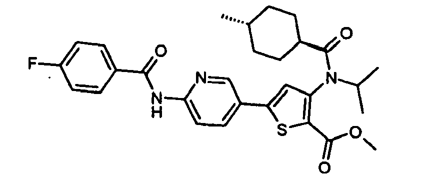

4-Fluorobenzoic acid (101 mg) was dissolved in DMF (4 mL). HATU (293 mg) and DIPEA (248 mg) were added and the mixture was stirred at room temperature for 10 mins. A solution of Intermediate 4 (200 mg) in DMF (2 mL) was added and the reaction mixture stirred at room temperature for 3 days. The reaction mixture was evaporated *in vacuo* and the residue partitioned between DCM and saturated sodium bicarbonate solution. The organic phase was separated and dried by passing through a hydrophobic frit and evaporated *in vacuo.* The crude material was purified by ISCO Companion^{™} silica chromatography, eluting with a gradient of 3-100% EtOAc in cyclohexane to give the title compound.
MS calcd for (C₂₉H₃₂FN₃O₄S + H)⁺: 537
MS found (electrospray): (M+H)⁺ = 537

### Intermediate 45

### Methyl 5-{6-[(2-furanylcarbonyl)amino]-3-pyridinyl}-3-[[(trans-4-methylcyclohexyl)carbonyl](1-methylethyl)amino]-2-thiophenecarboxylate

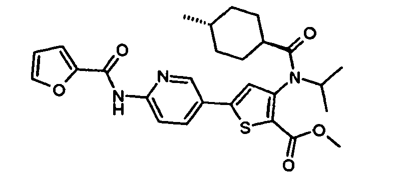

2-Furancarboxylic acid (81 mg) was dissolved in DMF (4 mL). HATU (293 mg) and DIPEA (248 mg) were added and the mixture was stirred at room temperature for 10 mins. A solution of Intermediate 4 (200 mg) in DMF (2 mL) was added and the reaction mixture stirred at room temperature for 3 days. The reaction mixture was evaporated *in vacuo* and the residue partitioned between DCM and saturated sodium bicarbonate solution. The organic phase was separated and dried by passing through a hydrophobic frit and evaporated *in vacuo.* The crude material was purified by ISCO Companion^{™} silica chromatography, eluting with a gradient of 3-100% EtOAc in cyclohexane to give the title compound.
MS calcd for (C₂₇H₃₁N₃O₅S + H)⁺: 509
MS found (electrospray): (M+H)⁺ = 509

### Intermediate 46

### Methyl 3-{[(trans-4-methylcyclohexyl)carbonyl]amino}-2-thiophenecarboxylate

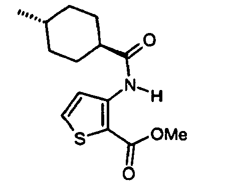

To methyl 3-amino-2-thiophenecarboxylate (22.6 g, 144 mmol) in dry DCM (400 mL) under nitrogen was added triethylamine (30 mL, 21.8 g, 216 mmol) dropwise, followed by *trans*-4-methylcyclohexanecarbonyl chloride (34.7 g, 216 mmol ) a synthesis of which is described as Intermediate 1) dropwise, maintaining the temperature below 30°C. The mixture was stirred overnight, and then poured into saturated sodium bicarbonate solution (-250 mL). The phases were separated and the aqueous phase washed twice with DCM. The combined organic fractions were washed successively with saturated sodium bicarbonate solution, water, and brine, and dried (Na₂SO₄). The solution was evaporated *in vacuo* and purified by ISCO Companion^{™} silica chromatography eluting with 0-30% EtOAc in cyclohexane to give the title compound.
MS calcd for (C₁₄H₁₉NO₃S + H)⁺: 282
MS found (electrospray): (M+H)⁺ = 282

### Intermediate 47

### Methyl 5-iodo-3-{[(trans-4-methylcyclohexyl)carbonyl]amino}-2-thiophenecarboxylate

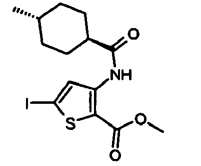

To dry diisopropylamine (3.0 mL) under nitrogen was added n-butyllithium (14.7 mL, 1 M) dropwise and the reaction stirred under nitrogen at room temperature for 2 h. This solution was cooled to -70° C then a solution of methyl 3-{[(*trans*-4-methylcyclohexyl)carbonyl]amino}-2-thiophenecarboxylate (2.00 g, a synthesis of which is described as Intermediate 46) in dry THF (15 mL) was added dropwise, keeping the temperature below -60°C. The reaction was stirred at -70°C under nitrogen for 1½ h, and then a solution of iodine (3.6 g) in dry THF (10 mL) was added dropwise. The reaction was stirred at room temperature under nitrogen for 18 h then poured into ammonium chloride solution (50 mL). The mixture was concentrated *in vacuo* to remove the THF, extracted with EtOAc (2 x 50 mL), dried using a hydrophobic frit and evaporated to dryness. The crude product was purified by 120 g silica Silicycle cartridge using ISCO Companion^{™} chromatography eluted with 0 - 25% EtOAc in cyclohexane to give the title compound.
MS calcd for (C₁₄H₁₈1NO₃S + H)⁺: 408
MS found (electrospray): (M+H)⁺ = 408

### Intermediate 48

### Methyl 3-{(2,2-difluoroethyl)[(trans-4-methylcyclohexyl)carbonyl]amino}-5-iodo-2-thiophenecarboxylate

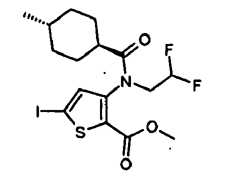

A mixture of Intermediate 47 (468 mg) and sodium hydride (69 mg) in dry DMF (1.5 mL) was stirred under nitrogen over an ice-bath for 1 h. 2,2-Difluoroethyl trifluoromethanesulphonate (368 mg) was added and the reaction heated to 70°C under nitrogen for 2 h, then stirred at room temperature for 18 h. The reaction was poured into citric acid solution (15 mL), extracted twice with EtOAc (2 x 20 mL), dried using a hydrophobic frit and evaporated to dryness to give an orange solid. The crude product was purified by 12 g silica Silicycle cartridge using ISCO Companion^{™} chromatography eluted with 0-20% EtOAc in cyclohexane to give the title compound.
MS calcd for (C₁₆H₂₀F₂INO₃S + H)⁺: 472
MS found (electrospray): (M+H)⁺ = 472

### Intermediate 49

### {4-[(1-Methylethyl)amino]-5-[(methyloxy)carbonyl]-2-thienyl}boronic acid

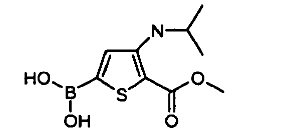

A stirred solution of {4-[[(*trans*-4-methylcyclohexyl)carbonyl](1-methylethyl)amino]-5-[(methyloxy)carbonyl]-2-thienyl}boronic acid (0.12 g, a synthesis of which is described as intermediate 4) in TFA (3 mL) was heated at reflux with stirring for 1.5 h. The reaction was evaporated *in vacuo*. The material was purified by MDAP HPLC to give the title compound.
MS calcd for (C₉H₁₄BNO₄S + H)⁺: 244
MS found (electrospray): (M+H)⁺ = 244

### Intermediate 50

### Methyl 3-{(2,2-difluoroethyl)[(trans-4-methylcyclohexyl)carbonyl]amino}-5-{4-[(1,3-thiazol-4-ylcarbonyl)amino]phenyl}-2-thiophenecarboxylate

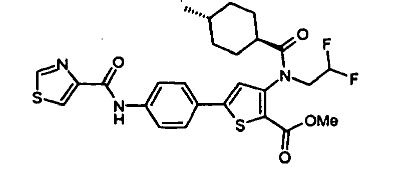

Intermediate 48 (109 mg), methyl 3-{ethyl[(trans-4-methylcyclohexyl)carbonyl]amino}-5-{4-[(1,3-thiazol-4-ylcarbonyl)amino]phenyl}-2-thiophenecarboxylate [a synthesis of which is described above as Intermediate 54 (92 mg)], sodium carbonate (74 mg) and tetrakis(triphenylphosphino)palladium (22 mg) in 1,4-dioxane (1.5 mL) and water (0.5 mL) were heated in a microwave at 300 W and 110°C for 15 mins. The reaction was evaporated to dryness, water (10 mL) was added, the mixture extracted with DCM (2 x 20 mL), the combined organic layers dried using a hydrophobic frit and evaporated to dryness. The crude product was purified by 12 g silica Silicycle cartridge using ISCO Companion^{™} chromatography eluted with 0 - 100% EtOAc in cyclohexane to give the title compound.
MS calcd for (C₂₆H₂₇F₂N₃O₄S₂ + H)⁺: 548
MS found (electrospray): (M+H)⁺ = 548

### Intermediate 51

### Methyl 3-{ethyl[(trans-4-methylcyclohexyl)carbonyl]amino}-2-thiophenecarboxylate

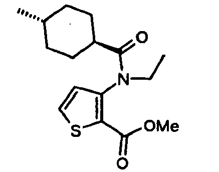

To a stirred suspension of methyl 3-{[(*trans*-4-methylcyclohexyl)carbonyl]amino}-2-thiophenecarboxylate (5.03 g, a synthesis of which is described above as Intermediate 49) in dry DMF (60 mL) under nitrogen was added sodium hydride (0.79 g, 60% dispersion in mineral oil) portion-wise over 5 mins. The mixture was stirred for 2 h at room temperature before iodoethane (1.59 mL) was added. The reaction was allowed to stir at room temperature for 23 h and was evaporated *in vacuo.* The residue was partitioned between DCM and water and the organics were separated using a hydrophobic frit. The organic phases were evaporated *in vacuo* and the crude material was purified by ISCO Companion^{™} silica chromatography, eluting with a gradient 0-30% EtOAc in cyclohexane to give the title compound.
MS calcd for (C₁₆H₂₃NO₃S + H)⁺: 310
MS found (electrospray): (M+H)⁺ = 310

### Intermediate 52

### Methyl 3-{ethyl[(trans-4-methylcyclohexyl)carbonyl]amino}-5-iodo-2-thiophene carboxylate

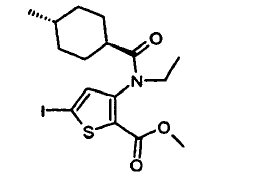

A solution of LDA (1.8M solution in THF/heptane/ethyl benzene, 6.1 mL) was cooled to -76°C under an atmosphere of nitrogen. A solution of Intermediate 51 (1.14 g) in dry THF (12 mL) was added dropwise over 30 mins, keeping the internal temperature below -70°C. The reaction mixture was then stirred for 2 h. Iodine (1.87 g) in dry THF (12 mL) was then added dropwise over 30 mins keeping the internal temperature below -70°C. The reaction mixture was stirred for 1 h before being slowly quenched with saturated ammonium chloride solution (2 mL) and aqueous sodium thiosulphate solution (1.0 g in 12 mL of water). The reaction was allowed to warm to room temperature and was diluted with EtOAc and water. The aqueous phase was separated off and was extracted with EtOAc (x 2). The organic phases were combined and were washed with water, dried over sodium sulphate and evaporated *in vacuo*. The crude material was purified using a silica Biotage cartridge, eluting with 20% EtOAc in cyclohexane to give the title compound.
MS calcd for (C₁₆H₂₂INO₃S + H)⁺ : 436
MS found (electrospray) : (M+H)⁺ = 436

### Intermediate 53

### N-[4-(4,4,5,5-Tetramethyl-1,3,2-dioxaborolan-2-yl)phenyl]-1,3-thiazole-4-carboxamide

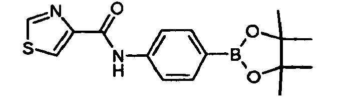

To thiazole 4-carboxylic acid (2.5 g) was added dry DMF (68 mL), DIPEA (6.67 mL) and HATU. (13.1 g). The reaction was stirred at room temperature for 1 hour under a nitrogen atmosphere. 4-(4,4,5,5-Tetramethyl-1,3,2-dioxaborolan-2-yl)aniline (5.1 g) was added and stirring continued for 24 hours. The cooled reaction was partitioned between EtOAc and saturated sodium bicarbonate. The phases were separated and the combined organic fractions washed further with saturated sodium bicarbonate, then water, then 2N HCl (x2) and then saturated brine. The organic fraction was passed through a hydrophobic frit and concentrated. The crude material was purified using a 330 g silica Biotage cartridge, eluting a 0% to 100% EtOAc in cyclohexane gradient to give the title compound.
MS calcd for (C₁₆H₁₉BN₂O₃S + H)⁺: 331
MS found (electrospray) : (M+H)⁺ = 331

### Intermediate 54

### Methyl 3-{ethyl[(trans-4-methylcyclohexyl)carbonyl]amino}-5-{4-[(1,3-thiazol-4-ylcarbonyl)amino]phenyl}-2-thiophenecarboxylate

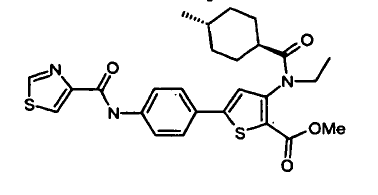

To Intermediate 52 (100 mg) was added Intermediate 53 (91 mg), potassium phosphate (K₃PO₄), tetrakis(triphenylphosphine)palladium(0) (26.5 mg), DMF (2 mL) and water (0.5 mL). The reaction was stirred at 80°C under a nitrogen atmosphere for 16 hours. The cooled reaction was partitioned between DCM and water. The phases were separated with a hydrophobic frit and the organic phases were concentrated. The crude material was purified using a 40 g silica Biotage cartridge, eluting a 0% to 100% EtOAc in cyclohexane gradient to give the title compound.
MS calcd for (C₂₆H₂₉N₃O₄S₂ + H)⁺ : 512
MS found (electrospray) : (M+H)⁺ = 512

### Intermediate 55

### Ethyl 4-methyl-3-cyclohexene-1-carboxylate

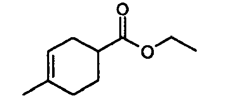

A mixture of ethyl 2-propenoate (3.70 g) and_2-methyl-1,3-butadiene (5.03 g) was added to a preformed complex of [AlCl₃ (0.24 g)+ 2THF (0.26 g)] with stirring at room temperature under air atmosphere. After 3 days the reaction mixture was diluted with DCM, filtered through a hydrophobic frit and evaporated *in vacuo* to afford the title compound.
¹H NMR (CDCl₃): δ 5.38 (1H, bs), 4.13 (2H, q), 2.52-2.42 (1H, m), 2.27-2.17 (2H, m), 2.11-1.91 (4H, m), 1.74-1.54 (3H, m, overlapped with H₂O), 1.25 (3H, t).

### Intermediate 56

### Ethyl cis-4-fluoro-4-methylcyclohexanecarboxylate

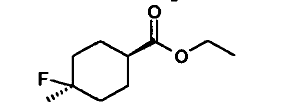

To the stirred intermediate 55 (0.51 g) in a plastic vial was added hydrogen fluoride-pyridine (1 mL). The reaction was stirred at room temperature for 1 h. DCM was added followed by dropwise addition of saturated aqueous sodium bicarbonate solution followed by solid sodium bicarbonate. The organic phase was separated using a hydrophobic frit, washed with 2N HCl (x 2) and was evaporated *in vacuo*. The crude material was purified using a 40 g silica Reside cartridge, eluting with a gradient of EtOAc in cyclohexane to give the title compound.
¹H NMR (CDCl₃): δ 4.14 (2H, q), 2.30-2.17 (1H, m), 2.02-1.90 (2H, m), 1.90-1.75 (4H, m), 1.47-1.32 (2H, m, overlapped), 1.34 (3H, d), 1.26 (3H, t).

### Intermediate 57

### cis-4-Fluoro-4-methylcyclohexanecarboxylic acid

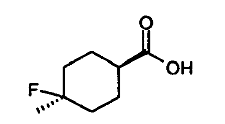

Intermediate 56 (0.25 g) in THF (2 mL) and ethanol (2 mL) was treated with 2N sodium hydroxide (1 mL). The resultant solution was stirred for 20 h. The mixture was evaporated *in vacuo* and the residue was partitioned between 2N HCl and DCM. The organic phase was separated using a hydrophobic frit and evaporated *in vacuo* to give the title compound.
¹H NMR (CDCl₃): δ 2.34-2.23 (1H, m), 2.05-1.93 (2H, m), 1.92-1.77 (4H, m), 1.52-1.32 (2H, m overlapped), 1.35 (3H, d), exchangeable not seen.

### Intermediate 58

### cis-4-Fluoro-4-methylcyclohexanecarbonyl chloride

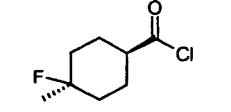

Intermediate 57 (0.2 g) was dissolved in oxalyl chloride (2 mL). *N,N*-diethylformamide (1 drop ) was added with stirring. The reaction mixture was allowed to stand at room temperature for 21 h then evaporated *in vacuo* to afford the title compound.
¹H NMR (CDCl₃): δ 2.77-2.63 (1H, bt), 2.11-1.31 (8H,m, overlapped), 1.36 (3H, d).

### Intermediate 59

### Methyl 3-[(1-methylethyl)amino]-5-{4-[(1,3-thiazol-4-ylcarbonyl)amino]phenyl}-2-thiophenecarboxylate

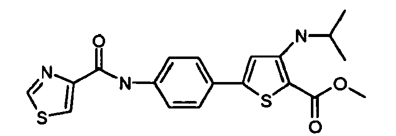

To a stirred mixture of *N*-(4-iodophenyl)-1,3-thiazole-4-carboxamide (0.35 g, a synthesis of which is described as Intermediate 5), tetrakis(triphenylphosphine)palladium (0) (0.124 g), cesium fluoride (1.137 g) in DME (12 mL) and water (12 mL) at 80°C under nitrogen was added Intermediate 49 (0.26 g) dropwise in DME (15 mL) over 15 min. The reaction mixture was heated at 80°C for 15 h. The reaction was cooled to room temperature and was then partitioned between saturated aqueous sodium bicarbonate solution and ethyl acetate. The organic was separated, dried over sodium sulphate and evaporated *in vacuo*. The crude material was purified using a 80 g silica Reside cartridge, eluting with a gradient of 0-25% EtOAc in cyclohexane to give the title compound.
MS calcd for (C₁₉H₁₉N₃O₃S₂ + H)⁺ : 402
MS found (electrospray) : (M+H)⁺ = 402

### Intermediate 60

### Methyl 3-[[(cis-4-fluoro-4-methylcyclohexyl)carbonyl](1-methylethyl)amino]-5-{4-[(1,3-thiazol-4-ylcarbonyl)amino]phenyl}-2-thiophenecarboxylate

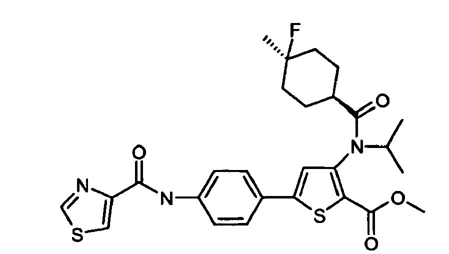

To a stirred solution of Intermediate 59 (0.22 g) in DCE (8.3 mL) was added Intermediate 58 (0.147 g) and the reaction mixture was heated at 80°C for 19 h. Further Intermediate 58 (0.08 g) was added and the reaction mixture was heated at 90°C for 4 h then allowed to cool to room temperature. The reaction mixture was evaporated *in vacuo*. The crude material was purified using a 40 g silica Reside cartridge, eluting with a gradient of 0 - 50% EtOAc in cyclohexane to give the title compound.
MS calcd for (C₂₇H₃₀FN₃O₄S₂ + H)⁺ : 544
MS found (electrospray) : (M+H)⁺ = 544

### Intermediate 61

### Methyl 3-{(cyclopropylmethyl)[(trans-4-ethylcyclohexyl)carbonyl]amino}-2-thiophenecarboxylate

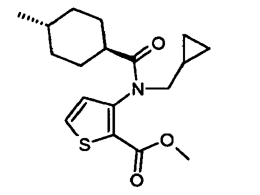

To a solution of methyl 3-[(cydopropylmethyl)amino]-2-thiophenecarboxylate (1.83 g, a synthesis of which is described as Intermediate 17) in DCE (35 mL) was added *trans*-4-methylcyclohexanecarbonyl chloride (0.815 g, a synthesis of which is described as Intermediate 1). The reaction mixture was then heated at 86°C under nitrogen for 18 h and was then allowed to cool to room temperature. The reaction was then quenched with saturated sodium bicarbonate solution. The organic layer was separated and the aqueous extracted with DCM. The combined organic phases were passed through a hydrophobic frit and were evaporated *in vacuo* to give an oil (3.03 g). The crude material was purified using a silica Biotage cartridge, eluting with 0-100% ethyl acetate in cyclohexane to give the title compounds.
MS calcd for (C₁₈H₂₅NO₃S + H)⁺ : 336
MS found (electrospray) : (M+H)⁺ = 336

### Intermediate 62

### Methyl 3-{(cyclopropylmethyl)[(trans-4-methylcyclohexyl)carbonyl]amino}-5-iodo-2-thiophenecarboxylate

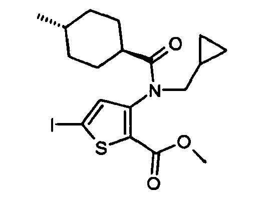

A solution of LDA (1.8 M solution in THF/heptane/ethyl benzene, 12.1 mL) was cooled to - 78°C under an atmosphere of nitrogen. A solution of Intermediate 61 (2.44 g) in dry THF (30 mL) was added dropwise over 15 mins, keeping the internal temperature below -70°C. The reaction mixture was then stirred for 30 mins at -78°C. Iodine (3.68 g) in dry THF (30 mL) was then added dropwise over 30 mins keeping the internal temperature below -70°C. The reaction mixture was stirred for 10 mins before being slowly quenched with saturated ammonium chloride solution (45 mL) and 5% aqueous sodium thiosulphate solution. The reaction was allowed to warm to room temperature and was diluted with EtOAc and water. The aqueous phase was separated and was extracted with EtOAc (x 2). The organic phases were combined, dried through a hydrophobic frit and evaporated *in vacuo*. The crude material was purified using a silica Biotage cartridge, eluting with 0-100% EtOAc in cyclohexane to give the title compound.
MS calcd for (C₁₈H₂₄INO₃S + H)⁺ : 462
MS found (electrospray) : (M+H)⁺ = 462

### Intermediate 63

### Methyl 3-{(cyclopropylmethyl)[(trans-4-methylcyclohexyl)carbonyl]amino}-5-[4-({[(1,1-dimethylethyl)oxy]carbonyl}amino)phenyl]-2-thiophenecarboxylate

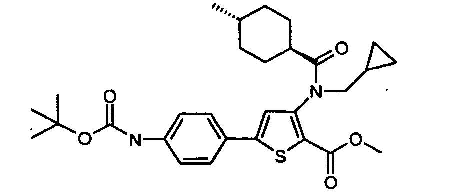

[4-({[(1,1-Dimethylethyl)oxy]carbonyl}amino)phenyl]boronic acid (1.23 g), Intermediate 62 (2 g) and sodium carbonate (1.84 g) were dissolved in DMF (30 mL) and water (5 mL). Tetrakis(triphenylphosphine)palladium(0) (0.50 g) was added and the reaction mixture was stirred at 100°C under nitrogen for 2 h. The reaction mixture was evaporated *in vacuo* and was partitioned between DCM and water. The aqueous layer was washed with DCM (2 x -25 mL), the organic phase was combined, dried by passing through a hydrophobic frit and evaporated *in vacuo*. The crude material was loaded onto a 330 g silica ISCO cartridge eluting with a gradient of 5 - 100% EtOAc in cyclohexane to give the title compound.
MS calcd for (C₂₉H₃₈N₂O₅S + H)⁺ : 527
MS found (electrospray) : (M+H)⁺ = 527

### Intermediate 64

### Methyl 5-(4-aminophenyl)-3-{(cyclopropylmethyl)[(trans-4-methylcyclohexyl)carbonyl]amino}-2-thiophenecarboxylate

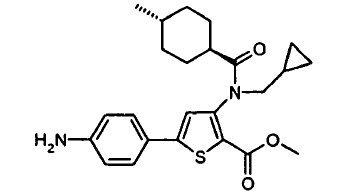

Intermediate 63 (2.2 g) was dissolved in DCM (30 mL). TFA (5 mL) was added and the reaction mixture was stirred under nitrogen at room temperature for 2 h. The reaction mixture was evaporated *in vacuo* and was partitioned between saturated sodium bicarbonate solution and DCM. The organic phase was collected and dried by passing through a hydrophobic frit and evaporated *in vacuo* to give the title compound.
MS calcd for (C₂₄H₃₀N₂O₃S + H)⁺ : 427
MS found (electrospray) : (M+H)⁺ = 427

### Intermediate 65

### Methyl 3-{(cyclopropylmethyl)[(trans-4-methylcyclohexyl)carbonyl]amino}-5-{4-[(2-pyridinylcarbonyl)amino]phenyl}-2-thiophenecarboxylate

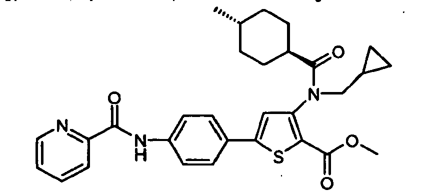

2-Pyridinecarboxylic acid (47.6 mg) was dissolved in a solution of DMF (3 mL), DIPEA (245 µL) and HATU (201 mg), and the reaction was stirred at room temperature for 15 mins. Intermediate 64 (150 mg) in DMF (1 mL) was added and the mixture was stirred at 50°C under nitrogen for 22 h. The reaction mixture was evaporated *in vacuo* and was partitioned between DCM and saturated sodium bicarbonate solution. The organic layer was collected and dried by passing through a hydrophobic frit and evaporated *in vacuo*. The crude material was purified using a 12 g silica ISCO cartridge eluting with a gradient of 5 - 100% EtOAc in cyclohexane to give the title compound.
MS calcd for (C₃₀H₃₃N₃O₄S + H)⁺ : 532
MS found (electrospray) : (M+H)⁺ = 532

### Intermediate 66

### Methyl 3-{(cyclopropylmethyl)[(trans-4-methylcyclohexyl)carbonyl]amino}-5-{4-[(2-pyrazinylcarbonyl)amino]phenyl}-2-thiophenecarboxylate

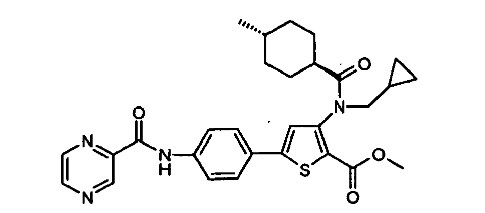

2-Pyrazinecarboxylic acid (48 mg) was dissolved in a solution of DMF (3 mL), DIPEA (245 µL) and HATU (201 mg), the reaction was stirred at room temperature for 15 mins. Intermediate 64 (150 mg) in DMF (1 mL) was added and the reactions were stirred at 50°C under nitrogen for 22 h. The reaction mixture was evaporated *in vacuo* and was partitioned between DCM and saturated sodium bicarbonate solution. The organic phase was collected and dried by passing through a hydrophobic frit and evaporated *in vacuo*. The crude material was purified using a 12 g silica ISCO cartridge eluting with a gradient of 5 - 100% EtOAc in cyclohexane to give the title compound.
MS calcd for (C₂₉H₃₂N₄O₄S + H)⁺ : 533
MS found (electrospray) : (M+H)⁺ = 533

### Intermediate 67

### Methyl 3-{(cyclopropylmethyl)[(trans-4-methylcyclohexyl)carbonyl]amino}-5-(4-{[(1-methyl-1H-imidazol-4-yl)carbonyl]amino}phenyl)-2-thiophenecarboxylate

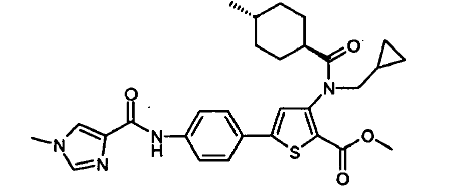

1-Methyl-1*H*-imidazole-4-carboxylic acid (48.8 mg) was dissolved in a solution of DMF (3 mL), DIPEA (182 µL) and HATU (201 mg), and the reaction was stirred at room temperature for 15 mins. Intermediate 64 (150 mg) in DMF (1 mL) was added and the mixture was stirred at 50°C under nitrogen for 22 h. The reaction mixture was evaporated *in vacuo* and was partitioned between DCM and saturated sodium bicarbonate solution. The organic phase was collected and dried by passing through a hydrophobic frit and evaporated *in vacuo*. The crude material was purified using a 12 g silica ISCO cartridge eluting with a gradient of 5 - 100% EtOAc in cyclohexane to give the title compound.
MS calcd for (C₂₉H₃₄N₄O₄S + H)⁺ : 535
MS found (electrospray) : (M+H)⁺ = 535

### Intermediate 68

### Methyl 3-{(cyclopropylmethyl)[(trans-4-methylcyclohexyl)carbonyl]amino}-5-{4-[(3-pyridazinylcarbonyl)amino]phenyl}-2-thiophenecarboxylate

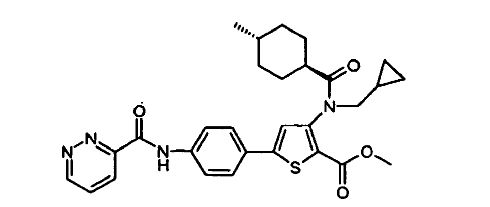

3-Pyridazinecarboxylic acid (48 mg) was dissolved in a solution of DMF (3 mL), DIPEA (182 µL) and HATU (201 mg), the reaction was stirred at room temperature for 15 mins. Intermediate 64 (150 mg) in DMF (1 mL) was added and the mixture was stirred at 50°C under nitrogen for 22 h. The reaction mixture was evaporated *in vacuo* and was partitioned between DCM and saturated sodium bicarbonate solution. The organic phase was collected and dried by passing through a hydrophobic frit and evaporated *in vacuo*. The crude material was purified using a 12 g silica ISCO cartridge eluting with a gradient of 5 - 100% EtOAc in cyclohexane to give the title compound.
MS calcd for (C₂₉H₃₂N₄O₄S + H)⁺ : 533
MS found (electrospray) : (M+H)⁺ = 533

### Intermediate 69

### Methyl 3-{(cyclopropylmethyl)[(trans-4-methylcyclohexyl)carbonyl]amino}-5-(4-{[(1-methyl-1H-pyrazol-3-yl)carbonyl]amino}phenyl)-2-thiophenecarboxylate

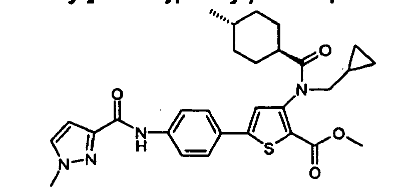

1-Methyl-1*H*-pyrazole-3-carboxylic acid (48.8 mg) was dissolved in a solution of DMF (3 mL), DIPEA (182 µL) and HATU (201 mg), the reaction was stirred at room temperature for 15 mins. Intermediate 64 (150 mg) in DMF (1 mL) was added and the reactions were stirred at 50°C under nitrogen for 22 h. The reaction mixture was evaporated *in vacuo* and was partitioned between DCM and saturated sodium bicarbonate solution. The organic phase was collected and dried by passing through a hydrophobic frit and evaporated *in vacuo*. The crude material was purified using a 12 g silica ISCO cartridge eluting with a gradient of 5 - 100% EtOAc in cyclohexane to give the title compound.
MS calcd for (C₂₉H₃₄N₄O₄S + H)⁺ : 535
MS found (electrospray) : (M+H)⁺ = 535

### Intermediate 70

### Methyl 3-{(cyclopropylmethyl)[(trans-4-methylcyclohexyl)carbonyl]amino}-5-(4-{[(1,5-dimethyl-1H-pyrazol-3-yl)carbonyl]amino}phenyl)-2-thiophenecarboxylate

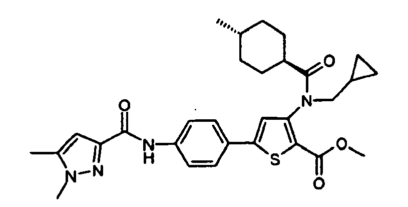

1,5-Dimethyl-1*H*-pyrazole-3-carboxylic acid (54.2 mg) was dissolved in a solution of DMF (3 mL), DIPEA (182 µL) and HATU (201 mg), the reaction was stirred at room temperature for 15 mins. Intermediate 64 (150 mg) in DMF (1 mL) was added and the reactions were stirred at 50°C under nitrogen for 22 h. The reaction mixture was evaporated *in vacuo* and was partitioned between DCM and saturated sodium bicarbonate solution. The organic phase was collected and dried by passing through a hydrophobic frit and evaporated *in vacuo*. The crude material was purified using a 12 g silica ISCO cartridge eluting with a gradient of 5 - 100% EtOAc in cyclohexane to give the title compound.
MS calcd for (C₃₀H₃₆N₄O₄S + H)⁺ : 549
MS found (electrospray) : (M+H)⁺ = 549

### Intermediate 71

### N-[4-Bromo-2-(trifluoromethyl)phenyl]-1,3-thiazole-4-carboxamide

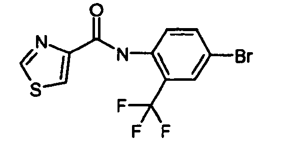

To 4-thiazolecarboxylic acid (300 mg) and HATU (1.06 g) in dry DCM (10 mL) was added DIPEA (1.2 mL) and the reaction mixture was stirred at room temperature under nitrogen for 30 mins. 4-Bromo-2-(trifluoromethyl)aniline (0.33 mL) was added and the reaction mixture was heated at reflux for 20 h. The reaction mixture was evaporated *in vacuo* and then purified using a 12 g silica Silicycle cartridge eluting with a gradient of 0 - 100% EtOAc in cyclohexane to give the title compound.
MS calcd for (C₁₁H₆Br F₃N₂OS + H)⁺ : 351/353
MS found (electrospray) : (M+H)⁺ = 351/353

### Intermediate 72

### Methyl 3-[[(trans-4-methylcyclohexyl)carbonyl](1-methylethyl)amino]-5-[4-[(1,3-thiazol-4-ylcarbonyl)amino]-3-(trifluoromethyl)phenyl]-2-thiophenecarboxylate

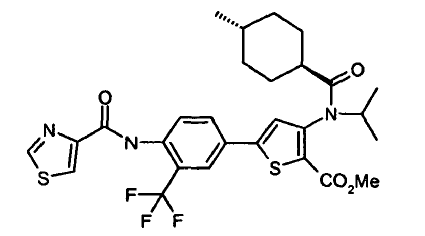

A mixture of {4-[[(*trans*-4-methylcyclohexyl)carbonyl](1-methylethyl)amino]-5-[(methyloxy)carbonyl]-2-thienyl}boronic acid (145 mg, a synthesis of which is described as Intermediate 4), Intermediate 71 (200 mg), sodium carbonate (100 mg) and tetrakis(triphenylphosphine)palladium (0) (36.2 mg) in 1,4-dioxane (1.5 mL) and water (0.5 mL) was heated in a microwave at 300W at 100°C for 30 mins. The reaction mixture was evaporated *in vacuo*, water (15 mL) was added and the organic phases extracted twice with DCM (2 x 20 mL). The combined organic fractions was dried by passing through a hydrophobic frit and evaporated *in vacuo*. The crude material was purified using a 12 g silica Silicycle cartridge eluting with a gradient of 0 - 50% EtOAc in cyclohexane to give the title compound.
MS calcd for (C₂₈H₃₀F₃N₃O₄S₂ + H)⁺ : 594
MS found (electrospray) : (M+H)⁺ = 594

### Intermediate 73

### N-[4-Bromo-2-(methyloxy)phenyl]-1,3-thiazole+4-carboxamide

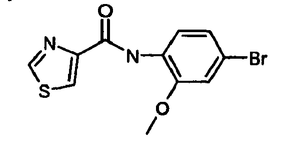

To a solution of 4-thiazolecarboxylic acid (0.24 g) and HATU (0.70 g) in *N,N-*dimethylformamide (5 mL) was added DIPEA (0.80 mL) and the reaction was stirred at room temperature under nitrogen for 10 mins. 4-Bromo-2-(methyloxy)phenyl]amine (0.31 g) was added and the reaction stirred at room temperature under nitrogen for 20 h. The reaction mixture was evaporated *in vacuo* and loaded onto a 5 g aminopropyl cartridge in DCM. The cartridge was eluted with DCM (3 column volumes) and methanol (3 column volumes). The fractions were evaporated *in vacuo*. The crude material was purified using a 40 g silica Silicycle cartridge eluting with a gradient of 0 - 100% EtOAc in cyclohexane to give the title compound.
MS calcd for (C₂₆H₃₀F₃N₃O₄S₂ + H)⁺: 313/315
MS found (electrospray) : (M+H)⁺ = 313/315

### Intermediate 74

### Methyl 3-[[(trans-4-methylcyclohexyl)carbonyl](1-methylethyl)amino]-5-{3-(methyloxy)-4-[(1,3-thiazol-4-ylcarbonyl)amino]phenyl}-2-thiophenecarboxylate

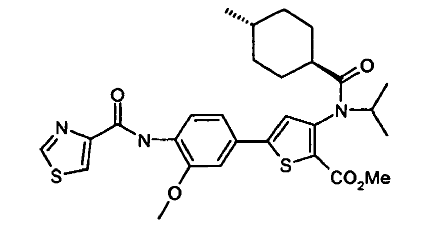

A mixture of {4-[[(*trans*-4-methylcyclohexyl)carbonyl](1-methylethyl)amino]-5-[(methyloxy)carbonyl]-2-thienyl}boronic acid (237 mg, a synthesis of which is described as Intermediate 4), Intermediate 73 (160 mg), sodium carbonate (162 mg) and tetrakis(triphenylphosphine)palladium (0) (59 mg) in 1,4-dioxane (1.5 mL) and water (0.5 mL) was heated in a microwave at 300W at 100°C for 10 mins. The reaction mixture was evaporated *in vacuo*, water (15 mL) was added and the organic phases extracted with DCM (2 x 20 mL). The organic phase was dried by passing through a hydrophobic frit and evaporated *in vacuo*. The crude material was purified using a 12 g silica Silicycle cartridge eluting with a gradient of 0 - 100% EtOAc in cyclohexane to give the title compound.
MS calcd for (C₂₈H₃₃N₃O₅S₂ + H)⁺ 556
MS found (electrospray) : (M+H)⁺ = 556

### Intermediate 75

### [2-{[Tris(1-methylethyl)silyl]oxy}-1-({[tris(1-methylethyl)silyl]oxy}methyl) ethyl]amine

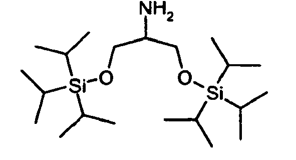

To a stirred suspension of 2-amino-1,3-propanediol hydrochloride (5 g) in DCM (100 mL) was added chloro[tris(1-methylethyl)]silane (15.11 g) followed by pyridine (9.3 g) dropwise. The reaction mixture was stirred at room temperature for 19 h. DMAP (0.48 g) was added with stirring and the reaction was stirred for 5 h. The reaction mixture was washed with water (3 x 100 mL) and the organic was applied to SCX cartridges. The cartridges were eluted with methanol followed by 5% NH₃ in methanol to afford the title compound.
¹H NMR (CDCl₃): δ 3.74 (2H, dd), 3.62 (2H, dd), 2.93 (1H, pentet), 1.14-1.03 (42H m), 2 exchangeables not seen.

### Intermediate 76

### Methyl 3-{[2-{[tris(1-methylethyl)silyl]oxy}-1-({[tris(1-methylethyl)silyl]oxy}methyl)ethyl]amino}-2-thiophenecarboxylate

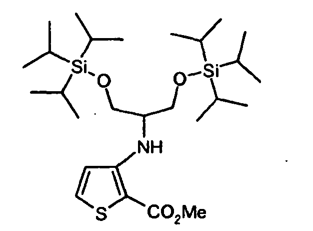

Nitrogen was bubbled through a mixture of intermediate 75 (4.57 g), methyl 3-bromo-2-thiophenecarboxylate (2.50 g), cesium carbonate (11.05 g) and Pd₂(dba)₃ in 1,4-dioxane (37 mg) for 6 mins. Racemic BINAP (1.20 g) was added and the mixture was stirred at 80°C for 24 h. The reaction mixture was filtered through celite with EtOAc and evaporated *in vacuo*. The crude material was purified using a 120 g silica Redisep^{™} cartridge eluting with a gradient of EtOAc in cyclohexane. The crude material was further purified using a 330 g silica Redisep^{™} cartridge eluting with a gradient of EtOAc in cyclohexane to give the title compound.
¹H NMR (CDCl₃): δ 7.30 (1H, d), 6.66 (1H, d), 3.92-3.83 (4H, m), 3.80 (s, 3H), 3.57-3.49 (1H, m), 1.16-1.02 (42H, m) (exchangeable unassigned)

### Intermediate 77

### Methyl 3-{[(trans-4-methylcyclohexyl)carbonyl][2-{[tris(1-methylethyl)silyl]oxy}-1-({[tris(1-methylethyl)silyl]oxy}methyl)ethyl]amino}-2-thiophenecarboxylate

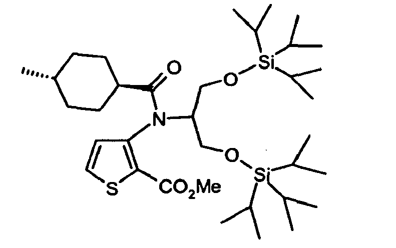

To a stirred solution of the Intermediate 76 (4.5 g) and pyridine (1.37 g) in toluene (15 mL) was added *trans*-4-methylcyclohexanecarbonyl chloride (2.66 g, a synthesis of which is described as Intermediate 1). The mixture was heated to 100°C for 7 days. The reaction mixture was partitioned between saturated sodium bicarbonate solution and ethyl acetate. The aqueous phase was separated and extracted with ethyl acetate. The organic phase was combined, washed with water, dried using sodium sulphate and evaporated *in vacuo*. The crude was purified using a 330 g silica Redisep^{™} cartridge eluting with a gradient of EtOAc in cyclohexane to give the title compound.
¹H NMR (CDCl₃): δ 7.49 (1H, d), 7.30 (1H, d), 4.56 (1H, m), 4.20-3.43 (4H, m, rotamers), 3.81 (3H, s overlapped), 2.06-0.50 (55H, m).

### Intermediate 78

### Methyl 5-iodo-3-{[(trans-4-methylcyclohexyl)carbonyl][2-{[tris(1-methylethyl)silyl]oxy}-1-({[tris(1-methylethyl)silyl]oxy}methyl)ethyl]amino}-2-thiophenecarboxylate

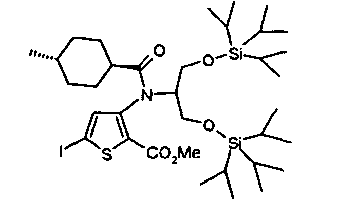

A solution of lithium diisopropylamide (5.75 mL, 2M in heptane/THF/ethyl benzene) was cooled to -78°C under nitrogen. Intermediate 77 (2.56 g) in anhydrous THF (12.5 mL) was added dropwise over 0.5 h keeping the temperature <-65°C. The reaction mixture was stirred at -78°C for 2 h. A solution of iodine (1.95 g) in anhydrous THF (12.5 mL) was added dropwise over 0.5 h keeping the internal temperature <-65°C. After 2 h the mixture was allowed to warm to -50°C and saturated aqueous ammonium chloride and aqueous sodium thiosulphate were added with rapid stirring. The reaction mixture was allowed to warm to room temperature and was diluted with EtOAc and saturated aqueous ammonium chloride. The organic phase was separated, washed with water, dried and evaporated *in vacuo* to give the title compound.
¹H NMR (CDCl₃): δ 7.53 (1H, d), 4.46 (1H, m), 3.79 (3H, s), 4.20-3.46 (4H, m, overlapped), 2.00-0.59 (55H, m).

### Intermediate 79

### Methyl 3-{[2-hydroxy-1-(hydroxymethyl)ethyl][(trans-4-methylcyclohexyl)carbonyl]amino}-5-iodo-2-thiophenecarboxylate

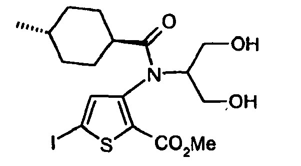

A stirred solution of Intermediate 78 (3.23 g) in THF (50 mL) was treated with pyridine.HF (5 mL). The mixture was allowed to stand at room temperature for 22 h. The mixture was diluted with EtOAc (-100 mL) and the solution was slowly added to stirred solid sodium bicarbonate. The mixture was stirred for 2.5 h, filtered and evaporated *in vacuo*. The crude material was purified using ISCO Companion^{™} silica chromatography eluting with a gradient of 0-100% EtOAc in cyclohexane to give the title compound.
MS calcd for (C₁₇H₂₄INO₅S + H)⁺: 482
MS found (electrospray) : (M+H)⁺ = 482

### Intermediate 80

### Methyl 3-{1,3-dioxan-5-yl[(trans-4-methylcyclohexyl)carbonyl]amino}-5-iodo-2-thiophenecarboxylate

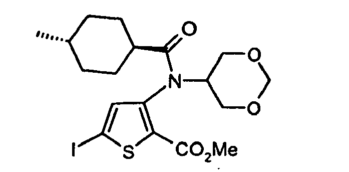

A mixture of Intermediate 79 (0.6 g), paraformaldehyde (0.18 g) and boron trifluoride-diethyl etherate (0.51 g) in 1,4-dioxane (18 mL) was stirred at 80°C for 20 min. The reaction was allowed to cool to room temperature and was diluted with EtOAc. Saturated aqueous sodium bicarbonate was added with stirring. The organic phase was separated washed with water, dried with sodium sulphate and evaporated *in vacuo*. The crude material was purified using ISCO Companion^{™} silica chromatography eluting with a gradient of 0-90% EtOAc in cyclohexane to give the title compound.
MS calcd for (C₁₈H₂₄INO₅S + H)⁺ : 494
MS found (electrospray) : (M+H)⁺ = 494

### Intermediate 81

### Methyl 3-{1,3-dioxan-5-yl[(trans-4-methylcyclohexyl)carbonyl]amino}-5-{4-[(1,3-thiazol-4-ylcarbonyl)amino]phenyl}-2-thiophenecarboxylate

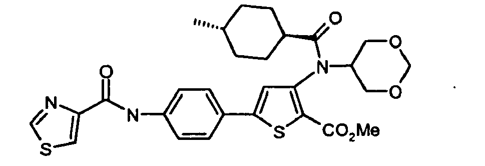

A mixture of Intermediate 80 (0.36 g), N-[4-(4,4,5,5-tetramethyl-1,3,2-dioxaborolan-2-yl)phenyl]-1,3-thiazole-4-carboxamide (0.29 g, a synthesis of which is described as Intermediate 53), potassium phosphate (0.31 g) and Pd(PPh₃)₄ (0.084 g) in DME (7 mL) and water (1.75 mL) was heated to 80°C under nitrogen for 16 h. The reaction mixture was allowed to cool and was partitioned between DCM and water. The organic phase was separated using a hydrophobic frit and evaporated *in vacuo*. The crude material was purified using ISCO Companion^{™} silica chromatography eluting with a gradient of 0-100% EtOAc in cyclohexane to give the title compound.
MS calcd for (C₂₈H₃₁N₃O₆S₂ + H)⁺ 570
MS found (electrospray) : (M+H)⁺ = 570

### Intermediate 82

### Methyl 3-(cyclobutylamino)-2-thiophenecarboxylate

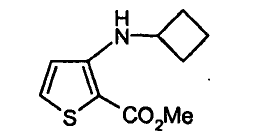

Methyl 3-amino-2-thiophenecarboxylate (3.5 g) and cyclobutanone (7.71 g) were stirred in anhydrous DCM (75 mL) with glacial acetic acid (5.28 g) under nitrogen at room temperature. Sodium triacetoxyborohydride (11.66 g) was added and the mixture was stirred for 5 h. Aqueous sodium bicarbonate was added and the mixture was stirred vigorously until the aqueous was basic. The organic phase was collected by passing through a hydrophobic frit and evaporated *in vacuo*. The crude material was purified using ISCO Companion^{™} silica chromatography eluting with a gradient of 0-5% EtOAc in cyclohexane to give the title compound.
MS calcd for (C₁₀H₁₃NO₂S + H)⁺ : 212
MS found (electrospray) : (M+H)⁺ = 212

### Intermediate 83

### Methyl 3-{cyclobutyl[(trans-4-methylcyclohexyl)carbonyl]amino}-2-thiophenecarboxylate

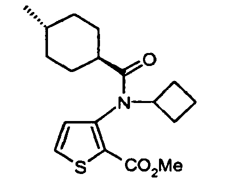

Intermediate 82 (2.2 g) and *trans*-4-methylcyclohexanecarbonyl chloride (3.2 g, a synthesis of which is described as Intermediate 1) were heated in anhydrous DCE (10 mL) under nitrogen atmosphere at 100°C for 21 h. *trans*-4-Methylcyclohexanecarbonyl chloride (1.6 g, a synthesis of which is described as Intermediate 1) was added and the reaction was stirred for 24 h. The solution was partitioned between DCM and sodium bicarbonate solution. The organic phase was separated and evaporated *in vacuo*. The crude material was purified using ISCO Companion^{™} silica chromatography eluting with a gradient of 5-40% EtOAc in cyclohexane. The appropriate fractions were combined, evaporated, dissolved in MeOH and passed through an aminopropyl SPE cartridge eluting with MeOH to give the title compound.
MS calcd for (C₁₈H₂₅NO₃S + H)⁺ : 336
MS found (electrospray) : (M+H)⁺ = 336

### Intermediate 84

### {4-{Cyclobutyl[(trans-4-methylcyclohexyl)carbonyl]amino}-5-[(methyloxy)carbonyl]-2-thienyl}boronic acid

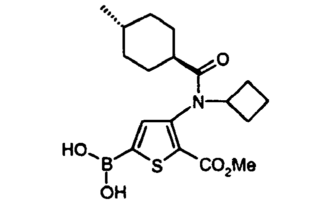

To Intermediate 83 (2 g) and trimethyl borate (1.3 mL) in dry THF (40 mL) at -78°C under nitrogen was added dropwise LDA (10 mL, 1.8M in THF/heptane /ethylbenzene) keeping the internal temperature <-70°C. After stirring for 1 h the reaction was quenched with 2M HCl, warmed to room temperature and evaporated *in vacuo*. The residue was partitioned between saturated ammonium chloride and DCM. The organic phases were separated, evaporated and the residue recrystallised from hot aqueous isopropanol. The solid was filtered off, washed with cold aqueous isopropanol, and then dried *in vacuo* to give the title compound.
MS calcd for (C₁₈H₂₆BNO₅S + H)⁺ : 380
MS found (electrospray) : (M+H)⁺ = 380

### Intermediate 85

### Methyl 3-{cyclobutyl[(trans-4-methylcyclohexyl)carbonyl]amino}-5-{4-[(1,3-thiazol-4-ylcarbonyl)amino]phenyl}-2-thiophenecarboxylate

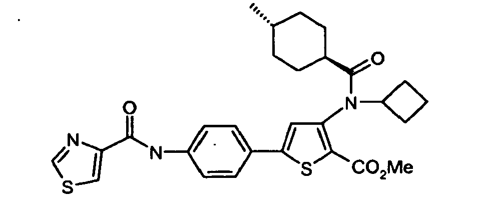

A solution of *N*-(4-iodophenyl)-1,3-thiazole-4-carboxamide (0.1 g, a synthesis of which is described as Intermediate 5), {4-{cyclobutyl[(*trans*-4-methylcyclohexyl)carbonyl]amino}-5-[(methyloxy)carbonyl]-2-thienyl}boronic acid (0.138g, a synthesis of which is described as Intermediate 84), sodium carbonate (32 mg) and tetrakis(triphenylphosphine)palladium(0) (35 mg) in 1,4-dioxane (1.5 mL) and water (0.5 mL) was heated in the microwave at 110°C for 5 mins. The reaction was evaporated *in vacuo* and partitioned between DCM and water. The organic phase was separated and evaporated *in vacuo*. The crude material was purified using ISCO Companion^{™} silica chromatography eluting with a gradient of EtOAc in cyclohexane to give the title compound.
MS calcd for (C₂₈H₃₁N₃O₄S₂ + H)⁺ : 538
MS found (electrospray) : (M+H)⁺ = 538

### Intermediate 86

### Methyl 3-{[1-methyl-2-(methyloxy)ethyl]amino}-2-thiophenecarboxylate

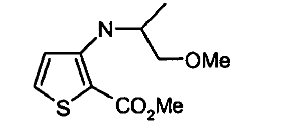

To methyl 3-amino-2-thiophenecarboxylate (10 g) in DCM (300 mL), 1-(methyloxy)-2-propanone (11.2 g), titanium isopropoxide (21.7 g) and sodium triacetoxyborohydride (27.0 g). The reaction was stirred at reflux under nitrogen for 4 days. The reaction was cautiously quenched with saturated sodium bicarbonate solution, and then extracted with DCM. The organic phase was separated, dried by passing through a hydrophobic frit and evaporated in *vacuo*. The crude material was purified using a 330g ISCO Companion^{™} silica cartridge eluting with a gradient of 5-50% EtOAc in cyclohexane to give the title compound.
MS calcd for (C₁₀H₁₅NO₃S + H)⁺ : 230
MS found (electrospray) : (M+H)⁺ = 230

### Intermediate 87

### Methyl 3-{[(trans-4-methylcyclohexyl)carbonyl][1-methyl-2-(methyloxy)ethyl]amino}-2-thiophenecarboxylate

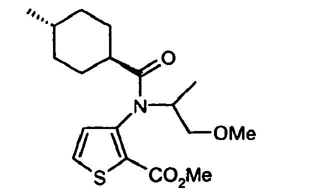

To Intermediate 86 (4 g) in DCE (60 mL) was added *trans*-4-methylcyclohexanecarbonyl chloride (3.2 g, a synthesis of which is described as Intermediate 1) and the reaction was stirred at 90°C for 18 h. The reaction mixture was cooled and quenched with saturated sodium bicarbonate solution. The phases were separated and the aqueous was washed with DCM x 2. The organic phase was combined and dried by passing through a hydrophobic frit and evaporated *in vacuo*. The crude material was purified using a 330g ISCO Companion^{™} silica cartridge eluting with a gradient of 5-100% EtOAc in cyclohexane to give the title compound.
MS calcd for (C₁₈H₂₇NO₄S + H)⁺ : 354
MS found (electrospray) : (M+H)⁺ = 354

### Intermediate 88

### Methyl 5-iodo-3-{[(trans-4-methylcyclohexyl)carbonyl][1-methyl-2-(methyloxy)ethyl]amino}-2-thiophenecarboxylate

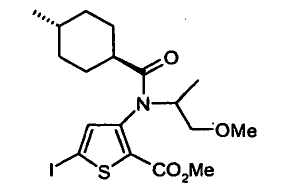

LDA (12 mL, 2M in THF/heptane/ ethyl benzene) was added dropwise to a solution of Intermediate 87 (2.86 g) and iodine (4.1 g) in THF (80 mL) at -78°C. The reaction mixture was stirred at -78°C for 2.5 h. The reaction mixture was quenched at -70°C with saturated ammonium chloride solution (40 mL) and the reaction mixture was allowed to warm to room temperature. The reaction mixture was washed with 5% sodium thiosulphate, the phases were separated and the aqueous phase was washed with EtOAc (x 2.) The combined organic phases were dried by passing through a hydrophobic frit and evaporated *in vacuo.* The crude material was purified using a 120g ISCO Companion^{™} silica cartridge eluting with a gradient of 5-50% EtOAc in cyclohexane to give the title compound.
MS calcd for (C₁₈H₂₆INO₄S + H)⁺ : 480
MS found (electrospray) : (M+H)⁺ = 480

### Intermediate 89

### Methyl 3-{[(trans-4-methylcyclohexyl)carbonyl][1-methyl-2-(methyloxy)ethyl]amino}-5-{4-[(1,3-thiazol-4-ylcarbonyl)amino]phenyl}-2-thiophenecarboxylate

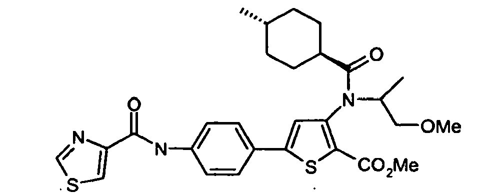

N-[4-(4,4,5,5-Tetramethyl-1,3,2-dioxaborolan-2-yl)phenyl]-1,3-thiazole-4-carboxamide (248 mg, a synthesis of which is described as intermediate 53), Intermediate 88 (300 mg) and sodium carbonate (265 mg) were dissolved in DMF (5 mL) and water (1 mL). Tetrakis(triphenylphosphine)palladium(0) (72.3 mg) was added and the reaction mixture was stirred at 100°C under nitrogen for 1.5 h. The reaction mixture was cooled and evaporated in *vacuo*. The residue was partitioned between DCM and water. The aqueous phase was washed with DCM (x 2.) The organic phases were combined, dried by passing through a hydrophobic frit and evaporated *in vacuo*. The crude material was purified using a 40 g ISCO Companion^{™} silica cartridge eluting with a gradient of 5-100% EtOAc in cyclohexane to give the title compound.
MS calcd for (C₂₈H₃₃N₃O₅S₂ + H)⁺ : 556
MS found (electrospray) : (M+H)⁺ = 556

### Intermediate 90

### Methyl 3-([1-methyl-2-(methyloxy)ethyl]{[trans-4-(trifluoromethyl)cyclohexyl]carbonyl}amino)-2-thiophenecarboxylate

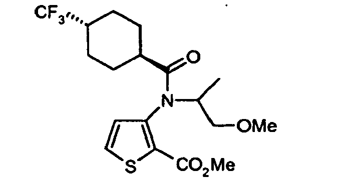

To Intermediate 86 (706 mg) in DCE (12 mL) was added *trans*-4-(trifluoromethyl)cyclohexanecarbonyl chloride (1.0 g, a synthesis of which is described above as Intermediate 22) and the reaction was stirred at 90°C for 18 h. The reaction mixture was cooled and quenched with saturated sodium bicarbonate solution. The phases were separated and the aqueous phase was washed with DCM. The organic phase was combined and dried by passing through a hydrophobic frit and evaporated *in vacuo*. The crude material was purified using a 40g ISCO Companion^{™} silica cartridge eluting with a gradient of 5-100% EtOAc in cyclohexane to give the title compound.
MS calcd for (C₁₈H₂₄F₃NO₄S + H)⁺ : 408
MS found (electrospray) : (M+H)⁺ = 408

### Intermediate 91

### Methyl 5-iodo-3-([1-methyl-2-(methyloxy)ethyl]{[trans-4-(trifluoromethyl)cyclohexyl]carbonyl}amino)-2-thiophenecarboxylate

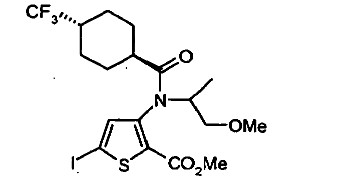

LDA (3.2 mL, 2M in THF/heptane/ ethyl benzene) was added dropwise to a solution of Intermediate 90 (1.0 g) and iodine (1.2 g) in THF (25 mL) at -78°C. The reaction mixture was stirred at -78°C for 2 h. The reaction mixture was quenched at -70°C with saturated ammonium chloride solution (2 mL) and the reaction mixture was allowed to warm to room temperature. The reaction mixture was washed with 5% sodium thiosulphate, the phases were separated and the aqueous phase was washed with EtOAc (x 2). The combined organic phases were evaporated *in vacuo*. The crude material was purified using an 80 g ISCO Companion^{™} silica cartridge eluting with a gradient of 5-50% EtOAc in cyclohexane to give the title compound.
MS calcd for (C₁₈H₂₃F₃INO₄S + H)⁺ : 534
MS found (electrospray) : (M+H)⁺ = 534

### Intermediate 92

### Methyl 3-([1-methyl-2-(methyloxy)ethy)]{[trans-4-(trifluoromethyl)cyclohexyl]carbonyl}amino)-5-{4-[(1,3-thiazol-4-ylcarbonyl)amino]phenyl}-2-thiophenecarboxylate

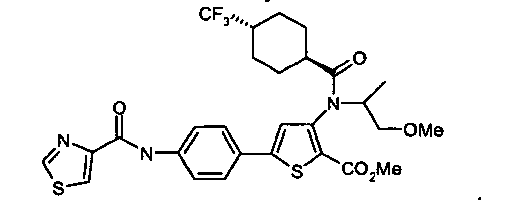

N-[4-(4,4,5,5-Tetramethyl-1,3,2-dioxaborolan-2-yl)phenyl]-1,3-thiazole-4-carboxamide (223 mg, a synthesis of which is described as Intermediate 53), Intermediate 91 (300 mg) and sodium carbonate (238 mg) were dissolved in DMF (5 mL) and water (1 mL). Tetrakis(triphenylphosphine)palladium(0) (65.0 mg) was added and the reaction mixture was stirred at 100°C under nitrogen for 1.5 h. The reaction mixture was cooled and evaporated *in vacuo*. The residue was partitioned between DCM and water. The aqueous phase was washed with DCM (x 2). The organic phases were combined, dried by passing through a hydrophobic frit and evaporated *in vacuo*. The crude material was purified using a 40 g ISCO Companion^{™} silica cartridge eluting with a gradient of 5-100% EtOAc in cyclohexane to give the title compound.
MS calcd for (C₂₈H₃₀F₃N₃O₅S₂ + H)⁺ : 610
MS found (electrospray) : (M+H)⁺ = 610

### Intermediate 93

### Methyl 5-(6-amino-5-methyl-3-pyridinyl)-3-[[(trans-4-methylcyclohexyl)carbonyl](1-methylethyl)amino]-2-thiophenecarboxylate

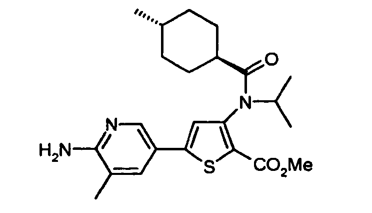

A solution of 5-bromo-3-methyl-2-pyridinamine (150 mg), {4-[[(*trans-*4-methylcyclohexyl)carbonyl](1-methylethyl)amino]-5-[(methyloxy)carbonyl]-2-thienyl}boronic acid (0.35 g, a synthesis of which is described as Intermediate 4), sodium carbonate (0.25 g) and tetrakis(triphenylphosphine)palladium(0) (90 mg) in 1,4-dioxane (3 mL) and water (1 mL) was heated in the microwave at 110°C for 5 mins. The reaction was evaporated *in vacuo* and partitioned between DCM and water. The organic phase was evaporated *in vacuo.* The crude material was purified using a 12 g ISCO Companion^{™} silica cartridge eluting with a gradient of 0-100% EtOAc in cyclohexane to give the title compound.
MS calcd for (C₂₃H₃₁N₃O₃S₂+ H)⁺ : 430
MS found (electrospray) : (M+H)⁺ = 430

### Intermediate 94

### Methyl 3-[[(trans-4-methylcyclohexyl)carbonyl](1-methylethyl)amino]-5-{5-methyl-6-[(1,3-thiazol-4-ylcarbonyl)amino]-3-pyridinyl}-2-thiophenecarboxylate

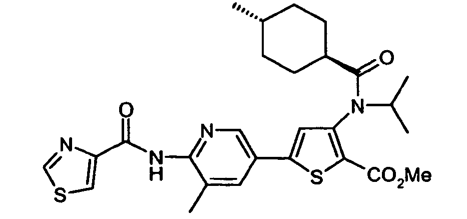

A solution of 1,3-thiazole-4-carboxylic acid (45 mg), Intermediate 93 (150 mg), DIPEA (135 mg) and HATU (160 mg) in DMF (2 mL) was stirred at room temperature under nitrogen for 16 h. The reaction mixture was left for a further 2 days. 1,3-Thiazole-4-carboxylic acid (23 mg) and HATU (67 mg) was added to the reaction mixture which was stirred for 1 day. 1,3-Thiazole-4-carboxylic acid (135 mg) were added and stirred for 1 day. The reaction was evaporated *in vacuo* and partitioned between HCl and DCM. The organic phase was washed with sodium bicarbonate, separated and evaporated *in vacuo.* The crude material was purified using a 12 g ISCO Companion^{™} silica cartridge eluting with a gradient of EtOAc in cyclohexane to give the title compound.
MS calcd for (C₂₇H₃₂N₄O₄S₂+ H)⁺ : 541
MS found (electrospray) : (M+H)⁺ = 541

### Intermediate 95

### N-(5-Bromo-3-methyl-2-pyridinyl)-4-fluorobenzamide

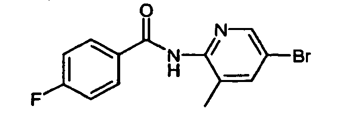

To a cold solution of 5-bromo-3-methyl-2-pyridinamine (1 g) and triethylamine (1.04 mL) in DCM (5.35 mL) was added dropwise a solution of 4-fluorobenzoyl chloride (0.85 g) in DCM (4 mL). The reaction mixture was stirred for 0.5 h and was evaporated *in vacuo.* The residue was washed with water and neutralised with sodium bicarbonate. The aqueous phase was extracted with DCM and the organic layer was evaporated *in vacuo.* The crude material was purified using a 80 g ISCO Companion^{™} silica cartridge eluting with a gradient of 0-100% EtOAc in cyclohexane to give the title compound.
MS calcd for (C₁₃H₁₀BrFN₂O+ H)⁺ : 309/310
MS found (electrospray) : (M+H)⁺ = 309/310

### Intermediate 96

### Methyl 5-(6-{[(4-fluorophenyl)carbonyl]amino}-5-methyl-3-pyridinyl)-3-[[(trans-4-methylcyclohexyl)carbonyl](1-methylethyl)amino]-2-thiophenecarboxylate

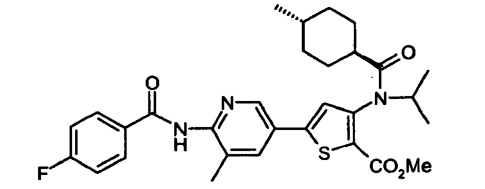

A solution of Intermediate 95 (150 mg), {4-[[(*trans-*4-methylcyclohexyl)carbonyl](1-methylethyl)amino]-5-[(methyloxy)carbonyl]-2-thienyl}boronic acid (0.214 g, a synthesis of which is described as Intermediate 4), sodium carbonate (0.15 g), tetrakis(triphenylphosphine(0) (56 mg) in 1,4-dioxane (3 mL) and water (1 mL) was heated in a microwave at 110°C for 5 mins. The reaction mixture was evaporated *in vacuo* and partitioned between DCM and water. The organic layer was evaporated *in vacuo.* The crude material was purified using a 12 g ISCO Companion^{™} silica cartridge eluting with a gradient of 30-100% EtOAc in cyclohexane to give the title compound.
MS calcd for (C₃₀H₃₄FN₃O₄S+ H)⁺ : 552
MS found (electrospray) : (M+H)⁺ = 552

### Intermediate 97

### N-[4-(4,4,5,5-tetramethyl-1,3,2-dioxaborolan-2-yl)phenyl]-1H-pyrazole-3-carboxamide

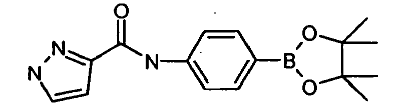

DIPEA (4.67 mL) was added to a solution of 1H-pyrazole-3-carboxylic acid (1 g), [4-(4,4,5,5-tetramethyl-1,3,2-dioxaborolan-2-yl)phenyl]amine (1.96 g) and HATU (4.07 g) in dry DMF (10 mL). The reaction mixture was stirred at room temperature for 24 h, evaporated *in vacuo* and dissolved in DCM (50 mL). The organic phase was washed with saturated sodium bicarbonate solution (3 x 10 mL), dried using a hydrophobic frit and evaporated *in vacuo.* The residue was dissolved in DCM and applied to a 50 g Si SPE cartridge eluting with a gradient of 0-50% EtOAc in cyclohexane. The appropriate fractions were evaporated *in vacuo* and dissolved in DCM. Undissolved solid was filtered off and air dried to give the title compound.
MS calcd for (C₁₆H₂₀BN₃O₃+ H)⁺ : 314
MS found (electrospray) : (M+H)⁺ = 314

### Intermediate 98

### N-[4-(4,4,5,5-Tetramethyl-1,3,2-dioxaborolan-2-yl)phenyl]-1-(triphenylmethyl)-1H-pyrazole-3-carboxamide

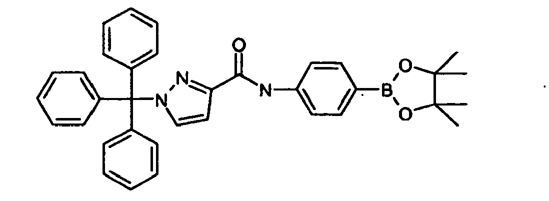

1,1',1"-(Chloromethanetriyl)tribenzene (606 mg) was added to a solution of Intermediate 97 (648 mg) and triethylamine (0.30 mL) in anhydrous DCM (10 mL) under nitrogen and stirred for 24 h. The reaction was evaporated *in vacuo*, dissolved in DCM and applied to a 20 g SPE cartridge eluting with a gradient of 0-30% EtOAc in cyclohexane to give the title compound.
¹H NMR (d₆-DMSO): δ 9.87 (1H, s), 7.78 (2H, d), 7.63 (2H, d), 7.45-7.35 (10H, m), 7.16-7.08 (6H, m), 6.91 (1H, m), 1.28 (12H, s).

### Intermediate 99

### Methyl 3-{cyclobutyl[(trans-4-methylcyclohexyl)carbonyl]amino}-5-iodo-2-thiophenecarboxylate

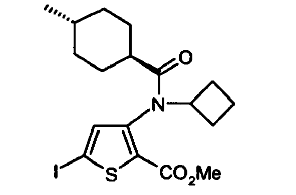

LDA (5.05 mL, 2M solution in heptane/THF/ethylbenzene) was added dropwise maintaining the temperature below -65°C to a solution of methyl 3-{cyclobutyl[(*trans-*4-methylcyclohexyl)carbonyl]amino}-2-thiophenecarboxylate (1.13 g, a synthesis of which is described as Intermediate 83) and iodine (1.7 g) in THF (20 mL) at -78°C under nitrogen. The reaction was stirred at -78°C for 1 h. The reaction was quenched by dropwise addition of saturated ammonium chloride solution and was allowed to warm to room temperature. The aqueous phase was extracted with EtOAc x 2. The combined organic phases were dried with sodium sulphate and evaporated *in vacuo.* The crude material was purified using a 120 g ISCO Companion^{™} silica cartridge eluting with a gradient of EtOAc in cyclohexane to give the title compound.
MS calcd for (C₁₈H₂₄INO₃S+ H)⁺ : 462
MS found (electrospray) : (M+H)⁺ = 462

### Intermediate 100

### Methyl 3-{cyclobutyl[(trans-4-methylcyclohexyl)carbonyl]amino}-5-[4-({[1-(triphenylmethyl)-1H-pyrazol-3-yl]carbonyl}amino)phenyl]-2-thiophenecarboxylate

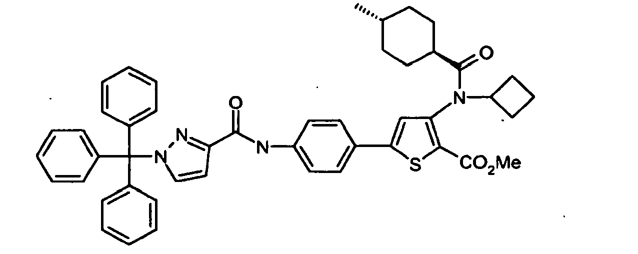

Intermediate 99 (100 mg), Intermediate 98 (132 mg), sodium carbonate (68.9 mg) and tetrakis(triphenylphosphine)palladium(0) (12.52 mg) in 1,4-dioxane (3 mL) and water (1 mL) was heated to 90°C under nitrogen for 2 h. On cooling the reaction was poured into DCM (10 mL) / water (5 mL). The layers were separated using a hydrophobic frit and evaporated *in vacuo.* The crude material was purified using a 40 g ISCO Companion^{™} silica cartridge eluting with a gradient of 0-100% EtOAc in cyclohexane to give the title compound.
MS calcd for (C₄₇H₄₆N₄O₄S+ H)⁺ : 763
MS found (electrospray) : (M+H)⁺ = 763

### Intermediate 101

### Methyl 3-{cyclobutyl[(trans-4-methylcyclohexyl)carbonyl]amino}-5-{4-[(1H-pyrazol-3-ylcarbonyl)amino]phenyl}-2-thiophenecarboxylate

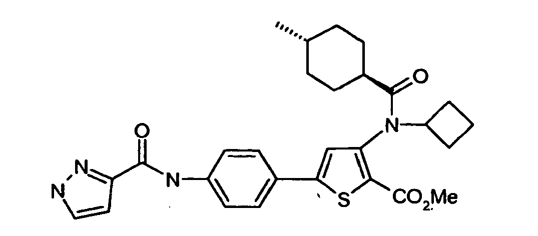

4N Hydrogen chloride in 1,4-dioxane (5 mL) was added to a solution of Intermediate 100 (175 mg) in 1,4-dioxane (2 mL) at room temperature and was stirred overnight. The solvent was evaporated *in vacuo* and the residue was partitioned between DCM (10 mL) and saturated sodium bicarbonate solution (5 mL). The aqueous phase was extracted with DCM (5 mL). The combined organic phases were dried by passing through a hydrophobic frit and evaporated *in vacuo.* The crude material was purified using a 40 g ISCO Companion^{™} silica cartridge eluting with a gradient of 0-100% EtOAc in cyclohexane to give the title compound.
MS calcd for (C₂₈H₃₂N₄O₄S+ H)⁺ : 521
MS found (electrospray) : (M+H)⁺ = 521

### Intermediate 102

### Methyl 3-{ethyl[(trans-4-methylcyclohexyl)carbonyl]amino}-5-[4-({[1-(triphenylmethyl)-1H-pyrazol-3-yl]carbonyl}amino)phenyl]-2-thiophenecarboxylate

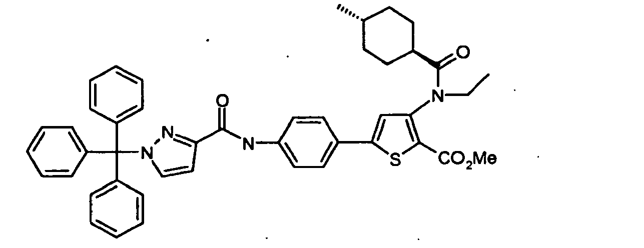

Methyl 3-{ethyl[(*trans-*4-methylcyclohexyl)carbonyl]amino}-5-iodo-2-thiophene carboxylate (100 mg, a synthesis of which is described as intermediate 52), Intermediate 98 (140 mg), sodium carbonate (73 mg) and tetrakis(triphenylphosphine)palladium(0) (13.27 mg) in 1,4-dioxane (3 mL) and water (1 mL) was heated to 90°C under nitrogen for 2 h. On cooling the reaction was poured into DCM (10 mL) and washed with saturated sodium bicarbonate solution (5 mL). The layers were separated using a hydrophobic frit and evaporated *in vacuo.* The crude material was purified using a 40 g ISCO Companion^{™} silica cartridge eluting with a gradient of 0-100% EtOAc in cyclohexane to give the title compound.
MS calcd for (C₄₅H₄₄N₄O₄S+ H)⁺ : 737
MS found (electrospray) : (M+H)⁺ = 737

### Intermediate 103

### Methyl 3-{ethyl[(trans-4-methylcyclohexyl)carbonyl]amino}-5-{4-[(1H-pyrazol-3-ylcarbonyl)amino]phenyl}-2-thiophenecarboxylate

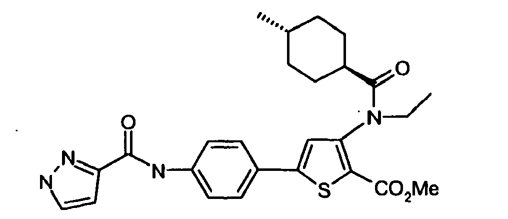

4N Hydrogen chloride in 1,4-dioxane (5 mL) was added to a solution of Intermediate 102 (149 mg) in 1,4-dioxane (3 mL) at room temperature and was stirred for 5 h. The solvent was evaporated *in vacuo* and the residue was partitioned between DCM (10 mL) and saturated sodium bicarbonate solution (5 mL). The aqueous phase was re-extracted with DCM (5 mL). The combined organic phases were dried using a hydrophobic frit and evaporated *in vacuo.* The crude material was purified using a 40 g ISCO Companion^{™} silica cartridge eluting with a gradient of 0-100% EtOAc in cyclohexane to give the title compound.
MS calcd for (C₂₆H₃₀N₄O₄S+ H)⁺ : 495
MS found (electrospray) : (M+H)⁺ = 495

### Intermediate 104

### Methyl 3-{[(trans-4-methylcyclohexyl)carbonyl][2-(methyloxy)ethyl]amino}-2-thiophenecarboxylate

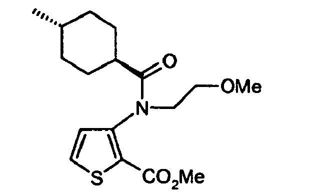

A solution of methyl 3-{[(*trans-*4-methylcyclohexyl)carbonyl]amino}-2-thiophenecarboxylate (12 g, a synthesis of which is described as Intermediate 49) in anhydrous DMF (120 mL) was stirred at 21°C under nitrogen and treated in small portions with 60% NaH dispersion in oil (2.56 g). The reaction was stirred at 21°C for 10 mins then treated with a solution of bromoethyl methyl ether (6.41 mL) in DMF (10 mL). The solution was stirred at 60°C overnight. The reaction was cooled and evaporated *in vacuo.* The residue was partitioned between EtOAc and water. The aqueous phase was separated and extracted with EtOAc (x 3). The combined organic phases were washed with brine, dried with sodium sulphate and evaporated *in vacuo.* The crude material was purified using a 330 g ISCO Companion^{™} silica cartridge eluting with a gradient of 0-70% EtOAc in cyclohexane to give the title compound.
MS calcd for (C₁₇H₂₅NO₄S+ H)⁺ : 340
MS found (electrospray) : (M+H)⁺ = 340

### Intermediate 105

### Methyl 5-iodo-3-{[(trans-4-methylcyclohexyl)carbonyl](2-(methyloxy)ethyl]amino}-2-thiophenecarboxylate

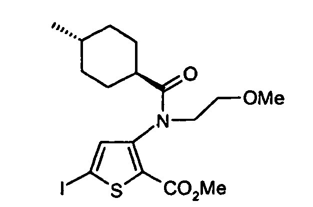

A solution of LDA (2M, 16.44 mL) was stirred and cooled to -78°C under nitrogen and was treated dropwise with a solution of Intermediate 104 (3.72 g) in dry THF (40 mL), keeping the internal temperature at <-70°C. The reaction was stirred for 2 h then treated dropwise with a solution of iodine (5.56 g) in THF (40 mL) keeping the internal temperature <-70°C. The solution was stirred at -78°C for 1 h. A saturated solution of ammonium chloride (75 mL) was added slowly. The mixture was warmed to 0°C then treated with a 5% solution of sodium thiosulphate (75 mL). The phases were separated and the aqueous extracted with EtOAc (x 2). The combined organic phases were washed with brine, dried with sodium sulphate and evaporated *in vacuo.* The crude material was purified using a 330 g ISCO Companion^{™} silica cartridge eluting with a gradient of 0-30% EtOAc in cyclohexane to give the title compound.
MS calcd for (C₁₇H₂₄INO₄S+ H)⁺ : 466
MS found (electrospray) : (M+H)⁺ = 466

### Intermediate 106

### Methyl 3-{[(trans-4-methylcyclohexyl)carbonyl][2-(methyloxy)ethyl]amino}-5-{4-[(1,3-thiazol-4-ylcarbonyl)amino]phenyl}-2-thiophenecarboxylate

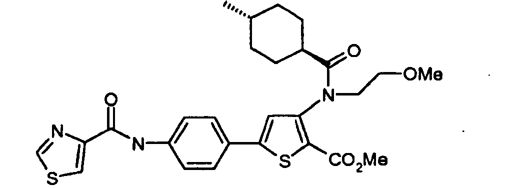

To Intermediate 105 (100 mg) was added N-[4-(4,4,5,5-tetramethyl-1,3,2-dioxaborolan-2-yl)phenyl]-1,3-thiazole-4-carboxamide (0.29 g, a synthesis of which is described as Intermediate 53), potassium phosphate (91 mg), tetrakis(triphenylphosphine)palladium(0) (25 mg), DME (2 mL) and water (0.5 mL). The reaction was stirred at 80°C under nitrogen for 24 h. The cooled reaction was partitioned between water and DCM. The phases were separated with a hydrophobic frit and the organic phase was evaporated *in vacuo.* The crude material was purified using a 40 g ISCO Companion^{™} silica cartridge eluting with a gradient of 0-100% EtOAc in cyclohexane to give the title compound.
MS calcd for (C₂₇H₃₁N₃O₅S₂+ H)⁺ : 542
MS found (electrospray) : (M+H)⁺ = 542

### Intermediate 107

### 1-Methyl-N-[4-(4,4,5,5-tetramethyl-1,3,2-dioxaborolan-2-yl)phenyl]-1H-pyrazole-3-carboxamide

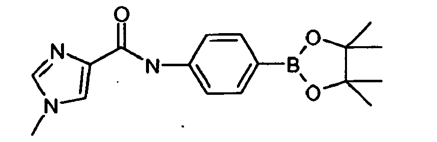

A solution of 1-methyl-1H-imidazole-4-carboxylic acid (1 g), [4-(4,4,5,5-tetramethyl-1,3,2-dioxaborolan-2-yl)phenyl]amine (1.74 g), HATU (3.62g) and DIPEA (4.15 mL) in DMF (10 mL) was stirred at room temperature for 24 h. The solvent was evaporated and the residue was dissolved in DCM (50 mL), washed with saturated sodium bicarbonate solution (3 x 10 mL) dried by passing through a hydrophobic frit and evaporated *in vacuo.* The crude material was purified using a 120 g ISCO Companion^{™} silica cartridge eluting with a gradient of 0-100% EtOAc in cyclohexane to give the title compound.
MS calcd for (C₁₇H₂₂BN₃O₃+ H)⁺ : 328
MS found (electrospray) : (M+H)⁺ = 328

### Intermediate 108

### Methyl 3-{[(trans-4-methylcyclohexyl)carbonyl][1-methyl-2-(methyloxy)ethyl]amino}-5-(4-{[(1-methyl-1H-imidazol-4-yl)carbonyl]amino}phenyl)-2-thiophenecarboxylate

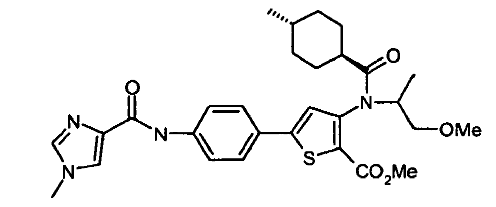

A solution of methyl 5-iodo-3-{[(*trans-*4-methylcyclohexyl)carbonyl][1-methyl-2-(methyloxy)ethyl]amino}-2-thiophenecarboxylate (0.22 g, a synthesis of which is described as Intermediate 88), Intermediate 107 (97 mg) and tetrakis(triphenylphosphine)palladium(0) (35 mg) in 1,4-dioxane (3 mL) and water (1 mL) was heated in the microwave at 110°C for 5 mins. The reaction mixture was evaporated *in vacuo* and partitioned between DCM and water. The organic phase was separated and evaporated *in vacuo.* The crude material was purified using a 12 g ISCO Companion^{™} silica cartridge eluting with a gradient of 0-100% EtOAc in cyclohexane followed by 100% MeOH to give the title compound.
MS calcd for (C₂₉H₃₆N₄O₅S+ H)⁺ : 553
MS found (electrospray) : (M+H)⁺ = 553

### Intermediate 109

### Methyl 3-{[(trans-4-methylcyclohexyl)carbonyl][2-(methyloxy)ethyl]amino}-5-(4-{[(1-methyl-1H-imidazol-4-yl)carbonyl]amino}phenyl)-2-thiophenecarboxylate

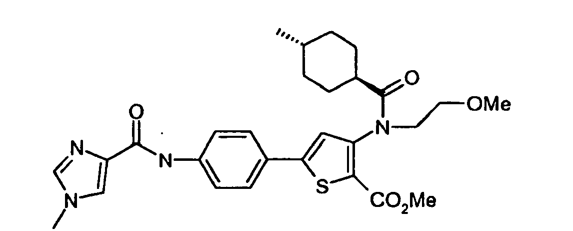

A solution of methyl 5-iodo-3-{[(*trans-*4-methylcyclohexyl)carbonyl][1-methyl-2-(methyloxy)ethyl]amino}-2-thiophenecarboxylate (0.213 g, a synthesis of which is described as Intermediate 88), Intermediate 107 (0.1 mg) and tetrakis(triphenylphosphine)palladium(0) (35 mg) in 1,4-dioxane (3 mL) and water (1 mL) was heated at 90°C for 2 h. The reaction mixture was evaporated *in vacuo* and partitioned between DCM and water. The organic phase was separated and evaporated *in vacuo.* The crude material was purified using a 12 g ISCO Companion^{™} silica cartridge eluting with a gradient of 0-100% EtOAc in cyclohexane. The compound was further purified using a 12 g ISCO Companion^{™} silica cartridge eluting with a gradient of 0-40% methanol in DCM to give the title compound.
MS calcd for (C₂₈H₃₄N₄O₅S+ H)⁺ : 539
MS found (electrospray) : (M+H)⁺ = 539

### Intermediate 110

### Methyl 5-iodo-3-[[(trans-4-methylcyclohexyl)carbonyl](2,2,2-trifluoroethyl)amino]-2-thiophenecarboxylate

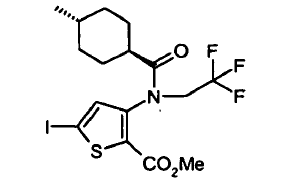

A mixture of methyl 5-iodo-3-{[(*trans-*4-methylcyclohexyl)carbonyl]amino}-2-thiophenecarboxylate (314 mg, a synthesis of which is described as Intermediate 46) and sodium hydride (46 mg) in anhydrous DMF (1.5 mL) was stirred over an ice bath under nitrogen for 30 mins. 2,2,2-Trifluoroethyl trifluoromethanesulfonate (167 uL) was added and the reaction mixture was heated to 70°C under nitrogen for 1.5 h. Further 2,2,2-trifluoroethyl trifluoromethanesulfonate (56 uL) was added and the reaction mixture was heated at 70°C under nitrogen for 1.5 h. The reaction mixture was cooled to room temperature, poured into citric acid (15 mL), extracted twice with EtOAc (2 x 15 mL), dried with a hydrophobic frit and evaporated *in vacuo.* The compound was purified using a 12 g ISCO Companion^{™} silica silicycle cartridge eluting with a gradient of 0-20% EtOAc in cyclohexane to give the title compound.
MS calcd for (C₁₆H₁₉F₃INO₃S+ H)⁺ : 490
MS found (electrospray) : (M+H)⁺ = 490

### Intermediate 111

### Methyl 3-[[(trans-4-methylcyclohexyl)carbonyl](2,2,2-trifluoroethyl)amino]-5-{4-[(1,3-thiazol-4-ylcarbonyl)amino]phenyl}-2-thiophenecarboxylate

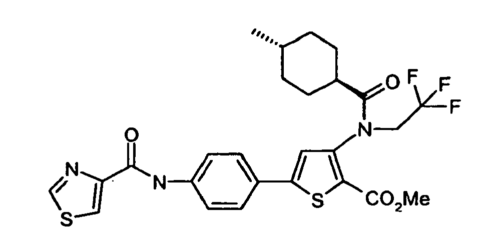

To Intermediate 110 (100 mg) was added N-[4-(4,4,5,5-tetramethyl-1,3,2-dioxaborolan-2-yl)phenyl]-1,3-thiazole-4-carboxamide (0.29 g, a synthesis of which is described as Intermediate 53), potassium phosphate (91 mg), tetrakis(triphenylphosphine)palladium(0) (25 mg), DME (2 mL) and water (0.5 mL). The reaction was stirred at 80°C under nitrogen for 24 h. The cooled reaction was partitioned between water and DCM. The phases were separated with a hydrophobic frit and the organic phase was evaporated *in vacuo.* The crude material was purified using a 40 g ISCO Companion^{™} silica cartridge eluting with a gradient of 0-100% EtOAc in cyclohexane to give the title compound.
MS calcd for (C₂₆H₂₆F₃N₃O₄S₂ + H)⁺ : 566
MS found (electrospray) : (M+H)⁺ = 566

### Intermediate 112

### Methyl 3-{(cyclopropylmethyl)[(trans-4-methylcyclohexyl)carbonyl}amino}-5-{4-[(1H-pyrazol-3-ylcarbonyl)amino]phenyl}-2-thiophenecarboxylate

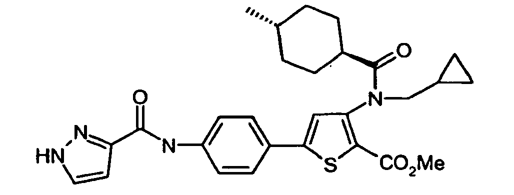

1*H-*Pyrazole-3-carboxylic acid (43.4 mg) was dissolved in a solution of DMF (3 mL), DIPEA (245 µL) and HATU (201 mg) were added and the reaction was stirred at room temperature for 15 mins. Intermediate 64 (150 mg) in DMF (1 mL) was added and the reaction was stirred at 50°C under nitrogen for 22 h. The reaction mixture was evaporated *in vacuo* and was partitioned between DCM and saturated sodium bicarbonate solution. The organic phase was collected and dried by passing through a hydrophobic frit and evaporated *in vacuo*. The crude material was purified using a 12 g silica ISCO cartridge eluting with a gradient of 5 - 100% EtOAc in cyclohexane to give the title compound.
MS calcd for (C₂₈H₃₂N₄O₄S + H)⁺ : 521
MS found (electrospray) : (M+H)⁺ = 521

### Intermediate 113

### Methyl 3-{cyclobutyl[(trans-4-methylcyclohexyl)carbonyl]amino}-5-(4-{[(1-methyl-1H-imidazol-4-yl)carbonyl]amino}phenyl)-2-thiophenecarboxylate

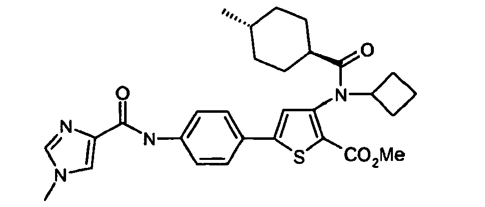

Methyl 3-{cyclobutyl[(*trans-*4-methylcyclohexyl)carbonyl]amino}-5-iodo-2-thiophenecarboxylate (100mg, a synthesis of which is described as Intermediate 99), Intermediate 107 (85 mg), sodium carbonate (68.9 mg) and tetrakis(triphenylphosphine)palladium (0) (12.52 mg) in 1,4-dioxane (3 mL) and water (1 mL) was heated at 90°C under nitrogen for 3 h. On cooling the mixture was partitioned between DCM (20 mL) and saturated sodium bicarbonate solution (5 mL). The layers were separated using a hydrophobic frit and evaporated *in vacuo* to give an oil. The crude material was purified using a 40 g silica ISCO cartridge eluting with a gradient of 0-100% EtOAc in cyclohexane followed by 0-40% MeOH in DCM to give the title compound.
MS calcd for (C₂₉H₃₄N₄O₄S + H)⁺ : 535
MS found (electrospray) : (M+H)⁺ = 535

### Intermediate 114

### Methyl 3-{ethyl[(trans-4-methylcyclohexyl)carbonyl]amino}-5-(4-{[(1-methyl-1H-imidazol-4-yl)carbonyl]amino}phenyl)-2-thiophenecarboxylate

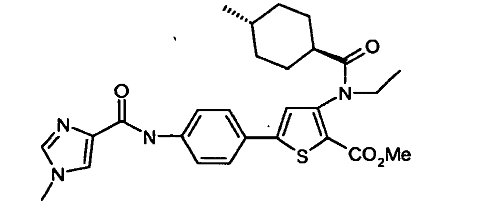

Methyl 3-{ethyl[{*trans-*4-methylcyclohexyl}carbonyl]amino}-5-iodo-2-thiophene carboxylate (100 mg, a synthesis of which is described as Intermediate 52), Intermediate 107 (75 mg), sodium carbonate (73.0 mg) and tetrakis(triphenylphosphine)palladium (0) (13.27 mg) in 1,4-dioxane (3 mL) and water (1 mL) was heated at 90°C under nitrogen for 3 h. On cooling the mixture was partitioned between DCM (20 mL) and saturated sodium bicarbonate solution (5 mL). The layers were separated by hydrophobic frit and evaporated *in vacuo* to give a yellow oil. The crude material was purified using a 40 g silica ISCO cartridge eluting with a gradient of 0-30% MeOH in DCM to give the title compound.
MS calcd for (C₂₇H₃₂N₄O₄S + H)⁺ : 509
MS found (electrospray) : (M+H)⁺ = 509

### Intermediate 115

### N-(6-Bromo-4-methyl-3-pyridinyl)-1,3-thiazole-4-carboxamide

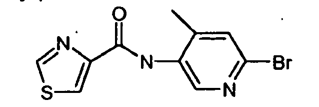

A solution of 1,3-thiazole-4-carbonyl chloride (142 mg) and 6-bromo-4-methyl-3-pyridinamine (18 mg) in DCM (1 mL) was stirred in a Reactivial^{™} under nitrogen at 40°C for 16 h. The reaction mixture was evaporated *in vacuo* and partitioned between DCM and water. The organic phase was separated and evaporated *in vacuo.* The crude material was purified using a 4 g silica ISCO cartridge eluting with a gradient of 0-30% EtOAc to give the title compound.
MS calcd for (C₁₀H₈BrN₃OS + H)⁺ : 298 / 300
MS found (electrospray) : (M+H)⁺ = 298 / 300

### Intermediate 116

### Methyl 3-[[(trans-4-methylcyclohexyl)carbonyl](1-methylethyl)amino]-5-{4-methyl-5-[(1,3-thiazol-4-ylcarbonyl)amino]-2-pyridinyl}-2-thiophenecarboxylate

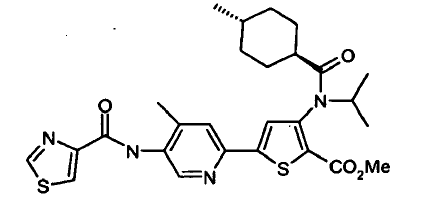

A solution of {4-[[(*trans-*4-methylcyclohexyl)carbonyl](1-methylethyl)amino]-5-[(methyloxy)carbonyl]-2-thienyl}boronic acid (280 mg, a synthesis of which is described as Intermediate 4), Intermediate 115 (150 mg), sodium carbonate (160 mg) and tetrakis(triphenylphosphine)palladium (0) (5.81 mg) in 1,4-dioxane (1.5 mL) and water (5 mL) was stirred under nitrogen at 110°C in a microwave for 5 min. {4-[[(*trans-*4-Methylcyclohexyl)carbonyl](1-methylethyl)amino]-5-[(methyloxy)carbonyl]-2-thienyl}boronic acid (373 mg, a synthesis of which is described as Intermediate 4) was added and the reaction was stirred under nitrogen at 110°C in a microwave for 5 min. The reaction mixture was evaporated *in vacuo* and partitioned between DCM and water. The organic phase was separated and evaporated *in vacuo.* The crude material was purified using a 12 g silica ISCO cartridge eluting with a gradient of 0-100% EtOAc to give the title compound.
MS calcd for (C₂₇H₃₂N₄O₄S₂ + H)⁺ : 541
MS found (electrospray) : (M+H)⁺ = 541

### Intermediate 117

### N-[4-(4,4,5,5-Tetramethyl-1,3,2-dioxaborolan-2-yl)phenyl]-3-pyridazinecarboxamide

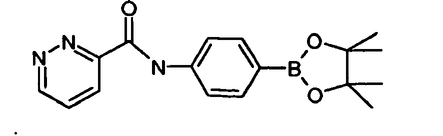

DIPEA (1.92 mL) was added to a suspension of 3-pyridazinecarboxylic acid (500 mg) and HATU (1.53 g) in DMF (10 mL) at room temperature under nitrogen. After 10 min [4-(4,4,5,5-tetramethyl-1,3,2-dioxaborolan-2-yl)phenyl]amine (802 mg) was added and the reaction was heated to 50°C for 4 h. The reaction was cooled evaporated *in vacuo* and partitioned between DCM (20 mL) and sodium bicarbonate solution (10 mL). The DCM was washed with sodium bicarbonate, dried by passing through a hydrophobic frit and evaporated *in vacuo.* The crude material was purified using a 120 g silica ISCO cartridge eluting with a gradient of 0-100% EtOAc in cyclohexane to give the title compound.
MS calcd for (C₁₇H₂₀BN₃O₃ + H)⁺ : 326
MS found (electrospray) : (M+H)⁺ = 326

### Intermediate 118

### Methyl 3-{cyclobutyl[(trans-4-methylcyclohexyl)carbonyl]amino}-5-{4-[(3-pyridazinylcarbonyl)amino]phenyl}-2-thiophenecarboxylate

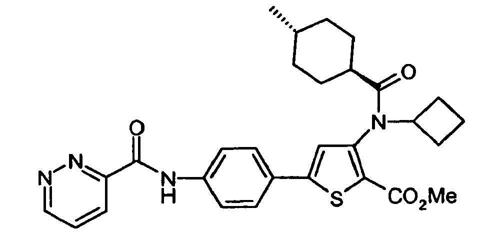

Methyl 3-{cyclobutyl[(*trans-*4-methylcyclohexyl)carbonyl]amino}-5-iodo-2-thiophenecarboxylate (100 mg, a synthesis of which is described as Intermediate 99), Intermediate 117 (70.5 mg), sodium carbonate (68.9 mg) and tetrakis(triphenylphosphine)palladium(0) (12.5 mg) in 1,4-dioxane (3 mL) and water (1 mL) was heated to 90°C under nitrogen for 2 h. The reaction was cooled, partitioned between DCM (10 mL) and saturated sodium bicarbonate solution (5 mL). The layers were separated, the organic layer dried using a hydrophobic frit and evaporated *in vacuo*. The crude material was purified using a 40 g silica ISCO cartridge eluting with a gradient of 0-100% EtOAc in cyclohexane to give the title compound.
MS calcd for (C₂₉H₃₂N₄O₄S + H)⁺ : 533
MS found (electrospray) : (M+H)⁺ = 533

### Intermediate 119

### 5-Bromo-3-chloro-2-pyridinamine

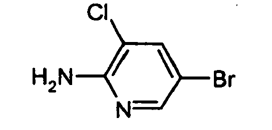

5-Bromo-2-pyridinamine (2 g) was added in small portions to stirred concentrated hydrochloric acid (9 mL), the solution was cooled to 0°C under nitrogen. Hydrogen peroxide (30% wt, 1.3 mL) was added dropwise to the stirred solution then the reaction was warmed to 21°C and stirred for 105 mins. A suspension formed and the mixture was allowed to stand overnight. A white solid was removed by filtration and the filtrate was neutralised by careful addition of saturated sodium carbonate solution. The mixture was extracted with DCM then the organic phase was separated using a hydrophobic frit and evaporated *in vacuo.* The crude material was purified using a silica ISCO cartridge eluting with a gradient of 0-15% EtOAc in cyclohexane to give the title compound.
MS calcd for (C₅H₅BrCIN₂+ H)⁺ : 207/209/211
MS found (electrospray) : (M+H)⁺ = 207/209/211

### Intermediate 120

### N-(5-Bromo-3-chloro-2-pyridinyl)-1,3-thiazole-4-carboxamide

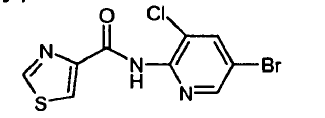

1,3-Thiazole-4-carbonyl chloride (50 mg) and Intermediate 119 (70 mg) in DMF (3 mL) were stirred under nitrogen at room temperature for 16 h. The reaction mixture was evaporated to dryness and washed with HCl. The organic layer was separated and washed with water followed by sodium bicarbonate. The organic layer was evaporated *in vacuo.* The crude material was purified using a silica ISCO cartridge eluting with a gradient of 0-100% EtOAc to give the title compound.
MS calcd for (C₉H₅BrClN₃OS+ H)⁺ : 318/320/322
MS found (electrospray) : (M+H)⁺ = 318/320/322

### Intermediate 121

### Methyl 5-{5-chloro-6-[(1,3-thiazol-4-ylcarbonyl)amino]-3-pyridinyl}-3-[[(trans-4-methylcyclohexyl)carbonyl](1-methylethyl)amino]-2-thiophenecarboxylate

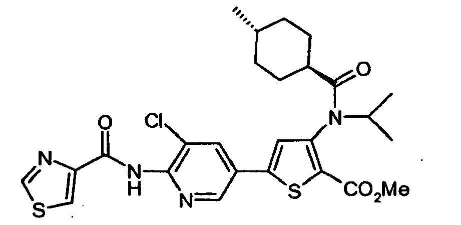

A solution of {4-[[(*trans-*4-methylcyclohexyl)carbonyl](1-methylethyl)amino]-5-[(methyloxy)carbonyl]-2-thienyl}boronic acid (115 mg, a synthesis of which is described as Intermediate 4), Intermediate 120 (20 mg), and tetrakis(triphenylphosphine)palladium (0) (7.25 mg), in 1,4-dioxane (1.5 mL) and water (5 mL) was heated in a microwave at 110°C for 45 mins. The reaction mixture was evaporated to dryness and then partitioned between DCM and water. The organic phase was separated and evaporated *in vacuo.* The crude material was purified using a silica ISCO cartridge eluting with a gradient of 0-100% EtOAc in cyclohexane to give the title compound.
MS calcd for (C₂₆H₂₉ClN₄O₄S₂+ H)⁺ : 561/563
MS found (electrospray) : (M+H)⁺ = 561/563

### Intermediate 122

### Methyl 5-(4-{[(5-fluoro-3-pyridinyl)carbonyl]amino}phenyl)-3-[[(trans-4-methylcyclohexyl)carbonyl](1-methylethyl)amino]-2-thiophenecarboxylate

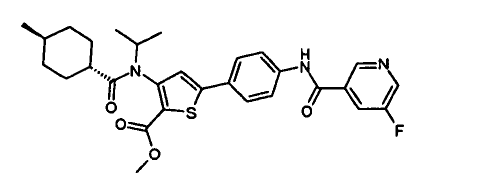

To 5-fluoro-3-pyridinecarboxylic acid (46 mg) was added anhydrous DMF (2 mL), DIPEA ' (114 uL) and then HATU (135 mg). The reaction mixture was stirred at room temperature for 10 mins. Methyl 5-(4-aminophenyl)-3-[[(*trans-*4-methylcyclohexyl}carbonyl](1-methylethyl)amino]-2-thiophenecarboxylate (135 mg, a synthesis of which is described as Intermediate 9) was added and the stirring continued for 16 h. The reaction was partitioned between DCM and saturated sodium bicarbonate solution. The organic phase was collected by passing through a hydrophobic frit and the aqueous phase was further extracted with DCM. The combined organic phases were concentrated to give a brown oil. The residue was loaded onto a 12 g silica ISCO cartridge. The cartridge was eluted with a gradient 5-100% EtOAc in cyclohexane to give the title compound.
MS calcd for (C₂₉H₃₂FN₃O₄S + H)⁺: 538
MS found (electrospray): (M+H)⁺ = 538

### Intermediate 123

### Methyl 3-[[(trans-4-methylcyclohexyl)carbonyl](1-methylethyl)amino]-5-{4-[(1,3-oxazol-5-ylcarbonyl)amino]phenyl}-2-thiophenecarboxylate

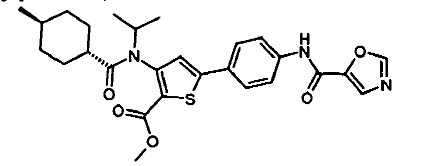

To a solution of 1,3-oxazole-5-carboxylic acid (30 mg) in DMF (4 mL) was added DIPEA (125 mg), followed by HATU (138 mg). The reaction was stirred at room temperature for 15 mins. Methyl 5-(4-aminophenyl)-3-[[(*trans-*4-methylcyclohexyl)carbonyl](1-methylethyl)amino]-2-thiophenecarboxylate (100 mg, a synthesis of which is described as Intermediate 9) was added and the reaction mixture was stirred at 50°C for 5 h. The reaction mixture was cooled and concentrated *in vacuo*. The residue was partitioned between DCM and sodium bicarbonate. The organic phase was collected and dried by passing through a hydrophobic frit and was concentrated *in vacuo.* The residue was loaded onto a 40g silica ISCO cartridge. The cartridge was eluted with a gradient 5-100% EtOAc in cyclohexane to give the title compound.
MS calcd for (C₂₇H₃₁N₃O₅S + H)⁺: 510
MS found (electrospray): (M+H)⁺ = 510

### Intermediate 124

### Methyl 3-[[(trans-4-methylcyclohexyl)carbonyl](1-methylethyl)amino]-5-{4-[(1,3-oxazol-4-ylcarbonyl)amino]phenyl}-2-thiophenecarboxylate

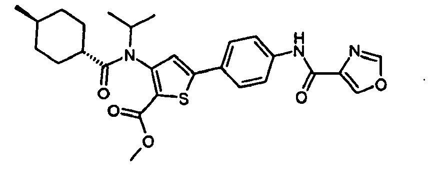

To a solution of 1,3-oxazole-4-carboxylic acid (30 mg) in DMF (4 mL) was added DIPEA (125 mg), followed by HATU (138 mg). The reaction was stirred at room temperature for 15 mins. Methyl 5-(4-aminophenyl)-3-[[(*trans-*4-methylcyclohexyl)carbonyl](1-methylethyl)amino]-2-thiophenecarboxylate (100 mg, a synthesis of which is described as Intermediate 9) was added and the reaction mixture was stirred at 50°C for 5 h. The reaction mixture was cooled and concentrated *in vacuo.* The residue was partitioned between DCM and sodium bicarbonate. The organic phase was collected and dried by passing through a hydrophobic frit and was concentrated *in vacuo.* The residue was loaded onto a 40g silica ISCO cartridge. The cartridge was eluted with a gradient 5-100% EtOAc in cyclohexane to give the title compound.
MS calcd for (C₂₇H₃₁N₃O₅S + H)⁺: 510
MS found (electrospray): (M+H)⁺ = 510

### Example 1

### 3-[[(trans-4-Methylcyclohexyl)carbonyl](1-methylethyl)amino]-5-{4-[(1,3-thiazol-4-ylcarbonyl)amino]phenyl}-2-thiophenecarboxylic acid

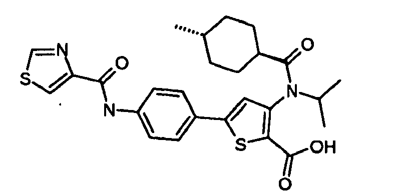

Intermediate 6 (310 mg) was dissolved in MeOH (3 mL) and THF (3 mL). 2N Sodium hydroxide solution (2 mL) was added and the reaction mixture was stirred at room temperature overnight. The mixture was diluted with DCM (15 mL) and 2N HCl (5 mL) was added. The mixture was stirred at room temperature for 30 mins. The organic phases were separated using a hydrophobic frit, and were evaporated *in vacuo.* The residue was purified by NH₂ SPE cartridge, eluting with MeOH (x 5 volumes), and 10% 2N HCl in MeOH to give the title compound.
MS calcd for (C₂₆H₂₉N₃O₄S₂ + H)⁺: 512
MS found (electrospray): (M+H)⁺ = 512
¹H NMR (CDCl₃): δ 9.44 (1H, s), 8.86 (1H, d), 8.35 (1H, d), 7.86 (2H, d), 7.69 (2H, d), 7.08 (1H, s), 5.05-4.93 (1H, m), 2.16-2.02 (1H, m), 1.79-1.55 (5H, m), 1.54-1.27 (2H, m), 1.24 (3H, d), 1.01 (3H, d), 0.79 (3H, d), 0.77-0.59 (2H, m), carboxylic acid proton not seen.

### Example 2

### Sodium 3-[[(trans-4-methylcyclohexyl)carbonyl](1-methylethyl)amino]-5-{4-[(1,3-thiazol-4-ylcarbonyl)amino]phenyl}-2-thiophenecarboxylate

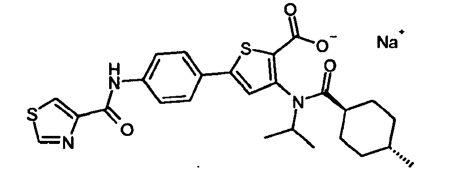

3-[[(*trans-*4-Methylcyclohexyl)carbonyl](1-methylethyl)amino]-5-{4-[(1,3-thiazol-4-ylcarbonyl)amino]phenyl}-2-thiophenecarboxylic acid (492 mg, a synthesis of which is described as Example 1) was dissolved in isopropanol (10 mL) with heating. 0.496 N Sodium hydroxide solution (2.04 mL) was added and the reaction mixture was left stirring at room temperature for 3 days. A solid precipitated out and this was collected by vacuum filtration. The solid was washed with ice cold isopropanol/water (5:1) and was collected and dried at 50°C under vacuum for 4 h to give the title compound.
MS calcd for (C₂₆H₂₈N₃O₄S₂ + H)⁺: 512
MS found (electrospray): (M+H)⁺ = 512
¹H NMR (d₆-DMSO): δ 10.46 (1H, s), 9.28 (1H, d), 8.53 (1H, d), 7.91 (2H, d), 7.64 (2H, d), 7.10 (1H, s), 4.67 (1H, quintet), 2.14 (1H, tt), 1.81 (1H, d), 1.61-1.48 (3H, m), 1.43-1.30 (1H, m), 1.27-1.14 (2H, m), 1.09 (3H, d), 0.92 (3H, d), 0.74 (3H, d), 0.69-0.46 (2H, m).

**Table 1: Characteristic X-Ray powder diffraction (XRPD) angles and d spacings for sodium 3-[[(trans-4-methylcyclohexyl)carbonyl](1-methylethyl)amino]-5-{4-[(1,3-thiazol-4-ylcarbonyl)amino]phenyl}-2-thiophenecarboxylate.**

| Pos. [°2Th.] | d-spacing [Å] |
|---|---|
| 4.2 | 20.9 |
| 6.3 | 14.0 |
| 8.4 | 10.5 |
| 8.7 | 10.2 |
| 12.6 | 7.0 |
| 15.0 | 5.9 |
| 16.9 | 5.2 |
| 17.3 | 5.1 |
| 18.6 | 4.8 |
| 20.6 | 4.3 |
| 21.1 | 4.2 |
| 2.1 | 4.0 |
| 22.6 | 3.9 |
| 23.2 | 3.8 |
| 23.7 | 3.8 |
| 24.9 | 3.6 |
| 25.3 | 3.5 |
| 26.3 | 3.4 |
| 29.1 | 3.1 |

Data obtained for sodium 3-[[(*trans-*4-methylcyclohexyl)carbonyl](1-methylethyl)amino]-5-{4-[(1,3-thiazol-4-ylcarbonyl)amino]phenyl}-2-thiophenecarboxylate can be seen in Figure 1.

### Example 3

### 3-[[(trans-4-Methylcyclohexyl)carbonyl](1-methylethyl)amino]-5-{4-[(2-pyridinylcarbonyl)amino]phenyl}-2-thiophenecarboxylic acid

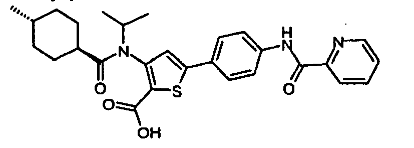

To a solution of Intermediate 10 (195 mg) in THF/MeOH (1:1, 6 mL) was added 2N sodium hydroxide (2 mL) and the reaction was stirred at room temperature for 18 h. DCM was added and the mixture was acidified with 2N HCl to pH 5.5 (using a pH meter). The organic phases were separated using a hydrophobic frit and were passed through a SCX SPE cartridge, eluting with DCM followed by MeOH. The fractions were evaporated *in vacuo* to give the title compound.
MS calcd for (C₂₈H₃₁N₃O₄S + H)⁺: 506
MS found (electrospray): (M+H)⁺ = 506
¹H NMR (CD₃OD): δ 8.72 (1H, d), 8.23 (1H, dt), 8.04 (1H, dt), 7.93 (2H, d), 7.76 (2H, d), 7.65-7.60 (1H, m), 7.33 (1H, s), 4.83 (1H, m, obscured by water peak), 2.11 (1H, tt), 1.80-1.49 (5H, m), 1.44-1.18 (5H, m), 1.00 (3H, d), 0.80-0.55 (5H, m), 2 exchangeable protons not seen.

### Example 4

### 5-{4-[(2-Furanylcarbonyl)amino]phenyl}-3-[[(trans-4-methylcyclohexyl)carbonyl](1-methylethyl)amino]-2-thiophenecarboxylic acid

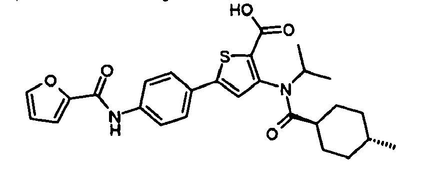

To a solution of Intermediate 11 (204 mg) in THF/MeOH (1:1, 6 mL) was added 2N sodium hydroxide (2 mL) and the reaction was stirred at room temperature for 18 h. DCM was added and the mixture was acidified with 2N HCl to pH 2. The organic phases were separated using a hydrophobic frit and were passed through a SCX SPE cartridge, eluting with DCM followed by MeOH. The fractions were evaporated *in vacuo* to give the title compound.
MS calcd for (C₂₇H₃₀N₂O₅S + H)⁺: 495
MS found (electrospray): (M+H)⁺ = 495
¹H NMR (CD₃OD): δ 7.83 (2H, d), 7.76-7.68 (3H, m), 7.31 (1H, s), 7.28 (1H, d), 6.64 (1H, dd), 4.85 (1H, m, partially obscured by water peak), 2.10 (1H, tt), 1.80-1.48 (5H, m), 1.44-1.17 (5H, m), 0.99 (3H, d), 0.79-0.53 (5H, m), 2 exchangeable protons not seen.

### Example 5

### 3-[[(trans-4-Methylcyclohexyl)carbonyl](1-methylethyl)amino]-5-(4-{[(2-methyl-1,3-thiazol-4-yl)carbonyl]amino}phenyl)-2-thiophenecarboxylic acid

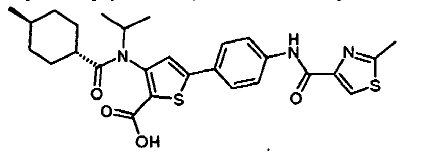

To a solution of Intermediate 12 (191 mg) in THF/MeOH (1:1, 6 mL) was added 2N sodium hydroxide (2 mL) and the reaction was stirred at room temperature for 18 h. DCM was added and the mixture was acidified with 2N HCl to pH 2. The organic phases were separated using a hydrophobic frit and were passed through a SCX SPE cartridge, eluting with DCM followed by MeOH. The fractions were evaporated *in vacuo* to give the title compound.
MS calcd for (C₂₇H₃₁N₃O₄S₂ + H)⁺: 526
MS found (electrospray): (M+H)⁺ = 526
¹H NMR (CD₃OD): δ 8.18 (1H, s), 7.87 (2H, d), 7.74 (2H, d), 7.32 (1H, s), 4.83 (1H, m, partially obscured by water peak), 2.78 (3H, s), 2.11 (1H, tt), 1.80-1.51 (5H, m), 1.43-1.20 (5H, m), 1.00 (3H, d), 0.81-0.56 (5H, m), 2 exchangeable protons not seen.

### Example 6

### 3-[[(trans-4-Methylcyclohexyl)carbonyl](1-methylethyl)amino]-5-{4-[(2-thienylcarbonyl)amino]phenyl}-2-thiophenecarboxylic acid

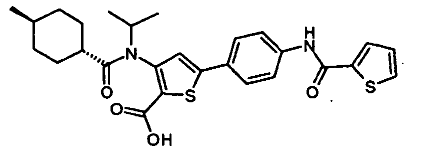

To a solution of Intermediate 13 (254 mg) in THF/MeOH (1:1, 6 mL) was added 2N sodium hydroxide (2 mL) and the reaction was stirred at room temperature for 18 h. DCM was added and the mixture was acidified with 2N HCl to pH 2. The organic phases were separated using a hydrophobic frit and were passed through a SCX SPE cartridge, eluting with DCM followed by MeOH. The fractions were evaporated *in vacuo* to give the title compound.
MS calcd for (C₂₇H₃₀N₂O₄S₂ + H)⁺: 511
MS found (electrospray): (M+H)⁺ = 511
¹H NMR (CD₃OD): δ 7.92 (1H, dd), 7.82 (2H, d), 7.77-7.71 (3H, m), 7.32 (1H, s), 7.18 (1H, dd), 4.83 (1H, m, obscured by water peak), 2.11 (1H, tt), 1.80-1.51 (5H, m), 1.45-1.19 (5H, m), 1.00 (3H, d), 0.81-0.56 (5H, m), 2 exchangeable protons not seen.

### Example 7

### 3-[[(trans-4-Methylcyclohexyl)carbonyl](1-methylethyl)amino]-5-{4-[(2-pyrazinylcarbonyl)amino]phenyl}-2-thiophenecarboxylic acid

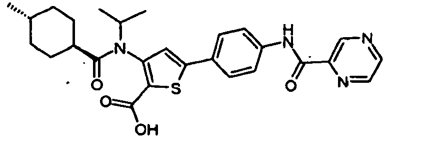

To a solution of Intermediate 14 (114 mg) in THF/MeOH (1:1, 6 mL) was added 2N sodium hydroxide (2 mL) and the reaction was stirred at room temperature for 18 h. DCM was added and the mixture was acidified with 2N HCl to pH 2. The organic phases were separated using a hydrophobic frit and were passed through a SCX SPE cartridge, eluting with DCM followed by MeOH. The fractions were evaporated *in vacuo.* The material was purified further by MDAP HPLC to give the title compound.
MS calcd for (C₂₇H₃₀N₄O₄S + H)⁺: 507
MS found (electrospray): (M+H)⁺ = 507
¹H NMR (CD₃OD): δ 9.37 (1H, d), 8.85 (1H, d), 8.77 (1H, dd), 8.16 (1H, br), 7.96 (2H, d), 7.79 (2H, d), 7.33 (1H, s), 4.86 (1H, m, obscured by water peak), 2.14 (1H, tt), 1.83-1.51 (5H, m), 1.45-1.21 (5H, m), 1.02 (3H, d), 0.82-0.57 (5H, m), carboxylic acid proton not seen.

### Example 8

### 5-(4-{[(4-Fluorophenyl)carbonyl]amino}phenyl)-3-[[(trans-4-methylcyclohexyl)carbonyl](1-methylethyl)amino]-2-thiophenecarboxylic acid

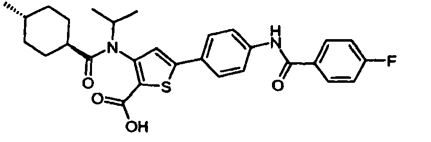

To a solution of Intermediate 15 (117 mg) in THF/MeOH (1:1, 6 mL) was added 2N sodium hydroxide (2 mL) and the reaction was stirred at room temperature for 18 h. DCM was added and the mixture was acidified with 2N HCl to pH 2. The organic phases were separated using a hydrophobic frit and were passed through a SCX SPE cartridge, eluting with DCM followed by MeOH. The fractions were evaporated *in vacuo* to give the title compound.
MS calcd for (C₂₉H₃₁FN₂O₄S + H)⁺: 523
MS found (electrospray): (M+H)⁺ = 523
¹H NMR (CD₃OD): δ 8.10-8.02 (2H, m), 7.90 (2H, d), 7.82(2H, d), 7.49 (1H, s), 7.42-7.35 (2H, m), 4.75 (1H, quintet), 2.04-1.93 (1H, m), 1.67-1.40 (5H, m), 1.32-1.08 (6H, m), 0.90 (3H, d), 0.77-0.48 (5H, m), carboxylic acid proton not seen.

### Example 9

### 3-[[(trans-4-Methylcyclohexyl)carbonyl](1-methylethyl)amino]-5-{4-[(1,3-oxazol-2-ylcarbonyl)amino]phenyl}-2-thiophenecarboxylic acid

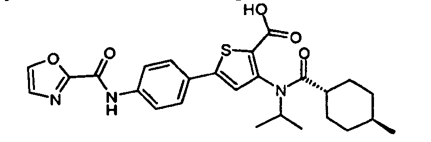

To a solution of Intermediate 16 (142 mg) in THF/MeOH (1:1, 6 mL) was added 2N sodium hydroxide (2 mL) and the reaction was stirred at room temperature for 18 h. DCM was added and the mixture was acidified with 2N HCl to pH 2. The organic phases were separated using a hydrophobic frit and were passed through a SCX SPE cartridge, eluting with DCM followed by MeOH. The fractions were evaporated *in vacuo.* The material was purified further by MDAP HPLC to give the title compound.
MS calcd for (C₂₆H₂₉N₃O₅S + H)⁺: 496
MS found (electrospray): (M+H)⁺ = 496
¹H NMR (CD₃OD): δ 8.20 (1H, br), 8.15 (1H, d), 7.87 (2H, d), 7.76 (2H, d), 7.43 (1H, d), 7.31 (1H, s), 4.84 (1H, m, obscured by water peak), 2.13 (1H, tt), 1.84-1.49 (5H, m), 1.44-1.19 (5H, m), 1.01 (3H, d), 0.81-0.56 (5H, m), carboxylic acid proton not seen.

### Example 10

### 3-{(Cyclopropylmethyl)[(trans-4-methylcyclohexyl)carbonyl]amino}-5-{4-[(1,3-thiazol-4-ylcarbonyl)amino]phenyl}-2-thiophenecarboxylic acid

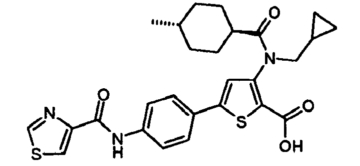

To Intermediate 20 (150 mg) was added DCE (2.25 mL) and *trans-*4-methylcyclohexanecarbonyl chloride (88 mg, a synthesis of which is described as Intermediate 1). The reaction was heated at reflux for 16 h. The cooled reaction was diluted with MeOH, passed through a Strata cartridge and evaporated *in vacuo.* To the residue was added THF (3 mL), MeOH (3 mL) and 2M lithium hydroxide (3 mL). The reaction was stirred for 16 h at room temperature. The reaction was neutralised with 2N HCl (3 mL) and was partitioned between DCM and water. The organic phase was separated and purified by reverse phase ISCO Companion^{™} chromatography, using a C18 cartridge, to give the title compound.
MS calcd for (C₂₇H₂₉N₃O₄S₂ + H)⁺: 524
MS found (electrospray): (M+H)⁺ = 524
¹H NMR (d₆-DMSO): δ 13.20 (1H, br), 10.58 (1H, s), 9.29 (1H, d), 8.54 (1H, d), 7.99 (2H, d), 7.78 (2H, d), 7.57 (1H, s), 3.61 (1H, dd), 2.14 (1H, tt), 1.73-1.53 (4H, m), 1.48-1.17 (3H, m), 0.92-0.82 (1H, m), 0.75 (3H, d), 0.72-0.56 (2H, m), 0.37-0.30 (2H, m), 0.03- -0.05 (2H, m), one proton not seen, obscured by water peak.

### Example 11

### 3-((Cyclopropylmethyl){[trans-4-(trifluoromethyl)cyclohexyl]carbonyl}amino)-5-{4-[(1,3-thiazol-4-ylcarbonyl)amino]phenyl}-2-thiophenecarboxylic acid

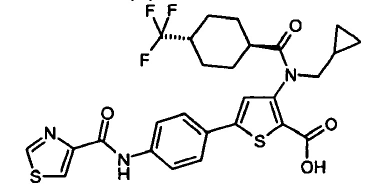

To Intermediate 20 (150 mg) was added DCE (2.25 mL) and *trans*-4-(trifluoromethyl)cyclohexanecarbonyl chloride (188 mg, a synthesis of which is described as Intermediate 22). The reaction was heated at reflux for 16 h. The cooled reaction was diluted with MeOH, passed through a Strata cartridge and was evaporated *in vacuo*. To the residue was added THF (3 mL), MeOH (3 mL) and 2M lithium hydroxide (3 mL). The reaction was stirred for 16 h at room temperature. The reaction was neutralised with 2N HCl (3 mL) and was partitioned between DCM and water. The organic phase was separated and purified by reverse phase ISCO Companion^{™} chromatography, using a C18 cartridge, to give the title compound.
MS calcd for (C₂₇H₂₆F₃N₃O₄S₂ + H)⁺: 578
MS found (electrospray): (M+H)⁺ = 578
¹H NMR (d₆-DMSO): δ 13.21 (1H, br), 10.58 (1H, s), 9.29 (1H, d), 8.54 (1H, d), 8.00 (2H, d), 7.78 (2H, d), 7.57 (1H, s), 3.64-3.56 (1H, m), 2.30-2.16 (2H, m), 1.85-1.66 (4H, m), 1.57-1.32 (2H, m), 1.10-0.81 (3H, m), 0.77-0.20 (2H, m), 0.04- -0.04 (2H, m), 1 proton not seen, obscured by water peak.

### Example 12

### 3-((1-Methylethyl){[trans-4-(trifluoromethyl)cyclohexyl]carbonyl}amino)-5-{4-[(1,3-thiazol-4-ylcarbonyl)amino]phenyl}-2-thiophenecarboxylic acid

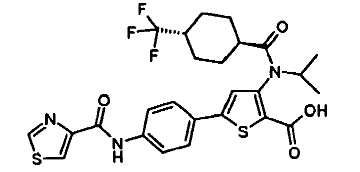

A solution of Intermediate 25 (138.4 mg), 2M sodium hydroxide solution (1 mL), MeOH (1 mL) and THF (1 mL) was stirred at room temperature for 20 h. The reaction mixture was partitioned between DCM (1 mL) and 2M HCl (1 mL). The organic phases were separated and the aqueous layer was washed with DCM (1 mL). The organic phases were evaporated *in vacuo.* The crude material was purified by SCX cartridge, followed by NH₂ SPE cartridge to give the title compound.
MS calcd for (C₂₆H₂₆F₃N₃O₄S₂ + H)⁺: 566
MS found (electrospray): (M+H)⁺ = 566
¹H NMR (CD₃OD): δ 10.57 (1H, s), 9.29 (1H, d), 8.54 (1H, d), 8.00 (2H, d), 7.81 (2H, d), 7.51 (1H, s), 4.75 (1H, quintet), 2.27-2.13 (1H, m), 2.12-2.02 (1H, m), 1.85-1.68 (4H, m), 1.63-1.49 (1H, m), 1.40-1.26 (1H, m), 1.15 (3H, d), 1.09-0.87 (5H, m), carboxylic acid proton not seen.

### Example 13

### 3-[[(trans-4-Methylcyclohexyl)carbonyl](1-methylethyl)amino]-5-{4-[(3-pyridazinylcarbonyl)amino]phenyl}-2-thiophenecarboxylic acid

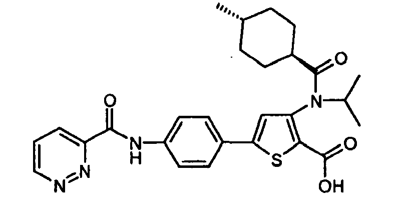

To a solution of Intermediate 26 (196 mg) in MeOH (3 mL) and THF (3 mL) was added 2N sodium hydroxide solution (1.5 mL) and the reaction was stirred at room temperature for 1 h. The mixture was diluted with DCM and acidified using 2N HCl. The organic phases were collected and dried using a hydrophobic frit. The crude material was purified using a SCX SPE cartridge, eluting with MeOH. The material was purified further by MDAP HPLC. The material was purified further by NH₂ SPE cartridge, eluting with 10% AcOH in MeOH. The appropriate fractions were concentrated *in vacuo* and then azeotroped with toluene (x 2). The material was freeze dried to give the title compound.
MS calcd for (C₂₇H₃₀N₄O₄S + H)⁺: 507
MS found (electrospray): (M+H)⁺ = 507
¹H NMR (CD₃OD): δ 13.30 (1H br), 11.29 (1H, s), 9.49 (1H, dd), 8.34 (1H, dd), 8.08 (2H, d), 8.00 (1H, dd), 7.83 (2H, d), 7.47 (1H, br), 4.74 (1H, quintet), 2.05-1.96 (1H, m), 1.69-1.39 (5H, m), 1.30-1.17 (2H, m), 1.14 (3H, d), 0.91 (3H, d), 0.74 (3H, d), 0.71-0.48 (2H, m).

### Example 14

### 3-[[(trans-4-Methylcyclohexyl)carbonyl](1-methylethyl)amino]-5-{4-[(4-pyrimidinylcarbonyl)amino]phenyl}-2-thiophenecarboxylic acid

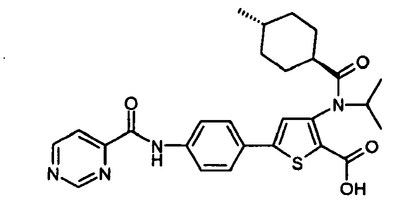

To a solution of Intermediate 27 (278 mg) in MeOH (3 mL) and THF (3 mL) was added 2N sodium hydroxide solution (1.5 mL) and the reaction was stirred at room temperature for 1 h. The mixture was diluted with DCM and acidified using 2N HCl. The organic phases were collected and dried using a hydrophobic frit. The crude material was purified using a SCX SPE cartridge, eluting with MeOH. The material was purified further by MDAP HPLC. The material was purified further by NH₂ SPE cartridge, eluting with 10% AcOH in MeOH and was azeotroped with toluene (x 2). The material was freeze dried to give the title compound.
MS calcd for (C₂₇H₃₀N₄O₄S + H)⁺: 507
MS found (electrospray): (M+H)⁺ = 507
¹H NMR (CD₃OD): δ 13.31 (1H, br), 11.06 (1H, s), 9.44 (1H, d), 9.15 (1H, d), 8.16 (1H, dd), 8.05 (2H, d), 7.83 (2H, d), 7.47 (1H, s), 4.74 (1H, quintet), 2.04-1.95 (1H, m), 1.68-1.39 (5H, m), 1.31-1.17 (2H, m), 1.13 (3H, d), 0.90 (3H, d), 0.74 (3H, d), 0.71-0.47 (2H, m).

### Example 15

### 5-{3-Chloro-4-[(1,3-thiazol-4-ylcarbonyl)amino]phenyl}-3-[[(trans-4-methylcyclohexyl)carbonyl](1-methylethyl)amino]-2-thiophenecarboxylic acid

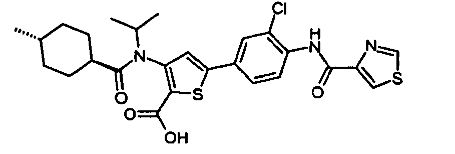

To a solution of Intermediate 29 (61 mg) in MeOH (2 mL) and THF (2 mL) was added 2N sodium hydroxide solution (1 mL) and the reaction was stirred at room temperature for 15 h. The mixture was diluted with DCM and acidified with 2N HCl. The organic phases were separated, dried using a hydrophobic frit and evaporated *in vacuo.* The crude material was purified by MDAP HPLC but the sample had to be retrieved from the waste. The sample was re-purified by reverse phase HPLC using a Supelco ABZ + plus 100 x 21.2 mm, 5 um column, flow rate 20 mL/min, 2 injections of 0.5 mL each, eluting with 75-99% MeCN (0.05% formic acid) in water (0.1% formic acid). The material was freeze-dried to give the title compound.
MS calcd for (C₂₆H₂₈ClN₃O₄S₂ + H)⁺: 546/548
MS found (electrospray): (M+H)⁺ = 546/548
¹H NMR (CD₃OD): δ 9.13 (1H, d), 8.60 (1H, d), 8.49 (1H, d), 7.96 (1H, d), 7.79 (1H, dd), 7.45 (1H, s), 4.87 (1H, m, obscured by water peak), 2.17-2.08 (1H, m), 1.83-1.52 (5H, m), 1.47-1.21 (5H, m), 1.02 (3H, d), 0.83-0.60 (5H, m), 2 exchangeable protons not seen.

### Example 16

### 3-[[(trans-4-Methylcyclohexyl)carbonyl](1-methylethyl)amino]-5-{4-[(3-thienylcarbonyl)amino]phenyl}-2-thiophenecarboxylic acid

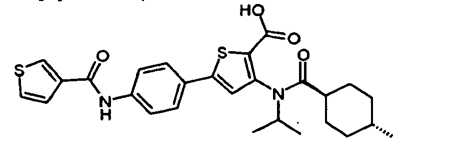

To a solution of Intermediate 30 (181 mg) in MeOH (3 mL) and THF (3 mL) was added 2N sodium hydroxide solution (1.5 mL) and the reaction was stirred at room temperature for 1 h. The mixture was diluted with DCM and acidified with 2N HCl. The organic phases were separated, dried using a hydrophobic frit and were applied to a SCX SPE cartridge which was eluted with MeOH. The material was purified further by MDAP HPLC to give the title compound.
MS calcd for (C₂₇H₃₀N₂O₄S₂ + H)⁺: 511
MS found (electrospray): (M+H)⁺ = 511
¹H NMR (CD₃OD): δ 8.27 (1H, dd), 7.85 (2H, d), 7.76 (2H, d), 7.66 (1H, dd), 7.55 (1H, dd), 7.33 (1H, s), 4.87 (1H, m, partially obscured by water peak), 2.14 (1H, tt), 1.85-1.52 (5H, m) 1.46-1.21 (5H, m), 1.02 (3H, d), 0.84-0.58 (5H, m), 2 exchangeable protons not seen.

### Example 17

### 5-(3-Chloro-4-{[(4-fluorophenyl)carbonyl]amino}phenyl)-3-[[(trans-4-methylcyclohexyl)carbonyl](1-methylethyl)amino]-2-thiophenecarboxylic acid

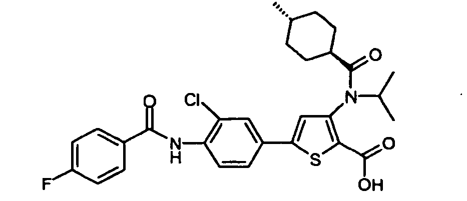

To a solution of Intermediate 31 (226 mg) in MeOH (2 mL) and THF (2 mL) was added 2N sodium hydroxide solution (1.0 mL) and the reaction was stirred at room temperature for 8 h. The mixture was diluted with DCM and acidified with 2N HCl. The organic phases were separated, dried using a hydrophobic frit and evaporated *in vacuo.* The crude material was purified by reverse phase HPLC using a Luna C18(2) 100 x 21.2 mm, 5 um column, flow rate 20 mL/min, 9.5 mg loading, eluting with 55-99% MeCN (0.05% TFA) in water (0.1% TFA) to give the title compound.
MS calcd for (C₂₉H₃₀ClFN₂O₄S + H)⁺: 557/559
MS found (electrospray): (M+H)⁺ = 557/559
¹H NMR (d₆-DMSO): δ 13.38 (1H, br), 10.20 (1H, s), 8.15-8.05 (3H, m), 7.86-7.78 (1H, m), 7.75-7.66 (2H, m), 7.45-7.35 (2H, m), 4.75 (1H, quintet), 2.02-1.91 (1H, m), 1.69-1.41 (5H, m), 1.32-1.11 (5H, m), 0.99 (3H, d), 0.81-0.49 (5H, m).

### Example 18

### 4-[[(trans-4-Methylcyclohexyl)carbonyl](1-methylethyl)amino]-5'-[(2-pyridinylcarbonyl)amino]-2,2'-bithiophene-5-carboxylic acid

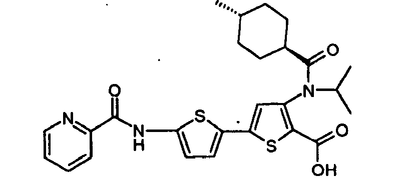

To a solution of Intermediate 34 (110 mg) in MeOH (3 mL) and THF (3 mL) was added 2N sodium hydroxide solution (1.5 mL) and the reaction was stirred at room temperature for 4 days. The mixture was diluted with DCM and acidified with 2N HCl. The organic phases were separated using a hydrophobic frit and were applied to a NH₂ SPE cartridge. The cartridge was washed with MeOH (x 6 column volumes) and was eluted with 10% AcOH in MeOH. The material was evaporated *in vacuo* and the residue was azeotroped with toluene (x 2), then freeze dried to give the title compound.
MS calcd for (C₂₆H₂₉N₃O₄S₂ + H)⁺: 512
MS found (electrospray): (M+H)⁺ = 512
¹H NMR (d₆-DMSO): δ 13.25 (1H, br), 12.33 (1H, s), 8.78 (1H, d), 8.17 (1H, d), 8.13-8.07 (1H, m), 7.74-7.69 (1H, m), 7.41-7.34 (1H, m), 7.19-7.10 (2H, m), 4.72 (1H, quintet), 2.07-1.95 (1H, m), 1.70-1.35 (5H, m), 1.31-1.08 (5H, m), 0.91 (3H, d), 0.79-0.50 (5H, m).

### Example 19

### 3-[[(trans-4-Methylcyclohexyl)carbonyl](1-methylethyl)amino]-5-{4-{[(1-methyl-1H-pyrazol-4-yl)carbonyl]amino}phenyl)-2-thiophenecarboxylic acid

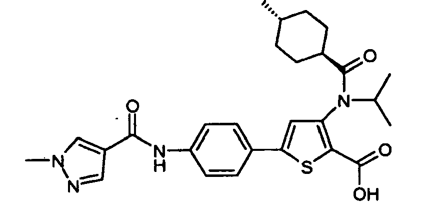

Intermediate 35 (202 mg) was dissolved in pyridine (1.5 mL). Lithium iodide (517 mg) was added and the reaction was stirred at 130°C in a Reactivial^{™} for 7 h, then at room temperature over a weekend. The mixture was partitioned between DCM and 2N HCl The organic phases were separated, dried using a hydrophobic frit and evaporated *in vacuo.* The crude material was purified by MDAP HPLC to give the title compound.
MS calcd for (C₂₇H₃₂N₄O₄S + H)⁺: 509
MS found (electrospray): (M+H)⁺= 509
¹H NMR (CD₃OD): δ 8.20 (1H, s), 8.04 (1 h, s), 7.83-7.69 (4H, m), 7.28 (1H, s), 4.83 (1H, m, obscured by water peak), 3.95 (3H, s), 2.19-2.08 (1H, m), 1.85-1.49 (5H, m), 1.45-1.18 (5H, m), 1.01 (3H, d), 0.82-0.55 (5H, m), 2 exchangeable protons not seen.

### Example 20

### 3-[[(trans-4-Methylcyclohexyl)carbonyl](1-methylethyl)amino]-5-(4-{[(5-methyl-3-isoxazolyl)carbonyl]amino}phenyl)-2-thiophenecarboxylic acid

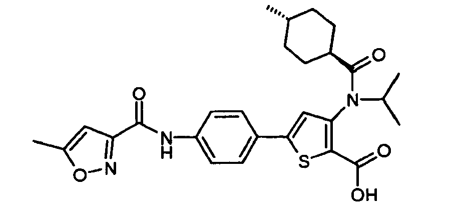

Intermediate 36 (194 mg) was dissolved in pyridine (1.5 mL). Lithium iodide (743 mg) was added and the reaction was stirred at 130°C in a Reactivial^{™} for 6 h. The mixture was partitioned between DCM and 2N HCI. The organics were separated, and the aqueous was re-extracted with DCM. The combined organic phases were dried using a hydrophobic frit and evaporated *in vacuo.* The crude material was purified by NH₂ SPE cartridge, eluting with 10% 2N HCl in MeOH to give the title compound.
MS calcd for (C₂₇H₃₁N₃O₅S + H)⁺: 510
MS found (electrospray): (M+H)⁺ = 510
¹H NMR (d₆-DMSO): δ 13.27 (1H, br), 10.85 (1H, s), 7.98-7.78 (4H, m), 7.50 (1H, s), 6.69 (1H, s), 4.74 (1H, quintet), 2.51 (3H, m, partially obscured by solvent peak), 2.03-1.91 (1H, m), 1.72-1.40 (5H, m), 1.34-1.07 (5H, m), 0.90 (3H, d), 0.79-0.47 (5H, m).

### Example 21

### 4-[[(trans-4-Methylcyclohexyl)carbonyl](1-methylethyl)amino]-5'-[(1,3-thiazol-4-ylcarbonyl)amino]-2,2'-bithiophene-5-carboxylic acid

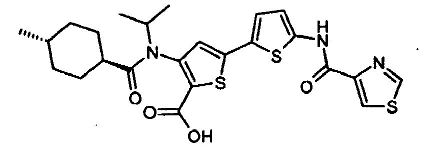

To a solution of Intermediate 37 (96 mg) in MeOH (3 mL) and THF (3 mL) was added 2N sodium hydroxide solution (1 mL) and the reaction was stirred at room temperature for 50 h. The mixture was diluted with DCM and acidified with 2N HCl. The organic phases were separated, dried using a hydrophobic frit and evaporated *in vacuo.* The crude material was purified by MDAP HPLC and was freeze-dried to give the title compound.
MS calcd for (C₂₄H₂₇N₃O₄S₃ + H)⁺: 518
MS found (electrospray): (M+H)⁺= 518
¹H NMR (CD₃OD): δ 9.09 (1H, d), 8.44 (1H, d), 7.29 (1H, d), 7.06 (1H, s), 6.97 (1H, d), 4.83 (1H, m, partially obscured by water peak), 2.13 (1H, tt), 1.81-1.50 (5H, m), 1.44-1.18 (5H, m), 1.00 (3H, d), 0.82-0.59 (5H, m), 2 exchangeable protons not seen.

### General method for Examples 22-41

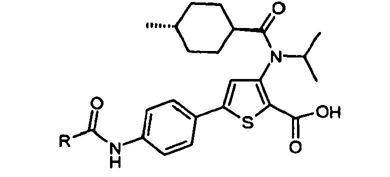

Carboxylic acid (0.266 mmol), DMF (0.5 mL) and DIPEA (0.084 mL) were added to a Greenhouse Plus tube. HATU (101 mg) in DMF (1 mL) was added and the reaction was stirred for 1 h. Methyl 5-(4-aminophenyl)-3-[[(trans-4-methylcyclohexyl)carbonyl](1-methylethyl)amino]-2-thiophenecarboxylate (100 mg, a synthesis of which is described as Intermediate 9) in DMF (1 mL) was then added and the reaction was stirred overnight at room temperature. The solvent was evaporated *in vacuo* using a Genevac vacuum centrifuge. To the residue was added THF (1 mL), MeOH (1 mL) and 2M lithium hydroxide solution (1 mL). The reaction was stirred for 24 h. The mixture was neutralised with 2N HCl (1 mL), then diluted with DCM (3 mL) and water (1 mL). The mixture was stirred for 1 h then separated using a hydrophobic frit. If the samples were not fully soluble in DCM the aqueous was extracted further with EtOAc and the organic phases separated and combined). The organic phases were evaporated *in vacuo* and the crude material was purified by MDAP HPLC to give the title compound.

### Example 22

### 3-[[(trans-4-Methylcyclohexyl)carbonyl](1-methylethyl)amino)-5-{4-[(1H-pyrazol-3-ylcarbonyl)amino]phenyl}-2-thiophenecarboxylic acid

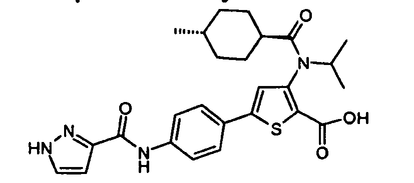

MS calcd for (C₂₆H₃₀N₄O₄S + H)⁺: 495
MS found (electrospray): (M+H)⁺= 495
¹H NMR (d₆-DMSO): δ 13.50 (1H, br), 10.28 (1H, s), 7.99-7.91 (2H, m), 7.88 (1H, s), 7.77 (2H, d), 7.44 (1H. s), 6.81 (1H, s), 4.72 (1H, quintet), 2.00 (1H, tt), 1.66-1.40 (5H, m), 1.29-1.18 (2H, m), 1.14 (3H, d), 0.90 (3H, d), 0.73 (3H, d), 0.69-0.48 (2H, m), 1 exchangeable proton not seen.

### Example 23

### 3-[[(trans-4-Methylcyclohexyl)carbonyl](1-methylethyl)amino]-5-(4-{[(5-methyl-1H-pyrazol-3-yl)carbonyl]amino}phenyl)-2-thiophenecarboxylic acid

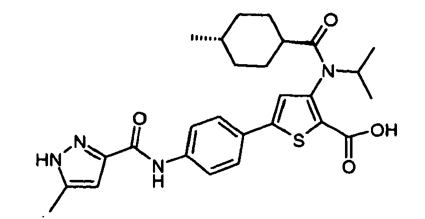

MS calcd for (C₂₇H₃₂N₄O₄S + H)⁺: 509
MS found (electrospray): (M+H)⁺= 509
¹H NMR (d₆-DMSO): δ 13.15 (1H, br), 10.17 (1H, s), 7.94 (2H, d), 7.75 (2H, d), 7.40 (1H, s), 6.54 (1H, s), 4.73 (1H, quintet), 2.29 (3H, s), 1.98 (1H, t), 1.70-1.49 (5H, m), 1.29-1.07 (5H, m), 0.88 (3H, d), 0.70 (3H, d), 0.69-0.48 (2H, m), 1 exchangeable proton not seen.

### Example 24

### 5-{4-[(1H-Imidazol-4-ylcarbonyl)amino]phenyl}-3-[[(trans-4-methylcyclohexyl)carbonyl](1-methylethyl)amino]-2-thiophenecarboxylic acid

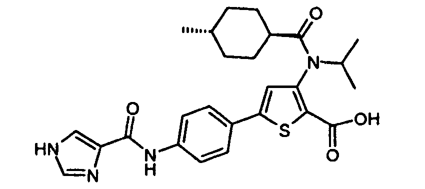

MS calcd for (C₂₆H₃₀N₄O₄S + H)⁺: 495
MS found (electrospray): (M+H)⁺= 495
¹H NMR (d₆-DMSO): δ 12.78 (1H, br), 10.08 (1H, s), 7.97 (2H, d), 7.85 (2H, d), 7.75 (2H, d), 7.42 (1H, s), 4.73 (1H, quintet), 1.99 (1H, t), 1.69-1.40 (5H, m), 1.31-1.10 (5H, m), 0.90 (3H, d), 0.72 (3H, d), 0.70-0.47 (2H, m), 1 exchangeable proton not seen.

### Example 25

### 5-(4-{[(1,5-Dimethyl-1H-pyrazol-3-yl)carbonyl]amino}phenyl)-3-[[(trans-4-methylcyclohexyl)carbonyl](1-methylethyl)amino]-2-thiophenecarboxylic acid

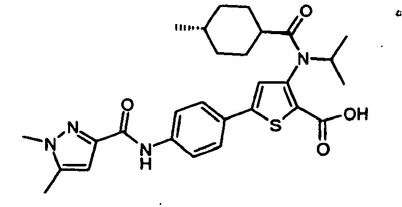

MS calcd for (C₂₈H₃₄N₄O₄S + H)⁺: 523
MS found (electrospray): (M+H)⁺ = 523
¹H NMR (d₆-DMSO): δ 10.11 (1H, s), 7.91 (2H, d), 7.74 (2H, d), 7.41 (1H, s), 6.56 (1H, s), 4.70 (1H, quintet), 3.81 (3H, s), 2.30 (3H, s), 1.97 (1H, t), 1.68-1.38 (5H, m), 1.28-1.10 (5H, m), 0.88 (3H, d), 0.73 (3H, d), 0.69-0.48 (2H, m), 1 exchangeable proton not seen.

### Example 26

### 3-[[(trans-4-Methylcyclohexyl)carbonyl](1-methylethyl)amino]-5-(4-{[(5-methyl-2-pyrazinyl)carbonyl]amino}phenyl)-2-thiophenecarboxylic acid

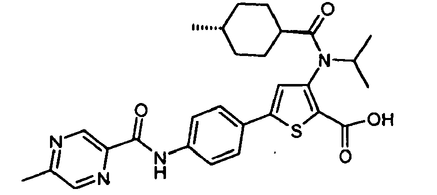

MS calcd for (C₂₈H₃₂N₄O₄S + H)⁺: 521
MS found (electrospray): (M+H)⁺= 521
¹H NMR (d₆-DMSO): δ 10.87 (1H, s), 9.19 (1H, s), 8.73 (1H, s), 8.03 (2H, d), 7.79 (2H, d), 7.38 (1H, s), 4.72 (1H, quintet), 2.66 (3H, s), 2.03 (1H, t), 1.77-1.38 (5H, m), 1.30-1.09 (5H, m), 0.89 (3H, d), 0.73 (3H, d), 0.71-0.48 (2H, m), 1 exchangeable proton not seen.

### Example 27

### 3-[[(trans-4-Methylcyclohexyl)carbonyl](1-methylethyl)amino]-5-(4-{[(3-methyl-5-isoxazolyl)carbonyl]amino}phenyl)-2-thiophenecarboxylic acid

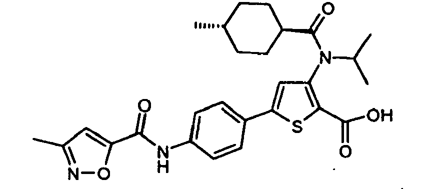

MS calcd for (C₂₇H₃₁N₃OₛS + H)⁺: 510
MS found (electrospray): (M+H)⁺ = 510
¹H NMR (d₆-DMSO): δ 10.82 (1H, s), 7.86 (2H, d), 7.71 (2H, d), 7.24 (1H, s), 7.17 (1H, s), 4.71 (1H, quintet), 2.49 (3H, s); 2.14-2.03 (1H, m),1.60-1.30 (5H, m), 1.28-1.15 (2H, m), 1.10 (3H, d), 0.90 (3H, d), 0.72 (3H, d), 0.67-0.45 (2H, m), 1 exchangeable proton not seen.

### Example 28

### 3-[[(trans-4-Methylcyclohexyl)carbonyl](1-methylethyl)amino]-5-(4-{[(4-methyl-1,3-thiazol-5-yl)carbonyl]amino}phenyl)-2-thiophenecarboxylic acid

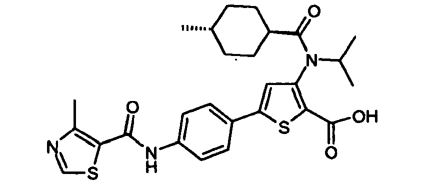

MS calcd for (C₂₇H₃₁N₃O₄S₂ + H)⁺: 526
MS found (electrospray): (M+H)⁺ = 526
¹H NMR (d₆-DMSO): δ 10.42 (1H, s), 9.13 (1H, s), 7.80 (4H, s), 7.44 (1H, s), 4.52 (1H, quintet), 2.62 (3H, s), 1.99 (1H, t), 1.69-1.40 (5H, m), 1.30-1.10 (5H, m), 0.90 (3H, d), 0.77 (3H, d), 0.70-0.47 (2H, m), 1 exchangeable proton not seen.

### Example 29

### 3-[[(trans-4-Methylcyclohexyl)carbonyl](1-methylethyl)amino]-5-(4-{[(3-methyl-2-pyridinyl)carbonyl]amino}phenyl)-2-thiophenecarboxylic acid

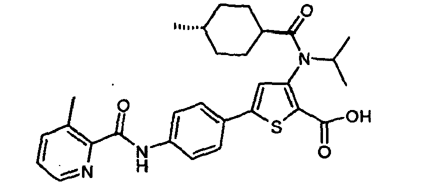

MS calcd for (C₂₉H₃₃N₃O₄S + H)⁺: 520
MS found (electrospray): (M+H)⁺= 520
¹H NMR (d₆-DMSO): δ 10.74 (1H, s), 8.56 (1H, d), 7.96 (2H, d), 7.83-7.71 (3H, m), 7.57-7.48 (1H, m), 7.45 (1H, s), 4.73 (1H, quintet), 2.59 (3H, s), 2.0 (1H, t), 1.71-1.40 (5H, m), 1.30-1.08 (5H, m), 0.88 (3H, d), 0.71 (3H, d), 0.70-0.47 (2H, m), 1 exchangeable proton not seen.

### Example 30

### 5-{4-[(1H-Imidazol-2-ylcarbonyl)amino]phenyl)-3-[[(trans-4-methylcyclohexyl)carbonyl](1-methylethyl)amino]-2-thiophenecarboxylic acid

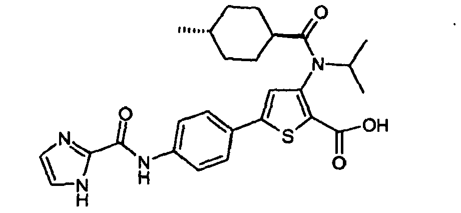

MS calcd for (C₂₆H₃₀N₄O₄S + H)⁺: 495
MS found (electrospray): (M+H)⁺ = 495
¹H NMR (d₆-DMSO): δ 13.30 (1H, br), 10.60 (1H, s), 7.95 (2H, d), 7.79 (2H, d), 7.46 (1H, s), 7.30 (2H, br), 4.77 (1H, quintet), 2.01 (1H, t), 1.70-1.40 (5H, m), 1.32-1.07 (5H, m), 0.90 (3H, d), 0.74 (3H, d), 0.72-0.50 (2H, m), 1 exchangeable proton not seen.

### Example 31

### 3-[[(trans-4-Methylcyclohexyl)carbonyl](1-methylethyl)amino]-5-(4-{[(6-methyl-2-pyridinyl)carbonyl]amino}phenyl)-2-thiophenecarboxylic acid

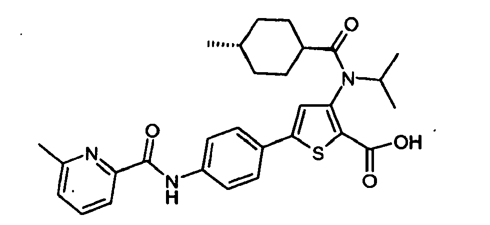

MS calcd for (C₂₉H₃₃N₃O₄S + H)⁺: 520
MS found (electrospray): (M+H)⁺ = 520
¹H NMR (d₆-DMSO): δ 10.62 (1H, s), 8.00 (2H, d), 7.96 (2H, d), 7.83 (2H, d), 7.53 (1H, d), 7.48 (1H, s), 4.74 (1H, t), 2.65 (3H, s), 2.00 (1H, t), 1.72-1.40 (5H, m), 1.30-1.10 (5H, m), 0.40 (3H, d), 0.73 (3H, d), 0.70-0.50 (2H, m), 1 exchangeable proton not seen.

### Example 32

### 3-[[(trans-4-Methylcyclohexyl)carbonyl](1-methylethyl)amino]-5-(4-{[(1-methyl-1H-imidazol-4-yl)carbonyl]amino}phenyl)-2-thiophenecarboxylic acid

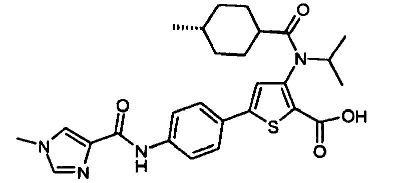

MS calcd for (C₂₇H₃₂N₄O₄S + H)⁺: 509
MS found (electrospray): (M+H)⁺= 509
¹H NMR (d₆-DMSO): δ 10.01 (1H, s), 7.98 (2H, d), 7.87 (1H, s), 7.78 (1H, s), 7.74 (2H, d), 7.41 (1H, s), 4.72 (1H, quintet), 3.73 (3H, s), 1.99 (1H, t), 1.68-1.38 (5H, m), 1.31-1.07 (5H, m), 0.40 (3H, d), 0.72 (3H, d), 0.69-0.46 (2H, m), 1 exchangeable proton not seen.

### Example 33

### 3-[[(trans-4-Methylcyclohexyl)carbonyl](1-methylethyl)amino]-5-(4-{[(2-methyl-4-pyridinyl)carbonyl]amino}phenyl)-2-thiophenecarboxylic acid

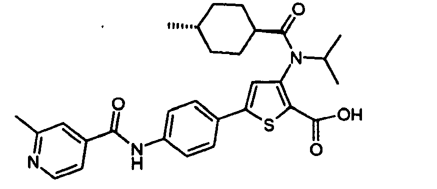

MS calcd for (C₂₉H₃₃N₃O₄S+ H)⁺: 520
MS found (electrospray): (M+H)⁺= 520
¹H NMR (d₆-DMSO): δ 10.62 (1H, s), 8.63 (1H, d), 7.88 (2H, d), 7.80-7.70 (3H, m), 7.67 (1H, d), 7.34 (1H, s), 4.71 (1H, quintet), 2.59 (3H, s), 2.04 (1H, t), 1.74-1.38 (5H, m), 1.30-1.06 (5H, m), 0.90 (3H, d), 0.77 (3H, d), 0.70-0.48 (2H, m), 1 exchangeable proton not seen.

### Example 34

### 5-(4-{[(3-Fluorophenyl)carbonyl]amino}phenyl)-3-[[(trans-4-methylcyclohexyl)carbonyl](1-methylethyl)amino]-2-thiophenecarboxylic acid

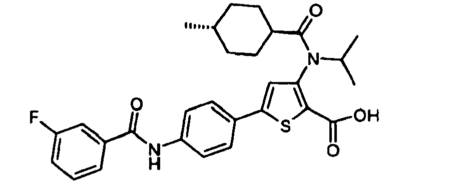

MS calcd for (C₂₉H₃₁FN₂O₄S + H)⁺:523
MS found (electrospray): (M+H)⁺ = 523
¹H NMR (d₆-DMSO): δ 10.53 (1H, s), 7.90 (2H, d), 7.88-7.75 (4H, m), 7.63-7.54 (1H, m), 7.48-7.40 (2H, m), 4.74 (1H, quintet), 1.96 (1H, t), 1.67-1.40 (5H, m), 1.30-1.10 (5H, m), 0.89 (3H, d), 0.70 (3H, d), 0.69-0.47 (2H, m), 1 exchangeable proton not seen.

### Example 35

### 3-[[(trans-4-Methylcyclohexyl)carbonyl](1-methylethyl)amino]-5-(4-{[(1-methyl-1H-imidazol-2-yl)carbonyl]amino}phenyl)-2-thiophenecarboxylic acid

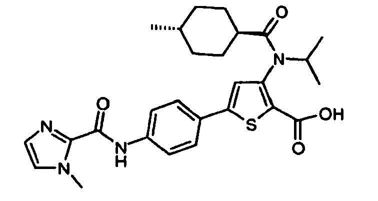

MS calcd for (C₂₇H₃₂N₄O₄S + H)⁺: 509
MS found (electrospray): (M+H)⁺ = 509
¹H NMR (d₆-DMSO): δ 10.53 (1H, s), 7.95 (2H, d), 7.78 (2H, d), 7.44 (2H, d), 7.10 (1H, s), 4.73 (1H, quintet), 4.03 (3H, s), 2.02 (1H, t), 1.70-1.40 (5H, m), 1.30-1.09 (5H, m), 0.90 (3H, d), 0.73 (3H, d), 0.70-0.48 (2H, m), 1 exchangeable proton not seen.

### Example 36

### 3-[[(trans-4-Methylcyclohexyl)carbonyl](1-methylethyl)amino]-5-(4-{[(1-methyl-1H-pyrazol-3-yl)carbonyl]amino}phenyl)-2-thiophenecarboxylic acid

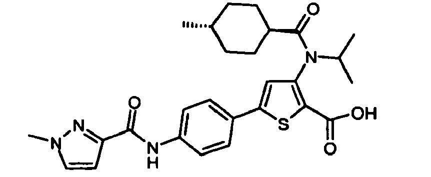

MS calcd for (C₂₇H₃₂N₄O₄S + H)⁺: 509
MS found (electrospray): (M+H)⁺ = 509
¹H NMR (d₆-DMSO): δ 9.23 (1H, s), 7.94 (2H, d), 7.86 (1H, s), 7.76 (2H, d), 7.42 (1H, s), 6.79 (1H, s), 4.73 (1H, quintet), 3.96 (3H, s), 2.00 (1H, t), 1.68-1.38 (5H, m), 1.30-1.08 (5H, m), 0.90 (3H, d), 0.76 (3H, d), 0.70-0.48 (2H, m), 1 exchangeable proton not seen.

### Example 37

### 5-(4-{[(5-Ethyl-3-isoxazolyl)carbonyl]amino}phenyl)-3-[[(trans-4-methylcyclohexyl)carbonyl](1-methylethyl)amino]-2-thiophenecarboxylic acid

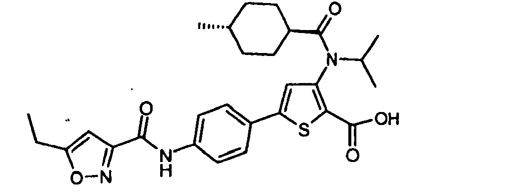

MS calcd for (C₂₈H₃₃N₃O₅S + H)⁺: 524
MS found (electrospray): (M+H)⁺ = 524
¹H NMR (d₆-DMSO): δ 10.79 (1H, s), 7.88 (2H, d), 7.73 (2H, d), 7.30 (1H, s), 6.72 (1H, s), 4.71 (1H, quintet), 2.86 (2H, q), 2.05 (1H, t), 1.80-1.32 (5H, m), 1.30-1.06 (8H, m), 0.88 (3H, d), 0.75 (3H, d), 0.69-0.47 (2H, m); 1 exchangeable proton not seen.

### Example 38

### 5-{4-[(3-Furanylcarbonyl)amino]phenyl}-3-[[(trans-4-methylcyclohexyl)carbonyl](1-methylethyl)amino]-2-thiophenecarboxylic acid

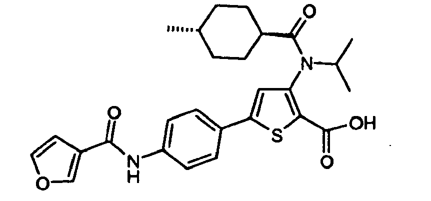

MS calcd for (C₂₇H₃₀N₂O₅S + H)⁺: 495
MS found (electrospray): (M+H)⁺ = 495
¹H NMR (d₆-DMSO): δ 10.13 (1H, s), 8.42 (1H, s), 7.88-7.74 (5H, m), 7.46 (1H, s), 7.01 (1H, s), 4.74 (1H, quintet), 1.98 (1H, t), 1.69-1.40 (5H, m), 1.30-1.09 (5H, m), 0.88 (3H, d), 0.73 (3H, d), 0.71-0.48 (2H, m), 1 exchangeable proton not seen.

### Example 39

### 5-(4-{[(2-Fluorophenyl)carbonyl]amino}phenyl)-3-[[(trans-4-methylcyclohexyl)carbonyl](1-methylethyl)amino]-2-thiophenecarboxylic acid

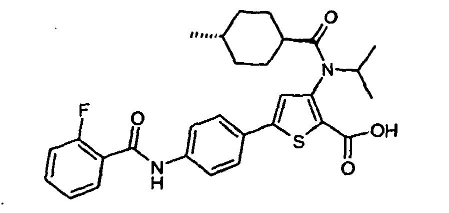

MS calcd for (C₂₉H₃₁FN₂O₄S + H)⁺: 523
MS found (electrospray): (M+H)⁺ = 523
¹H NMR (d₆-DMSO): δ 10.62 (1H, s), 7.87-7.74 (4H, m), 7.72-7.64 (1H, m), 7.60 (1H, d), 7.45 (1H, s), 7.40-7.30 (2H, m), 4.74 (1H, quintet), 2.00 (1H, t), 1.70-1.39 (5H, m), 1.31-1.06 (5H, m), 0.87 (3H, d), 0.70 (3H, d), 0.69-0.46 (2H, m), 1 exchangeable proton not seen.

### Example 40

### 5-(4-{[(2,6-Difluorophenyl)carbonyl]amino}phenyl)-3-1[(trans-4-methylcyclohexyl)carbonyl](1-methylethyl)amino]-2-thiophenecarboxylic acid

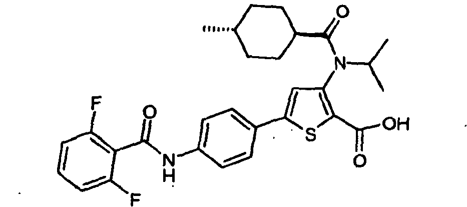

MS calcd for (C₂₉H₃₀F₂N₂O₄S + H)⁺: 541
MS found (electrospray): (M+H)⁺= 541
¹H NMR (d₆-DMSO): δ 11.00 (1H, s), 7.87-7.73 (4H, m), 7.68-7.52 (1H, m), 7.45 (1H, s), 7.30-7.20 (2H, m), 4.73 (1H, quintet), 1.99 (1H, t), 1.69-1.39 (5H, m), 1.30-1.06 (5H, m), 0.89 (3H, d); 0.72 (3H, d), 0.69-0.47 (2H, m), 1 exchangeable proton not seen.

### Example 41

### 3-[[(trans-4-Methylcyclohexyl)carbonyl](1-methylethyl)amino]-5-(4-{[(3-methyl-2-furanyl)carbonyl]amino}phenyl)-2-thiophenecarboxylic acid

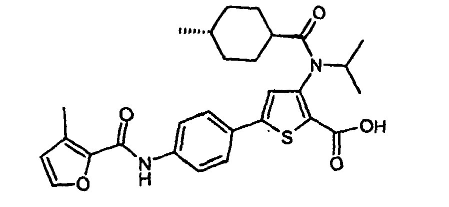

MS calcd for (C₂₈H₃₂N₂O₅S + H)⁺: 509
MS found (electrospray): (M+H)⁺= 509
¹H NMR (d₆-DMSO): δ 10.25 (1H, s), 7.88 (2H, d), 7.77 (1H, s), 7.73 (2H, d), 7.45 (1H, s), 6.59 (1H, s), 4.76 (1H, quintet), 2.34 (3H, s), 1.98 (1H, t), 1.68-1.38 (5H, m), 1.30-1.06 (5H, m), 0.89 (3H, d), 0.72 (3H, d), 0.69-0.48 (2H, m), 1 exchangeable proton not seen.

### Example 42

### 5-(4-{[(2-Amino-1,3-thiazol-4-yl)carbonyl]amino}phenyl)-3-[[(trans-4-methylcyclohexyl)carbonyl](1-methylethyl)amino]-2-thiophenecarboxylic acid

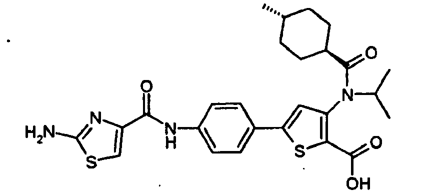

Intermediate 39 (310 mg) was dissolved in pyridine (2.5 mL). Lithium iodide (1.2 g) was added and the reaction was stirred at 130°C in a Reactivial^{™} for 6 h. The mixture was partitioned between DCM and 2N HCl. The organic phases were separated, and the aqueous was re-extracted with DCM. The combined organic phases were dried using a hydrophobic frit and evaporated *in vacuo.* The crude material was purified by NH₂ SPE cartridge, eluting with 10% 2N HCl in MeOH to give the title compound.
MS calcd for (C₂₆H₃₀N₄O₄S₂ + H)⁺: 527
MS found (electrospray): (M+H)⁺ = 527
¹H NMR (d₆-DMSO): δ 13.25 (1H, br), 10.06 (1H, s), 7.97-7.75 (4H, m), 7.58-7.44 (2H, m), 4.74 (1H, quintet), 2.03-1.92 (1H, m), 1.70-1.40 (5H, m), 1.33-1.07 (5H, m), 0.90 (3H, d), 0.79-0.46 (5H, m), 2 exchangeable protons not seen.

### General method for Examples 43-48

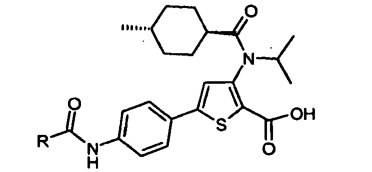

Carboxylic acid (0.266 mmol), DMF (0.5 mL) and DIPEA (0.084 mL) were added to a Greenhouse Plus tube. HATU (101 mg) in DMF (1 mL) was added and the reaction was stirred for 1 h. Methyl 5-(4-aminophenyl)-3-[[(*trans*-4-methylcyclohexyl)carbonyl](1-methylethyl)amino]-2-thiophenecarboxylate (100 mg, a synthesis of which is described as Intermediate 9) in DMF (1 mL) was then added and the reaction was stirred at room temperature for 24 h. The solvent was evaporated *in vacuo* using a Genevac vacuum centrifuge. To the residue was added THF (1 mL), MeOH (1 mL) and 2M lithium hydroxide solution (1 mL). The reaction was stirred for 24 h and was then neutralised with 2N HCl (1 mL). The mixture was partitioned between EtOAc and water and the organic phases were separated and dried using a hydrophobic frit. The organic phases were evaporated *in vacuo* using a Genevac vacuum centrifuge and the crude material was purified by MDAP HPLC to give the title compound.

### Example 43

### 5-(4-{[(3-Fluoro-2-pyridinyl)carbonyl]amino}phenyl)-3-[[(trans-4-methylcyclohexyl)carbonyl](1-methylethyl)amino]-24hiophenecarboxylic acid

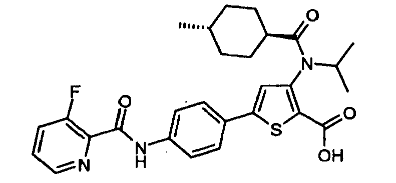

MS calcd for (C₂₈H₃₀FN₃O₄S + H)⁺: 524
MS found (electrospray): (M+H)⁺ = 524
¹H NMR (d₆-DMSO): δ 10.81 (1H, s), 8.59 (1H, s), 8.00-7.68 (6H, m), 7.43 (1H, s), 4.73 (1H, quintet), 2.01 (1H, t), 1.70-1.39 (5H, m), 1.31-1.10 (5H, m), 0.90 (3H, d), 0.73 (3H, d), 0.71-0.48 (2H, m), 1 exchangeable proton not seen.

### Example 44

### 5-(4-{[(1-Ethyl-1H-pyrazol-3-yl)carbonyl]amino}phenyl)-3-[[(trans-4-methylcyclohexyl)carbonyl](1-methylethyl)amino]-2-thiophenecarboxylic acid

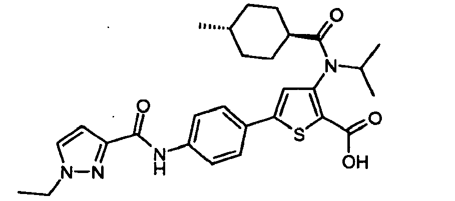

MS calcd for (C₂₈H₃₄N₄O₄S + H)⁺: 523
MS found (electrospray): (M+H)⁺ = 523
¹H NMR (d₆-DMSO): δ 10.19 (1H, s), 8.00-7.81 (3H, m), 7.76 (2H, d), 7.43 (1H, s), 6.78 (1H, s), 4.73 (1H, quintet), 4.25 (2H, q), 1.99 (1H, t), 1.68-1.37 (8H, m), 1.31-1.06 (5H, m), 0.92 (3H, d), 0.73 (3H, d), 0.71-0.45 (2H, m), 1 exchangeable proton not seen.

### Example 45

### 3-[[(trans-4-Methylcyclohexyl)carbonyl](1-methylethyl)amino]-5-(4-{[(5-methyl-2-furanyl)carbonyl]amino}phenyl)-2-thiophenecarboxylic acid

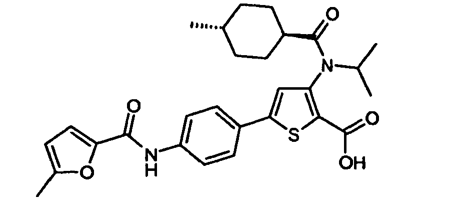

MS calcd for (C₂₈H₃₂N₂O₅S + H)⁺: 509
MS found (electrospray): (M+H)⁺ = 509
¹H NMR (d₆-DMSO): δ 10.24 (1H, s), 7.93-7.69 (4H, m), 7.42 (1H, s), 7.26 (1H, s), 6.32 (1H, s), 4.72 (1H, quintet), 2.48 (3H, s), 2.00 (1H, t), 1.68-1.40 (5H, m), 1.31-1.06 (5H, m), 0.89 (3H, d), 0.75 (3H, d), 0.72-0.47 (2H, m), 1 exchangeable proton not seen.

### Example 46

### 5-[4-({[5-(Hydroxymethyl)-2-furanyl]carbonyl}amino)phenyl]-3-[[(trans-4-methylcyclohexyl)carbonyl](1-methylethyl)amino]-2-thiophenecarboxylic acid

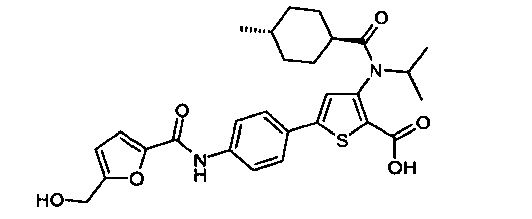

MS calcd for (C₂₈H₃₂N₂O₆S + H)⁺: 525
MS found (electrospray): (M+H)⁺ = 525
¹H NMR (d₆-DMSO): δ 10.30 (1H, s), 7.91-7.70 (4H, m), 7.49-7.38 (2H, m), 7.34 (1H, s), 6.50 (1H, s), 4.73 (1H, quintet), 4.48 (2H, s), 1.98 (1H, t), 1.68-1.39 (5H, m), 1.31-1.06 (5H, m), 0.88 (3H, d), 0.70 (3H, d), 0.69-0.45 (2H, m), 1 exchangeable proton not seen.

### Example 47

### 3-([(trans-4-Methylcyclohexyl)carbonyl](1-methylethyl)amino]-5-(4-{[(4-methyl-3-pyridinyl)carbonyl]amino}phenyl)-2-thiophenecarboxylic acid

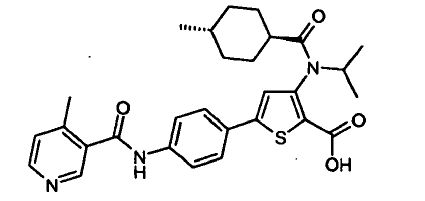

MS calcd for (C₂₉H₃₃N₃P₄S+ H)⁺: 520
MS found (electrospray): (M+H)⁺ = 520
¹H NMR (d₆-DMSO): δ 10.70 (1H, s), 8.67 (1H, s), 8.56 (1H, d), 7.90-7.73 (4H, m), 7.45 (1H, s), 7.39 (1H, d), 4.73 (1H, quintet), 2.42 (3H, s), 1.97 (1H, t), 1.70-1.40 (5H, m), 1.31-1.06 (5H, m), 0.89 (3H, d), 0.74 (3H, d), 0.70-0.45 (2H, m), 1 exchangeable proton not seen.

### Example 48

### 3-[[(trans-4-Methylcyclohexyl)carbonyl](1-methylethyl)amino]-5-(4-{[(3-methyl-4-isoxazolyl)carbonyl]amino}phenyl)-2-thiophenecarboxylic acid

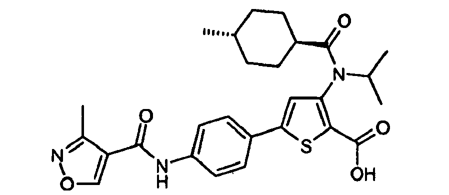

MS calcd for (C₂₇H₃₁N₃O₅S+ H)⁺: 510
MS found (electrospray): (M+H)⁺ = 510
¹H NMR (d₆-DMSO): δ 10.4 (1H, s), 9.5 (1H, s), 7.7 (2H, d), 7.6 (2H, d), 7.1 (1H, s), 4.65 (1H, m), 2.4 (3H, s), 2.1 (1H, m), 1.75 (1H, m), 1.65-1.15 (6H, m), 1.05 (3H, d), 0.9 (3H, d), 0.7 (3H, d), 0.67-0.5 (2H, m), 1 exchangeable proton not seen.

### Example 49

### 3-[[(trans-4-Methylcyclohexyl)carbonyl](1-methylethyl)amino]-5-{6-[(1,3-thiazol-4-ylcarbonyl)amino]-3-pyridinyl}-2-thiophenecarboxylic acid

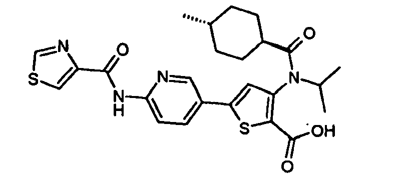

A mixture of Intermediate 41 (56 mg), lithium iodide (285 mg) and pyridine (0.5 mL) was heated in a Reactivial^{™} at 130°C for 4 h. The reaction mixture was allowed to cool and partitioned between DCM and 0.5N aqueous HCl solution. The organic phase was separated and dried by passing through a hydrophobic frit and evaporated *in vacuo.* The residue was purified by MDAP to give the title compound.
MS calcd for (C₂₅H₂₈N₄O₄S₂ + H)⁺: 513
MS found (electrospray): (M+H)⁺= 513
¹H NMR (CDCl3): δ 10.19 (1H, br. s), 8.92 (1H, d), 8.67 (1H, d), 8.55 (1H, d), 8.39 (1H, d), 8.04 (1H, dd), 7.13 (1H, s), 5.10-4.96 (1H, m), 2.18-2.04 (1H, m), 1.84-1.57 (5H, m), 1.56-1.27 (2H, m), 1.25 (3H, d), 1.03 (3H, d), 0.77 (3H, d), 0.75-0.60 (2H, m), carboxylic acid proton not seen.

### Example 50

### 3-[[(trans-4-Methylcyclohexyl)carbonyl](1-methylethyl)amino]-5-{6-[(2-pyridinylcarbonyl)amino]-3-pyridinyl}-2-thiophenecarboxylic acid

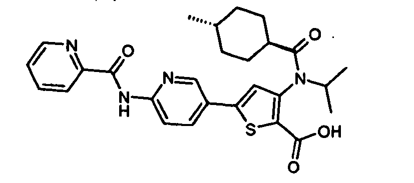

A mixture of Intermediate 42 (95 mg), lithium iodide (245 mg) and pyridine (1.0 mL) was heated in a Reactivial^{™} at 130°C for 4 h. The reaction mixture was allowed to cool and partitioned between DCM and 0.5N aqueous HCl solution. The organic phase was separated and dried by passing through a hydrophobic frit and evaporated *in vacuo.* The residue was purified by MDAP to give the title compound.
MS calcd for (C₂₇H₃₀N₄O₄S + H)⁺: 507
MS found (electrospray): (M+H)⁺ = 507
¹H NMR (CDCl₃): δ 10.77 (1H, br.s), 8.69 (2H, br.), 8.56 (1H, d), 8.31 (1H, d), 8.03 (1H, dd), 7.95 (1H, dt), 7.58-7.51 (1H, m), 7.13 (1H, s), 6.64 (1H excess, br), 5.09-4.94 (1H, m), 2.18-2.05 (1H, m), 1.83-1.56 (5H, m), 1.55-1.28 (2H, m), 1.25 (3H, d), 1.03 (3H, d), 0.77 (3H, d), 0.75-0.59 (2H, m)

### Example 51

### 3-[[(trans-4-Methylcyclohexyl)carbonyl](1-methylethyl)amino]-5-{6-[(2-pyrazinylcarbonyl)amino]-3-pyridinyl}-2-thiophenecarboxylic acid

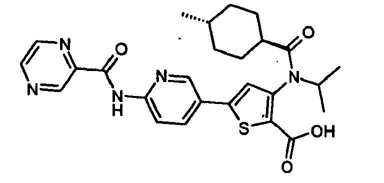

A mixture of Intermediate 43 (30 mg), lithium iodide (77 mg) and pyridine (0.5 mL) was heated in a Reactivial^{™} at 130°C for 4 h. The reaction mixture was allowed to cool and partitioned between DCM and 0.5N aqueous HCl solution. The organic phase was separated and dried by passing through a hydrophobic frit and evaporated *in vacuo*. The residue was purified by MDAP to give the title compound.
MS calcd for (C₂₆H₂₉N₅O₄S + H)⁺: 508
MS found (electrospray): (M+H)⁺ = 508
¹H NMR (d₆-DMSO): δ 10.54 (1H, s), 9.36 (1H, s), 8.99 (1H, s), 8.88 (2H, d), 8.35 (2H, br. s), 7.65 (1H, s), 4.79-4.71 (1H, m), 2.03-1.93 (1H, m), 1.68-1.39 (5H, m), 1.32-1.18 (2H, m), 1.15 (3H, d), 0.90 (3H, d), 0.70 (3H, d), 0.68-0.52 (2H, m), carboxylic acid proton not seen.

### Example 52

### 5-(6-{[(4-Fluorophenyl)carbonyl]amino}-3-pyridinyl)-3-[[(trans-4-methylcyclohexyl)carbonyl](1-methylethyl)amino]-2-thiophenecarboxylic acid

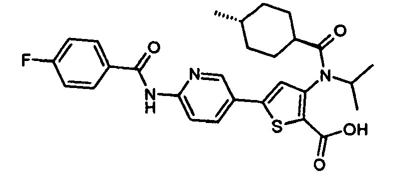

A mixture of Intermediate 44 (76 mg), lithium iodide (240 mg) and pyridine (1.0 mL) was heated in a Reactivial^{™} at 130°C for 4 h. The reaction mixture was allowed to cool and partitioned between DCM and 0.5N aqueous HCl solution. The organic phase was separated and dried by passing through a hydrophobic frit and evaporated *in vacuo.* The residue was purified by MDAP to give the title compound.
MS calcd for (C₂₈H₃₀FN₃O₄S + H)⁺: 524
MS found (electrospray): (M+H)⁺ = 524
¹H NMR (d₆-DMSO): δ 13.33 (1H, br. s), 11.13 (1H, s), 8.88 (1H, s), 8.29 (2H, br. s), 8.17-8.10 (2H, m), 7.65 (1H, s), 7.40-7.32 (2H, m), 4.79-4.71 (1H, m), 2.03-1.92 (1H, m), 1.68-1.44 (5H, m), 1.32-1.18 (2H, m), 1.15 (3H, d), 0.91 (3H, d), 0.75 (3H, d), 0.71-0.50 (2H, m)

### Example 53

### 5-{6-[(2-Furanylcarbonyl)amino]-3-pyridinyl}-3-[[(trans-4-methylcyclohexyl)carbonyl](1-methylethyl)amino]-2-thiophenecarboxylic acid

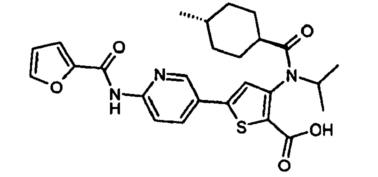

A mixture of Intermediate 45 (39 mg), lithium iodide (102 mg) and pyridine (0.5 mL) was heated in a Reactival^{™} at 130°C for 4 h. The reaction mixture was allowed to cool and partitioned between DCM and 0.5N aqueous HCl solution. The organic phase was separated and dried by passing through a hydrophobic frit and evaporated *in vacuo.* The residue was purified by MDAP to give the title compound.
MS calcd for (C₂₆H₂₉N₃O₅S + H)⁺: 496
MS found (electrospray): (M+H)⁺= 496
¹H NMR (d₆-DMSO): δ 13.38 (1H, br. s), 10.88 (1H, s), 8.87 (1H, s), 8.30-8.21 (2H, m), 7.98 (1H, s), 7.67 (1H, d), 7.64 (1H, s), 6.74-6.70 (1H, m), 4.79-4.70 (1H, m), 2.02-1.92 (1H, m), 1.68-1.42 (5H, m), 1.33-1.18 (2H, m), 1.15 (3H, d), 0.90 (3H, d), 0.74 (3H, d), 0.70-0.49 (2H, m).

### Example 54

### 3-{(2,2-Difluoroethyl)[(trans-4-methylcyclohexyl)carbonyl]amino}-5-{4-[(1,3-thiazol-4-ylcarbonyl)amino]phenyl}-2-thiophenecarboxylic acid

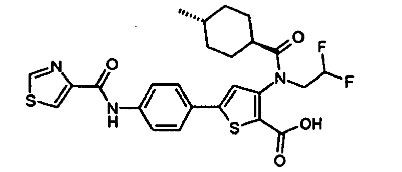

To a solution of Intermediate 50 (87 mg) in methanol (2 mL) and THF (4 mL) was added 2M sodium hydroxide (0.5 mL) and the reaction stirred at room temperature for 20 h. The reaction was evaporated to dryness, taken into water, acidified with 2M HCl, extracted twice with EtOAc (2 x 15 mL), dried using a hydrophobic frit and evaporated to dryness. The crude product was purified by MDAP to give the title compound.
MS calcd for (C₂₅H₂₅F₂N₃O₄S₂ + H)⁺: 534
MS found (electrospray): (M+H)⁺ = 534
¹H NMR (d₆-DMSO): δ 13.1 - 13.7 (1H, brs), 10.5 (1H, s), 9.29 (1H, d), 8.54 (1H, d), 8.00 (2H, d), 7.77 (2H, d), 7.56 (1H, s), 6.11 (1H, m), 4.15 (1H, m), 3.79 (1H, m), 2.18 (1H, m), 1.71 (1H, m), 1.58 (3H, m), 1.15 - 1.48 (3H, m), 0.75 (3H, d), 0.57 - 0.75 (2H, m)

### Example 55

### 3-{Ethyl[(trans-4-methylcyclohexyl)carbonyl]amino}-5-{4-[(1,3-thiazol-4-ylcarbonyl)amino]phenyl}-2-thiophenecarboxylic acid

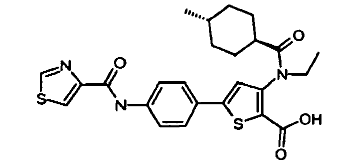

To Intermediate 54 (76 mg) was added THF (2 mL), methanol (2 mL) and 2M lithium hydroxide (2 mL). The reaction was stirred at room temperature for 16 hours. The reaction was neutralised with 2N HCl (2 mL), then partitioned between water and DCM. The phases were separated using a hydrophobic frit and the organic fractions were concentrated. The crude product was purified by a 13 g C18 reverse phase ISCO Companion^{™} flash chromatography, eluted with a gradient of MeCN (0.05% formic acid) in water (0.1% formic acid), to give the title compound.
MS calcd for (C₂₅H₂₇N₃O₄S + H)⁺: 498
MS found (electrospray): (M+H)⁺ = 498
¹H-NMR (d₆-DMSO): δ 13.2 (1H, br s), 10.6 (1H, s), 9.3 (1H, s), 8.55 (1H, s), 8.00 (2H, d), 7.75 (2H, d), 7.55 (1H, s), 3.75 (1H, m), 3.5 (1H, m), 2.1 (1H, m), 1.75-1.50 (4H, br m), 1.40 (1H, m), 1.25 (2H, m), 1.00 (3H, m), 0.75 (3H, d), 0.60 (2H, m)

### Example 56

### 3-[[(cis-4-Fluoro-4-methylcyclohexyl)carbonyl](1-methylethyl)amino]-5-{4-[(1,3-thiazol-4-ylcarbonyl)amino]phenyl}-2-thiophenecarboxylic acid

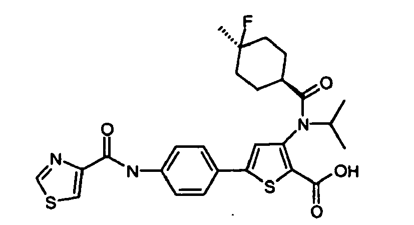

To a stirred solution of Intermediate 60 (0.05 g) in THF (0.75 mL) and ethanol (0.75 mL) was added sodium hydroxide (0.75 mL). The reaction mixture was stirred for 21 h and was then evaporated *in vacuo.* The residue was partitioned between DCM and 2N HCl. The organic phase was separated using a hydrophobic frit, washed with 2N HCl and water (x 2) and evaporated *in vacuo* to give the title compound.
MS calcd for (C₂₆H₂₈FN₃O₄S₂ + H)⁺: 530
MS found (electrospray): (M+H)⁺= 530

### Example 57

### 3-{(Cyclopropylmethyl)[(trans-4-methylcyclohexyl)carbonyl]amino}-5-{4-[(2-pyridinylcarbonyl)amino]phenyl}-2-thiophenecarboxylic acid

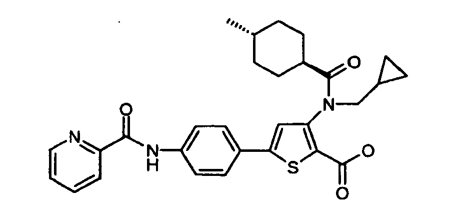

A mixture of Intermediate 65 (101 mg), lithium iodide (254 mg) and pyridine (1 mL) was heated in a Reactivial^{™} at 130°C for 4 h. The reaction mixture was allowed to cool and partitioned between DCM and 2N HCl solution. The organic phase was separated and dried by passing through a hydrophobic frit and evaporated. The residue was purified by MDAP to give the title compound.
MS calcd for (C₂₉H₃₁N₃O₄S + H)⁺: 518
MS found (electrospray): (M+H)⁺ = 518

### Example 58

### 3-{(Cyclopropylmethyl)[(trans-4-methylcyclohexyl)carbonyl]amino}-5-{4-[(2-pyrazinylcarbonyl)amino]phenyl}-2-thiophenecarboxylic acid

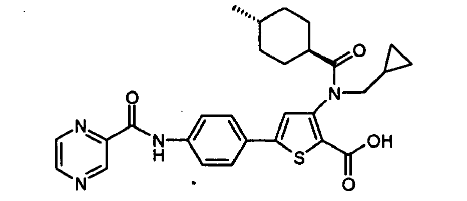

A mixture of Intermediate 66 (11 mg), lithium iodide (254 mg) and pyridine (1 mL) was heated in a Reactivial^{™} at 130°C for 4 h. The reaction mixture was allowed to cool and partitioned between DCM and 2N HCl solution. The organic phase was separated and dried by passing through a hydrophobic frit and evaporated. The crude material was applied to a 5 g aminopropyl cartridge which was washed with methanol. The cartridge was eluted with 10% 2N HCl in MeOH and the appropriate fractions were evaporated *in vacuo* to afford the title compound.
MS calcd for (C₂₈H₃₀N₄O₄S + H)⁺: 519
MS found (electrospray): (M+H)⁺ = 519

### Example 59

### 3-{(Cyclopropylmethyl)[(trans-4-methylcyclohexyl)carbonyl]amino}-5-(4-{[(1-methyl-1H-imidazol-4-yl)carbonyl]amino}phenyl)-2-thiophenecarboxylic acid

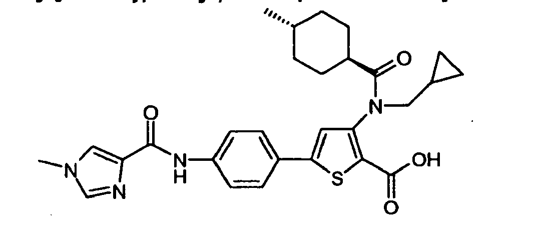

A mixture of Intermediate 67 (167 mg), lithium iodide (415 mg) and pyridine (1 mL) was heated in a Reactivial^{™} at 130°C for 4 h. The reaction mixture was allowed to cool and partitioned between DCM and 2N HCl solution. The organic phase was separated and dried by passing through a hydrophobic frit and evaporated. The residue was purified by MDAP to give the title compound.
MS calcd for (C₂₈H₃₂N₄O₄S + H)⁺: 521
MS found (electrospray): (M+H)⁺ = 521

### Example 60

### 3-((Cyclopropylmethyl)[(trans-4-methylcyclohexyl)carbonyl]amino}-5-{4-[(3-pyridazinylcarbonyl)amino]phenyl}-2-thiophenecarboxylic acid

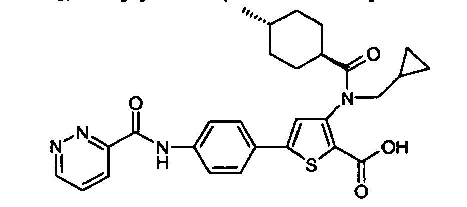

A mixture of Intermediate 68 (12 mg), lithium iodide (415 mg) and pyridine (1 mL) was heated in a Reactivial^{™} at 130°C for 4 h. The reaction mixture was allowed to cool and partitioned between DCM and 2N HCl solution. The organic phase was separated and dried by passing through a hydrophobic frit and evaporated. The residue was purified by MDAP to give the title compound.
MS calcd for (C₂₈H₃₀N₄O₄S + H)⁺: 519
MS found (electrospray): (M+H)⁺= 519

### Example 61

### 3-{(Cyclopropylmethyl)[(trans-4-methylcyclohexyl)carbonyl]amino}-5-(4-{[(1-methyl-1H-pyrazol-3-yl)carbonyl]amino}phenyl)-2-thiophenecarboxylic acid

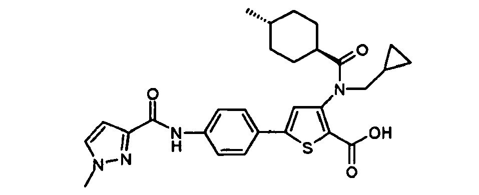

A mixture of Intermediate 69 (118 mg), lithium iodide (415 mg) and pyridine (1 mL) was heated in a Reactivial^{™} at 130°C for 4 h. The reaction mixture was allowed to cool and partitioned between DCM and 2N HCl solution. The organic phase was separated and dried by passing through a hydrophobic frit and evaporated. The residue was purified by MDAP to give the title compound.
MS calcd for (C₂₈H₃₂N₄O₄S + H)⁺: 521
MS found (electrospray): (M+H)⁺ = 521

### Example 62

### 3-{(Cyclopropylmethyl)[(trans-4-methylcyclohexyl)carbonyl]amino}-5-(4-{[(1,5-dimethyl-1H-pyrazol-3-yl)carbonyl]amino}phenyl)-2-thiophenecarboxylic acid

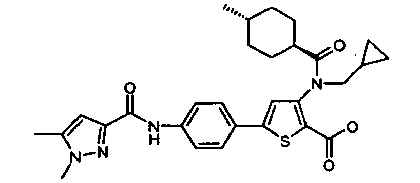

A mixture of Intermediate 70 (88 mg), lithium iodide (415 mg) and pyridine (1 mL) was heated in a Reactivial^{™} at 130°C for 4 h. The reaction mixture was allowed to cool and partitioned between DCM and 2N HCl solution. The organic phase was separated and dried by passing through a hydrophobic frit and evaporated. The crude material was applied to a 5 g aminopropyl cartridge which was washed with methanol. The cartridge was eluted with 10% 2N HCl in MeOH and the appropriate fractions were evaporated *in vacuo* to afford the title compound.
MS calcd for (C₂₉H₃₄N₄O₄S + H)⁺: 535
MS found (electrospray): (M+H)⁺ = 535

### Example 63

### 3-[[(trans-Methylcyclohexyl)carbonyl](1-methylethyl)amino]-5-[4-[(1,3-thiazol-4-ylcarbonyl)amino]-3-(trifluoromethyl)phenyl]-2-thlophenecarboxylic acid

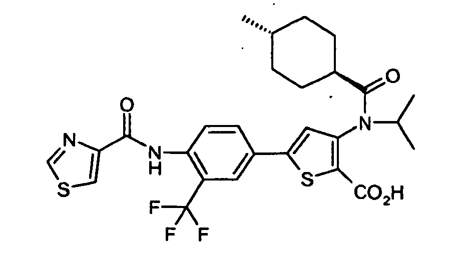

To a solution of Intermediate 72 (89 mg) in MeOH (2 mL) and THF (2 mL) was added sodium hydroxide (0.5 mL) and the reaction mixture was stirred at room temperature for 1 day. The reaction mixture was evaporated *in vacuo,* taken into water (10 mL) and acidified with 2M HCl. The organic phase was extracted with EtOAc (x 2), dried using a hydrophobic frit and evaporated *in vacuo.* The residue was purified by MDAP and freeze dried from 1,4-dioxane to give the title compound.
MS calcd for (C₂₇H₂₈F₃N₃O₄S₂ + H)⁺: 580
MS found (electrospray): (M+H)⁺= 580
¹H NMR (d₆-DMSO): δ 13.43 (1H, br), 10.08 (1H, s), 9.32 (1H, m), 8.63 (1H, m), 8.31-8.22 (1H, m), 8.19-8.13 (2H, m), 7.76 (1H, s), 4.74 (1H, quintet), 1.97 (1H, m), 1.70-1.40 (5H, m), 1.30-1.18 (2H, m), 1.16 (3H, d), 0.91 (3H, d), 0.74 (3H, d), 0.71-0.48 (2H, m).

### Example 64

### 3-[[(trans-4-Methylcyclohexyl)carbonyl](1-methylethyl)amino]-5-{3-(methyloxy)-4-[(1,3-thiazol-4-ylcarbonyl)amino]phenyl}-2-thiophenecarboxylic acid

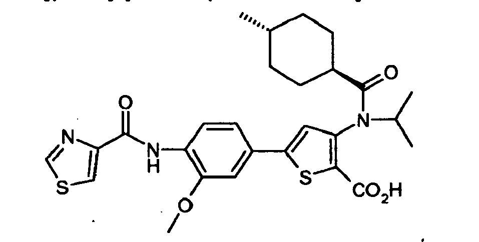

To a solution of Intermediate 74 (89 mg) in MeOH (2 mL) and THF (1 mL) was added sodium hydroxide (0.5 mL) and the reaction mixture was stirred at room temperature for 1 day. The reaction mixture was evaporated *in vacuo,* taken into water (10 mL) and acidified with 2M HCl. The organic phase was extracted with EtOAc (x 2), dried using a hydrophobic frit and evaporated *in vacuo.* The residue was purified by MDAP and freeze dried from 1,4-dioxane to give the title compound.
MS calcd for (C₂₇H₃₁N₃O₅S₂ + H)⁺: 542
MS found (electrospray): (M+H)⁺= 542

### Example 65

### 3-{1,3-Dioxan-5-yl[(trans-4-methylcyclohexyl)carbonyl]amino}-5-{4-[(1,3-thiazol-4-ylcarbonyl)amino]phenyl}-2-thiophenecarboxylic acid

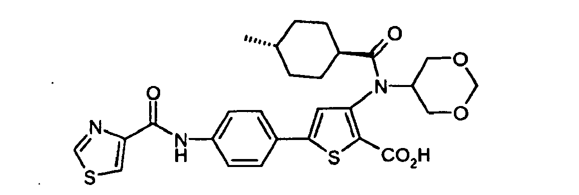

To a stirred suspension of Intermediate 81 (0.36 g) in THF (10 mL) and ethanol (10 mL) was added sodium hydroxide (2.5 mL). After 18 h the mixture was evaporated *in vacuo* and partitioned between 2N HCl and DCM. The organic phase was separated using a hydrophobic frit and evaporated *in vacuo.* The material was crystallised from DMSO/MeOH (1:1). The crystals were filtered off, washed with methanol and dried *in vacuo* to give the title compound.
MS calcd for (C₂₇H₂₉N₃O₆S₂ + H)⁺: 556
MS found (electrospray): (M+H)⁺= 556
¹H NMR (d₆-DMSO): δ 13.9-12.9 (1H, bs), 10.58 (1H, s), 9.29 (1H, d), 8.54 (1H, d), 8.00 (2H, d), 7.79 (2H, d), 7.55 (1H, d), 4.77 (1H, d), 4.53-4.44 (2H, m), 4.12 (1H, dd), 4.00 (1H, bdd), 3.67 (1H, dd), 3.43 (1H, dd, overlapped), 2.02-1.92 (1H, m) 1.68-1.38 (5H, m), 1.32-1.15 (2H, m), 0.73 (3H, d), 0.70-0.48 (2H, m).

### Example 66

### 3-{Cyclobutyl[(trans-4-methylcyclohexyl)carbonyl]amino}-5-{4-[(1,3-thiazol-4-ylcarbonyl)amino]phenyl}-2-thiophenecarboxylic acid

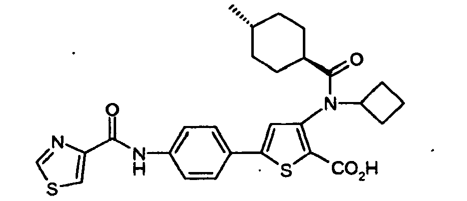

A solution of Intermediate 85 (58 mg) and lithium iodide (14 mg) in pyridine (0.5 mL) was stirred at 90°C for 24 h. The reaction mixture was evaporated *in vacuo* and was partitioned between 2N HCl and DCM. The organic layer was separated, evaporated *in vacuo* and purified by aminopropyl SPE ion exchange chromatography eluting with methanol and then 10% 2N HCl in methanol to give the title compound.
MS calcd for (C₂₇H₂₉N₃O₄S₂ + H)⁺: 524
MS found (electrospray): (M+H)⁺ = 524
¹H NMR (CD30D): δ 9.10 (1H, br.s), 8.42 (1H, br.m), 7.90(2H, d), 7.77 (2H, d), 7.38 (1H, s), 4.80 (1H, under water), 1.26 (1H, m), 2.17-1.21 (13H, m + excess), 0.78 (3H, d), 0.77-0.60 (2H, m), 2 exchangeable protons not seen.

### Example 67

### 3-{[(trans-4-Methylcyclohexyl)carbonyl][1-methyl-2-(methyloxy)ethyl]amino}-5-{4-[(1,3-thiazol-4-ylcarbonyl)amino]phenyl}-2-thiophenecarboxylic acid

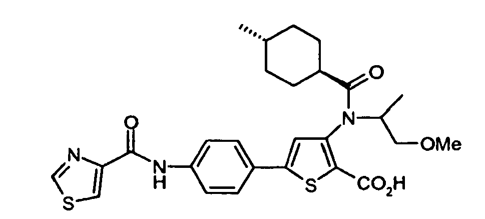

A mixture of Intermediate 89 (240 mg), lithium iodide (578 mg) and pyridine (1.5 mL) was heated in a Reactivial^{™} at 130°C for 5 h. The reaction mixture was allowed to cool and partitioned between DCM and 2N HCl solution. The organic phase was separated, dried by passing through a hydrophobic frit and evaporated *in vacuo.* The residue was purified by MDAP HPLC and freeze dried from 1,4-dioxane to give the title compound.
MS calcd for (C₂₇H₃₁N₃O₅S₂ + H)⁺: 542
MS found (electrospray): (M+H)⁺ = 542
¹H NMR (d₆-DMSO): δ 13.21 (1H, br), 10.55 (1H, m), 9.28 (1H, m), 8.54 (1H, m), 8.00 (2H, m), 7.78 (2H, m), 7.49 (⅓H, s), 7.35 (⅓H, s), 4.84 (%H, m), 4.56 (⅓H, m), 3.45-3.35 (1H, m), 3.30 (3H under water), 3.16-3.08 (1H, m), 2.01 (1H, m), 1.71-1.38 (5H, m), 1.35-1.19 (2H, m), 1.87 (1H, d), 0.88 (2H, d), 0.74 (3H, d), 0.71-0.43 (2H, m)

### Example 68

### 3-([1-Methyl-2-(methyloxy)ethyl]{[trans-4-(trifluoromethyl)cyclohexyl]carbonyl}amino)-5-{4-[(1,3-thiazol-4-ylcarbonyl)amino]phenyl}-2-thiophenecarboxylic acid

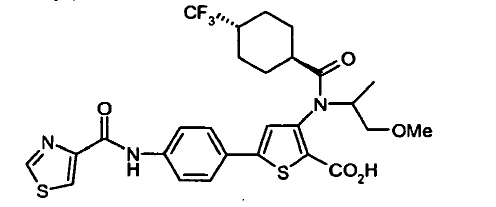

A mixture of Intermediate 92 (216 mg), lithium iodide (474 mg) and pyridine (1.5 mL) was heated in a Reactivial^{™} at 130°C for 5 h. The reaction mixture was allowed to cool and partitioned between DCM and 2N HCl solution. The organic phase was separated, dried by passing through a hydrophobic frit and evaporated *in vacuo.* The residue was purified by MDAP HPLC and freeze dried from 1,4-dioxane to give the title compound.
MS calcd for (C₂₇H₂₈F₃N₃OₛS₂ + H)⁺: 596
MS found (electrospray): (M+H)⁺ = 596

### Example 69

### 3-[[(trans-4-Methylcyclohexyl)carbonyl](1-methylethyl)amino]-5-{5-methyl-6-[(1,3-thiazol-4-ylcarbonyl)amino]-3-pyridinyl}-2-thiophenecarboxylic acid

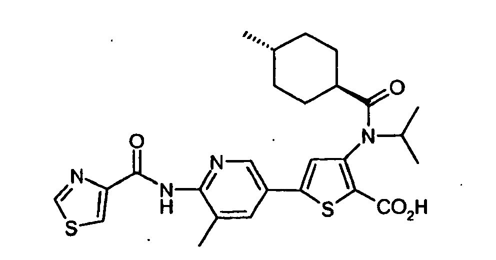

A solution of Intermediate 94 (23 mg) and lithium iodide (5.69 mg) in pyridine (0.5 mL) was stirred under nitrogen at room temperature for 2 days. The reaction mixture was evaporated *in vacuo* and was washed with 2M HCl and DCM. The organic layer was evaporated *in vacuo* and purified by aminopropyl SPE ion exchange chromatography eluting with methanol and then 10% 2N HCl in methanol to give the title compound.
MS calcd for (C₂₆H₃₀N₄O₄S₂ + H)⁺: 527
MS found (electrospray): (M+H)⁺= 527
¹H NMR (CD3OD): δ 9.21 (1H, m), 8.84-8.72 (3H, m), 7.70 (1H, s), 4.85 (1H, m), 2.67 (3H, s), 2.11-2.03 (1H, m), 1.80-1.52 (5H, m), 1.45-1.27 (2H, m), 1.25 (3H, d), 1.00 (3H, d), 0.78 (3H, d), 0.73-0.54 (2H, m), 2 exchangeable protons not seen.

### Example 70

### 5-(6-{[(4-Fluorophenyl)carbonyl]amino}-5-methyl-3-pyridinyl)-3-[[(trans-4-methylcyclohexyl)carbonyl](1-methylethyl)amino]-2-thiophenecarboxylic acid

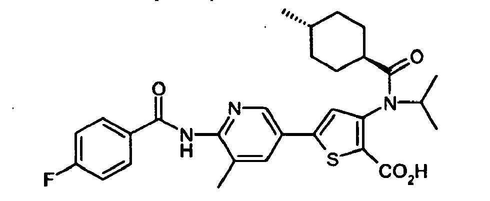

A solution of Intermediate 96 (238 mg), 2M sodium hydroxide (2 mL), methanol (2 mL), THF (2 mL) was left at room temperature for 4 days. The reaction was purified by ion exchange chromatography. The fractions were evaporated *in vacuo* and the crude material was purified by MDAP HPLC to give the title compound.
MS calcd for (C₂₉H₃₂FN₃O₄S + H)⁺: 538
MS found (electrospray): (M+H)⁺ = 538

### Example 71

### 3-{Cyclobutyl[(trans-4-methylcyclohexyl)carbonyl]amino}-5-{4-[(1H-pyrazol-3-ylcarbonyl)amino]phenyl}-2-thiophenecarboxylic acid

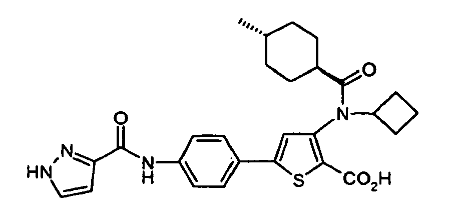

2N Lithium hydroxide (1 mL) was added to a solution of Intermediate 101 (50 mg) in methanol (2 mL) and THF (1 mL) and was stirred at room temperature for 6 h. The reaction was evaporated *in vacuo* and was acidified to pH 1.0 with 2N HCl. The suspension was applied to a 1 g OASIS cartridge eluting with water (5 column volumes) then methanol (5 column volumes). The appropriate fraction was evaporated *in vacuo* and freeze dried from 1,4-dioxane to give the title compound.
MS calcd for (C₂₇H₃₀N₄O₄S + H)⁺: 507
MS found (electrospray): (M+H)⁺= 507
¹H NMR (d₆-DMSO): δ 13.52 (1H; br), 10.21 (1H, br), 7.90 (3H, m), 7.68 (2H, d), 7.25 (1H, s), 6.80 (1H, br), 4.81 (1H, m), 2.12-2.01 (2H, m), 1.94-1.14 (12H, m), 0.74 (3H, d), 0.68-0.49 (2H, m), carboxylic acid proton not seen.

### Example 72

### 3-{Ethyl[(trans-4-methylcyclohexyl)carbonyl]amino}-5-{4-[(1H-pyrazol-3-ylcarbonyl)amino]phenyl}-2-thiophenecarboxylic acid

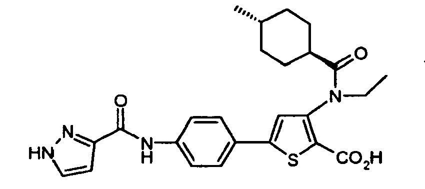

2N Lithium hydroxide (1 mL) was added to a solution of Intermediate 103 (48 mg) in methanol (2 mL) and THF (1 mL) and was stirred at room temperature for 4 h. The reaction was evaporated *in vacuo* and was acidified to pH 1.0 with 2N HCl. The suspension was applied to a 1 g OASIS cartridge eluting with water (5 column volumes) then methanol (5 column volumes). The appropriate fraction was evaporated *in vacuo* and freeze dried from 1,4-dioxane to give the title compound.
MS calcd for (C₂₅H₂₈N₄O₄S + H)⁺: 481
MS found (electrospray): (M+H)⁺= 481

### Example 73

### 3-{[(trans-4-Methylcyclohexyl)carbonyl][2-(methyloxy)ethyl]amino}-5-{4-[(1,3-thiazol-4-ylcarbonyl)amino]phenyl}-2-thiophenecarboxylic acid

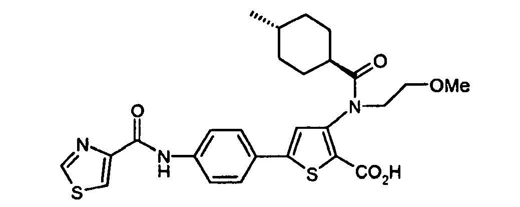

To Intermediate 106 (52 mg) was added THF (1 mL), MeOH (1 mL) and 2M LiOH (1 mL). The reaction was stirred at room temperature for 5 h. The reaction was neutralised with 2N HCl (1 mL), partitioned between DCM and water and separated with a hydrophobic frit. The organic phases were evaporated *in vacuo* to give the title compound.
MS calcd for (C₂₆H₂₉N₃O₅S₂ + H)⁺: 528
MS found (electrospray): (M+H)⁺ = 528
¹H NMR (CD₃OD): δ 9.08 (1H, d), 8.41 (1H, d), 7.89 (2H, m), 7.74 (2H, m), 7.40 (1H, s), 4.15-4.06 (1H, m), 3.62-3.44 (3H, m), 3.27 (3H, s), 2.24 (1H, m), 1.80-1.16 (7H, m), 0.80 (3H, d), 0.75-0.60 (2H, m) 2 exchangeable protons not seen.

### Example 74

### 3-{[(trans-4-Methylcyclohexyl)carbonyl][1-methyl-2-(methyloxy)ethyl]amino}-5-(4-{[(1-methyl-1H-imidazol-4-yl)carbonyl]amino}phenyl)-2-thiophenecarboxylic acid

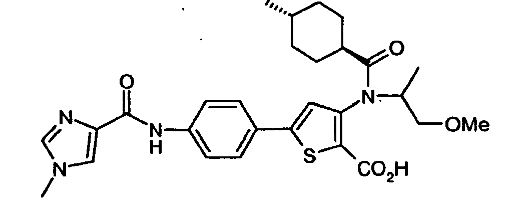

A solution of Intermediate 108 (0.07 g) and sodium hydroxide (0.317 mL) in methanol (0.7 mL) and THF (0.7 mL) was stirred under nitrogen at room temperature overnight. The reaction mixture was evaporated in vacuo and washed with 2M HCl and DCM. The organic layer was separated and evaporated *in vacuo.* The crude material was purified using a 5 g aminopropyl cartridge. The residue was further purified using a 4 g ISCO Companion^{™} silica cartridge eluting with a gradient of 0-100% EtOAc in DCM followed by 100% MeOH to give the title compound.
MS calcd for (C₂₈H₃₄N₄O₅S + H)⁺: 539
MS found (electrospray): (M+H)⁺ = 539

### Example 75

### 3-{[(trans-4-Methylcyclohexyl)carbonyl][2-(methyloxy)ethyl]amino}-5-(4-{[(1-methyl-1H-imidazol-4-yl)carbonyl]amino}phenyl)-2-thiophenecarboxylic acid

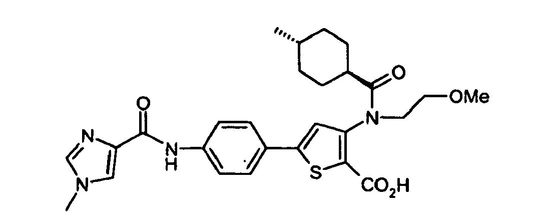

A solution of Intermediate 109 (0.1g) and sodium hydroxide (0.278 mL) in methanol (0.775 mL) and THF (0.775 mL) was stirred under nitrogen at room temperature overnight. The reaction mixture was evaporated *in vacuo* and washed with 2M HCl and DCM. The organic layer was evaporated *in vacuo* and purified by NH₂ SPE to give the title compound.
MS calcd for (C₂₇H₃₂N₄O₅S + H)⁺: 525
MS found (electrospray): (M+H)⁺ = 525

### Example 76

### 3-[[(trans-4-Methylcyclohexyl)carbonyl](2,2,2-trifluoroethyl)amino]-5-{4-[(1,3-thiazol-4-ylcarbonyl)amino]phenyl}-2-thiophenecarboxylic acid

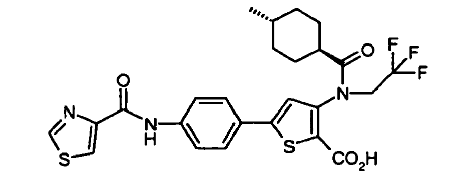

A solution of Intermediate 111 (36 mg) in MeOH (2 mL) and THF (2 mL) was added 2M sodium hydroxide (0.5 mL) and the reaction mixture was stirred at room temperature for 20 h. The reaction mixture was evaporated to dryness, taken into water (10 mL), acidified with 2M HCl, extracted with EtOAc (2 x 15 mL), dried using a H-frit and evaporated *in vacuo.* The residue was purified by MDAP HPLC and freeze dried from 1,4-dioxane to give the title compound.
MS calcd for (C₂₅H₂₄F₃N₃O₄S₂ + H)⁺: 552
MS found (electrospray): (M+H)⁺ = 552

### Example 77

### Lysine 3-[[(trans-4-methylcyclohexyl)carbonyl](1-methylethyl)amino]-5-{4-[(1,3-thiazol-4-ylcarbonyl)amino]phenyl}-2-thiophenecarboxylate

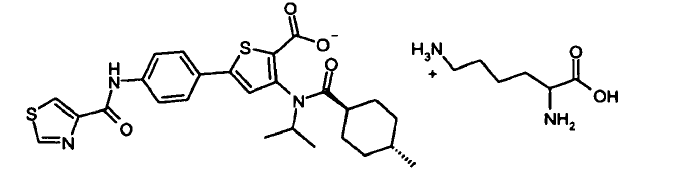

3-[[(*trans*-4-Methylcyclohexyl)carbonyl](1-methylethyl)amino]-5-{4-[(1,3-thiazol-4-ylcarbonyl)amino]phenyl}-2-thiophenecarboxylic acid (306 mg, a synthesis of which is described as Example 1) was placed in acetone (10 mL), and a solution of L-lysine (15 mg, 1.17 mL of a 0.5M solution in water) was added. The mixture was temperature cycled between room temperature and 40°C for 7 days (4 hour cycles), the solid isolated by filtration and dried at 40°C under vacuum for 24 h to give the title compound.
¹H NMR (d₆-DMSO): δ 10.46 (1H, s), 9.29 (1H, d), 8.53 (1H, d), 8.50-7.50 (2H, br), 7.93 (2H, d), 7.67 (2H, d), 7.1 (1H, s), 4.70 (1H, quintet), 3.20 (2H, t), 2.67 (2H, t), 2.14 (1H, tt), 1.8-1.3 (11H, m), 1.27-1.14 (2H, m), 1.09 (3H, d), 0.92 (3H, d), 0.74 (3H, d), 0.69-0.46 (2H, m). Further exchangeable protons presumed to be under broad water peak at 3.5-3.0.

**Table 2: Characteristic X-Ray powder diffraction (XRPD) angles and d spacings for lysine 3-[[(trans-4-methylcyclohexyl)carbonyl](1-methylethyl)amino]-5-{4-[(1,3-thiazol-4-ylcarbonyl)amino]phenyl}-2-thiophenecarboxylate.**

| Pos. [°2Th.] | d-spacing [Å] |
|---|---|
| 6.1 | 14.6 |
| 8.9 | 10.0 |
| 11.7 | 7.6 |
| 12.1 | 7.3 |
| 13.0 | 6.8 |
| 13.7 | 6.4 |
| 14.3 | 6.2 |
| 17.1 | 5.2 |
| 17.8 | 5.0 |
| 18.7 | 4.7 |
| 20.5 | 4.3 |
| 21.2 | 4.2 |
| 21.7 | 4.1 |
| 23.0 | 3.9 |
| 26.7 | 3.3 |
| 28.7 | 3.1 |

Data obtained for lysine 3-[[(*trans*-4-methylcyclohexyl)carbonyl](1-methylethyl)amino]-5-{4-[(1,3-thiazol-4-ylcarbonyl)amino]phenyl}-2-thiophenecarboxylate can be seen in Figure 2.

### Example 78

### Ammonium 3-[[(trans-4-methylcyclohexyl)carbonyl](1-methylethyl)amino]-5-{4-[(1,3-thiazol-4-ylcarbonyl)amino]phenyl}-2-thiophenecarboxylate

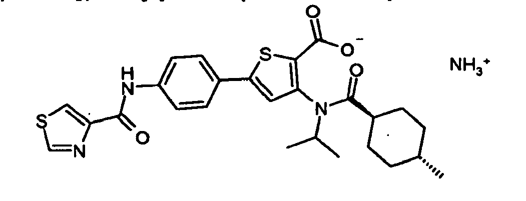

Isopropanol (6 mL) was added to 3-[[(*trans*-4-methylcyclohexyl)carbonyl](1-methylethyl)amino]-5-{4-[(1,3-thiazol-4-ylcarbonyl)amino]phenyl}-2-thiophenecarboxylic acid (300 mg, a synthesis of which is described as Example 1) followed by ammonium hydroxide solution (28%, 82 uL). An additional 6 mL of isopropanol was added and the mixture was temperature cycled between room temperature and 40°C for 48 hours (4 hour cycles). The product was collected by filtration and dried at 70°C for 48 hours to give the title compound.
¹H NMR (d₆-DMSO): δ 10.5 (1H, s), 9.28 (1H, d), 8.53 (1H, d), 7.92 (2H, d), 7.66 (2H, d), 7.5-7.0 (3H, br), 7.15 (1H, s), 4.69 (1H, quintet), 2.14 (1H, tt), 1.81 (1H, d), 1.61-1.48 (3H, m), 1.43-1.30 (1H, m), 1.27-1.14 (2H, m), 1.09 (3H, d), 0.92 (3H, d), 0.74 (3H, d), 0.69-0.46 (2H, m).

**Table 3: Characteristic X-Ray powder diffraction (XRPD) angles and d spacings for ammonium 3-[[(trans-4-methylcyclohexyl)carbonyl](1-methylethyl)amino]-5-{4-[(1,3-thiazol-4-ylcarbonyl)amino]phenyl}-2-thiophenecarboxylate.**

| Pos. [°2Th.] | d-spacing [Å] |
|---|---|
| 3.9 | 22.9 |
| 7.7 | 11.5 |
| 8.8 | 10.1 |
| 11.5 | 7.7 |
| 13.8 | 6.4 |
| 15.0 | 5.9 |
| 16.5 | 5.4 |
| 17.6 | 5.0 |
| 19.2 | 4.6 |
| 20.9 | 4.2 |
| 21.5 | 4.1 |
| 26.7 | 3.3 |

Data obtained for ammonium 3-[[(*trans*-4-methylcyclohexyl)carbonyl](1-methylethyl)aminol-5-{4-[(1,3-thiazol-4-ylcarbonyl)amino]phenyl}-2-thiophenecarboxylate can be seen in Figure 3.

### Example 79

### Ethanolamine 3-[[(trans-4-methylcyclohexyl)carbonyl](1-methylethyl)amino]-5-{4-[(1,3-thiazol-4-ylcarbonyl)amino]phenyl}-2-thiophenecarboxylate

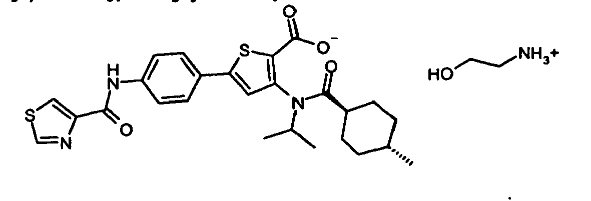

To 3-[[(*trans*-4-methylcyclohexyl)carbonyl](1-methylethyl)amino]-5-{4-[(1,3-thiazol-4-ylcarbonyl)amino]phenyl}-2-thiophenecarboxylic acid (50 mg, a synthesis of which is described as Example 1) in acetonitrile (3 mL), was added ethanolamine (6 uL). The mixture was then temperature cycled between room temperature and 40°C for 48 hours (4 hour cycles). The product was collected by filtration and dried at 70°C for 48 hours to give the title compound.
¹H NMR (d₆-DMSO): δ 10.46 (1H, s), 9.28 (1H, d), 8.53 (1H, d), 7.93 (2H, d), 7.67 (2H, d), 7.17 (1H, s), 5.50 (1H, br), 4.70 (1H, quintet), 3.56 (2h, t), 2.83 (2H, t), 2.15 (1H, tt), 1.77 (1H, d), 1.61-1.48 (3H, m), 1.43-1.30 (1H, m), 1.27-1.14 (2H, m), 1.09 (3H, d), 0.92 (3H, d), 0.74 (3H, d), 0.69-0.46 (2H, m).

**Table 4: Characteristic X-Ray powder diffraction (XRPD) angles and d spacings for ethanolamine 3-[[(trans-4-methylcyclohexyl)carbonyl](1-methylethyl)amino]-5-{4-[(1,3-thiazol-4-ylcarbonyl)amino]phenyl}-2-thiophenecarboxylate.**

| Pos. [°2Th.] | d-spacing [Å] |
|---|---|
| 7.3 | 12.2 |
| 7.9 | 11.1 |
| 9.6 | 9.2 |
| 10.6 | 8.3 |
| 13.4 | 6.6 |
| 14.6 | 6.1 |
| 16.2 | 5.5 |
| 17.4 | 5.1 |
| 18.5 | 4.8 |
| 19.3 | 4.6 |
| 21.5 | 4.1 |
| 23.9 | 3.7 |
| 26.0 | 3.4 |
| 27.1 | 3.3 |
| 28.1 | 3.2 |

Data obtained for ethanolamine 3-[[(*trans*-4-methylcyclohexyl)carbonyl](1-methylethyl)amino]-5-{4-[(1,3-thiazol-4-ylcarbonyl)amino]phenyl}-2-thiophenecarboxylate can be seen in Figure 4.

### Example 80

### N-Methyl-D-glucamine 3-[[(trans-4-methylcyclohexyl)carbonyl](1-methylethyl)amino]-5-{4-[(1,3-thiazol-4-ylcarbonyl)amino]phenyl}-2-thiophenecarboxylate

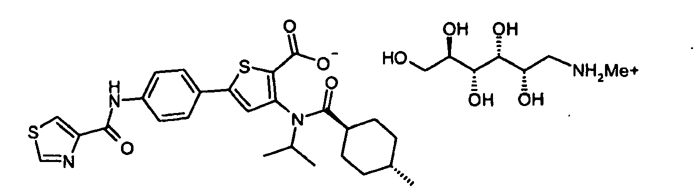

3-[[(*trans-*4-Methylcyclohexyl)carbonyl](1-methylethyl)amino]-5-{4-[(1,3-thiazol-4-ylcarbonyl)amino]phenyl}-2-thiophenecarboxylic acid (306 mg, a synthesis of which is described as Example 1) and N-methyl-D-glucamine (124 mg) in acetone (2 mL) was stirred for approximately 24 h. The mixture was then temperature cycled between room temperature and 40°C for 4 days. The product was collected by filtration and dried at 70°C for 24 hours to give the title compound.
¹H NMR (d₆-DMSO): δ 10.46 (1H, s), 9.28 (1H, d), 8.52 (1H, d), 7.90 (2H, d), 7.64 (2H, d), 7.07 (1H, s), 4.67 (1H, quintet), 3.85 (1H, m), 3.65 (1H, m), 3.60 (1H, dd), 3.55-3.2 (6H, m), 3.9 (2H, m) 2.14 (1H, tt), 1.81 (1H, d), 1.61-1.48 (3H, m), 1.43-1.30 (1H, m), 1.27-1.14 (2H, m), 1.09 (3H, d), 0.92 (3H, d), 0.74 (3H, d), 0.69-0.46 (2H, m). Hydroxy and amino protons not clearly visible.

**Table 5: Characteristic X-Ray powder diffraction (XRPD) angles and d spacings for N-methyl-D-glucamine 3-[[(trans-4-methylcyclohexyl)carbonyl](1-methylethyl)amino]-5-{4-[(1,3-thiazol-4-ylcarbonyl)amino]phenyl}-2-thiophenecarboxylate.**

| Pos. [°2Th.] | d-spacing [Å] |
|---|---|
| 3.7 | 23.7 |
| 7.4 | 12.0 |
| 9.5 | 9.3 |
| 11.0 | 8.1 |
| 14.6 | 6.1 |
| 14.7 | 6.0 |
| 15.4 | 5.7 |
| 16.2 | 5.5 |
| 17.1 | 5.2 |
| 17.5 | 5.1 |
| 18.2 | 4.9 |
| 19.4 | 4.6 |
| 21.9 | 4.0 |

Data obtained for N-methyl-D-glucamine 3-[[(*trans-*4-methylcyclohexyl)carbonyl](1-methylethyl)amino]-5-{4-[(1,3-thiazol-4-ylcarbonyl)amino]phenyl}-2-thiophenecarboxylate can be seen in Figure 5.

### Example 81

### Potassium 3-[[(trans-4-methylcyclohexyl)carbonyl](1-methylethyl)amino]-5-{4-[(1,3-thiazol-4-ylcarbonyl)amino]phenyl}-2-thiophenecarboxylate

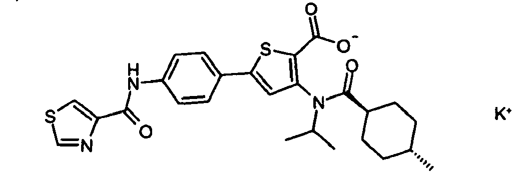

To 3-[[(*trans-*4-methylcyclohexyl)carbonyl](1-methylethyl)amino]-5-{4-[(1,3-thiazol-4-ylcarbonyl)amino]phenyl}-2-thiophenecarboxylic acid (50 mg, a synthesis of which is described as Example 1) in acetonitrile (2 mL), was added potassium hydroxide solution (8.37 uL, 45% in water). The mixture was then temperature cycled between room temperature and 40°C for 48 hours (4 hour cycles). The product was collected by filtration and dried at 40°C for 24 hours to give the title compound.
¹H NMR (d₆-DMSO): δ 10.46 (1H, s), 9.28 (1H, d), 8.52 (1H, d), 7.90 (2H, d), 7.64 (2H, d), 7.07 (1H, s), 4.67 (1H, quintet), 2.14 (1H, tt), 1.81 (1H, d), 1.61-1.48 (3H, m), 1.43-1.30 (1H, m), 1.27-1.14 (2H, m), 1.09 (3H, d), 0.92 (3H, d), 0.74 (3H, d), 0.69-0.46 (2H, m).

**Table 6: Characteristic X-Ray powder diffraction (XRPD) angles and d spacings for potassium 3-[[(trans-4-methylcyclohexyl)carbonyl](1-methylethyl)amino]-5-{4-[(1,3-thiazol-4-ylcarbonyl)amino]phenyl}-2-thiophenecarboxylate.**

| Pos. [°2Th.] | d-spacing [Å] |
|---|---|
| 3.9 | 22.5 |
| 6.9 | 12.8 |
| 8.8 | 10.0 |
| 11.2 | 7.9 |
| 11.6 | 7.6 |
| 13.8 | 6.4 |
| 15.1 | 5.9 |
| 16.6 | 5.4 |
| 17.6 | 5.0 |
| 19.4 | 4.6 |
| 21.3 | 4.2 |

Data obtained for potassium 3-[[(*trans-*4-methylcyclohexyl)carbonyl](1-methylethyl)amino]-5-{4-[(1,3-thiazol-4-ylcarbonyl)amino]phenyl}-2-thiophenecarboxylate can be seen in Figure 6.

### Example 82

### Choline 3-[[(trans-4-methylcyclohexyl)carbonyl](1-methylethyl)amino]-5-{4-[(1,3-thiazol-4-ylcarbonyl)amino]phenyl}-2-thiophenecarboxylate

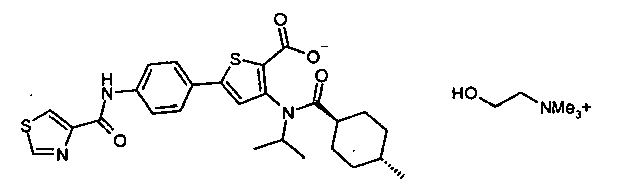

To 3-[[(*trans-*4-methylcyclohexyl)carbonyl](1-methylethyl)amino]-5-{4-[(1,3-thiazol-4-ylcarbonyl)amino]phenyl}-2-thiophenecarboxylic acid (57 mg, a synthesis of which is described as Example 1) in toluene (2.5 mL), was added choline hydroxide solution (28 uL, 45% in methanol). The mixture was stirred at room temperature for 24 hours and then temperature cycled between room temperature and 40°C for 48 hr. The product was collected by filtration and dried at 120°C for 24 hours to give the title compound.
¹H NMR (d₆-DMSO): δ 10.46 (1H, s), 9.28 (1H, d), 8.53 (1H, d), 7.91 (2H, d), 7.64 (2H, d), 7.16 (1H, s), 6.0-5.8 (1H, br), 4.68 (1H, quintet), 3.85 (2H, m), 3.50 (2H, m), 3.12 (9H, s), 2.14 (1H, tt), 1.81 (1H, d), 1.61-1.48 (3H, m), 1.43-1.30 (1H, m), 1.27-1.14 (2H, m), 1.09 (3H, d), 0.92 (3H, d), 0.74 (3H, d), 0.69-0.46 (2H, m).

**Table 7: Characteristic X-Ray powder diffraction (XRPD) angles and d spacings for choline 3-[[(trans-4-methylcyclohexyl)carbonyl](1-methylethyl)amino]-5-{4-[(1,3-thiazol-4-ylcarbonyl)amino]phenyl}-2-thiophenecarboxylate.**

| Pos. [°2Th.] | d-spacing [Å] |
|---|---|
| 5.7 | 15.5 |
| 6.8 | 13.1 |
| 11.5 | 7.7 |
| 11.9 | 7.4 |
| 15.3 | 5.8 |
| 17.0 | 5.2 |
| 19.2 | 4.6 |
| 20.7 | 4.3 |

Data obtained for choline 3-[[(*trans-*4-methylcyclohexyl)carbonyl](1-methylethyl)amino]-5-{4-[(1,3-thiazol-4-ylcarbonyl)amino]phenyl}-2-thiophenecarboxylate can be seen in Figure 7.

### Example 83

### L-Arginine 3-[[(trans-4-methylcyclohexyl)carbonyl](1-methylethyl)amino]-5-{4-[(1,3-thiazol-4-ylcarbonyl)amino]phenyl}-2-thiophenecarboxylate

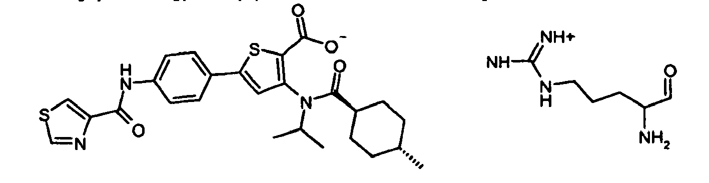

A mixture of 3-[[(*trans-*4-methylcyclohexyl)carbonyl](1-methylethyl)amino]-5-{4-[(1,3-thiazol-4-ylcarbonyl)amino]phenyl}-2-thiophenecarboxylic acid (298 mg, a synthesis of which is described as Example 1) and L-arginine (109 mg) in acetone (5 mL) was temperature cycled between room temperature and 40°C for 3 days. The product was collected by filtration and dried at 40°C under vacuum for 48 hours to give the title compound.
¹H NMR (d₆-DMSO): δ 10.46 (1H, s), 9.28 (1H, d), 8.53 (1H, d), 8.0-7.5 (6H, br), 7.93 (2H, d), 7.67 (2H, d), 7.20 (1H, s), 4.70 (1H, quintet), 3.25 (1H, t), 3.05 (2H, m) 2.14 (1H, tt), 1.80-1.45 (8H, m), 1.43-1.30 (1H, m), 1.27-1.14 (2H, m), 1.09 (3H, d), 0.92 (3H, d), 0.74 (3H, d), 0.69-0.46 (2H, m).

**Table 8: Characteristic X-Ray powder diffraction (XRPD) angles and d spacings for L-arginine 3-[[(trans-4-methylcyclohexyl)carbonyl](1-methylethyl)amino]-5-{4-[(1,3-thiazol-4-ylcarbonyl)amino]phenyl}-2-thiophenecarboxylate. Note that this material is mainly amorphous.**

| Pos. [°2Th.] | d-spacing [Å] |
|---|---|
| 18.4 | 4.8 |
| 19.2 | 4.6 |
| 19.5 | 4.5 |
| 23.2 | 3.8 |
| 27.5 | 3.2 |

Data obtained for L-arginine 3-[[(*trans-*4-methylcyclohexyl)carbonyl](1-methylethyl)amino]-5-{4-[(1,3-thiazol-4-ylcarbonyl)amino]phenyl}-2-thiophenecarboxylate can be seen in Figure 8.

### Example 84

### 3-{(Cyclopropylmethyl)[(trans-4-methylcyclohexyl)carbonyl]amino}-5-{4-[(1H-pyrazol-3-ylcarbonyl)amino]phenyl}-2-thiophenecarboxylic acid

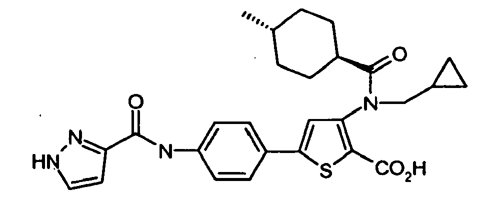

Intermediate 112 (35 mg) was dissolved in pyridine (1 mL) in a Reactivial^{™}. Lithium iodide (254 mg) was added and the reaction was stirred at 130°C for 4 h. The reaction was cooled and partitioned between 2N HCl and DCM. The organic phase was collected and dried by passing through a hydrophobic frit and blown down to dryness. The crude material was purified using a 5 g aminopropyl cartridge. The cartridge was washed with MeOH followed by 10% 2N HCl in MeOH, then freeze dried from 1,4-dioxane to give the title compound.
MS calcd for (C₂₇H₃₀N₄O₄S + H)⁺: 507
MS found (electrospray): (M+H)⁺ = 507
¹H NMR (CD₃OD): δ 7.93-7.82 (2H, br.m), 7.79-7.71 (3H, br.m), 7.43 (1H, s), 6.91 (1H, br.s), 3.74-3.68 (1H, m), 3.43-3.38 (1H, m), 2.27-2.20 (1H, m), 1.80-1.70 (2H, m), 1.69-1.62 (2H, m), 1.59-1.38 (2H, m), 1.34-1.25 (1H, m), 1.01-0.91 (1H, m), 0.80 (3H, d), 0.77-0.65 (2H, m), 0.46-0.39 (2H, m), 0.10-0.03 (2H, m), 3 exchangeable protons not seen.

### Example 85

### 3-{Cyclobutyl[(trans-4-methylcyclohexyl)carbonyl]amino}-5-(4-{[(1-methyl-1H-imidazol-4-yl)carbonyl]amino}phenyl)-2-thiophenecarboxylic acid

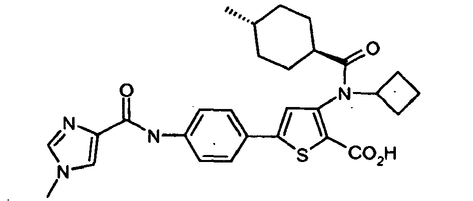

2N Lithium Hydroxide solution (1 mL) was added to a solution of Intermediate 113 (90 mg) in MeOH (2 mL) and tetrahydrofuran (1 mL) at room temperature. The reaction was stirred for 5 h, the solvent was evaporated and the residue acidified to pH 1 with 2N HCl. Extraction with EtOAc failed and the suspension was evaporated *in vacuo.* The residue was purified by MDAP HPLC and freeze dried from 1,4-dioxane to give the title compound.
MS calcd for (C₂₈H₃₂N₄O₄S + H)⁺: 521
MS found (electrospray): (M+H)⁺= 521
¹H NMR (d₆-DMSO): δ 13.20 (1H, br.s), 10.09 (1H, br.s), 7.97 (2H, d), 7.89-7.83 (2H, br.m), 7.77 (2H, d), 7.51 (1H, s), 4.83 (1H, m), 3.75 (3H, s), 2.16-2.05 (1H, m), 2.04-1.75 (3H, m), 1.72-1.12 (10H, m), 0.74 (3H, d), 0.69-0.50 (2H, m).

### Example 86

### 3-{Ethyl[(trans-4-methylcyclohexyl)carbonyl]amino}-5-(4-{[(1-methyl-1H-imidazol-4-yl)carbonyl]amino}phenyl)-2-thiophenecarboxylic acid

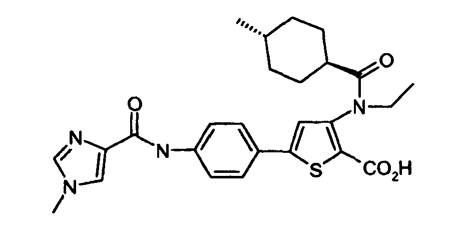

2N Lithium hydroxide solution (1 mL) was added to a solution of Intermediate 114 (121 mg) in MeOH (2 mL) and tetrahydrofuran (1 mL) at room temperature. The reaction was stirred at room temperature for 5 h, the solvent was evaporated and the residue acidified to pH 1 with 2N HCl. The suspension was evaporated *in vacuo* and the residue was purified by MDAP HPLC and freeze dried from 1,4-dioxane to give the title compound.
MS calcd for (C₂₆H₃₀N₄O₄S + H)⁺: 495
MS found (electrospray): (M+H)⁺ = 495
¹H NMR (d₆-DMSO): δ 13.23 (1H, br.s), 10.13 (1H, br.s), 8.00-7.86 (4H, m), 7.75 (2H, d), 7.56 (1H, s), 3.81-3.66 (4H, m), 3.56-3.44 (1H, m, + water), 2.10 (1H, m), 1.70-1.51 (4H, m), 1.49-1.17 (3H, m), 1.03-0.96 (3H, t), 0.74 (3H, d), 0.72-0.53 (2H, m).

### Example 87

### 3-[[(trans-4-Methylcyclohexyl)carbonyl](1-methylethyl)amino]-5-{4-methyl-5-[(1,3-thiazol-4-ylcarbonyl)amino]-2-pyridinyl}-2-thiophenecarboxylic acid

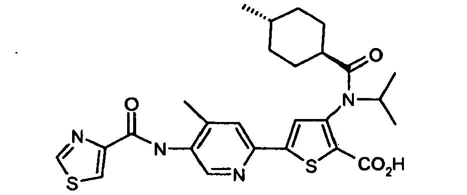

A solution of Intermediate 116 (3 mg) and sodium hydroxide (2.77 uL) in MeOH (775 uL) and tetrahydrofuran (775 uL) was stirred under nitrogen at room temperature for 2 days. The reaction mixture was evaporated to dryness and partitioned between 2M HCl and DCM. The organic layer was separated and evaporated *in vacuo.* The crude material was purified using a 1 g aminopropyl cartridge to give the title compound.
MS calcd for (C₂₆H₃₀N₄O₄S₂ + H)⁺: 527
MS found (electrospray): (M+H)⁺ = 527
¹H NMR (CD₃OD): δ 9.14 (2H, br.s), 8.51 (1H, br.s), 8.15 (1H, s), 7.75 (1H, s), 4.83 (1H, obscured by solvent), 2.58 (3H, s), 2.12-2.02 (1H, m), 1.80-1.20 (10H, m, + excess), 1.00 (3H, d), 0.78 (3H, d), 0.76-0.53 (2H, m), 2 exchangeable not seen.

### Example 88

### 3-{Cyclobutyl[(trans-4-methylcyclohexyl)carbonyl]amino}-5-{4-[(3-pyridazinylcarbonyl)amino]phenyl}-2-thiophenecarboxylic acid

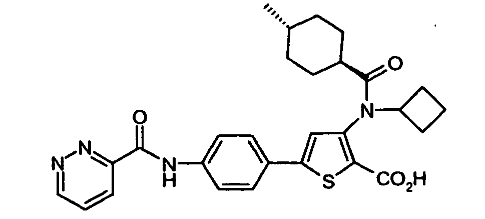

2N Lithium hydroxide solution (1 mL) was added to a solution of Intermediate 118 (75 mg) in MeOH (2 mL) and THF (1 mL). The reaction mixture was left to stir at room temperature for 4 h. The solvent was evaporated and the residue acidified to pH 1.0 with 2N hydrochloric acid, extracted with EtOAc (3 x 10 mL) and evaporated *in vacuo.* The residue was applied in MeOH to a 1 g aminopropyl cartridge which was eluted with MeOH (5 column volumes) followed by 10% 2N HCl in MeOH (5 column volumes). The appropriate fractions were concentrated *in vacuo,* purified by MDAP HPLC and freeze dried from 1,4-dioxane to give the title compound.
MS calcd for (C₂₈H₃₀N₄O₄S + H)⁺: 519
MS found (electrospray): (M+H)⁺ = 519
¹H NMR (d₆-DMSO): δ 13.23 (1H, br.s), 11.32 (1H, s), 9.49 (1H, dd), 8.35 (1H, dd), 8.10 (2H, d), 8.03-7.97 (1H, m), 7.86 (2H, d), 7.57 (1H, s), 4.84 (1H, m), 2.17-2.06 (1H, m), 2.04-1.75 (3H, m), 1.74-1.15 (10H, m), 0.74 (3H, d), 0.70-0.51 (2H, m).

### Example 89

### 5-{5-Chloro-6-[(1,3-thiazol-4-ylcarbonyl)amino]-3-pyridinyl}-3-[[(trans-4-methylcyclohexyl)carbonyl](1-methylethyl)amino]-2-thiophenecarboxylic acid

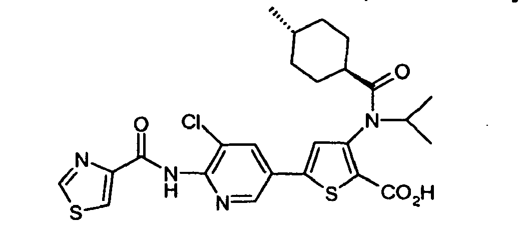

A solution of Intermediate 121 (33 mg) and sodium hydroxide (147 uL) in MeOH (775 uL) and THF (775 uL) was stirred under nitrogen at 25°C for 1 day. The reaction mixture was evaporated *in vacuo* and was washed with 2M HCl and DCM. The organic layer was separated and evaporated *in vacuo.* The residue was applied to a 2 g aminopropyl cartridge which was eluted with MeOH followed by 10% 2N HCl in MeOH. The crude material was further purified using a 4 g silica ISCO Companion^{™} cartridge eluting with a gradient of 0-15% MeOH in DCM to give the title compound.
MS calcd for (C₂₅H₂₇ClN₄O₄S₂ + H)⁺: 547/549
MS found (electrospray): (M+H)⁺ = 547/549
¹H NMR (CD₃OD): δ 8.15 (1H, br.s), 8.06 (1H, br.m), 7.75 (1H, br.m), 7.50 (1H, br.s), 7.22 (1H, br.m), 4.8 (1H, obscured by solvent), 2.14-1.98 (1H, m), 1.89-1.49 (5H, br.m), 1.43-1.17 (5H, br.m, + excess), 1.10-0.90 (3H, br.m), 0.85-0.47 (5H, m), 2 exchangeable protons not seen.

### Example 90

### 5-(4-{[(5-Fluoro-3-pyridinyl)carbonyl]amino}phenyl)-3-[[(trans-4-methylcyclohexyl)carbonyl](1-methylethyl)amino]-2-thiophenecarboxylic acid

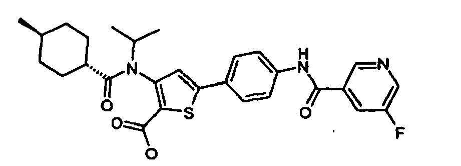

A Reactivial^{™} was charged with Intermediate 122 (50 mg), pyridine (0.5 mL) and lithium iodide (124 mg). The reaction was stirred at 110°C for 24 h. The reaction was cooled and solvent removed. The crude material was purified by MDAP HPLC to give the title compound.
MS calcd for (C₂₈H₃₀FN₃O₄S + H)⁺: 524
MS found (electrospray): (M+H)⁺ = 524

### Example 91

### 3-[[(trans-4-Methylcyclohexyl)carbonyl](1-methylethyl)amino]-5-{4-[(1,3-oxazol-4-ylcarbonyl)amino]phenyl}-2-thiophenecarboxylic acid

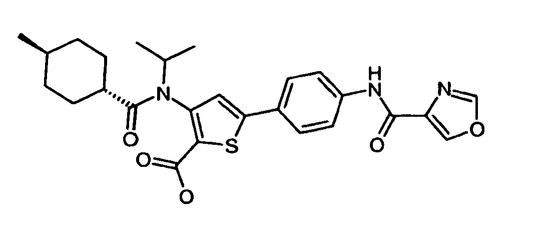

A mixture of Intermediate 123 (63 mg), lithium iodide (80 mg) and pyridine (1 mL) was heated in a Reactivial^{™} at 130°C for 4 h. The reaction mixture was allowed to cool and partitioned between DCM and 2N HCl solution. The organic phase was separated, dried by passing through a hydrophobic frit and evaporated *in vacuo.* The organic phase was applied to a 5 g aminopropyl cartridge pre-conditioned with methanol. The cartridge was washed with methanol and then the product was eluted with 10% 2N HCl in methanol. The organic phases were evaporated *in vacuo* and the crude material was further purified by MDAP HPLC to give the title compound.
MS calcd for (C₂₆H₂₉N₃O₅S + H)⁺: 496
MS found (electrospray): (M+H)⁺ = 496

### Example 92

### 3-[[(trans-4-Methylcyclohexyl)carbonyl](1-methylethyl)amino]-5-{4-[(1,3-oxazol-5-ylcarbonyl)amino]phenyl}-2-thiophenecarboxylic acid

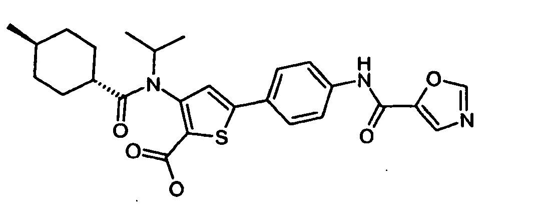

A mixture of Intermediate 124 (83 mg), lithium iodide (80 mg) and pyridine (1 mL) was heated in a Reactivial^{™} at 130°C for 4 h. The reaction mixture was allowed to cool and partitioned between DCM and 2N HCl solution. The organic phase was separated, dried by passing through a hydrophobic frit and evaporated *in vacuo.* The organic phase was applied to a 5 g aminopropyl cartridge pre-conditioned with methanol. The cartridge was washed with methanol and then the product was eluted with 10% 2N HCl in methanol. The organic phases were evaporated *in vacuo* and the crude material was further purified by MDAP HPLC to give the title compound.
MS calcd for (C₂₆H₂₉N₃O₅S + H)⁺: 496
MS found (electrospray): (M+H)⁺ = 496

Examples 93-115 were prepared using analogous methods to those Examples described above and are exemplified below in Table 9.

**Table 9**

| **Example No.** | **Name** | **Structure** | **Mass Spectroscopy** | |
|---|---|---|---|---|
| | | | (M+H)⁺ calcd | (M+H)⁺ found |
| 93 | 5-(4-{[(3,5-dimethyl-4-isoxazolyl)carbonyl]amino}ph enyl)-3-[[(*trans-*4-methylcyclohexyl)carbonyl](1-methylethyl)amino]-2-thiophenecarboxylic acid | 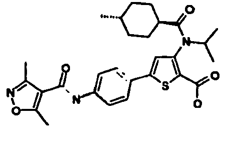 | 524 | 524 |
| 94 | 5-(4-{[(1-ethyl-1*H-*pyrazol-5-yl)carbonyl]amino}phenyl)-3-[[(*trans-*4-methylcyclohexyl)carbonyl](1-methylethyl)amino]-2-thiophenecarboxylic acid | 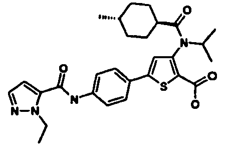 | 523 | 523 |
| 95 | 3-[[(*trans-*4-methylcyclohexyl)carbonyl](1-methylethyl)amino]-5-(4-{[(1-methyl-1*H-*imidazol-5-yl)carbonyl]amino}phenyl)-2-thiophenecarboxylic acid | 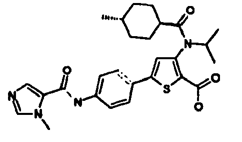 | 509 | 509 |
| 96 | 3-[[(*trans-*4-methylcyclohexyl)carbonyl](1 -methylethyl)amino]-5-(4-{[(3-methyl-4-pyridinyl)carbonyl]amino}phe nyl)-2-thiophenecarboxylic acid | 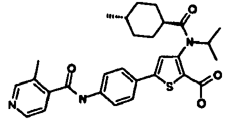 | 520 | 520 |
| 97 | *3-[[(*trans-*4-*methylcyclohexyl)carbonyl](1-methylethyl)amino]-5-{4-[(1,3-thiazol-5-ylcarbonyl)amino]phenyl}-2-thiophenecarboxylic acid | 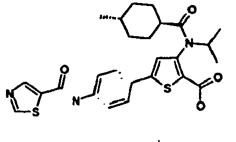 | 512 | 512 |
| 98 | 3-[[(*trans-*4-methylcyclohexyl)carbonyl](1-methylethyl)amino]-5-(4-{[(1-methyl-1*H-*pyrazol-5-yl)carbonyl]amino}phenyl)-2-thiophenecarboxylic acid | 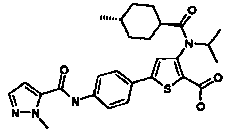 | 509 | 509 |
| 99 | 5-(4-{[(2,4-dimethyl-1,3-oxazol-5-yl)carbonyl]amino}phenyl)-3-[[(trans-*4-* methylcyclohexyl)carbonyl](1-methylethyl)amino]-2-thiophenecarboxylic acid | 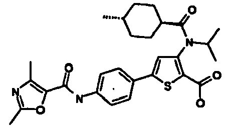 | 524 | 524 |
| 100 | 5-(4-{[(1,3-dimethyl-1*H-*pyrazol-5-yl)carbonyl]amino}phenyl)-3-*[[(*trans-*4-* methylcyclohexyl)carbonyl](1-methylethyl)amino]-2-thiophenecarboxylic acid | 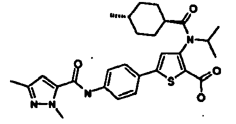 | 523 | 523 |
| 101 | *3-[[(*trans-*4-*methylcyclohexyl)carbonyl](1-methylethyl)amino]-5-(4-{[(4-methyl-1,3-oxazol-5-yl)carbonyl]amino}phenyl)-2-thiophenecarboxylic acid | 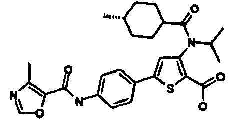 | 510 | 510 |
| 102 | 5-(4-{[(1,5-dimethyl-1*H-*pyrazol-4-yl)carbonyl]amino}phenyl)-3-[[(*trans-*4-methylcyclohexyl)carbonyl](1-methylethyl)aminol-2-thiophenecarboxylic acid | 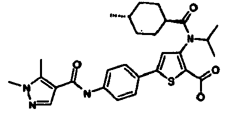 | 523 | 523 |
| 103 | *3-[[(trans-4-*methylcyclohexyl)carbonyl](1-methylethyl)amino]-5-(4-{[(2-methyl-3-pyridinyl)carbonyl]amino}phe nyl)-2-thiophenecarboxylic acid | 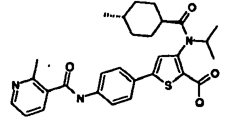 | 520 | 520 |
| 104 | 3-[[(*trans*-4-methylcyclohexyl)carbonyl](1-methylethyl)amino]-5-(4-{[(6-methyl-3-pyridinyl)carbonyl]amino}phe nyl)-2-thiophenecarboxylic acid | 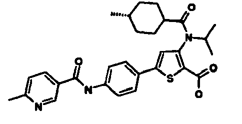 | 520 | 520 |
| 105 | 3-[[(*trans*-4- methylcyclohexyl)carbonyl](1-methylethyl)amino]-5-{4-[(1*H-*pyrazol-4-ylcarbonyl)amino]phenyl}-2-thiophenecarboxylic acid | 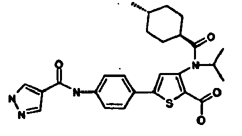 | 495 | 495 |
| 106 | 5'-[(2-furanylcarbonyl)amino]-4-[[(*trans*-4-methylcyclohexyl)carbonyl](1-methylethyl)amino]-2,2'-bithiophene-5-carboxylic acid | 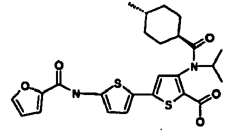 | 501 | 501 |
| 107 | 3-[[(*trans-*4-methylcyclohexyl)carbonyl](1 -methylethyl)amino]-5-(4-{[(5-methyl-4-isoxazolyl)carbonyl]amino}ph enyl)-2-thiophenecarboxylic acid | 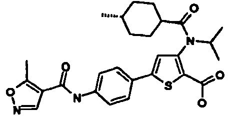 | 510 | 510 |
| 108 | 5-(4-{[(1-ethyl-1*H-*pyrazol-4-yl)carbonyl]amino}phenyl)-3-[[(*trans*-4-methylcyclohexyl)carbonyl](1-methylethyl)amino]-2-thiophenecarboxylic acid | 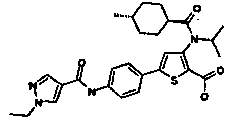 | 523 | 523 |
| 109 | 3-[[(*trans-*4-methylcyclohexyl)carbonyl](1-methylethyl)amino]-5-(4-{[(5-methyl-3-pyridinyl)carbonyl]amino}phe nyl)-2-thiophenecarboxylic acid | 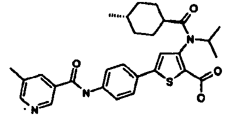 | 520 | 520 |
| 110 | 5'-{[(4-fluorophenyl)carbonyl]amino} -4-[[(*trans*-4-methylcyclohexyl)carbonyl](1-methylethyl)amino]-2,2'-bithiophene-5-carboxylic acid | 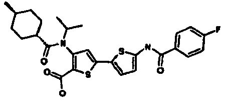 | 529 | 529 |
| 111 | 3-[[(*trans*-4-methylcyclohexyl)carbonyl](1-methylethyl)amino]-5-{5-[(1,3-thiazol-4-ylcarbonyl)amino]-2-pyridinyl}-2-thiophenecarboxylic acid | 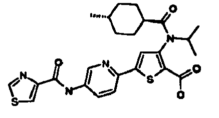 | 513 | 513 |
| 112 | 5-(4-{[(1,3-dimethyl-1*H-*pyrazol-4-yl)carbonyl]amino}phenyl)-3-[[(*trans-4-*methylcyclohexyl)carbonyl](1-methylethyl)amino]-2-thiophenecarboxylic acid | 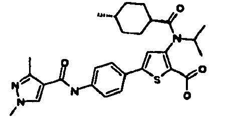 | 523 | 523 |
| 113 | 5-(4-{[(1-ethyl-1*H-*imidazol-5-yl)carbonyl]amino}phenyl)-3-[[(*trans-*4-methylcyclohexyl)carbonyl](1-methylethyl)amino]-2-thiophenecarboxylic acid | 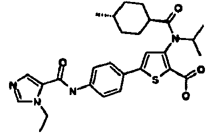 | 523 | 523 |
| 114 | 3-[[(*trans-*4-methylcyclohexyl)carbonyl](1-methylethyl)amino]-5-(4-{[(3-methyl-1*H-*pyrazol-4-yl)carbonyl]amino}phenyl)-2-thiophenecarboxylic acid | 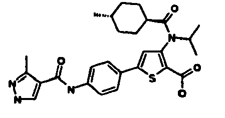 | 509 | 509 |
| 115 | 5-{4-[(5-isothiazolylcarbonyl)amino]ph enyl}-3-[[(*trans-*4-methylcyclohexyl)carbonyl](1-methylethyl)amino]-2-thiophenecarboxylic acid | 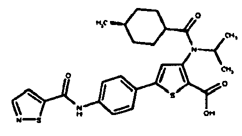 | 512 | 512 |

### Example 116

### Magnesium 3-[[(trans-4-methylcyclohexyl)carbonyl](1-methylethyl)amino]-5-{4-[(1,3-thiazol-4-ylcarbonyl)amino]phenyl}-2-thiophenecarboxylate

3-[[(*trans-*4-Methylcyclohexyl)carbonyl](1-methylethyl)amino]-5-{4-[(1,3-thiazol-4-ylcarbonyl) amino]phenyl}-2-thiophenecarboxylic acid (approximately 25 mg, a synthesis of which is described as Example 1) and magnesium hydroxide (2.9 mg) were dispensed into each reaction vessel. Three solvents yielded solids for this salt: IPA, IPA:water (5%), and methyl isobutyl ketone (MIBK). A volume of 1.25 mL of each solvent was used. The reaction vessels were shaken and temperature cycled between 0-40°C (in 1 hour blocks) for a minimum of 48 hours. The resulting solids and solutions were isolated by filtration. All solids were analysed by polarised light microscopy (PLM), Raman spectroscopy and XRPD. These data indicated that partially crystalline material which was not consistent with the free acid was obtained for these three experiments.

### X-Ray Powder Diffraction

X-Ray Powder Diffraction (XRPD) data was acquired on a PANalytical X'Pert PRO powder diffractometer, model PW3040/60, serial number DY1850 using an XCelerator detector. The acquisition conditions were: radiation: Cu Ka, generator tension: 40 kV, generator current: 45 mA, start angle: 2.0° 2θ, end angle: 40.0° 2θ, step size: 0.0167° 2θ, time per step: 31.75 seconds. The sample was prepared by mounting a few milligrams of sample on a Si wafer (zero background) plates, resulting in a thin layer of powder. Characteristic XRPD angles and d-spacings are recorded in the Tables 1 to 8. These values were determined by using Highscore software. The margin of error is approximately ± 0.1° 2θ for each of the peak assignments.

The compounds of Formula (I) may be formulated for administration in any convenient way, and the invention therefore also includes within its scope pharmaceutical compositions for use in therapy, comprising a compound of Formula (I) or a pharmaceutically acceptable salt, solvate or ester thereof, for example salt or solvate, in admixture with one or more pharmaceutically acceptable diluents or carriers.

The compounds of Formula (I) can be administered by different routes including oral, parenteral (e.g. intravenous, intraperitoneal, subcutaneous, intramuscular), rectal, topical, transdermal, transmucosal, buccal, sublingual, intranasal or by inhalation administration. For systemic administration, oral administration is convenient.

For oral administration, for example, the compounds of Formula (I) can be formulated into conventional oral dosage forms such as capsules, tablets and liquid preparations such as syrups, elixirs, concentrated drops, ovules, solutions or suspensions, which may contain flavouring or colouring agents, for immediate-, delayed-, modified-, sustained-, pulsed-or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia.

Additionally, lubricating agents such as magnesium stearate, stearic acid, glyceryl behenate and talc may be included.

Solid compositions of a similar type may also be employed as fillers in gelatin capsules. Suitable excipients in this regard include lactose, starch, a cellulose, milk sugar or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

Alternatively, injection (parenteral administration) may be used, e.g., intravenously, intraarterially, intraperitoneally, intrathecally, intraventricularly, intraurethrally, intrasternally, intracranially, intramuscularly or subcutaneously administering the agent; and/or by using infusion techniques. For parenteral administration, the compound may be used in the form of a sterile aqueous solution which may contain other substances, for example, enough salts or glucose to make the solution isotonic with blood. Typical parenteral compositions consist of a solution or suspension of a compound of Formula (I) or a pharmaceutically acceptable salt, solvate or ester in a sterile aqueous or non-aqueous carrier optionally containing a parenterally acceptable oil, for example polyethylene glycol, polyvinylpyrrolidone, lecithin, arachis oil or sesame oil. The aqueous solutions should be suitably buffered (suitably to a pH of from 3 to 9), if necessary. The preparation of suitable parenteral formulations under sterile conditions is readily accomplished by standard pharmaceutical techniques well-known to those skilled in the art. For example, the compounds of Formula (I) may be formulated in liquid solutions, for example, in pharmaceutically compatible buffers or solutions, such as saline solution, Hank's solution, or Ringer's solution. In addition, the compounds of Formula (I) may be formulated in solid form and redissolved or suspended immediately prior to use. Lyophilized forms can also be produced.

Alternatively, the compounds of Formula (I) may be administered in the form of a suppository or pessary, or may be applied topically in the form of a gel, hydrogel, lotion, salve, solution, cream, ointment or dusting powder. The compounds of Formula (I) may also be dermally or transdermally administered, for example, by the use of a skin patch. They may also be administered by the pulmonary or rectal routes. They may also be administered by the ocular route. For ophthalmic use, the compounds may be formulated as micronised suspensions in isotonic, pH adjusted, sterile saline, or, suitably, as solutions in isotonic, pH adjusted, sterile saline, optionally in combination with a preservative such as a benzylalkonium chloride. Alternatively, they may be formulated in an ointment such as petrolatum.

For application topically to the skin, the compounds of Formula (I) may be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water.

Alternatively, it can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

Compositions for rectal administration are conveniently in the form of suppositories containing a conventional suppository base such as cocoa butter. A typical suppository formulation comprises a compound of Formula (I) or a pharmaceutically acceptable salt thereof which is active when administered in this way, with a binding and/or lubricating agent, for example polymeric glycols, gelatins, cocoa-butter or other low melting vegetable waxes or fats or their synthetic analogs.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, bile salts and fusidic acid derivatives. In addition, detergents may be used to facilitate permeation. Transmucosal administration, for example, may be through nasal sprays, rectal suppositories, or vaginal suppositories. Typical dermal and transdermal formulations comprise a conventional aqueous or non-aqueous vehicle, for example a cream, ointment, lotion or paste or are in the form of a medicated plaster, patch or membrane

Compositions suitable for buccal or sublingual administration include tablets, lozenges and pastilles, wherein the compound of Formula (I) is formulated with a carrier such as sugar and acacia, tragacanth, or gelatin and glycerin.

The compounds of Formula (I) can also be administered intranasally or by inhalation and is conveniently delivered in the form of a dry powder inhaler or an aerosol spray presentation from a pressurised container, pump, spray or nebuliser with the use of a suitable propellant, e. g. dichlorodifluoromethane, trichlorofluoromethane, dichlorotetrafluoroethane, a hydrofluoroalkane such as 1,1,1,2-tetrafluoroethane (HFA 134AT"") or 1,1,1,2,3,3,3-heptafluoropropane (HFA 227EA), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, e. g. using a mixture of ethanol and the propellant as the solvent, which may additionally contain a lubricant, e. g. sorbitan trioleate.

Capsules and cartridges (made, for example, from gelatin) for use in an inhaler or insufflator may be formulated to contain a powder mix of the compound and a suitable powder base such as lactose or starch.

The amounts of various compounds of Formula (I) to be administered can be determined by standard procedures taking into account factors such as the compound (IC₅₀) potency, (EC₅₀) efficacy, and the biological half-life (of the compound of Formula (I)), the age, size and weight of the patient, and the disease or disorder associated with the patient. The importance of these and other factors to be considered are known to those of ordinary skill in the art.

Amounts of the compounds of Formula (I) administered also depend on the routes of administration and the degree of oral bioavailability. For example, for compounds of Formula (I) with low oral bioavailability, relatively higher doses will have to be administered. Oral administration is a convenient method of administration of the present compounds of Formula (I).

Suitably the composition is in unit dosage form. For oral application, for example, a tablet, or capsule may be administered, for nasal application, a metered aerosol dose may be administered, for transdermal application, a topical formulation or patch may be administered and for transmucosal delivery, a buccal patch may be administered. In each case, dosing is such that the patient may administer a single dose.

Each dosage unit for oral administration contains suitably from 0.01 to 500 mg/Kg, for example from 0.1 to 50 mg/Kg, of a compound of Formula (I) or a pharmaceutically acceptable salt thereof, calculated as the free base. The daily dosage for parenteral, nasal, oral inhalation, transmucosal or transdermal routes contains suitably from 0.01 mg to 100 mg/Kg, of a compound of Formula(I). A topical formulation contains suitably 0.01 to 5.0% of a compound of Formula (I). The active ingredient may be administered from 1 to 6 times per day, for example once, sufficient to exhibit the desired activity, as is readily apparent to one skilled in the art.

Compounds of Formula (I) which are active when given orally can be formulated as syrups, tablets, capsules and lozenges. A syrup formulation will generally consist of a suspension or solution of the compound or salt in a liquid carrier for example, ethanol, peanut oil, olive oil, glycerine or water with a flavouring or colouring agent. Where the composition is in the form of a tablet, any pharmaceutical carrier routinely used for preparing solid formulations may be used. Examples of such carriers include magnesium stearate, terra alba, talc, gelatin, acacia, stearic acid, starch, lactose and sucrose. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils, and are incorporated in a soft gelatin capsule shell.

### ASSAYS

The potential for compounds of Formula (I) to inhibit NS5B wildtype HCV polymerase activity, genotype 1b, may be demonstrated, for example, using the following *in vitro* assay:

### In Vitro Detection of inhibitors of HCV RNA-dependent RNA Polymerase Activity

Incorporation of [³³P]-GMP into RNA was followed by absorption of the biotin labelled RNA polymer by streptavidin containing Scintillation Proximity Assay (SPA) beads. A synthetic template consisting of biotinylated 13mer-oligoG hybridised to polyrC was used as a homopolymer substrate.

### Genotype 1b Full-Length Enzyme

Reaction Conditions were 0.5 µM [³³P]-GTP (20 Ci/mMol), 1 mM dithiothreitol, 20 mM MgCl₂, 5mM MnCl₂, 20 mM Tris-HCl, pH7.5, 1.6 µg/mL polyC/0.256 µM biotinylated oligoG13, 10% glycerol, 0.01% NP-40, 0.2 u/µL RNasin and 50 mM NaCl.

HCV RNA Polymerase (Recombinant full-length NS5B (Lohmann et al, J. Virol. 71 (11), 1997, 8416. 'Biochemical properties of hepatitis C virus NS5B RNA-dependent RNA polymerase and identification of amino acid sequence motifs essential for enzymatic activity') expressed in baculovirus and purified to homogeneity) was added to 4 nM final concentration.

5x concentrated assay buffer mix was prepared using 1 M MnCl₂ (0.25 mL), glycerol (2.5mL), 10% NP-40 (0.025 mL) and water (7.225 mL), *Total 10* mL.

2x concentrated enzyme buffer contained 1M-Tris-HCl, pH7.5 (0.4 mL), 5M NaCl (0.2 mL), 1M-MgCl₂ (0.4 mL), glycerol (1 mL), 10% NP-40 (10 µL), 1M DTT (20 µL) and water (7.97 mL), *Total 10* mL.

Substrate Mix was prepared using 5x Concentrated assay Buffer mix (4µL), [³³P]-GTP (10 µCi/µL, 0.02µL), 25 µM GTP (0.4 µL), 40 u/µL RNasin (0.1 µL), 20 µg/mL polyrC/biotinylated-oligorG (1.6 µL), and water (3.94 µL), *Total* 10 µL.

Enzyme Mix was prepared by adding 1mg/ml full-length NS5B polymerase (1.5 µL) to 2.81 mL 2x-concentrated enzyme buffer.

The Assay was set up using compound (1µL), Substrate Mix (10 µL), and Enzyme Mix (added last to start reaction) (10 µL), *Total* 21 µL.

The reaction was performed in a U-bottomed, white, 96-well plate. The reaction was mixed on a plate-shaker, after addition of the Enzyme, and incubated for 1h at 22°C. After this time, the reaction was stopped by addition of 40 µL 1.875 mg/ml streptavidin SPA beads in 0.1 M EDTA. The beads were incubated with the reaction mixture for 1h at 22°C after which 120 µL 0.1 M EDTA in PBS was added. The plate was sealed, mixed centrifuged and incorporated radioactivity determined by counting in a TriLux Wallac^{™} Microbeta^{®} 1450 or Topcount^{®}(Packard) Scintillation Counter.

After subtraction of background levels without enzyme, any reduction in the amount of radioactivity incorporated in the presence of a compound, compared to that in the absence, was taken as a measure of the level of inhibition. Ten concentrations of compounds were tested in three- or fivefold dilutions. From the counts, percentage of inhibition at highest concentration tested or IC₅₀s for the compounds were calculated using GraFit 3™, GraFit 4™ or GraFit 5™ software packages or a data evaluation macro for Excel^{™} based on XLFit Software (IDBS).

The potential for compounds of Formula (I) to inhibit HCV replication, genotype 1a and genotype 1b, may be demonstrated, for example, using the following cell based assay:

### Replicon Luciferase cell based assay

### Method

A 40mM stock solution in DMSO of each test compound was further diluted into 50µL of DMSO in the first row of a 96 well, V-bottom microplate, to give 100 times the top concentration of the required dilution series. Aliquots of 25µL of DMSO were added to each well of the remaining rows, and doubling dilutions of compound were prepared by the serial transfer of 25µL volumes from the first row onwards. A PlateMate™ robot was used to transfer 1µL volumes from each dilution well into duplicate wells of a clear bottom, black walled, 96 well assay plate (COSTAR #3603). Control wells received 1 µL of DMSO alone.

Suspensions were prepared from cultures of Huh-7 cells stably transfected with sub-genomic HCV NS3-NS5B replicons of either genotype 1b (the ET subline described by Pietschmann,T., Lohmann, V., Kaul, A., Krieger, N., Rinck, G., Rutter, G., Strand, D. & Bartenschlager, R., Journal of Virology, 2002, 76, 4008-4021) or genotype 1a (subline 1.19 constructed in-house) linked to a firefly luciferase reporter gene. Monolayers nearing confluency were stripped from growth flasks with versene-trypsin solution and the cells resuspended in assay medium comprising DMEM (Invitrogen #41965-039) supplemented with 5% v/v foetal calf serum, 1% v/v non-essential amino acids solution, 100 units/ml penicillin, 100µg/ml streptomycin and 2mM L-glutamine. 100µL of suspension containing either 15,000 cells (genotype 1 b luciferase replicon) or 20,000 cells (genotype 1a luciferase replicon) were added to all wells, except medium controls, of the assay plate and the plate incubated for 48 hours at 37°C in a 5% CO₂ atmosphere.

One tablet of Resazurin (Fisher #R/0040/79) was dissolved in 50mL of phosphate buffered saline and 100µL of solution added to all wells. The plate was re-incubated at 37°C for a further 2- 4 hours, wrapped in aluminium foil, before reading in a FLUOStar Optima at 595nm. All growth medium and Resazurin was removed by aspiration, and an opaque mask applied to the bottom of the plate. A solution of SteadyLite™ cytolytic buffer/luciferase substrate (Perkin-Elmer #6016987) was prepared according to the manufacturer's instructions, and 25µL added to each well. The plate was then read for luminescence on a TopCount^{®} NXT™.

### Data Analysis

Toxicity : The Resazurin absorbance values from duplicate wells were averaged and expressed as a percentage of the mean absorbance of compound free control wells to determine comparative cell viability. Compound cytotoxicity was expressed either as the lowest concentration at which a significant reduction in viability was observed or a 50% toxic concentration (CCID₅₀) was determined by plotting percentage cytotoxicity against compound concentration using Grafit^{™} software (Erithacus Software Ltd.).

Potency : The luminescence values from all compound-free wells containing cells were averaged to obtain a positive control value. The mean luminescence value from the compound-free wells that had received no cells was used to provide the negative (background) control value. The readings from the duplicate wells at each compound concentration were averaged and, after the subtraction of the mean background from all values, were expressed as a percentage of the positive control signal. The quantifiable and specific reduction of luciferase signal in the presence of a drug is a direct measure of replicon inhibition. GraFit™ software was used to plot the curve of percentage inhibition against compound concentration and derive the 50% inhibitory concentration (IC₅₀) for the compound.

Examples 1-89 demonstrate an activity of IC₅₀ < 500nM in the 1a luciferase replicon assay and in the 1b luciferase replicon assay. Preferred compounds of Formula (I) demonstrate an activity of IC₅₀ < 100nM in at least one of the 1 a luciferase replicon assay and in the 1b luciferase replicon assay. The compound of Example 1 demonstrated an activity of IC₅₀ < 100nM in the 1 a luciferase replicon assay and in the 1b luciferase replicon assay. Examples 93-115 (Table 2) demonstrate an activity of IC₅₀ > 500nM in the 1a luciferase replicon assay.

The compounds of Formula (I) which have been tested demonstrate a surprisingly high potency as HCV polymerase inhibitors, as shown by the IC₅₀ values in the cell-based assays across both of the 1 a and 1 b genotypes of HCV. Accordingly, the compounds of Formula (I) are of great potential therapeutic benefit in the treatment and prophylaxis of HCV.

The pharmaceutical compositions according to the invention may also be used in combination with other therapeutic agents. The invention thus provides, in a further aspect, a combination comprising a compound of Formula (I) or a salt, solvate or ester thereof together with at least one other therapeutic agent.

When a compound of Formula (I) or a salt, solvate or ester thereof is used in combination with a second therapeutic agent active against the same disease state, the dose of each compound may differ from that when the compound is used alone. Appropriate doses will be readily appreciated by those skilled in the art. It will be appreciated that the amount of a compound of Formula (I) or a salt, solvate or ester thereof required for use in treatment will vary with the nature of the condition being treated and the age and the condition of the patient and will be ultimately at the discretion of the attendant physician or veterinarian. The compounds of Formula (I) or a salt, solvate or ester thereof may be used in combination with other therapeutic agents, for example immune therapies [e.g. interferon, such as interferon alpha-2a (ROFERON®-A; Hoffmann-La Roche), interferon alpha-2b (INTRON®A; Schering-Plough), interferon alfacon-1 (INFERGEN®; Intermune), peginterferon alpha-2b (PEGINTRON^{™}; Schering-Plough) or peginterferon alpha-2a (PEGASYS®; Hoffmann-La Roche)], therapeutic vaccines, antifibrotic agents, anti-inflammatory agents [such as corticosteroids or NSAIDs], bronchodilators [such as beta-2 adrenergic agonists and xanthines (e.g. theophylline)], mucolytic agents, anti-muscarinics, anti-leukotrienes, inhibitors of cell adhesion [e.g. ICAM antagonists], anti-oxidants [e.g. N-acetylcysteine], cytokine agonists, cytokine antagonists, lung surfactants and/or antimicrobial, anti-viral agents [e.g. ribavirin and amantidine], and anti-HCV agents [for example HCV NS3 protease inhibitors, e.g. VX950 (telapravir; Vertex) or SCH503034 (Schering Plough)], or HCV NS5b polymerase inhibitors [for example HCV796 (Wyeth) or R1626 (Roche)], RNAi agents or cyclophilin inhibitors. The compositions according to the invention may also be used in combination with gene replacement therapy.

The invention thus provides, in a further aspect, a combination comprising at least one compound of Formula (I) together with at least one other therapeutically active agent, especially Interferon, ribavirin and/or an additional anti-HCV agent.

The combinations referred to above may conveniently be presented for use in the form of a pharmaceutical formulation and thus pharmaceutical formulations comprising a combination as defined above together with a pharmaceutically acceptable carrier thereof represent a further aspect of the invention.

The individual components of such combinations may be administered either sequentially or simultaneously in separate or combined pharmaceutical formulations. Appropriate doses of known therapeutic agents will be readily appreciated by those skilled in the art.

## Claims

1. A compound of Formula (I) : 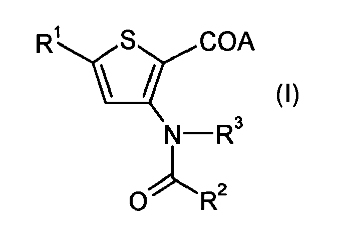 wherein:
A represents hydroxy;
R¹ represents -R^{X}-NHC(O)-R^{Y};
R^{X} represents phenyl (optionally substituted by one or more substituents selected independently from halo, methyl, ethyl, methoxy and trifluoromethyl), 5- or 6-membered heteroaryl (optionally substituted by one or more substituents selected independently from halo, methyl, ethyl, methoxy and trifluoromethyl) bonded through a carbon atom to the thiophene; the thiophene and amide groups being in a *para* relationship to each other;
R^{Y} represents phenyl (optionally substituted by one or more substituents selected independently from halo, methyl, ethyl, methoxy, trifluoromethyl, hydroxy and amino) or 5- or 6-membered heteroaryl;
R² represents C₅₋₇cycloalkyl (optionally substituted by one or more substituents selected independently from -C₁₋₆alkyl [optionally substituted by one or more substituents selected independently from fluoro and -OR^{A}], halo and -OR^{A});
R^{A} represents hydrogen or -C₁₋₄alkyl; and
R³ represents C₁₋₆alkyl (optionally substituted by one or more substituents selected independently from fluoro, hydroxyl, methoxy, ethoxy, C₃₋₆cycloalkyl, and a 5 or 6-membered heteroaryl and heterocyclyl), tetrahydrofuranyl, pyranyl, C₃₋₇cycloalkyl or dioxanyl;
or salts thereof.

2. A compound as claimed in claim 1 wherein R^{X} represents unsubstituted phenyl, 2-chlorophenyl, 3-(trifluoromethyl)phenyl, 3-methoxyphenyl, 2-pyridinyl, 3-pyridinyl, 5-methyl-3-pyridinyl, 4-methyl-2-pyridinyl or 5-chloro-3-pyridinyl, wherein the pyridinyl ring in all cases is bonded through a carbon atom to the thiophene, the thiophene and amide groups being in a para relationship to each other; or unsubstituted thienyl bonded to the amide group and the thiophene at the 2- and 5-positions.

3. A compound as claimed in claim 1 or claim 2 wherein R^{Y} represents phenyl (optionally substituted by one or more substituents selected from halo) or 5- or 6-membered heteroaryl.

4. A compound as claimed in any of claims 1 to 3 wherein R^{Y} represents fluorophenyl, unsubstituted pyridinyl, methylpyridinyl, fluoropyridinyl, unsubstituted pyrazinyl, methylpyrazinyl, unsubstituted pyridazinyl, unsubstituted pyrimidinyl, unsubstituted furanyl, methylfuranyl, hydroxymethylfuranyl, unsubstituted oxazolyl, methyloxazolyl, unsubstituted isoxazolyl, methylisoxazolyl, ethylisoxazolyl, unsubstituted thienyl, unsubstituted thiazolyl, unsubstituted isothiazolyl, methylthiazolyl, aminothiazolyl, pyrazolyl, methylpyrazolyl, ethylpyrazolyl, unsubstituted imidazolyl, methylimidazolyl or ethylimidazolyl.

5. A compound as claimed in any of claims 1 to 4 wherein R² represents unsubstituted cyclohexyl or cyclohexyl substituted by one or more substituents selected independently from methyl, trifluoromethyl and fluoro.

6. A compound as claimed in any of claims 1 to 5 wherein R³ represents C₁₋₆alkyl (optionally substituted by one or more substituents selected independently from fluoro, methoxy and C₃₋₆cycloalkyl), C₃₋₇cycloalkyl or dioxanyl.

7. A compound as claimed in claim 1 selected from the group consisting of:
3-[[(*trans-*4-Methylcyclohexyl)carbonyl](1-methylethyl)amino]-5-{4-[(1,3-thiazol-4-ylcarbonyl)amino]phenyl}-2-thiophenecarboxylic acid;
3-[[(*trans-*4-Methylcyclohexyl)carbonyl](1-methylethyl)amino]-5-{4-[(2-pyridinylcarbonyl)amino]phenyl}-2-thiophenecarboxylic acid;
5-{4-[(2-Furanylcarbonyl)amino]phenyl}-3-[[(*trans-*4-methylcyclohexyl)carbonyl](1-methylethyl)amino]-2-thiophenecarboxylic acid;
3-[[(*trans-*4-Methylcyclohexyl)carbonyl](1-methylethyl)amino]-5-(4-{[(2-methyl-1,3-thiazol-4-yl)carbonyl]amino}phenyl)-2-thiophenecarboxylic acid;
3-[[(*trans-*4-Methylcyclohexyl)carbonyl](1-methylethyl)amino]-5-{4-[(2-thienylcarbonyl)amino]phenyl}-2-thiophenecarboxylic acid;
3-[[(*trans-*4-Methylcyclohexyl)carbonyl](1-methylethyl)amino]-5-{4-[(2-pyrazinylcarbonyl)amino]phenyl}-2-thiophenecarboxylic acid;
5-(4-{[(4Fluorophenyl)carbonyl]amino}phenyl)-3-[[(*trans-*4-methylcyclohexyl)carbonyl](1-methylethyl)amino]-2-thiophenecarboxylic acid;
3-[[(*trans-*4-Methylcyclohexyl)carbonyl](1-methylethyl)amino]-5-{4-[(1,3-oxazol-2-ylcarbonyl)amino]phenyl}-2-thiophenecarboxylic acid;
3-{(Cyclopropyl methyl)[(*trans-*4-methylcyclohexyl)carbonyl]amino}-5-{4-[(1,3-thiazol-4-ylcarbonyl)amino]phenyl}-2-thiophenecarboxylic acid;
3-((Cyclopropylmethyl){[*trans-*4-(trifluoromethyl)cyclohexyl]carbonyl}amino)-5-{4-[(1,3-thiazol-4-ylcarbonyl)amino]phenyl}-2-thiophenecarboxylic acid;
3-((1-Methylethyl){[*trans-*4-(trifluoromethyl)cyclohexyl]carbonyl}amino)-5-{4-[(1,3-thiazol-4-ylcarbonyl)amino]phenyl}-2-thiophenecarboxylic acid;
3-[[(*trans-*4-Methylcyclohexyl)carbonyl](1-methylethyl)amino]-5-{4-[(3-pyridazinylcarbonyl)amino]phenyl}2-thiophenecarboxylic acid;
3-[[(*trans-*4-Methylcyclohexyl)carbonyl](1-methylethyl)amino]-5-{4-[(4-pyrimidinylcarbonyl)amino]phenyl}-2-thiophenecarboxylic acid;
5-{3-Chloro-4-[(1,3-thiazol-4-ylcarbonyl)amino]phenyl}-3-[[(*trans-*4-methylcyclohexyl)carbonyl](1-methylethyl)amino]-2-thiophenecarboxylic acid;
3-[[(*trans-*4-Methylcyclohexyl)carbonyl](1-methylethyl)amino]-5-{4-[(3-thienylcarbonyl)amino]phenyl}-2-thiophenecarboxylic acid;
5-(3-Chloro-4-{[(4-fluorophenyl)carbonyl]amino}phenyl)-3-[[(*trans-*4-methylcyclohexyl)carbonyl](1-methylethyl)amino]-2-thiophenecarboxylic acid;
4-[[(*trans-*4-Methylcyclohexyl)carbonyl](1-methylethyl)amino]-5'-[(2-pyridinylcarbonyl)amino]-2,2'-bithiophene-5-carboxylic acid;
3-[[(*trans-*4-Methylcyclohexyl)carbonyl](1-methylethyl)amino]-5-(4-{[(1-methyl-1*H-*pyrazol-4-yl)carbonyl]amino}phenyl)-2-thiophenecarboxylic acid;
3-[[(*trans-*4-Methylcyclexyl)carbonyl](1methylethyl)amino]-5-(4-{[(5-methyl-3-isoxazolyl)carbonyl]amino}phenyl)-2-thiophenecarboxylic acid;
4-[[(*trans-*4-Methylcyclohexyl)carbonyl](1-methylethyl)amino]-5'-[(1,3-thiazol-4-ylcarbonyl)amino]-2,2'-bithiophene-5-carboxylic acid;
3-[[(*trans-*4-Methylcyclohexyl)carbonyl](1-methylethyl)amino]-5-{4-[(1*H-*pyrazol-3-ylcarbonyl)amino]phenyl}-2-thiophenecarboxylic acid;
3-[[(*trans-*4-Methylcyclohexyl)carbonyl](1-methylethyl)amino]-5-(4-{[(5-methyl-1*H-*pyrazol-3-yl)carbonyl]amino}phenyl)-2-thiophenecarboxylic acid;
5-{4-[(1*H-*Imidazol-4-ylcarbonyl)amino]phenyl}-3-[[(*trans-*4-methylcyclohexyl)carbonyl](1-methylethyl)amino]-2-thiophenecarboxylic acid;
5-(4-{[(1,5-Dimethyl-1*H-*pyrazol-3-yl)carbonyl]amino}phenyl)-3-[[(*trans-*4-methylcyclohexyl)carbonyl](1-methylethyl)amino]-2-thiophenecarboxylic acid;
3-[[(*trans-*4-Methylcyclohexyl)carbonyl](1-methylethyl)amino]-5-(4-{[(5-methyl-2-pyrazinyl)carbonyl]amino}phenyl)-2-thiophenecarboxylic acid;
3-[[(*trans-*4-Methylcyclohexyl)carbonyl](1-methylethyl)amino]-5-(4-{[(3-methyl-5-isoxazolyl)carbonyl]amino}phenyl)-2-thiophenecarboxylic acid;
3-[[(*trans-*4-Methylcyclohexyl) carbonyl](1-methylethyl)amino]-5-(4-{[(4-methyl-1,3-thiazol-5-yl)carbonyl]amino}phenyl)-2-thiophenecarboxylic acid;
3-[[(*trans-*4-Methylcyclohexyl)carbonyl](1-methylethyl)amino]-5-(4-{[(3-methyl-2-pyridinyl)carbonyl]amino}phenyl)-2-thiophenecarboxylic acid;
5-{4-[(1*H-*Imidazol-2-ylcarbonyl)amino]phenyl}-3-[[(*trans-*4-methylcyclohexyl)carbonyl](1-methylethyl)amino]-2-thiophenecarboxylic acid;
3-[[(*trans-*4-Methylcyclohexyl)carbonyl](1-methylethyl)amino]-5-(4-{[(6-methyl-2-pyridinyl)carbonyl]amino}phenyl)-2-thiophenecarboxylic acid;
3-[[(*trans-*4-Methylcyclohexyl)carbonyl](1-methylethyl)amino]-5-(4-{[(1-methyl-1*H-*imidazol-4-yl)carbonyl]amino}phenyl)-2-thiophenecarboxylic acid;
3-[[(*trans-*4-Methylcyclohexyl)carbonyl](1-methylethyl)amino]-5-(4-{[(2-methyl-4-pyridinyl)carbonyl]amino}phenyl)-2-thiophenecarboxylic acid;
5-(4-{[(3-Fluorophenyl)carbonyl]amino}phenyl)-3-[[(*trans-*4-methylcyclohexyl)carbonyl](1-methylethyl)amino]-2-thiophenecarboxylic acid;
3-[[(*trans-*4-Methylcyclohexyl)carbonyl](1-methylethyl)amino]-5-(4-{[(1-methyl-1*H-*imidazol-2-yl)carbonyl]amino}phenyl)-2-thiophenecarboxylic acid;
3-[[(*trans-*4-Methylcydohexyl)carbonyl](1-methylethyl)amino]-5-(4-{[(1-methyl-1*H-*pyrazol-3-yl)carbonyl]amino}phenyl)-2-thiophenecarboxylic acid;
5-(4-{[(5-Ethyl-3-isoxazolyl)carbonyl]amino}phenyl)-3-[[(*trans-*4-methylcyclohexyl)carbonyl](1-methylethyl)amino]-2-thiophenecarboxylic acid;
5-{4-[(3-Furanylcarbonyl)amino]phenyl}-3-[[(*trans-*4-methylcyclohexyl)carbonyl](1-methylethyl)amino]-2-thiophenecarboxylic acid;
5-(4-{[(2-Fluorophenyl)carbonyl]amino}phenyl)-3-[[(*trans-*4-methylcyclohexyl)carbonyl](1-methylethyl)amino]-2-thiophenecarboxylic acid;
5-(4-{[(2,6-Difluorophenyl)carbonyl]amino}phenyl)-3-[[(*trans-*4-methylcyclohexyl)carbonyl](1-methylethyl)amino]-2-thiophenecarboxylic acid;
3-[[(*trans-*4-Methylcyclohexyl)carbonyl](1-methylethyl)amino]-5-(4-{[(3-methyl-2-furanyl)carbonyl]amino}phenyl)-2-thiophenecarboxylic acid;
5-(4-{[(2-Amino-1,3-thiazol-4-yl)carbonyl]amino}phenyl)-3-[[(*trans-*4-methylcyclohexyl)carbonyl](1-methylethyl)amino]-2-thiophenecarboxylic acid;
5-(4-{[(3-Fluoro-2-pyridinyl)carbonyl]amino}phenyl)-3-[[(*trans-*4-methylcyclohexyl)carbonyl](1-methylethyl)amino]-2-thiophenecarboxylic acid;
5-(4-{[(1-Ethyl-1*H-*pyrazol-3-yl)carbonyl]amino}phenyl)-3-[[(*trans-*4-methylcyclohexyl)carbonyl](1-methylethyl)amino]-2-thiophenecarboxylic acid;
3-[[(*trans-*4-Methylcyclohexyl)carbonyl](1-methylethyl)amino]-5-(4-{[(5-methyl-2-furanyl)carbonyl]amino}phenyl)-2-thiophenecarboxylic acid;
5-[4-({[5-(Hydroxymethyl)-2-furanyl]carbonyl}amino)phenyl]-3-[[(*trans-*4-methylcyclohexyl)carbonyl](1-methylethyl)amino]-2-thiophenecarboxylic acid;
3-[[(*trans-*4-Methylcyclohexyl)carbonyl](1-methylethyl)amino]-5-(4-{[(4-methyl-3-pyridinyl)carbonyl]amino}phenyl)-2-thiophenecarboxylic acid;
3-[[(*trans-*4-Methylcyclohexyl)carbonyl](1-methylethyl)amino]-5-(4-{[(3-methyl-4-isoxazolyl)carbonyl]amino}phenyl)-2-thiophenecarboxylic acid;
3-[[(*trans-*4-Methylcyclohexyl)carbonyl](1-methylethyl)amino]-5-{6-[(1,3-thiazol-4-ylcarbonyl)amino]-3-pyridinyl}-2-thiophenecarboxylic acid;
3-[[(*trans-*4-Methylcyclohexyl)carbonyl](1-methylethyl)amino]-5-{6-[(2-pyridinylcarbonyl)amino]-3-pyridinyl}-2-thiophenecarboxylic acid;
3-[[(*trans-*4-Methylcyclohexyl)carbonyl](1-methylethyl)amino]-5-{6-[(2-pyrazinylcarbonyl)amino]-3-pyridinyl}-2-thiophenecarboxylic acid;
5-(6-{[(4-Fluorophenyl)carbonyl]amino}-3-pyridinyl)-3-[[(*trans-*4-methylcyclohexyl)carbonyl](1-methylethyl)amino]-2-thiophenecarboxylic acid;
5-{6-[(2-Furanylcarbonyl)amino]-3-pyridinyl}-3-[[(*trans-*4-methylcyclohexyl)carbonyl](1-methylethyl)amino]-2-thiophenecarboxylic acid;
3-{(2,2-Difluoroethyl)[(*trans-*4-methylcyclohexyl)carbonyl]amino}-5-{4-[(1,3-thiazol-4-ylcarbonyl)amino]phenyl}-2-thiophenecarboxylic acid; 3-{Ethyl[(*trans-*4-methylcyclohexyl)carbonyl]amino}-5-{4-[(1,3-thiazol-4-ylcarbonyl)amino]phenyl}-2-thiophenecarboxylic acid; 3-[[(*cis-*4-Fluoro-4-methylcyclohexyl)carbonyl](-methylethyl)amino]-5-{4-[(1,3-thiazol-4-ylcarbonyl)amino]phenyl}-2-thiophenecarboxylic acid; 3-{(Cyclopropylmethyl)[(*trans-*4-methylcyclohexyl)carbonyl]amino}-5-{4-[(2-pyridinylcarbonyl)amino]phenyl}-2-thiophenecarboxylic acid; 3-{(Cyclopropylmethyl)[(*trans-*4-methylcyclohexyl)carbonyl]amino}-5-{4-[(2-pyrazinylcarbonyl)amino]phenyl}-2-thiophenecarboxylic acid; 3-{(Cyclopropylmethyl)[(*trans-*4-methylcyclohexyl)carbonyl]amino}-5-(4-{[(1-methyl-1*H-*imidazol-4-yl)carbonyl]amino}phenyl)-2-thiophenecarboxylic acid; 3-{(Cyclopropylmethyl)[(*trans-*4-methylcyclohexyl)carbonyl]amino}-5-{4-[(3-pyridazinylcarbonyl)amino]phenyl}-2-thiophenecarboxylic acid; 3-{(Cyclopropylmethyl)[(*trans-*4-methylcyclohexyl)carbonyl]amino}-5-(4-{[(1-methyl-1*H-*pyrazol-3-yl)carbonyl]amino}phenyl)-2-thiophenecarboxylic acid;
3-{(Cyclopropyl methyl)[(*trans*-4-methylcyclohexyl)carbonyl]amino}-5-(4-{[(1,5-dimethyl-1*H-*pyrazol-3-yl)carbonyl]amino}phenyl)-2-thiophenecarboxylic acid; 3-[[(*trans-*4-Methylcyclohexyl)carbonyl](1-methylethyl)amino]-5-[4-[(1,3-thiazol-4-ylcarbonyl)amino]-3-(trifluoromethyl)phenyl]-2-thiophenecarboxylic acid; 3-[[(*trans-*4-Methylcyclohexyl)carbonyl](1-methylethyl)amino]-5-{3-(methyloxy)-4-[(1,3-thiazol-4-ylcarbonyl)amino]phenyl}-2-thiophenecarboxylic acid; 3-{1,3-Dioxan-5-yl[(*trans-*4-methylcyclohexyl)carbonyl]amino}-5-{4-[(1,3-thiazol-4-ylcarbonyl)amino]phenyl}-2-thiophenecarboxylic acid; 3-{Cyclobutyl[(*trans-*4-methylcyclohexyl)carbonyl]amino}-5-{4-[(1,3-thiazol-4-ylcarbonyl)amino]phenyl}-2-thiophenecarboxylic acid; 3-{[(*trans-*4-Methylcyclohexyl)carbonyl][1-methyl-2-(methyloxy)ethyl]amino}-5-{4-[(1,3-thiazol-4-ylcarbonyl)amino]phenyl}-2-thiophenecarboxylic acid; 3-([1-Methyl-2-(methyloxy)ethyl]{[*trans-*4-(trifluoromethyl)cyclohexyl]carbonyl}amino)-5-{4-[(1,3-thiazol-4-ylcarbonyl)amino]phenyl}-2-thiophenecarboxylic acid; 3-[[(*trans-*4-Methylcyclohexyl)carbonyl](1-methylethyl)amino]-5-{5-methyl-6-[(1,3-thiazol-4-ylcarbonyl)amino]-3-pyridinyl}-2-thiophenecarboxylic acid; 5-(6-{[(4-Fluorophenyl)carbonyl]amino}-5-methyl-3-pyridinyl)-3-[[(*trans-*4-methylcyclohexyl)carbonyl](1-methylethyl)amino]-2-thiophenecarboxylic acid; 3-{Cyclobutyl[(*trans-*4-methylcyclohexyl)carbonyl]amino}-5-{4-[(1*H-*pyrazol-3-ylcarbonyl)amino]phenyl}-2-thiophenecarboxylic acid; 3-{Ethyl[(*trans-*4-methylcyclohexyl)carbonyl]amino}-5-{4-[(1*H-*pyrazol-3-ylcarbonyl)amino]phenyl}-2-thiophenecarboxylic acid; 3-{[(*trans-*4-Methylcyclohexyl)carbonyl][2-(methyloxy)ethyl]amino}-5-{4-[(1,3-thiazol-4-ylcarbonyl)amino]phenyl}-2-thiophenecarboxylic acid; 3-{[(*trans-*4-Methylcyclohexyl)carbonyl][1-methyl-2-(methyloxy)ethyl]amino}-5-(4-{[(1-methyl-1*H-*imidazol-4yl)carbonyl]amino}phenyl)-2-thiophenecarboxylic acid;
3-{[(*trans-*4-Methylcyclohexyl)carbonyl][2-(methyloxy)ethyl]amino}-5-(4-{[(1-methyl-1*H-*imidazol-4-yl)carbonyl]amino}phenyl)-2-thiophenecarboxylic acid; 3-[[(*trans-*4-Methylcyclohexyl)carbonyl](2,2,2-trifluoroethyl)amino]-5-{4-[(1,3-thiazol-4-ylcarbonyl)amino]phenyl}-2-thiophenecarboxylic acid; 3-{(Cyclopropylmethyl)[(*trans-*4-methylcyclohexyl)carbonyl]amino}-5-{4-[(1*H-*pyrazol-3-ylcarbonyl)amino]phenyl}-2-thiophenecarboxylic acid; 3-{Cyclobutyl[(*trans-*4-methylcyclohexyl)carbonyl]amino}-5-(4-{[(1-methyl-1*H-*imidazol-4-yl)carbonyl]amino}phenyl)-2-thiophenecarboxylic acid; 3-{Ethyl[(*trans-*4-methylcyclohexyl)carbonyl]amino}-5-(4-{[(1-methyl-1*H-*imidazol-4-yl)carbonyl]amino}phenyl)-2-thiophenecarboxylic acid; 3-[[(*trans-*4-Methylcyclohexyl)carbonyl](1-methylethyl)amino]-5-{4-methyl-5-[(1,3-thiazol-4-ylcarbonyl)amino]-2-pyridinyl}-2-thiophenecarboxylic acid; 3-{Cyclobutyl[(*trans-*4-methylcyclohexyl)carbonyl]amino}-5-{4-[(3-pyridazinylcarbonyl)amino]phenyl}-2-thiophenecarboxylic acid; 5-{5-Chloro-6-[(1,3-thiazol-4-ylcarbonyl)amino]-3-pyridinyl}-3-[[(*trans-*4-methylcyclohexyl)carbonyl](1-methylethyl)amino]-2-thiophenecarboxylic acid; 5-(4-{[(3,5-Dimethyl-4-isoxazolyl)carbonyl]amino}phenyl)-3-[[(*trans-*4-methylcyclohexyl)carbonyl](1-methylethyl)amino]-2-thiophenecarboxylic acid; 5-(4-{[(1-Ethyl-1*H-*pyrazol-5-yl)carbonyl]amino}phenyl)-3-[[(*trans-*4-methylcyclohexyl)carbonyl](1-methylethyl)amino]-2-thiophenecarboxylic acid; 3-[[(*trans-*4-Methylcyclohexyl)carbonyl](1-methylethyl)amino]-5-(4-{[(1-methyl-1*H-*imidazol-5-yl)carbonyl]amino}phenyl)-2-thiophenecarboxylic acid; 3-[[(*trans-*4-Methylcyclohexyl)carbonyl](1-methylethyl)amino]-5-(4-{[(3-methyl-4-pyridinyl)carbonyl]amino}phenyl)-2-thiophenecarboxylic acid; 3-[[(*trans-*4-Methylcyclohexyl)carbonyl](1-methylethyl)amino]-5-{4-[(1,3-thiazol-5-ylcarbonyl)amino]phenyl}-2-thiophenecarboxylic acid; 3-[[(*trans-*4-Methylcyclohexyl)carbonyl](1-methylethyl)amino]-5-(4-{[(1-methyl-1*H-*pyrazol-5-yl)carbonyl]amino}phenyl)-2-thiophenecarboxylic acid; 5-(4-{[(2,4-Dimethyl-1,3-oxazol-5-yl)carbonyl]amino}phenyl)-3-[[(*trans-*4-methylcyclohexyl)carbonyl](1-methylethyl)amino]-2-thiophenecarboxylic acid; 5-(4-{[(1,3-Dimethyl-1*H-*pyrazol-5-yl)carbonyl]amino}phenyl)-3-[[(*trans-*4-methylcyclohexyl)carbonyl](1-methylethyl)amino]-2-thiophenecarboxylic acid; 3-[[(*trans-*4-Methylcyclohexyl)carbonyl](1-methylethyl)amino]-5-(4-{[(4-methyl-1,3-oxazol-5-yl)carbonyl]amino}phenyl)-2-thiophenecarboxylic acid;
5-(4-{[(1,5-Dimethyl-1*H-*pyrazol-4-yl)carbonyl]aminolphenyl)-3-[[(*trans-*4-methylcyclohexyl)carbonyl](1-methylethyl)amino]-2-thiophenecarboxylic acid; 3-[[(*trans-*4-Methylcyclohexyl)carbonyl](1-methylethyl)amino]-5-(4-{[(2-methyl-3-pyridinyl)carbonyl]amino}phenyl)-2-thiophenecarboxylic acid; 3-[[(*trans-*4-Methylcyclohexyl)carbonyl](1-methylethyl)amino]-5-(4-{[(6-methyl-3-pyridinyl)carbonyl]amino}phenyl)-2-thiophenecarboxylic acid; 3-[[(*trans-*4-Methylcyclohexyl)carbonyl](1-methylethyl)amino]-5-(4-[(1*H-*pyrazol-4-ylcarbonyl)amino]phenyl}-2-thiophenecarboxylic acid;
5'-[(2-Furanylcarbonyl)amino]-4-[[(*trans-*4-methylcyclohexyl)carbonyl](1-methylethyl)amino]-2,2'-bithiophene-5-carboxylic acid;
3-[[(*trans-*4-Methylcyclohexyl)carbonyl](1-methylethyl)amino]-5-(4-{[(5-methyl-4-isoxazolyl)carbonyl]amino}phenyl)-2-thiophenecarboxylic acid;
5-(4-{[(1-Ethyl-1*H-*pyrazol-4-yl)carbonyl]amino}phenyl)-3-[[(*trans-*4-methylcyclohexyl)carbonyl](1-methylethyl)amino]-2-thiophenecarboxylic acid;
3-[[(*trans-*4-Methylcyclohexyl)carbonyl](1-methylethyl)amino]-5-(4-{[(5-methyl-3-pyridinyl)carbonyl]amino}phenyl)-2-thiophenecarboxylic acid;
5'-{[(4-Fluorophenyl)carbonyl]amino}-4-[[(*trans-*4-methylcyclohexyl)carbonyl](1-methylethyl)amino]-2,2'-bithiophene-5-carboxylic acid;
3-[[(*trans-*4-Methylcyclohexyl)carbonyl](1-methylethyl)amino]-5-{5-[(1,3-thiazol-4-ylcarbonyl)amino]-2-pyridinyl}-2-thiophenecarboxylic acid;
5-(4-{[(1,3-Dimethyl-1*H-*pyrazol-4-yl)carbonyl]amino}phenyl)-3-[[(*trans-*4-methylcyclohexyl)carbonyl](1-methylethyl)amino]-2-thiophenecarboxylic acid;
5-(4-{[(1-Ethyl-1*H-*imidazol-5-yl)carbonyl]amino}phenyl)-3-[[(*trans-*4-methylcyclohexyl)carbonyl](1-methylethyl)amino]-2-thiophenecarboxylic acid;
3-[[(*trans-*4-Methylcyclohexyl)carbonyl](1-methylethyl)amino]-5-(4-{[(3-methyl-1*H-*pyrazol-4-yl)carbonyl]amino}phenyl)-2-thiophenecarboxylic acid;
and salts thereof.

8. A compound as claimed in claim 1 selected from the group consisting of:
3-[[(*trans-*4-Methylcyclohexyl)carbonyl](1-methylethyl)amino]-5-{4-[(1,3-thiazol-4-ylcarbonyl)amino]phenyl}-2-thiophenecarboxylic acid;
3-{(Cyclopropylmethyl)[(*trans-*4-methylcyclohexyl)carbonyl]amino}-5-{4-[(1,3-thiazol-4-ylcarbonyl)amino]phenyl}-2-thiophenecarboxylic acid;
3-[[(*trans-*4-Methylcyclohexyl)carbonyl](1-methylethyl)amino]-5-{4-[(3-pyridazinylcarbonyl)amino]phenyl}-2-thiophenecarboxylic acid;
3-[[(*trans-*4-Methylcyclohexyl)carbonyl](1-methylethyl)amino]-5-{4-[(1*H-*pyrazol-3-ylcarbonyl)amino]phenyl}-2-thiophenecarboxylic acid;
3-[[(*trans-*4-Methylcyclohexyl)carbonyl](1-methylethyl)amino]-5-(4-{[(1-methyl-1*H-*imidazol-4-yl)carbonyl]amino}phenyl)-2-thiophenecarboxylic acid;
3-{ethyl[(*trans-*4-Methylcyclohexyl)carbonyl]amino}-5-{4-[(1,3-thiazol-4-ylcarbonyl)amino]phenyl}-2-thiophenecarboxylic acid;
and salts thereof.

9. A compound as claimed in claim 1 selected from the group consisting of:
3-[[(*trans-*4-Methylcyclohexyl)carbonyl](1-methylethyl)amino]-5-{4-[(1,3-thiazol-4-ylcarbonyl)amino]phenyl}-2-thiophenecarboxylic acid
and salts thereof.

10. A compound as claimed in claim 9 selected from the group consisting of:
Sodium 3-[[(*trans-*4-methylcyclohexyl)carbonyl](1-methylethyl)amino]-5-{4-[(1,3-thiazol-4-ylcarbonyl)amino]phenyl}-2-thiophenecarboxylate;
Lysine 3-[[(*trans-*4-methylcyclohexyl)carbonyl](1-methylethyl)amino]-5-{4-[(1,3-thiazol-4-ylcarbonyl)amino]phenyl}-2-thiophenecarboxylate;
Ammonium 3-[[(*trans-*4-methylcyclohexyl)carbonyl](1-methylethyl)amino]-5-{4-[(1,3-thiazol-4-ylcarbonyl)amino]phenyl}-2-thiophenecarboxylate;
N-Methyl-D-glucamine 3-[[(*trans-*4-methylcyclohexyl)carbonyl](1-methylethyl)amino]-5-{4-[(1,3-thiazol-4-ylcarbonyl)amino]phenyl}-2-thiophenecarboxylate;
Potassium 3-[[(*trans-*4-methylcyclohexyl)carbonyl](1-methylethyl)amino]-5-{4-[(1,3-thiazol-4-ylcarbonyl)amino]phenyl}-2-thiophenecarboxylate;
Choline 3-[[(*trans-*4-methylcyclohexyl)carbonyl](1-methylethyl)amino]-5-{4-[(1,3-thiazol-4-ylcarbonyl)amino]phenyl}-2-thiophenecarboxylate; and
L-Arginine 3-[[(*trans-*4-methylcyclohexyl)carbonyl](1-methylethyl)amino]-5-{4-[(1,3-thiazol-4-ylcarbonyl)amino]phenyl}-2-thiophenecarboxylate.

11. A compound of Formula (I) as defined in any of claims 1 to 10 or pharmaceutically acceptable salts thereof, for use in medical therapy.

12. A compound of Formula (I) as claimed in claim 11 wherein the medical therapy is the treatment of viral infection.

13. A pharmaceutical formulation comprising a compound of Formula (I) as defined in any of claims 1 to 10 or pharmaceutically acceptable salts thereof in conjunction with at least one pharmaceutically acceptable diluent or carrier.

14. A process for the preparation of a compound of Formula (I) as defined in any of claims 1 to 10, comprising deprotection of a compound of Formula (II) 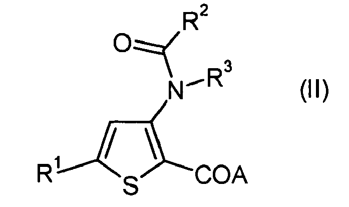 in which A is an alkoxy, benzyloxy or silyloxy group, and R¹, R² and R³ are as defined in claim 1.

15. A combination comprising a compound of Formula (I) as defined in any of claims 1 to 10 or a pharmaceutically acceptable salt thereof, together with at least one other therapeutically active agent.

16. A combination as claimed in Claim 15, wherein the other therapeutically active agent is selected from Interferon, ribavirin and/or an additional anti-HCV agent.

## Patentansprüche

1. Eine Verbindung der Formel (I): 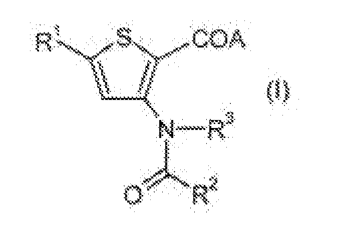 wobei:
A Hydroxy bedeutet;
R¹ -R^{X}-NHC(O)-R^{Y} bedeutet;
R^{X} Phenyl (gegebenenfalls substituiert mit einem oder mehreren Substituenten, unabhängig ausgewählt aus Halogen, Methyl, Ethyl, Methoxy und Trifluormethyl), 5- oder 6-gliedriges Heteroaryl (gegebenenfalls substituiert mit einem oder mehreren Substituenten, unabhängig ausgewählt aus Halogen, Methyl, Ethyl, Methoxy und Trifluormethyl), die über ein Kohlenstoffatom an das Thiophen gebunden sind, bedeutet; wobei sich der Thiophen- und der Amidrest in para-Stellung zueinander befinden;
R^{Y} Phenyl (gegebenenfalls substituiert mit einem oder mehreren Substituenten, unabhängig ausgewählt aus Halogen, Methyl, Ethyl, Methoxy, Trifluormethyl, Hydroxy und Amino) oder 5- oder 6-gliedriges Heteroaryl bedeutet;
R² C₅₋₇Cycloalkyl (gegebenenfalls substituiert mit einem oder mehreren Substituenten, unabhängig ausgewählt aus -C₁₋₆-Alkyl [gegebenenfalls substituiert mit einem oder mehreren Substituenten, unabhängig ausgewählt aus Fluor und -OR^{A}], Halogen und -OR^{A}) bedeutet;
R^{A} Wasserstoff oder-C₁₋₄-Alkyl bedeutet; und
R³ C₁₋₆-Alkyl (gegebenenfalls substituiert mit einem oder mehreren Substituenten, unabhängig ausgewählt aus Fluor, Hydroxyl, Methoxy, Ethoxy, C₃₋₆-Cycloalkyl und einem 5- oder 6-gliedrigen Heteroaryl und Heterocyclyl), Tetrahydrofuranyl, Pyranyl, C₃₋₇-Cycloalkyl oder Dioxanyl bedeutet;
oder Salze davon.

2. Eine Verbindung wie in Anspruch 1 beansprucht, wobei R^{X} unsubstituiertes Phenyl, 2-Chlorphenyl, 3-(Trifluormethyl)phenyl, 3-Methoxyphenyl, 2-Pyridinyl, 3-Pyridinyl, 5-Methyl-3-pyridinyl, 4-Methyl-2-pyridinyl oder 5-Chlor-3-pyridinyl, wobei der Pyridinylring in allen Fällen über ein Kohlenstoffatom an das Thiophen gebunden ist, wobei sich der Thiophen- und der Amidrest in para-Stellung zueinander befinden; oder unsubstituiertes Thienyl, das an den Positionen 2 und 5 an der Amidrest und das Thiophen gebunden ist, bedeutet.

3. Eine Verbindung wie in Anspruch 1 oder Anspruch 2 beansprucht, wobei R^{Y} Phenyl (gegebenenfalls substituiert mit einem oder mehreren Substituenten, ausgewählt aus Halogen) oder 5- oder 6-gliedriges Heteroaryl bedeutet.

4. Eine Verbindung wie in einem der Ansprüche 1 bis 3 beansprucht, wobei R^{Y} Fluorphenyl, unsubstituiertes Pyridinyl, Methylpyridinyl, Fluorpyridinyl, unsubstituiertes Pyrazinyl, Methylpyrazinyl, unsubstituiertes Pyridazinyl, unsubstituiertes Pyrimidinyl, unsubstituiertes Furanyl, Methylfuranyl, Hydroxymethylfuranyl, unsubstituiertes Oxazolyl, Methyloxazolyl, unsubstituiertes Isoxazolyl, Methylisoxazolyl, Ethylisoxazolyl, unsubstituiertes Thienyl, unsubstituiertes Thiazolyl, unsubstituiertes Isothiazolyl, Methylthiazolyl, Aminothiazolyl, Pyrazolyl, Methylpyrazolyl, Ethylpyrazolyl, unsubstituiertes Imidazolyl, Methylimidazolyl oder Ethylimidazolyl bedeutet.

5. Eine Verbindung wie in einem der Ansprüche 1 bis 4 beansprucht, wobei R² unsubstituiertes Cyclohexyl oder Cyclohexyl, substituiert mit einem oder mehreren Substituenten, unabhängig ausgewählt aus Methyl, Trifluormethyl und Fluor, bedeutet.

6. Eine Verbindung wie in einem der Ansprüche 1 bis 5 beansprucht, wobei R³ C₁₋₆-Alkyl (gegebenenfalls substituiert mit einem oder mehreren Substituenten, unabhängig ausgewählt aus Fluor, Methoxy und C₃₋₆-Cycloalkyl), C₃₋₇-Cycloalkyl oder Dioxanyl bedeutet.

7. Eine Verbindung wie in Anspruch 1 beansprucht, ausgewählt aus der Gruppe bestehend aus:
3-[[(trans-4-Methylcyclohexyl)carbonyl](1-methylethyl)amino]-5-{4-[(1,3-thiazol-4-ylcarbonyl)amino]phenyl}-2-thiophencarbonsäure;
3-[[(trans-4-Methylcyclohexyl)carbonyl](1-methylethyl)amino]-5-{4-[(2-pyridinylcarbonyl)amino]phenyl}-2-thiophencarbonsäure;
5-{4-[(2-Furanylcarbonyl)amino]phenyl}-3-[[(trans-4-methylcyclohexyl)carbonyl](1-methylethyl)amino]-2-thiophencarbonsäure;
3-[[(trans-4-Methylcyclohexyl)carbonyl](1-methylethyl)amino]-5-(4-{[(2-methyl-1,3-thiazol-4-yl)carbonyl]amino}phenyl)-2-thiophencarbonsäure;
3-[[(trans-4-Methylcyclohexyl)carbonyl](1-methylethyl)amino]-5-{4-[(2-thienylcarbonyl)amino]phenyl}-2-thiophencarbonsäure;
3-[[(trans-4-Methylcyclohexyl)carbonyl](1-methylethyl)amino]-5-{4-[(2-pyrazinylcarbonyl)amino]phenyl}-2-thiophencarbonsäure;
5-(4-{[(4-Fluorphenyl)carbonyl]amino}phenyl)-3-[[(trans-4-methylcyclohexyl)carbonyl](1-methylethyl)amino]-2-thiophencarbonsäure;
3-[[(trans-4-Methylcyclohexyl)carbonyl](1-methylethyl)amino]-5-{4-[(1,3-oxazol-2-ylcarbonyl)amino]phenyl}-2-thiophencarbonsäure;
3-{(Cyclopropylmethyl)[(trans-4-methylcyclohexyl)carbonyl]amino}-5-{4-[(1,3-thiazol-4-ylcarbonyl)amino]phenyl}-2-thiophencarbonsäure;
3-((Cyclopropylmethyl){[trans-4-(trifluormethyl)cyclohexyl]carbonyl}amino)-5-{4-[(1,3-thiazol-4-ylcarbonyl)amino]phenyl}-2-thiophencarbonsäure;
3-((1-Methylethyl){[trans-4-(trifluormethyl)cyclohexyl]carbonyl}amino)-5-{4-[(1,3-thiazol-4-ylcarbonyl)amino]phenyl}-2-thiophencarbonsäure;
3-[[(trans-4-Methylcyclohexyl)carbonyl](1-methylethyl)amino]-5-{4-[(3-pyridazinylcarbonyl)amino]phenyl}-2-thiophencarbonsäure;
3-[[(trans-4-Methylcyclohexyl)carbonyl](1-methylethyl)amino]-5-{4-[(4-pyrimidinylcarbonyl)amino]phenyl}-2-thiophencarbonsäure;
5-{3-Chlor-4-[(1,3-thiazol-4-ylcarbonyl)amino]phenyl}-3-[[(trans-4-methylcyclohexyl)carbonyl](1-methylethyl)amino]-2-thiophencarbonsäure;
3-[[(trans-4-Methylcyclohexyl)carbonyl](1-methylethyl)amino]-5-{4-[(3-thienylcarbonyl)amino]phenyl}-2-thiophencarbonsäure;
5-(3-Chlor-4-{[(4-fluorphenyl)carbonyl]amino}phenyl)-3-[[(trans-4-methylcyclohexyl)carbonyl](1-methylethyl)amino]-2-thiophencarbonsäure;
4-[[(trans-4-Methylcyclohexyl)carbonyl](1-methylethyl)amino]-5'-[(2-pyridinylcarbonyl)amino]-2,2'-bithiophen-5-carbonsäure;
3-[[(trans-4-Methylcyclohexyl)carbonyl](1-methylethyl)amino]-5-(4-{[(1-methyl-1H-pyrazol-4-yl)carbonyl]amino}phenyl)-2-thiophencarbonsäure;
3-[[(trans-4-Methylcyclohexyl)carbonyl](1-methylethyl)amino]-5-(4-{[(5-methyl-3-isoxazolyl)carbonyl]amino}phenyl)-2-thiophencarbonsäure;
4-[[(trans-4-Methylcyclohexyl)carbonyl](1-methylethyl)amino]-5'-[(1,3-thiazol-4-ylcarbonyl)amino]-2,2'-bithiophen-5-carbonsäure;
3-[[(trans-4-Methylcyclohexyl)carbonyl](1-methylethyl)amino]-5-{4-[(1H-pyrazol-3-ylcarbonyl)amino]phenyl}-2-thiophencarbonsäure;
3-[[(trans-4-Methylcyclohexyl)carbonyl](1-methylethyl)amino]-5-(4-{[(5-methyl-1H-pyrazol-3-yl)carbonyl]amino}phenyl)-2-thiophencarbonsäure;
5-{4-[(1H-Imidazol-4-ylcarbonyl)amino]phenyl}-3-[[(trans-4-methylcyclohexyl)carbonyl](1-methylethyl)amino]-2-thiophencarbonsäure;
5-(4-{[(1,5-Dimethyl-1H-pyrazol-3-yl)carbonyl]amino}phenyl)-3-[[(trans-4-methylcyclohexyl)carbonyl](1-methylethyl)amino]-2-thiophencarbonsäure;
3-[[(trans-4-Methylcyclohexyl)carbonyl](1-methylethyl)amino]-5-(4-{[(5-methyl-2-pyrazinyl)carbonyl]amino}phenyl)-2-thiophencarbonsäure;
3-[[(trans-4-Methylcyclohexyl)carbonyl](1methylethyl)amino]5-(4-{[(3-methyl-5-isoxazolyl)carbonyl]amino}phenyl)-2-thiophencarbonsäure;
3-[[(trans-4-Methylcyclohexyl)carbonyl](1-methylethyl)amino]-5-(4-{[(4-methyl-1,3-thiazol-5-yl)carbonyl]amino}phenyl)-2-thiophencarbonsäure;
3-[[(trans-4-Methylcyclohexyl)carbonyl](1-methylethyl)amino]-5-(4-{[(3-methyl-2-pyridinyl)carbonyl]amino}phenyl)-2-thiophencarbonsäure;
5-{4-[(1-Imidazol-2-ylcarbonyl)amino]phenyl}-3-[[(trans-4-methylcyclohexyl)carbonyl](1-methylethyl)amino]-2-thiophencarbonsäure;
3-[[(trans-4-Methylcyclohexyl)carbonyl](1-methylethyl)amino]-5-(4-{[(6-methyl-2-pyridinyl)carbonyl]amino}phenyl)-2-thiophencarbonsäure;
3-[[(trans-4-Methylcyclohexyl)carbonyl](1-methylethyl)amino]-5-(4-{[(1-methyl-1H-imidazol-4-yl)carbonyl]amino}phenyl)-2-thiophencarbonsäure;
3-[[(trans-4-Methylcyclohexyl)carbonyl](1-methylethyl)amino]-5-(4-{[(2-methyl-4-pyridinyl)carbonyl]amino}phenyl)-2-thiophencarbonsäure;
5-(4-{[(3-Fluorphenyl)carbonyl]amino}phenyl)-3-[[(trans-4-methylcyclohexyl)-carbonyl](1-methylethyl)amino]-2-thiophencarbonsäure;
3-[[(trans-4-Methylcyclohexyl)carbonyl](1-methylethyl)amino]-5-(4-{[(1-methyl-1H-imidazol-2-yl)carbonyl]amino}phenyl)-2-thiophencarbonsäure;
3-[[(trans-4-Methylcyclohexyl)carbonyl](1-methylethyl)amino]-5-(4-{[(1-methyl-1H-pyrazol-3-yl)carbonyl]amino}phenyl)-2-thiophencarbonsäure;
5-(4-{[(5-Ethyl-3-isoxazolyl)carbonyl]amino}phenyl)-3-[[(trans-4-methylcyclohexyl)carbonyl](1-methylethyl)amino]-2-thiophencarbonsäure;
5-{4-[(3-Furanylcarbonyl)amino]phenyl}-3-[[(trans-4-methylcyclohexyl)carbonyl](1-methylethyl)amino]-2-thiophencarbonsäure;
5-(4-{[(2-Fluorphenyl)carbonyl]amino}phenyl)-3-[[(trans-4-methylcyclohexyl)carbonyl](1-methylethyl)amino]-2-thiophencarbonsäure;
5-(4-{[(2,6-Difluorphenyl)carbonyl]amino}phenyl)-3-[[(trans-4-methylcyclohexyl)-carbonyl](1-methylethyl)amino]-2-thiophencarbonsäure;
3-[[(trans-4-Methylcyclohexyl)carbonyl](1-methylethyl)amino]-5-(4-{[(3-methyl-2-furanyl)carbonyl]amino}phenyl)-2-thiophencarbonsäure;
5-(4-{[(2-Amino-1,3-thiazol-4-yl)carbonyl]amino}phenyl)-3-[[(trans-4-methylcyclohexyl)carbonyl](1-methylethyl)amino]-2-thiophencarbonsäure;
5-(4-{[(3-Fluor-2-pyridinyl)carbonyl]amino}phenyl)-3-[[(trans-4-methylcyclohexyl)carbonyl](1-methylethyl)amino]-2-thiophencarbonsäure;
5-(4-{[(1-Ethyl-1H-pyrazol-3-yl)carbonyl]amino}phenyl)-3-[[(trans-4-methylcyclohexyl)carbonyl](1-methylethyl)amino]-2-thiophencarbonsäure;
3-[[(trans-4-Methylcyclohexyl)carbonyl](1-methylethyl)amino]-5-(4-{[(5-methyl-2-furanyl)carbonyl]amino}phenyl)-2-thiophencarbonsäure;
5-[4-({[5-(Hydroxymethyl)-2-furanyl]carbonyl}amino)phenyl]-3-[[(trans-4-methylcyclohexyl)carbonyl](1-methylethyl)amino]-2-thiophencarbonsäure;
3-[[(trans-4-Methylcyclohexyl)carbonyl](1-methylethyl)amino]-5-(4-{[(4-methyl-3-pyridinyl)carbonyl]amino}phenyl)-2-thiophencarbonsäure;
3-[[(trans-4-Methylcyclohexyl)carbonyl](1-methylethyl)amino]-5-(4-{[(3-methyl-4-isoxazolyl)carbonyl]amino}phenyl)-2-thiophencarbonsäure;
3-[[(trans-4-Methylcyclohexyl)carbonyl](1-methylethyl)amino]-5-{6-[(1,3-thiazol-4-ylcarbonyl)amino]-3-pyridinyl}-2-thiophencarbonsäure;
3-[[(trans-4-Methylcyclohexyl)carbonyl](1-methylethyl)amino]-5-{6-[(2-pyridinylcarbonyl)amino]-3-pyridinyl}-2-thiophencarbonsäure;
3-[[(trans-4-Methylcyclohexyl)carbonyl](1-methylethyl)amino]-5-{6-[(2-pyrazinylcarbonyl)amino]-3-pyridinyl}-2-thiophencarbonsäure;
5-(6-{[(4-Fluorphenyl)carbonyl]amino}-3-pyridinyl)-3-[[(trans-4-methylcyclohexyl)carbonyl](1-methylethyl)amino]-2-thiophencarbonsäure;
5-{6-[(2-Furanylcarbonyl)amino]-3-pyridinyl}-3-[[(trans-4-methylcyclohexyl)-carbonyl](1-methylethyl)amino]-2-thiophencarbonsäure;
3-{(2,2-Difluorethyl)[(trans-4-methylcyclohexyl)carbonyl]amino}-5-{4-[(1,3-thiazol-4-ylcarbonyl)amino]phenyl}-2-thiophencarbonsäure;
3-{Ethyl[(trans-4-methylcyclohexyl)carbonyl]amino}-5-{4-[(1,3-thiazol-4-ylcarbonyl)amino]phenyl}-2-thiophencarbonsäure;
3-[[(cis-4-Fluor-4-methylcyclohexyl)carbonyl](1-methylethyl)amino]-5-{4-[(1,3-thiazol-4-ylcarbonyl)amino]phenyl}-2-thiophencarbonsäure;
3-{(Cyclopropylmethyl)[(trans-4-methylcyclohexyl)carbonyl]amino}-5-{4-[(2-pyridinylcarbonyl)amino]phenyl}-2-thiophencarbonsäure;
3-{(Cyclopropylmethyl)[(trans-4-methylcyclohexyl)carbonyl]amino}-5-{4-[(2-pyrazinylcarbonyl)amino]phenyl}-2-thiophencarbonsäure;
3-{(Cyclopropylmethyl)[(trans-4-methylcyclohexyl)carbonyl]amino}-5-(4-{[(1-methyl-1H-imidazol-4-yl)carbonyl]amino}phenyl)-2-thiophencarbonsäure;
3-{(Cyclopropylmethyl)[(trans-4-methylcyclohexyl)carbonyl]amino}-5-{4-[(3-pyridazinylcarbonyl)amino]phenyl}-2-thiophencarbonsäure;
3-{(Cyclopropylmethyl)[(trans-4-methylcyclohexyl)carbonyl]amino}-5-(4-{[(1-methyl-1H-pyrazol-3-yl)carbonyl]amino}phenyl)-2-thiophencarbonsäure;
3-{(Cyclopropylmethyl)[(trans-4-methylcyclohexyl)carbonyl]amino}-5-(4-{[(1,5-dimethyl-1H-pyrazol-3-yl)carbonyl]amino}phenyl)-2-thiophencarbonsäure;
3-[[(trans-4-Methylcyclohexyl)carbonyl](1-methylethyl)amino]-5-[4-[(1,3-thiazol-4-ylcarbonyl)amino]-3-(trifluormethyl)phenyl]-2-thiophencarbonsäure;
3-[[(trans-4-Methylcyclohexyl)carbonyl](1-methylethyl)amino]-5-{3-(methyloxy)-4-[(1,3-thiazol-4-ylcarbonyl)amino]phenyl}-2-thiophencarbonsäure;
3-{1,3-Dioxan-5-yl[(trans-4-methylcyclohexyl)carbonyl]amino}-5-{4-[(1,3-thiazol-4-ylcarbonyl)amino]phenyl}-2-thiophencarbonsäure;
3-{Cyclobutyl[(trans-4-methylcyclohexyl)carbonyl]amino}-5-{4-[(1,3-thiazol-4-ylcarbonyl)amino]phenyl}-2-thiophencarbonsäure;
3-{[(trans-4-Methylcyclohexyl)carbonyl][1-methyl-2-(methyloxy)ethyl]amino}-5-{4-[(1,3-thiazol-4-ylcarbonyl)amino]phenyl}-2-thiophencarbonsäure;
3-([1-Methyl-2-(methyloxy)ethyl]{[trans-4-(trifluormethyl)cyclohexyl]carbonyl}amino)-5-{4-[(1,3-thiazol-4-ylcarbonyl)amino]phenyl}-2-thiophencarbonsäure;
3-[[(trans-4-Methylcyclohexyl)carbonyl](1-methylethyl)amino]-5-{5-methyl-6-[(1,3-thiazol-4-ylcarbonyl)amino]-3-pyridinyl}-2-thiophencarbonsäure;
5-(6-{[(4-Fluorphenyl)carbonyl]amino}-5-methyl-3-pyridinyl)-3-[[(trans-4-methylcyclohexyl)carbonyl](1-methylethyl)amino]-2-thiophencarbonsäure;
3-{Cyclobutyl[(trans-4-methylcyclohexyl)carbonyl]amino}-5-{4-[(1H-pyrazol-3-ylcarbonyl)amino]phenyl}-2-thiophencarbonsäure;
3-{Ethyl[(trans-4-methylcyclohexyl)carbonyl]amino}-5-{4-[(1H-pyrazol-3-ylcarbonyl)amino]phenyl}-2-thiophencarbonsäure;
3-{[(trans-4-Methylcyclohexyl)carbonyl][2-(methyloxy)ethyl]amino}-5-{4-[(1,3-thiazol-4-ylcarbonyl)amino]phenyl}-2-thiophencarbonsäure;
3-{[(trans-4-Methylcyclohexyl)carbonyl][1-methyl-2-(methyloxy)ethyl]amino}-5-(4-{[(1-methyl-1H-imidazol-4-yl)carbonyl]amino}phenyl)-2-thiophencarbonsäure;
3-{[(trans-4-Methylcyclohexyl)carbonyl][2-(methyloxy)ethyl]amino}-5-(4-{[(1-methyl-1H-imidazol-4-yl)carbonyl]amino}phenyl)-2-thiophencarbonsäure;
3-[[(trans-4-Methylcyclohexyl)carbonyl](2,2,2-trifluorethyl)amino]-5-{4-[(1,3-thiazol-4-ylcarbonyl)amino]phenyl}-2-thiophencarbonsäure;
3-{(Cyclopropylmethyl)[(trans-4-methylcyclohexyl)carbonyl]amino}-5-{4-[(1H-pyrazol-3-ylcarbonyl)amino]phenyl}-2-thiophencarbonsäure;
3-{Cyclobutyl[(trans-4-methylcyclohexyl)carbonyl]amino}-5-(4-{[(1-methyl-1H-imidazol-4-yl)carbonyl]amino}phenyl)-2-thiophencarbonsäure;
3-{Ethyl[(trans-4-methylcyclohexyl)carbonyl]amino}-5-(4-{[(1-methyl-1H-imidazol-4-yl)carbonyl]amino}phenyl)-2-thiophencarbonsäure;
3-[[(trans-4-Methylcyclohexyl)carbonyl](1-methylethyl)amino]-5-{4-methyl-5-[(1,3-thiazol-4-ylcarbonyl)amino]-2-pyridinyl}-2-thiophencarbonsäure;
3-{Cyclobutyl[(trans-4-methylcyclohexyl)carbonyl]amino}-5-{4-[(3-pyridazinylcarbonyl)amino]phenyl}-2-thiophencarbonsäure;
5-{5-Chlor-6-[(1,3-thiazol-4-ylcarbonyl)amino]-3-pyridiny}-3-[[(trans-4-methylcyclohexyl)carbonyl](1-methylethyl)amino]-2-thiophencarbonsäure;
5-(4-{[(3,5-Dimethyl-4-isoxazolyl)carbonyl]amino}phenyl)-3-[[(trans-4-methylcyclohexyl)carbonyl](1-methylethyl)amino]-2-thiophencarbonsäure;
5-(4-{[(1-Ethyl-1H-pyrazol-5-yl)carbonyl]amino}phenyl)-3-[[(trans-4-methylcyclohexyl)carbonyl](1-methylethyl)amino]-2-thiophencarbonsäure;
3-[[(trans-4-Methylcyclohexyl)carbonyl](1-methylethyl)amino]-5-(4-{[(1-methyl-1H-imidazol-5-yl)carbonyl]amino}phenyl)-2-thiophencarbonsäure;
3-[[(trans-4-Methylcyclohexyl)carbonyl](1-methylethyl)amino]-5-(4-{[(3-methyl-4-pyridinyl)carbonyl]amino}phenyl)-2-thiophencarbonsäure;
3-[[(trans-4-Methylcyclohexyl)carbonyl](1-methylethyl)amino]-5-{4-[(1,3-thiazol-5-ylcarbonyl)amino]phenyl}-2-thiophencarbonsäure;
3-[[(trans-4-Methylcyclohexyl)carbonyl](1-methylethyl)amino]-5-(4-{[(1-methyl-1H-pyrazol-5-yl)carbonyl]amino}phenyl)-2-thiophencarbonsäure;
5-(4-{[(2,4-Dimethyl-1,3-oxazol-5-yl)carbonyl]amino}phenyl)-3-[[(trans-4-methylcyclohexyl)carbonyl](1-methylethyl)amino]-2-thiophencarbonsäure;
5-(4-{[(1,3-Dimethyl-1H-pyrazol-5-yl)carbonyl]amino}phenyl)-3-[[(trans-4-methylcyclohexyl)carbonyl](1-methylethyl)amino]-2-thiophencarbonsäure;
3-[[(trans-4-Methylcyclohexyl)carbonyl](1-methylethyl)amino]-5-(4-{[(4-methyl-1,3-oxazol-5-yl)carbonyl]amino}phenyl)-2-thiophencarbonsäure;
5-(4-{[(1,5-Dimethyl-1H-pyrazol-4-yl)carbonyl]amino}phenyl)-3-[[(trans-4-methylcyclohexyl)carbonyl](1-methylethyl)amino]-2-thiophencarbonsäure;
3-[[(trans-4-Methylcyclohexyl)carbonyl](1-methylethyl)amino]-5-(4-{[(2-methyl-3-pyridinyl)carbonyl]amino}phenyl)-2-thiophencarbonsäure;
3-[[(trans-4-Methylcyclohexyl)carbonyl](1-methylethyl)amino]-5-(4-{[(6-methyl-3-pyridinyl)carbonyl]amino}phenyl)-2-thiophencarbonsäure;
3-[[(trans-4-Methylcyclohexyl)carbonyl](1-methylethyl)amino]-5-{4-[(1H-pyrazol-4-ylcarbonyl)amino]phenyl}-2-thiophencarbonsäure;
5'-[(2-Furanylcarbonyl)amino]-4-[[(trans-4-methylcyclohexyl)carbonyl](1-methylethyl)amino]-2,2'-bithiophen-5-carbonsäure;
3-[[(trans-4-Methylcyclohexyl)carbonyl](1-methylethyl)amino]-5-(4-{[(5-methyl-4-isoxazolyl)carbonyl]amino}phenyl)-2-thiophencarbonsäure;
5-(4-{[(1-Ethyl-1H-pyrazol-4-yl)carbonyl]amino}phenyl)-3-[[(trans-4-methylcyclohexyl)carbonyl](1-methylethyl)amino]-2-thiophencarbonsäure;
3-[[(trans-4-Methylcyclohexyl)carbonyl](1-methylethyl)amino]-5-(4-{[(5-methyl-3-pyridinyl)carbonyl]amino}phenyl)-2-thiophencarbonsäure;
5'-{[(4-Fluorphenyl)carbonyl]amino}-4-[[(trans-4-methylcyclohexyl)carbonyl](1-methylethyl)amino]-2,2'-bithiophen-5-carbonsäure;
3-[[(trans-4-Methylcyclohexyl)carbonyl](1-methylethyl)amino]-5-{5-[(1,3-thiazol-4-ylcarbonyl)amino]-2-pyridinyl}-2-thiophencarbonsäure;
5-(4-{[(1,3-Dimethyl-1H-pyrazol-4-yl)carbonyl]amino}phenyl)-3-[[(trans-4-methylcyclohexyl)carbonyl](1-methylethyl)amino]-2-thiophencarbonsäure;
5-(4-{[(1-Ethyl-1H-imidazol-5-yl)carbonyl]amino}phenyl)-3-[[(trans-4-methylcyclohexyl)carbonyl](1-methylethyl)amino]-2-thiophencarbonsäure;
3-[[(trans-4-Methylcyclohexyl)carbonyl](1-methylethyl)amino]-5-(4-{[(3-methyl-1H-pyrazol-4-yl)carbonyl]amino}phenyl)-2-thiophencarbonsäure;
und Salzen davon.

8. Eine Verbindung wie in Anspruch 1 beansprucht, ausgewählt aus der Gruppe bestehend aus:
3-[[(trans-4-Methylcyclohexyl)carbonyl](1-methylethyl)amino]-5-{4-[(1,3-thiazol-4-ylcarbonyl)amino]phenyl}-2-thiophencarbonsäure;
3-{(Cyclopropylmethyl)[(trans-4-methylcyclohexyl)carbonyl]amino}-5-{4-[(1,3-thiazol-4-ylcarbonyl)amino]phenyl}-2-thiophencarbonsäure;
3-[[(trans-4-Methylcyclohexyl)carbonyl](1-methylethyl)amino]-5-{4-[(3-pyridazinylcarbonyl)amino]phenyl}-2-thiophencarbonsäure;
3-[[(trans-4-Methylcyclohexyl)carbonyl](1-methylethyl)amino]-5-{4-[(1H-pyrazol-3-ylcarbonyl)amino]phenyl}-2-thiophencarbonsäure;
3-[[(trans-4-Methylcyclohexyl)carbonyl](1-methylethyl)amino]-5-(4-{[(1-methyl-1H-imidazol-4-yl)carbonyl]amino}phenyl)-2-thiophencarbonsäure;
3-{Ethyl[(trans-4-methylcyclohexyl)carbonyl]amino}-5-{4-[(1,3-thiazol-4-ylcarbonyl)amino]phenyl}-2-thiophencarbonsäure;
und Salzen davon.

9. Eine Verbindung wie in Anspruch 1 beansprucht, ausgewählt aus der Gruppe bestehend aus:
3-[[(trans-4-Methylcyclohexyl)carbonyl](1-methylethyl)amino]-5-{4-[(1,3-thiazol-4-ylcarbonyl)amino]phenyl}-2-thiophencarbonsäure
und Salzen davon.

10. Eine Verbindung wie in Anspruch 9 beansprucht, ausgewählt aus der Gruppe bestehend aus:
Natrium-3-[[(trans-4-methylcyclohexyl)carbonyl](1-methylethyl)amino]-5-{4-[(1,3-thiazol-4-ylcarbonyl)amino]phenyl}-2-thiophencarboxylat;
Lysin-3-[[(trans-4-methylcyclohexyl)carbonyl](1-methylethyl)amino]-5-{4-[(1,3 -thiazol-4-ylcarbonyl)amino]phenyl}-2-thiophencarboxylat;
Ammonium-3-[[(trans-4-methylcyclohexyl)carbonyl](1-methylethyl)amino]-5-{4-[(1,3-thiazol-4-ylcarbonyl)amino]phenyl}-2-thiophencarboxylat;
N-Methyl-D-glucamin-3-[[(trans-4-methylcyclohexyl)carbonyl](1-methylethyl)amino]-5-{4-[(1,3-thiazol-4-ylcarbonyl)amino]phenyl}-2-thiophencarboxylat;
Kalium-3-[[(trans-4-methylcyclohexyl)carbonyl](1-methylethyl)amino]-5-{4-[(1,3-thiazol-4-ylcarbonyl)amino]phenyl}-2-thiophencarboxylat;
Cholin-3-[[(trans-4-methylcyclohexyl)carbonyl](1-methylethyl)amino]-5-{4-[(1,3-thiazol-4-ylcarbonyl)amino]phenyl}-2-thiophencarboxylat; und
L-Arginin-3-[[(trans-4-methylcyclohexyl)carbonyl](1-methylethyl)amino]-5-{4-[(1,3-thiazol-4-ylcarbonyl)amino]phenyl}-2-thiophencarboxylat.

11. Eine Verbindung der Formel (I) wie in einem der Ansprüche 1 bis 10 definiert oder pharmazeutisch verträgliche Salze davon zur Verwendung in der medizinischen Therapie.

12. Eine Verbindung der Formel (I) wie in Anspruch 11 beansprucht, wobei die medizinische Therapie die Behandlung einer Virusinfektion ist.

13. Eine pharmazeutische Formulierung, umfassend eine Verbindung der Formel (I) wie in einem der Ansprüche 1 bis 10 definiert oder pharmazeutisch verträgliche Salze davon in Verbindung mit mindestens einem pharmazeutisch verträglichen Verdünnungsmittel oder Träger.

14. Ein Verfahren zur Herstellung einer Verbindung der Formel (I) wie in einem der Ansprüche 1 bis 10 definiert, umfassend Entfernen der Schutzgruppe von einer Verbindung der Formel (II) 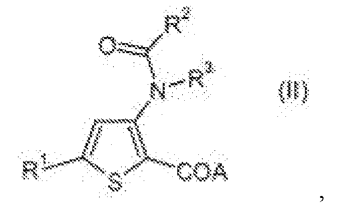 wobei A ein Alkoxy-, Benzyloxy- oder Silyloxyrest ist und R¹, R² und R³ wie in Anspruch 1 definiert sind.

15. Eine Kombination, umfassend eine Verbindung der Formel (I) wie in einem der Ansprüche 1 bis 10 definiert oder ein pharmazeutisch verträgliches Salz davon zusammen mit mindestens einem anderen therapeutischen Wirkstoff.

16. Eine Kombination wie in Anspruch 15 beansprucht, wobei der andere therapeutische Wirkstoff aus Interferon, Ribavirin und/oder einem zusätzlichen Anti-HCV-Mittel ausgewählt ist.

## Revendications

1. Composé de formule (I) : 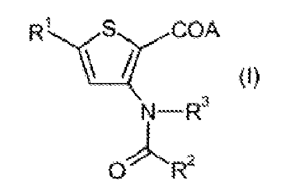 dans lequel :
A représente un groupe hydroxy ;
R¹ représente un groupe -R^{x}-NHC(O) -R^{y} ;
R^{x} représente un groupe phényle (facultativement substitué avec un ou plusieurs substituants choisis indépendamment entre des groupes halogéno, méthyle, éthyle, méthoxy et trifluorométhyle), hétéroaryle penta- ou hexagonal (facultativement substitué avec un ou plusieurs substituants choisis indépendamment entre des substituants halogéno, méthyle, éthyle, méthoxy et trifluorométhyle), lié par un atome de carbone au groupe thiophène ; les groupes thiophène et amide étant en position para l'un par rapport à l'autre ;
R^{y} représente un groupe phényle (facultativement substitué avec un ou plusieurs substituants choisis indépendamment entre des substituants halogéno, méthyle, éthyle, méthoxy, trifluorométhyle, hydroxy et amino) ou hétéroaryle penta- ou hexagonal ;
R² représente un groupe cycloalkyle en C⁵ à C⁷ (facultativement substitué avec un ou plusieurs substituants choisis indépendamment entre des groupes -alkyle en C₁ à C₆ [facultativement substitué avec un ou plusieurs substituants choisis indépendamment entre des substituants fluoro et -OR^{A}], halogéno et -OR^{A}) ;
R^{A} représente un atome d'hydrogène ou un -alkyle en C₁ à C₄ ; et
R³ représente un groupe alkyle en C₁ à C₆ (facultativement substitué avec un ou plusieurs substituants choisis indépendamment entre des substituants fluoro, hydroxyle, méthoxy, éthoxy, cycloalkyle en C₃ à C₆ et un substituant hétéroaryle ou hétérocyclyle penta- ou hexagonal), tétrahydrofurannyle, pyrannyle, cycloalkyle en C₃ à C₇, ou dioxannyle ;
ou ses sels.

2. Composé suivant la revendication 1, dans lequel R^{x} représente un groupe phényle non substitué, 2-chlorophényle, 3-(trifluorométhyl)phényle, 3-méthoxyphényle, 2-pyridinyle, 3-pyridinyle, 5-méthyl-3-pyridinyle, 4-méthyl-2-pyridinyle ou 5-chloro-3-pyridinyle, dans lequel le noyau pyridinyle dans tous les cas est lié par un atome de carbone au thiophène, les groupes thiophène et amide étant en position para l'un par rapport à l'autre, ou un groupe thiényle non substitué lié au groupe amide et au thiophène aux positions 2- et 5-.

3. Composé suivant la revendication 1 ou la revendication 2, dans lequel R^{y} représente un groupe phényle, (facultativement substitué avec un ou plusieurs substituants choisis parmi des substituants halogéno) ou hétéroaryle penta- ou hexagonal.

4. Composé suivant l'une quelconque des revendications 1 à 3, dans lequel R^{y} représente un groupe fluorophényle, pyridinyle non substitué, méthylpyridinyle, fluoropyridinyle, pyrazinyle non substitué, méthylpyrazinyle, pyridazinyle non substitué, pyrimidinyle non substitué, furannyle non substitué, méthylfurannyle, hydroxyméthylfurannyle, oxazolyle non substitué, méthyloxazolyle, isoxazolyle non substitué, méthylisoxazolyle, éthylisoxazolyle, thiényle non substitué, thiazolyle non substitué, isothiazolyle non substitué, méthylthiazolyle, aminothiazolyle, pyrazolyle méthylpyrazolyle, éthylpyrazolyle, imidazolyle non substitué, méthylimidazolyle ou éthylimidazolyle.

5. Composé suivant l'une quelconque des revendications 1 à 4, dans lequel R² représente un groupe cyclohexyle non substitué ou cyclohexyle substitué avec un ou plusieurs substituants choisis indépendamment entre des substituants méthyle, trifluorométhyle et fluoro.

6. Composé suivant l'une quelconque des revendications 1 à 5, dans lequel R³ représente un groupe alkyle en C₁ à C₆ (facultativement substitué avec un ou plusieurs substituants choisis indépendamment entre des substituants fluoro, méthoxy et cycloalkyle en C₃ à C₆), cycloalkyle en C₃ à C₇ ou dioxannyle.

7. Composé suivant la revendication 1, choisi dans le groupe consistant en :
l'acide 3-[[(*trans*-4-méthylcyclohexyl)carbonyl](1-méthyléthyl)amino]-5-{4-[(1,3-thiazole-4-ylcarbonyl)amino]-phényl}-2-thiophènecarboxylique ;
l'acide 3-[[(*trans*-4-méthylcyclohexyl)carbonyl](1-méthyléthyl)amino]-5-{4-[(2-pyridinylcarbonyl)amino]-phényl}-2-thiophènecarboxylique ;
l'acide 5-{4-[(2-furannylcarbonyl)amino]phényl}-3-[[(*trans*-4-méthylcyclohexyl)carbonyl](1-méthyléthyl)amino]-2-thiophènecarboxylique ;
l'acide 3-[[(*trans*-4-méthylcyclohexyl)carbonyl](1-méthyléthyl)amino]-5-(4-{[(2-méthyl-1,3-thiazole-4-yl)-carbonyl]amino}phényl)-2-thiophènecarboxylique ;
l'acide 3-[[(*trans*-4-méthylcyclohexyl)carbonyl](1-méthyléthyl)amino]-5-{4-[(2-thiénylcarbonyl)amino]phényl}-2-thiophènecarboxylique ;
l'acide 3-[[(*trans*-4-méthylcyclohexyl)carbonyl](1-méthyléthyl)amino]-5-{4-[(2-pyrazinylcarbonyl)amino]phényl}-2-thiophènecarboxylique ;
l'acide 5-(4-{[(4-fluorophényl)carbonyl]amino}phényl)-3-[[(*trans*-4-méthylcyclohexyl)carbonyl](1-méthyléthyl)-amino]-2-thiophènecarboxylique ;
l'acide 3-[[(*trans*-4-méthylcyclohexyl)carbonyl](1-méthyléthyl)amino]-5-{4-[(1,3-oxazole-2-ylcarbonyl)amino]-phényl}-2-thiophènecarboxylique ;
l'acide 3-{(cyclopropylméthyl)[(*trans*-4-méthylcyclohexyl)carbonyl]amino}-5-{4-[(1,3-thiazole-4-ylcarbonyl)-amino]phényl}-2-thiophènecarboxylique ;
l'acide 3-((cyclopropylméthyl){[*trans*-4-(trifluorométhyl)cyclohexyl]carbonyl}amino)-5-{4-[(1,3-thiazole-4-ylcarbonyl)amino]phényl}-2-thiophènecarboxylique ;
l'acide 3-((1-méthyléthyl){[*trans*-4-(trifluorométhyl)-cyclohexyl]carbonyl}amino)-5-{4-[(1,3-thiazole-4-yl-carbonyl)amino]phényl}-2-thiophènecarboxylique ;
l'acide 3-[[(*trans*-4-méthylcyclohexyl)carbonyl](1-méthyléthyl)amino]-5-{4-[(3-pyridazinylcarbonyl)amino]-phényl}-2-thiophènecarboxylique ;
l'acide 3-[[(*trans*-4-méthylcyclohexyl)carbonyl](1-méthyléthyl)amino]-5-{4-[(4-pyrimidinylcarbonyl)amino]-phényl}-2-thiophènecarboxylique ;
l'acide 5-{3-chloro-4-[(1,3-thiazole-4-ylcarbonyl)-amino]phényl}-3-[[(*trans*-4-méthylcyclohexyl)carbonyl](1-méthyléthyl)amino]-2-thiophènecarboxylique ;
l'acide 3-[[(*trans*-4-méthylcyclohexyl)carbonyl](1-méthyléthyl)amino]-5-{4-[(3-thiénylcarbonyl)amino]phényl}-2-thiophènecarboxylique ;
l'acide 5-(3-chloro-4-{[(4-fluorophényl)carbonyl]-amino}phényl)-3-[[(*trans*-4-méthylcyclohexyl)carbonyl](1-méthyléthyl)amino]-2-thiophènecarboxylique ;
l'acide 4-[[(*trans*-4-méthylcyclohexyl)carbonyl](1-méthyléthyl)amino]-5'-[(2-pyridinylcarbonyl)amino]-2,2'-bithiophène-5-carboxylique ;
l'acide 3-[[(*trans*-4-méthylcyclohexyl)carbonyl](1-méthyléthyl)amino]-5-(4-{[(1-méthyl-1H-pyrazole-4-yl)-carbonyl]amino}phényl)-2-thiophènecarboxylique ;
l'acide 3-[[(*trans*-4-méthylcyclohexyl)carbonyl](1-méthyléthyl)amino]-5-(4-{[(5-méthyl-3-isoxazolyl)carbonyl]-amino}phényl)-2-thiophènecarboxylique ;
l'acide 4-[[(*trans*-4-méthylcyclohexyl)carbonyl](1-méthyléthyl)amino]-5'-[(1,3-thiazole-4-ylcarbonyl)amino]-2,2'-bithiophène-5-carboxylique ;
l'acide 3-[[(*trans*-4-méthylcyclohexyl)carbonyl](1-méthyléthyl)amino]-5-{4-[(1*H*-pyrazole-3-ylcarbonyl)amino]-phényl}-2-thiophènecarboxylique ;
l'acide 3-[[(*trans*-4-méthylcyclohexyl)carbonyl](1-méthyléthyl)amino]-5-(4-{[(5-méthyl-1*H*-pyrazole-3-yl)-carbonyl]amino}phényl)-2-thiophènecarboxylique ;
l'acide 5-{4-[(1*H*-imidazole-4-ylcarbonyl)amino]phényl}-3-[[(*trans*-4-méthylcyclohexyl)carbonyl](1-méthyléthyl)-amino]-2-thiophènecarboxylique ;
l'acide 5-(4-{[(1,5-diméthyl-1*H*-pyrazole-3-yl)carbonyl]-amino}phényl)-3-[[(*trans*-4-méthylcyclohexyl)carbonyl](1-méthyléthyl)amino]-2-thiophènecarboxylique ;
l'acide 3-[[(*trans*-4-méthylcyclohexyl)carbonyl](1-méthyléthyl)amino]-5-(4-{[(5-méthyl-2-pyrazinyl)carbonyl]-amino}phényl)-2-thiophènecarboxylique ;
l'acide 3-[[[*trans*-4-méthylcyclohexyl)carbonyl](1-méthyléthyl)amino]-5-(4-{[(3-méthyl-5-isoxazolyl)carbonyl]-amino}phényl)-2-thiophènecarboxylique ;
l'acide 3-[[[*trans*-4-méthylcyclohexyl)carbonyl](1-méthyléthyl)amino]-5-(4-{[(4-méthyl-1,3-thiazole-5-yl)-carbonyl]amino}phényl)-2-thiophènecarboxylique ;
l'acide 3-[[[*trans*-4-méthylcyclohexyl)carbonyl](1-méthyléthyl)amino]-5-(4-{[(3-méthyl-2-pyridinyl)carbonyl]-amino}phényl)-2-thiophènecarboxylique ;
l'acide 5-{4-[(1*H*-imidazole-2-ylcarbonyl)amino]phényl}-3-[[(*trans*-4-méthylcyclohexyl)carbonyl](1-méthyléthyl)-amino]-2-thiophènecarboxylique ;
l'acide 3-[[(*trans*-4-méthylcyclohexyl)carbonyl](1-méthyléthyl)amino]-5-(4-{[(6-méthyl-2-pyridinyl)carbonyl]-amino}phényl)-2-thiophènecarboxylique ;
l'acide 3-[[(*trans*-4-méthylcyclohexyl)carbonyl](1-méthyléthyl)amino]-5-(4-{[(1-méthyl-1*H*-imidazole-4-yl)carbonyl]amino}phényl)-2-thiophènecarboxylique ;
l'acide 3-[[(*trans*-4-méthylcyclohexyl)carbonyl](1-méthyléthyl)amino]-5-(4-{[(2-méthyl-4-pyridinyl)carbonyl]-amino}phényl)-2-thiophènecarboxylique ;
l'acide 5-(4-{[(3-fluorophényl)carbonyl]amino}phényl)-3-[[(*trans*-4-méthylcyclohexyl)carbonyl](1-méthyléthyl)-amino]-2-thiophènecarboxylique ;
l'acide 3-[[(*trans*-4-méthylcyclohexyl)carbonyl](1-méthyléthyl)amino]-5-(4-{[(1-méthyl-1*H*-imidazole-2-yl)-carbonyl]amino}phényl)-2-thiophènecarboxylique ;
l'acide 3-[[(*trans*-4-méthylcyclohexyl)carbonyl](1-méthyléthyl)amino]-5-(4-{[(1-méthyl-1*H*-pyrazole-3-yl)-carbonyl]amino}phényl)-2-thiophènecarboxylique ;
l'acide 5-(4-{[(5-éthyl-3-isoxazolyl)carbonyl]amino}-phényl)-4-[[(*trans*-4-méthylcyclohexyl)carbonyl](1-méthyl-éthyl)amino]-2-thiophènecarboxylique ;
l'acide 5-{4-[(3-furannylcarbonyl)amino]phényl}-3-[[(*trans*-4-méthylcyclohexyl)carbonyl](1-méthyléthyl)amino]-2-thiophènecarboxylique ;
l'acide 5-(4-{[(2-fluorophényl)carbonyl]amino}phényl)-3-[[(*trans*-4-méthylcyclohexyl)carbonyl](1-méthyléthyl)-amino]-2-thiophènecarboxylique ;
l'acide 5-(4-{[(2,6-difluorophényl)carbonyl]amino}-phényl)-3-[[(*trans*-4-méthylcyclohexyl)carbonyl](1-méthyléthyl)amino]-2-thiophènecarboxylique ;
l'acide 3-[[(*trans*-4-méthylcyclohexyl)carbonyl](1-méthyléthyl)amino]-5-(4-{[(3-méthyl-2-furannyl)carbonyl]-amino}phényl)-2-thiophènecarboxylique ;
l'acide 5-(4-{[(2-amino-1,3-thiazole-4-yl)carbonyl]-amino}phényl)-3-[[(*trans*-4-méthylcyclohexyl)carbonyl](1-méthyléthyl)amino]-2-thiophènecarboxylique ;
l'acide 5-(4-{[(3-fluoro-2-pyridinyl)carbonyl]amino}-phényl)-3-[[(*trans*-4-méthylcyclohexyl)carbonyl](1-méthyléthyl)amino]-2-thiophènecarboxylique ;
l'acide 5-(4-{[(1-éthyl-1*H*-pyrazole-3-yl)carbonyl]-amino}phényl)-3-[[(*trans*-4-méthylcyclohexyl)carbonyl](1-méthyléthyl)amino]-2-thiophènecarboxylique ;
l'acide 3-[[(*trans*-4-méthylcyclohexyl)carbonyl](1-méthyléthyl)amino]-5-(4-{[(5-méthyl-2-furannyl)carbonyl]-amino}phényl)-2-thiophènecarboxylique ;
l'acide 5-[4-({[5-(hydroxyméthyl)-2-furannyl]carbonyl}-amino)phényl]-3-[[(*trans*-4-méthylcyclohexyl)carbonyl](1-méthyléthyl)amino]-2-thiophènecarboxylique ;
l'acide 3-[[(*trans*-4-méthylcyclohexyl)carbonyl](1-méthyléthyl)amino]-5-(4-{[(4-méthyl-3-pyridinyl)carbonyl]-amino}phényl)-2-thiophènecarboxylique ;
l'acide 3-[[(*trans*-4-méthylcyclohexyl)carbonyl](1-méthyléthyl)amino]-5-(4-{[(3-méthyl-4-isoxazolyl)carbonyl]-amino}phényl)-2-thiophènecarboxylique ;
l'acide 3-[[(*trans*-4-méthylcyclohexyl)carbonyl](1-méthyléthyl)amino]-5-{6-[(1,3-thiazole-4-ylcarbonyl)amino]-3-pyridinyl}-2-thiophènecarboxylique ;
l'acide 3-[[(*trans*-4-méthylcyclohexyl)carbonyl](1-méthyléthyl)amino]-5-{6-[(2-pyridinylcarbonyl)amino]-3-pyridinyl}-2-thiophènecarboxylique ;
l'acide 3-[[(*trans*-4-méthylcyclohexyl)carbonyl](1-méthyléthyl)amino]-5-{6-[(2-pyrazinylcarbonyl)amino]-3-pyridinyl}-2-thiophènecarboxylique ;
l'acide 5-(6-{[(4-fluorophényl)carbonyl]amino}-3-pyridinyl)-3-[[(*trans*-4-méthylcyclohexyl)carbonyl](1-méthyléthyl)amino]-2-thiophènecarboxylique ;
l'acide 5-{6-[(2-furannylcarbonyl)amino]-3-pyridinyl}-3-[[(*trans*-4-méthylcyclohexyl)carbonyl](1-méthyléthyl)-amino]-2-thiophènecarboxylique ;
l'acide 3-{(2,2-difluoroéthyl)[(*trans*-4-méthylcyclohexyl)carbonyl]amino}-5-{4-[(1,3-thiazole-4-ylcarbonyl)-amino]phényl}-2-thiophènecarboxylique ;
l'acide 3-{éthyl[(*trans*-4-méthylcyclohexyl)carbonyl]-amino}-5-{4-[(1,3-thiazole-4-ylcarbonyl)amino]phényl}-2-thiophènecarboxylique ;
l'acide 3-[[(cis-4-fluoro-4-méthylcyclohexyl)carbonyl]-(1-méthyléthyl)amino]-5-{4-[(1,3-thiazole-4-ylcarbonyl)-amino]phényl}-2-thiophènecarboxylique ;
l'acide 3-{(cyclopropylméthyl)[(*trans*-4-méthylcyclohexyl)carbonyl]amino}-5-{4-[(2-pyridinylcarbonyl)amino]-phényl}-2-thiophènecarboxylique ;
l'acide 3-{(cyclopropylméthyl)[(*trans*-4-méthylcyclohexyl)carbonyl]amino}-5-{4-[(2-pyrazinylcarbonyl)amino]-phényl}-2-thiophènecarboxylique ;
l'acide 3-{(cyclopropylméthyl)[(*trans*-4-méthylcyclohexyl)carbonyl]amino}-5-(4-{[(1-méthyl-1*H*-imidazole-4-yl)-carbonyl]amino}phényl)-2-thiophènecarboxylique ;
l'acide 3-{(cyclopropylméthyl)[(tarnsi-4-méthylcyclohexyl)carbonyl]amino}-5-{4-[(3-pyridazinylcarbonyl)amino]-phényl}-2-thiophènecarboxylique ;
l'acide 3-{(cyclopropylméthyl)[(*trans*-4-méthylcyclohexyl)carbonyl]amino}-5-(4-{[(1-méthyl-1*H*-pyrazole-3-yl)carbonyl]amino}phényl)-2-thiophènecarboxylique ;
l'acide 3-{(cyclopropylméthyl)[(*trans*-4-méthylcyclohexyl)carbonyl]amino}-5-(4-{[(1,5-diméthyl-1*H*-pyrazole-3-yl)carbonyl]amino}phényl)-2-thiophènecarboxylique ;
l'acide 3-[[(*trans*-4-méthylcyclohexyl)carbonyl](1-méthyléthyl)amino]-5-[4-[(1,3-thiazole-4-ylcarbonyl)amino]-3-(trifluorométhyl)phényl]-2-thiophènecarboxylique ;
l'acide 3-[[(*trans*-4-méthylcyclohexyl)carbonyl](1-méthyléthyl)amino]-5-{3-(méthyloxy)-4-[(1,3-thiazole-4-yl-carbonyl)amino]phényl}-2-thiophènecarboxylique ;
l'acide 3-{1,3-dioxanne-5-yl[(*trans*-4-méthylcyclohexyl)carbonyl]amino}-5-{4-[(1,3-thiazole-4-ylcarbonyl)-amino]phényl}-2-thiophènecarboxylique ;
l'acide 3-{cyclobutyl[(*trans*-4-méthylcyclohexyl)-carbonyl]amino}-5-{4-[(1,3-thiazole-4-ylcarbonyl)amino]-phényl}-2-thiophènecarboxylique ;
l'acide 3-{[(*trans*-4-méthylcyclohexyl)carbonyl][1-méthyl-2-(méthyloxy)éthyl]amino}-5-{4-[(1,3-thiazole-4-yl-carbonyl)amino]phényl}-2-thiophènecarboxylique ;
l'acide 3-([1-méthyl-2-(méthyloxy)éthyl]{[*trans*-4-(trifluorométhyl)cyclohexyl]carbonyl}amino)-5-{4-[(1,3-thiazole-4-ylcarbonyl)amino]phényl}-2-thiophènecarboxylique ;
l'acide 3-[[(*trans*-4-méthylcyclohexyl)carbonyl](1-méthyléthyl)amino]-5-{5-méthyl-6-[(1,3-thiazole-4-yl-carbonyl)amino]-3-pyridinyl}-2-thiophènecarboxylique ;
l'acide 5-(6-{[(4-fluorophényl)carbonyl]amino}-5-méthyl-3-pyridinyl)-3-[[(*trans*-4-méthylcyclohexyl)-carbonyl](1-méthyléthyl)amino]-2-thiophènecarboxylique ;
l'acide 3-{cyclobutyl[(*trans*-4-méthylcyclohexyl)-carbonyl]amino}-5-{4-[(1*H*-pyrazole-3-ylcarbonyl)amino]-phényl}-2-thiophènecarboxylique ;
l'acide 3-{éthyl[(*trans*-4-méthylcyclohexyl)carbonyl]-amino}-5-{4-[(1*H*-pyrazole-3-ylcarbonyl)amino]phényl}-2-thiophènecarboxylique ;
l'acide 3-{[(*trans*-4-méthylcyclohexyl)carbonyl][2-(méthyloxy)éthyl]amino}-5-{4-[(1,3-thiazole-4-ylcarbonyl)-amino]phényl}-2-thiophènecarboxylique ;
l'acide 3-{[(*trans*-4-méthylcyclohexyl)carbonyl][1-méthyl-2-(méthyloxy)éthyl]amino}-5-(4-{[(1-méthyl-1*H-*imidazole-4-yl)carbonyl]amino}phényl)-2-thiophènecarboxylique ;
l'acide 3-{[(*trans*-4-méthylcyclohexyl)carbonyl][2-(méthyloxy)éthyl]amino}-5-(4-{[(1-méthyl-1*H*-imidazole-4-yl)carbonyl]amino}phényl)-2-thiophènecarboxylique ;
l'acide 3-[[(*trans*-4-méthylcyclohexyl)carbonyl](2,2,2-trifluoroéthyl)amino]-5-{4-[(1,3-thiazole-4-ylcarbonyl)-amino]phényl}-2-thiophènecarboxylique ;
l'acide 3-{(cyclopropylméthyl)[(*trans*-4-méthylcyclohexyl)carbonyl]amino}-5-{4-[(1*H*-pyrazole-3-ylcarbonyl) - amino]phényl}-2-thiophènecarboxylique ;
l'acide 3-{cyclobutyl[(*trans*-4-méthylcyclohexyl)-carbonyl]amino}-5-(4-{[(1-méthyl-1*H*-imidazole-4-yl)-carbonyl]amino}phényl)-2-thiophènecarboxylique ;
l'acide 3-{éthyl[(*trans*-4-méthylcyclohexyl)carbonyl]-amino}-5-(4-{[(1-méthyl-1*H*-imidazole-4-yl)carbonyl]amino}-phényl)-2-thiophènecarboxylique ;
l'acide 3-[[(*trans*-4-méthylcyclohexyl)carbonyl](1-méthyléthyl)amino]-5-{4-méthyl-5-[(1,3-thiazole-4-yl-carbonyl)amino]-2-pyridinyl}-2-thiophènecarboxylique ;
l'acide 3-{cyclobutyl[(*trans*-4-méthylcyclohexyl)-carbonyl]amino}-5-{4-[(3-pyridazinylcarbonyl)amino]phényl}-2-thiophènecarboxylique ;
l'acide 5-{5-chloro-6-[(1,3-thiazole-4-ylcarbonyl)-amino]-3-pyridinyl}-3-[[(*trans*-4-méthylcyclohexyl)carbonyl]-(1-méthyléthyl)amino]-2-thiophènecarboxylique ;
l'acide 5-(4-{[(3,5-diméthyl-4-isoxazolyl)carbonyl]-amino}phényl)-3-[[(*trans*-4-méthylcyclohexyl)carbonyl](1-méthyléthyl)amino]-2-thiophènecarboxylique ;
l'acide 5-(4-{[(1-éthyl-1*H*-pyrazole-5-yl)carbonyl]-amino}phényl)-3-[[(*trans*-4-méthylcyclohexyl)carbonyl](1-méthyléthyl)amino]-2-thiophènecarboxylique ;
l'acide 3-[[(*trans*-4-méthylcyclohexyl)carbonyl](1-méthyléthyl)amino]-5-(4-{[(1-méthyl-1*H*-imidazole-5-yl)carbonyl]amino}phényl)-2-thiophènecarboxylique ;
l'acide 3-[[(*trans*-4-méthylcyclohexyl)carbonyl](1-méthyléthyl)amino]-5-(4-{[(3-méthyl-4-pyridinyl)carbonyl]-amino}phényl)-2-thiophènecarboxylique ;
l'acide 3-[[(*trans*-4-méthylcyclohexyl)carbonyl](1-méthyléthyl)amino]-5-{4-[(1,3-thiazole-5-ylcarbonyl)-amino]phényl}-2-thiophènecarboxylique ;
l'acide 3-[[(*trans*-4-méthylcyclohexyl)carbonyl](1-méthyléthyl)amino]-5-(4-{[(1-méthyl-1*H*-pyrazole-5-yl)carbonyl]amino}phényl)-2-thiophènecarboxylique ;
l'acide 5- (4-{[(2,4-diméthyl-1,3-oxazole-5-yl)carbonyl]-amino}phényl)-3-[[(*trans*-4-méthylcyclohexyl)carbonyl](1-méthyléthyl)amino]-2-thiophènecarboxylique ;
l'acide 5- (4 {[(1,3-diméthyl-1*H*-pyrazole-4-yl) carbonyl]-amino}phényl)-3-[[(*trans*-4-méthylcyclohexyl)carbonyl](1-méthyléthyl)amino]-2-thiophènecarboxylique ;
l'acide 3-[[(*trans*-4-méthylcyclohexyl)carbonyl](1-méthyléthyl)amino]-5-(4-{[(4-méthyl-1,3-oxazole-5-yl)carbonyl]amino}phényl)-2-thiophènecarboxylique ;
l'acide 5- (4-{[(1,5-diméthyl-1*H*-pyrazole-4-yl)carbonyl]-amino}phényl)-3-[[(*trans*-4-méthylcyclohexyl)carbonyl](1-méthyléthyl)amino]-2-thiophènecarboxylique ;
l'acide 3-[[(*trans*-4-méthylcyclohexyl)carbonyl](1-méthyléthyl)amino]-5-(4-{[(2-méthyl-3-pyridinyl)carbonyl]-amino}phényl)-2-thiophènecarboxylique ;
l'acide 3-[[(*trans*-4-méthylcyclohexyl)carbonyl](1-méthyléthyl)amino]-5-(4-{[(6-méthyl-3-pyridinyl)carbonyl]-amino}phényl)-2-thiophènecarboxylique ;
l'acide 3-[[(*trans*-4-méthylcyclohexyl)carbonyl](1-méthyléthyl)amino]-5-{4-[(1*H*-pyrazole-4-ylcarbonyl)amino]-phényl}-2-thiophènecarboxylique ;
l'acide 5'-[(2-furannylcarbonyl)amino]-4-[[(*trans*-4-méthylcyclohexyl)carbonyl](1-méthyléthyl)amino]-2,2'-bithiophène-5-carboxylique ;
l'acide 3-[[(*trans*-4-méthylcyclohexyl)carbonyl](1-méthyléthyl)amino]-5-(4-{[(5-méthyl-4-isoxazolyl)carbonyl]-amino}phényl)-2-thiophènecarboxylique ;
l'acide 5-(4-{[(1-éthyl-1*H*-pyrazole-4-yl)carbonyl]-amino}phényl)-3-[[(trans-4-méthylcyclohexyl)carbonyl](1-méthyléthyl)amino]-2-thiophènecarboxylique ;
l'acide 3-[[(*trans*-4-méthylcyclohexyl)carbonyl](1-méthyléthyl)amino]-5-(4-{[(5-méthyl-3-pyridinyl)carbonyl]-amino}phényl)-2-thiophènecarboxylique ;
l'acide 5'-{[(4-fluorophényl)carbonyl]amino}-4-[[(*trans*-4-méthylcyclohexyl)carbonyl](1-méthyléthyl)amino]-2,2'-bithiophène-5-carboxylique ;
l'acide 3-[[(*trans*-4-méthylcyclohexyl)carbonyl](1-méthyléthyl)amino]-5-{5-[(1,3-thiazole-4-ylcarbonyl)amino]-2-pyridinyl}-2-thiophènecarboxylique ;
l'acide 5-(4-{[(1,3-diméthyl-1*H*-pyrazole-4-yl)-carbonyl]amino}phényl)-3-[[(*trans*-4-méthylcyclohexyl)-carbonyl](1-méthyléthyl)amino]-2-thiophènecarboxylique ;
l'acide 5-(4-{[(1-éthyl-1*H*-imidazole-5-yl)carbonyl]-amino}phényl)-3-[[(*trans*-4-méthylcyclohexyl)carbonyl](1-méthyléthyl)amino]-2-thiophènecarboxylique ;
l'acide 3-[[(*trans*-4-méthylcyclohexyl)carbonyl](1-méthyléthyl)amino]-5-(4-{[(3-méthyl-1*H*-pyrazole-4-yl)carbonyl]amino}phényl)-2-thiophènecarboxylique ;
et ses sels.

8. Composé suivant la revendication 1, choisi dans le groupe consistant en :
l'acide 3-[[(*trans*-4-méthylcyclohexyl)carbonyl](1-méthyléthyl)amino]-5-{4-[(1,3-thiazole-4-ylcarbonyl)amino]-phényl}-2-thiophènecarboxylique ;
l'acide 3-{(cyclopropylméthyl)[(*trans*-4-méthylcyclohexyl)carbonyl]amino}-5-{4-[(1,3-thiazole-4-ylcarbonyl)-amino]phényl}-2-thiophènecarboxylique ;
l'acide 3-[[(*trans*-4-méthylcyclohexyl)carbonyl](1-méthyléthyl)amino]-5-{4-[(3-pyridazinylcarbonyl)amino]-phényl}-2-thiophènecarboxylique ;
l'acide 3-[[(*trans*-4-méthylcyclohexyl)carbonyl](1-méthyléthyl)amino]-5-{4-[(1*H*-pyrazole-3-ylcarbonyl)amino]-phényl}-2-thiophènecarboxylique ;
l'acide 3-[[(*trans*-4-méthylcyclohexyl)carbonyl](1-méthyléthyl)amino]-5-(4-{[(1-méthyl-1*H*-imidazole-4-yl)-carbonyl]amino}phényl)-2-thiophènecarboxylique ;
l'acide 3-{éthyl[(*trans*-4-méthylcyclohexyl)carbonyl]-amino}-5-{4-[(1,3-thiazole-4-ylcarbonyl)amino]phényl}-2-thiophènecarboxylique ;
et ses sels.

9. Composé suivant la revendication 1, choisi dans le groupe consistant en :
l'acide 3-[[(*trans*-4-méthylcyclohexyl)carbonyl](1-méthyléthyl)amino]-5-{4-[(1,3-thiazole-4-ylcarbonyl)amino]-phényl}-2-thiophènecarboxylique et ses sels.

10. Composé suivant la revendication 9, choisi dans le groupe consistant en :
le 3-[[(*trans*-4-méthylcyclohexyl)carbonyl](1-méthyléthyl)amino]-5-{4-[(1,3-thiazole-4-ylcarbonyl)amino]phényl}-2-thiophènecarboxylate de sodium ;
le 3-[[(*trans*-4-méthylcyclohexyl)carbonyl](1-méthyléthyl)amino]-5-{4-[(1,3-thiazole-4-ylcarbonyl)amino]-phényl}-2-thiophènecarboxylate de lysine ;
le 3-[[(*trans*-4-méthylcyclohexyl)carbonyl](1-méthyléthyl)amino]-5-{4-[(1,3-thiazole-4-ylcarbonyl)amino]-phényl}-2-thiophènecarboxylate d'ammonium ;
le 3-[[(*trans*-4-méthylcyclohexyl)carbonyl](1-méthyléthyl)amino]-5-{4-[(1,3-thiazole-4-ylcarbonyl)amino]-phényl}-2-thiophènecarboxylate de N-méthyl-D-glucamine ;
le 3-[[(*trans*-4-méthylcyclohexyl)carbonyl](1-méthyléthyl)amino]-5-{4-[(1,3-thiazole-4-ylcarbonyl)amino]-phényl}-2-thiophènecarboxylate de potassium ;
le 3-[[(*trans*-4-méthylcyclohexyl)carbonyl](1-méthyléthyl)amino]-5-{4-[(1,3-thiazole-4-ylcarbonyl)amino]-phényl}-2-thiophènecarboxylate de choline ; et
le 3-[[(*trans*-4-méthylcyclohexyl)carbonyl](1-méthyléthyl)amino]-5-{4-[(1,3-thiazole-4-ylcarbonyl)amino]-phényl}-2-thiophènecarboxylate de L-arginine.

11. Composé de formule (I) suivant l'une quelconque des revendications 1 à 10 ou ses sels pharmaceutiquement acceptables, pour une utilisation en thérapie médicale.

12. Composé de formule (I) suivant la revendication 11, la thérapie médicale étant le traitement d'une infection virale.

13. Composition pharmaceutique comprenant un composé de formule (I) tel que défini dans l'une quelconque des revendications 1 à 10 ou ses sels pharmaceutiquement acceptables conjointement avec au moins un diluant ou support pharmaceutiquement acceptable.

14. Procédé pour la préparation d'un composé deformule (I) tel que défini dans l'une quelconque des revendications 1 à 10, comprenant la suppression de protection d'un composé de formule (II) 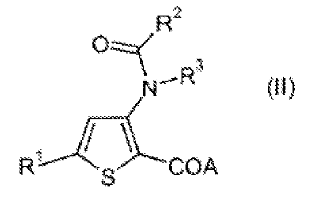 dans laquelle A représente un groupe alkoxy, benzyloxy ou silyloxy et R¹, R² et R³ sont tels que définis dans la revendication 1.

15. Association comprenant un composé de formule (I) tel que défini dans l'une quelconque des revendications 1 à 10 ou un de ses sels pharmaceutiquement acceptables, conjointement avec au moins un autre agent thérapeutiquement actif.

16. Association suivant la revendication 15, dans laquelle l'autre agent thérapeutiquement actif est choisi entre l'interféron, la ribavirine et/ou un agent anti-HCV supplémentaire.
